(19) Europäisches Patentamt
European Patent Office
Office européen des brevets

(11) EP 1 541 574 A1

(12) EUROPEAN PATENT APPLICATION
published in accordance with Art. 158(3) EPC

(43) Date of publication:
**15.06.2005 Bulletin 2005/24**

(21) Application number: **03753933.5**

(22) Date of filing: **17.09.2003**

(51) Int Cl.[7]: **C07D 471/10**, A61K 31/527, A61P 1/04, A61P 1/16, A61P 3/10, A61P 9/00, A61P 11/00, A61P 11/02, A61P 11/06, A61P 13/12, A61P 17/00, A61P 17/06, A61P 19/02, A61P 29/00, A61P 31/04, A61P 31/18, A61P 35/04, A61P 37/02, A61P 37/06, A61P 37/08, A61P 43/00

(86) International application number:
**PCT/JP2003/011834**

(87) International publication number:
**WO 2004/026873 (01.04.2004 Gazette 2004/14)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **18.09.2002 JP 2002270849**

(71) Applicant: **ONO PHARMACEUTICAL CO., LTD.**
**Osaka-shi, Osaka 541-8526 (JP)**

(72) Inventors:
- **TAKAOKA, Yoshikazu, Ono Pharmaceutical Co., Ltd. Mishima-gun, Osaka 618-8585 (JP)**
- **NISHIZAWA, Rena, Ono Pharmaceutical Co., Ltd. Mishima-gun, Osaka 618-8585 (JP)**
- **SHIBAYAMA, Shiro, Ono Pharmaceutical Co., Ltd. Tsukuba-shi, Ibaraki 300-4247 (JP)**
- **SAGAWA, Kenji, Ono Pharmaceutical Co., Ltd. Mishima-gun, Osaka 618-8585 (JP)**
- **MATSUO, Masayoshi, Ono Pharmaceutical Co., Ltd. Mishima-gun, Osaka 618-8585 (JP)**

(74) Representative: **Henkel, Feiler & Hänzel**
**Möhlstrasse 37**
**81675 München (DE)**

(54) **TRIAZASPIRO 5.5 UNDECANE DERIVATIVES AND DRUGS COMPRISI NG THE SAME AS THE ACTIVE INGREDIENT**

(57) Compounds represented by formula (I)

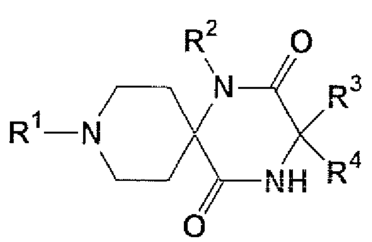

(I)

(wherein all of the symbols have the same meanings as defined in specification.), quaternary ammonium salts thereof, N-oxides thereof or salts thereof. The compounds represented by formula (I) are used for prevention and/or treatment of various inflammatory diseases (asthma, nephritis, nephropathy, hepatitis, arthritis, chronic rheumatoid arthritis, rhinitis, conjunctivitis, ulcerative colitis and the like), immunologic diseases (autoimmune diseases, transplant rejection, immunosuppression, psoriasis, multiple sclerosis and the like), human immunodeficiency virus (acquired immunodeficiency syndrome and the like), allergic diseases (atopic dermatitis, nettle rash, allergic bronchopulmonary aspergillosis, allergic eosinophilic gastroenteritis and the like), ischemia-reperfusion injury, acute respiratory distress syndrome, shock accompanied by bacterial infection, diabetes mellitus, or metastasis and the like.

EP 1 541 574 A1

**Description**

TECHNICAL FIELD

The present invention relates to (1) a compound represented by formula (I)

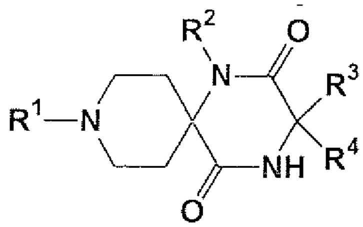

(I)

(wherein all symbols have the same meanings as defined hereinafter.), a quaternary ammonium salt thereof or an N-oxide thereof, or a salt thereof, and (2) a pharmaceutical composition for prevent and/or treatment for inflammatory diseases, immunologic diseases, human immunodeficiency virus, allergic diseases, ischemia-reperfusion injury, acute respiratory distress syndrome, shock accompanied by bacterial infection, diabetes mellitus, or metastasis *etc*. comprising, as an active ingredient, the compound represented by formula (I), the quaternary ammonium salt thereof or the N-oxide thereof, or the salt thereof.

BACKGROUND ART

Chemokine is known as a basic protein having endogeneous leukocyte chemotactic and activating abilities and strong heparin-binding abilities. At present, it is considered that chemokine is related to not only the control of infiltration of specific leukocyte at the time of inflammations and immune responses but also the development and homing of lymphocyte under physiological conditions and migration of hemocyte precursor cells and somatic cells.

Differentiation, proliferation and cell death of hemocytes are controlled by various types of cytokine. In the living body, inflammations are found topically and differentiation, maturation and the like of lymphocytes are carried out at certain specified sites. That is, various necessary cells migrate into certain specified sites and accumulate therein to cause a series of inflammations and immune responses. Accordingly, migration of cells is also an indispensable phenomenon in addition to differentiation, proliferation and death of cells.

Migration of hemocytes in the living body starts firstly in the development stage by the shift of hematopoiesis started in the AGM region into permanent hematopoiesis in bone marrow via fetal liver. Furthermore, precursor cells of T cells and thymus dendritic cells migrate from the fetal liver into the bone marrow and then into the thymus gland and cytodifferentiate under thymus environment. The T cell which received clone selection migrates into secondary lymphoid tissues and takes part in an immune response in the periphery. The Langerhans' cell of the skin activated and differentiated by capturing an antigen migrates into the T cell region of a topical lymph node and activates naive T cell therein as a dendritic cell. The memory T cell performs its homing again into the lymph node via lymphatic and blood vessels. Also, B cell, T cell in the intestinal epithelium, $\gamma\delta$ T cell, NKT cell and dendritic cell migrate from bone marrow without passing through the thymus gland and differentiate to take part in an immune response.

Chemokine is deeply related to the migration of these various cells. For example, MIP3$\beta$, SLC and its receptor CCR7 play an important role in the migration and homing of naive T cell, memory T cell and the mature dendritic cell which captured an antigen into a topical lymphoid tissue for the dendritic cells to encounter efficiently with the T cells. The T cell and dendritic cell necessary for controlling antigen-specific immune responses are hardly observed in the secondary lymph node of a PLT mouse having deficiency in the expression of SLC (*J. Exp. Med*., 189(3), 451 (1999)).

MDC, TARC and its receptor CCR4 play an important role in the migration of Th2 cell into topical sites in immune and inflammatory responses in which the Th2 cell is related. In a fat fulminant hepatitis model (P. acnes + LPS), an anti-TARC antibody suppressed increase of the amount of ALT in blood and increase of the expressing amounts of TNF$\alpha$ and FasL in the liver and also improved lethality of the rats (*J. Clin. Invest*., 102, 1933 (1998)). Also, an anti-MDC antibody decreased the number of eosinophils accumulated in the lung interstitium and suppressed airway hypersensitivity in a mouse OVA-induced airway hypersensitivity model (*J. Immunology*, 163, 403 (1999)).

MCP-1 and its receptor CCR2 are related to the infiltration of macrophage into inflammation sites. An anti-MCP-1 antibody showed an effect to suppress infiltration of monocyte and macrophage into glomerulus in a rat anti-Thy1.1 antibody glomerular nephritis model (*Kidney Int*., 51, 770 (1997)).

Thus, chemokine receptors are greatly related to the control of inflammation and immune responses through a mechanism in which they are expressed at certain specified periods in variously specific cells and the effector cells

are accumulated in a region where chemokine is produced.

Acquired immunodeficiency syndrome (called AIDS) which is induced by human immunodeficiency virus (hereinafter referred to as "HIV") is one of the diseases of which their therapeutic methods are most earnestly desired in recent years. Once infection with HIV is completed in a CD4-positive cell which is a principal target cell, HIV repeats its proliferation in the body of the patient and, sooner or later, completely destroys T cell which takes charge of the immunological function. During this process, the immunological function is gradually reduced to cause fever, diarrhea, lymph node enlargement and the like various immunodeficiency conditions which are apt to cause complications with pneumocystis carinii pneumonia and the like various opportunistic infections. Such conditions are the onset of AIDS, and it is well known that they induce and worsen Kaposi sarcoma and the like malignant tumors.

As the recent preventive and therapeutic methods for AIDS, attempts have been made to, e.g., (1) inhibit growth of HIV by the administration of a reverse transcriptase inhibitor or a protease inhibitor and (2) prevent or alleviate opportunistic infections by the administration of a drug having immunopotentiation activity.

Helper T cells which take charge of the central of immune system are mainly infected with HIV. It is known since 1985 that HIV uses the membrane protein CD4 expressing on the membrane of T cells in the infection (*Cell*, 52, 631 (1985)). The CD4 molecule is composed of 433 amino acid residues, and its expression can be found in macrophages, some B cells, vascular endothelial cells, Langerhans' cells in skin tissues, dendritic cells in lymphoid tissues, glia cells of the central nervous system and the like, in addition to the mature helper T cells. However, since it has been revealed that the infection with HIV is not completed by the CD4 molecule alone, a possibility has been suggested on the presence of factors other than the CD4 molecule, which are related to the infection of cells with HIV.

In 1996, a cell membrane protein called Fusin was identified as a factor other than the CD4 molecule, which is related to the HIV infection (*Science*, 272, 872 (1996)). It was confirmed that this Fusin molecule is a receptor (namely, CXCR4) of stromal derived factor-1 (hereinafter referred to as "SDF-1"). In addition, it was confirmed also *in vitro* that the SDF-1 specifically inhibits infection of T cell tropic (X4) HIV (*Nature*, 382, 829 (1996), *Nature*, 382, 833 (1996)). That is, it is considered that the HIV infection was inhibited by the binding of SDF-1 to CXCR4 preceding HIV, thereby depriving HIV of a foothold for infecting cells.

Also at that time, it was discovered that another chemokine receptor CCR5, which is a receptor ofRANTES, MIP-1$\alpha$ and MIP-1$\beta$, is also used at the time of the infection with a macrophage tropic (R5) HIV (*Science*, 272, 1955 (1996)).

Accordingly, substances which can compete with CXCR4 and CCR5 for HIV, or which can bind to HIV virus thus causing the virus unable to bind to CXCR4 and CCR5, could become HIV infection inhibitors. Also, there is a case in which a low molecular compound initially discovered as an HIV infection inhibitor was actually a CXCR4 antagonist (*Nature Medicine*, 4, 72 (1998)).

Based on the above, it is considered that the chemokine/chemokine receptors are deeply related to the inflammation, immune disease or HIV infection. For example, it is considered that they are related to various inflammatory diseases (asthma, nephritis, nephropathy, hepatitis, arthritis, chronic rheumatoid arthritis, rhinitis, conjunctivitis, ulcerative colitis and the like), immunologic diseases (autoimmune diseases, transplant rejection, immunosuppression, psoriasis, multiple sclerosis and the like), human immunodeficiency virus (acquired immunodeficiency syndrome and the like), allergic diseases (atopic dermatitis, nettle rash, allergic bronchopulmonary aspergillosis, allergic eosinophilic gastroenteritis and the like), ischemia-reperfusion injury, acute respiratory distress syndrome, shock accompanied by bacterial infection, diabetes mellitus, or metastasis and the like.

It is described that the compounds of formula (Z)

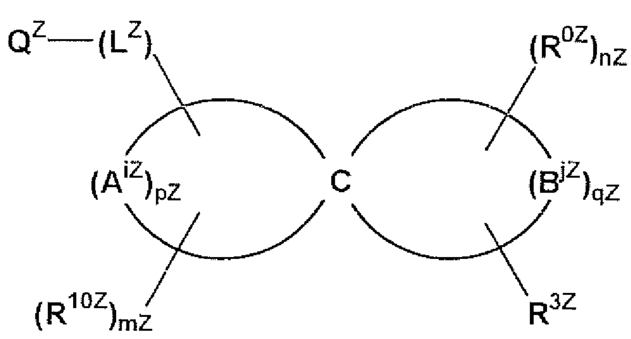

(Z)

(wherein the atoms $A^{iZ}$ and $B^{jZ}$ are independently selected from carbon, nitrogen, oxygen and sulfur, provided that at least one atom of $A^{iZ}$ is carbon, and at least one atom $B^{jZ}$ is carbon.); the rings of the spirobicycle formed by $A^{iZ}$ and

$B^{iZ}$, respectively, may optionally be partly unsaturated, pZ and qZ are independently numbers from 2 to 6, mZ is a number from 0 to pZ, $R^{10Z}$ is the same or different and is a non-interfering substituent independently selected from hydrogen, alkyl, halosubstituted alkyl, alkenyl, alkynyl, cycloalkyl, =O and =S *etc.*, nZ is a number from 0 to qZ, $R^{0Z}$ is the same or different and is a non-interfering substituent independently selected from hydrogen, alkyl, halosubstituted alkyl, alkenyl, alkynyl, cycloalkyl, =O, and =S *etc.*, the linking group -($L^Z$)- is a single bond or a divalent substituted or unsubstituted chain of from 1 to 10 atoms selected from the group consisting of carbon, nitrogen, sulfur, and oxygen, $Q^Z$ is a basic group containing one or more basic radical(s), and $R^{3Z}$ is an acidic group containing one or more acid radical(s)) are useful for inhibiting platelet aggregation (ref. the specification of WO97/11940).

Furthermore, it is described that the compounds of formula (Y)

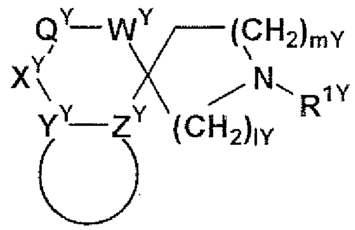

(Y)

(wherein, mY or 1Y are each independently 0, 1, 2, 3, 4 or 5, $R^{1Y}$ is hydrogen, C1-8 alkyl, C2-8 alkenyl, C2-8 alkynyl *etc.*, $W^Y$ is a single bond, C1-3 alkyl or C1-3 alkyl substituted with oxo *etc.*, $Q^Y$ is -$NR^2$-, -O-, -S-, -S(O)- or -$SO_2$-, $X^Y$ is a single bond, C1-3 alkyl or C1-3 alkyl substituted with oxo *etc.*, $Y^Y$-$Z^Y$ ring is phenyl, naphthyl or hetero aryl, and wherein the definition of each symbol is an excerpt partially.) are useful as modulators of the chemokine receptors(ref. the specification of WO98/25605).

On the other hand, it is reported that triazaspiro[5.5]undecane derivatives, quaternary ammonium salts thereof or N-oxides thereof, or salts thereof regulate the effect of chemokine/chemokine receptor, so they are used for prevention and/or treatment of various inflammatory diseases, asthma, atopic dermatitis, urticaria, allergic diseases (allergic bronchopulmonary aspergillosis or allergic eosinophilic gastroenteritis *etc.*), nephritis, nephropathy, hepatitis, arthritis, rheumatoid arthritis, psoriasis, rhinitis, conjunctivitis, ischemic reperfusion disorder, multiple sclerosis, ulcerative colitis, acute respiratory distress syndrome, cytotoxic shock, diabetes, autoimmune disease, in transplanted organ rejection reactions, immunosuppression, cancer metastasis and acquired immune deficiency syndrome(ref. the specification of WO01/40227).

Triazaspiro[5.5]undecane derivative compounds, their quaternary ammonium salts or their N-oxides, or their non-toxic salts described in the publication of International Publication No. 01/40227 are very useful compounds as agents for preventing and treating various diseases due to the regulation of the CCR interaction. However, it is still needed to improve their metabolic stabilities, liver and systemic clearance, extent of bioavailability or affinity to CCR. Therefore, it has been desired to provide a pharmaceutical product with improved metabolic stability, liver and systemic clearance, extent of bioavailability or affinity to CCR for clinical application, very meaningfully from both the standpoints of medicine and economy.

## DISCLOSURE OF THE INVENTION

The present inventors have made investigations so as to solve the problems. Consequently, the inventors have found that the compound represented by formula (I) (hereinafter referred to as "the inventive compound") can meet the object. Compared with the compound described in the publication of International Publication No. 01/40227, the inventive compound has improved metabolic stability, liver and systemic clearance, extent of bioavailability or affinity to CCR.

The present invention relates to the followings:

1. A compound represented by formula (I)

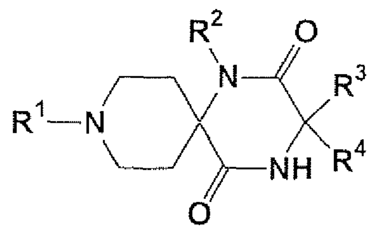

(I)

wherein $R^1$ represents (1) ring 1, or (2) C1-8 alkyl, C2-4 alkenyl or C2-4 alkynyl optionally substituted with 1-3 substituents selected from the following (a)-(i): (a) $-OR^5$, (b)$-COR^6$, (c) $-NR^7R^8$, (d) $-CONR^9R^{10}$, (e) $-NR^{11}COR^{12}$, (f) $-NR^{13}SO_2R^{14}$, (g) ring 1, (h) $=NR^{15}$, (i) $=NOR^{16}$,

$R^5$-$R^{13}$, $R^{15}$ and $R^{16}$ each represents (1) hydrogen, (2) C1-8 alkyl, (3) C2-8 alkenyl, (4) C2-8 alkynyl, (5) ring 1, or (6) C1-8 alkyl, C2-8 alkenyl or C2-8 alkynyl optionally substituted with 1-5 substituents selected from ring 1 and -O-ring 1,

$R^{14}$ represents C1-4 alkyl or ring 1,

ring 1 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 1 may be substituted with 1-5 substituents selected from (1) C1-8 alkyl, (2) C2-8 alkenyl, (3) C2-8 alkynyl, (4) halogen, (5) cyano, (6) ring 2, (7) $-OR^{17}$, (8) $-SR^{18}$, (9) - $NR^{19}R^{20}$, (10) $-COR^{21}$, (11) $-COOR^{22}$, (12) $-CONR^{23}R^{24}$, (13) $-NR^{25}COR^{26}$, (14) - $SO_2NR^{27}R^{28}$, (15) $-NR^{29}SO_2R^{30}$, (16) $-N(SO_2R^{31})_2$, (17) oxo, and (18) C1-8 alkyl, C2-8 alkenyl or C2-8 alkynyl optionally substituted with 1-5 substituents selected from the following (a) -(e): (a) halogen, (b) ring 2, (c) $-OR^{32}$, (d) $-NR^{33}COR^{34}$, (e) $=NOR^{35}$,

$R^{17}$-$R^{29}$ and $R^{32}$-$R^{35}$ each represents (1) hydrogen, (2) C1-8 alkyl, (3) C2-8 alkenyl, (4) C2-8 alkynyl, (5) ring 2, or (6) C1-8 alkyl, C2-8 alkenyl or C2-8 alkynyl optionally substituted with 1-3 substituents selected from the following (a)-(f): (a) ring 2, (b) $-OR^{36}$, (c) - $COOR^{37}$, (d) $-NR^{38}R^{39}$, (e) halogen, (f) $=NR^{40}$,

$R^{30}$ and $R^{31}$ each represents C1-4 alkyl,

$R^{36}$-$R^{40}$ each represents hydrogen or C1-4 alkyl optionally substituted with hydroxyl,

ring 2 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 2 may be substituted with 1-5 substituents selected from (1) C1-8 alkyl, (2) halogen, (3) $-OCF_3$, (4) cyano, (5) ring 3, (6) $-OR^{41}$, (7) $-NR^{42}R^{43}$, (8) $-COR^{44}$, (9) $-COOR^{45}$, (10) $-CONR^{46}R^{47}$, (11) $-NR^{48}COR^{49}$, (12) $-SO_2NR^{50}R^{51}$, (13) $-NR^{52}SO_2R^{53}$, and (14) - $C(NH_2)=NR^{54}$,

$R^{41}$-$R^{52}$ and $R^{54}$ each represents (1) hydrogen, (2) C1-8 alkyl, (3) C2-8 alkenyl, (4) C2-8 alkynyl, (5) ring 3, (6) $-OR^{55}$, or (7) C1-8 alkyl, C2-8 alkenyl or C2-8 alkynyl optionally substituted with 1-3 substituents selected from the following (a)-(d): (a) ring 3, (b) $-OR^{56}$, (c) $-COOR^{57}$, (d) $-NR^{58}R^{59}$,

$R^{53}$ represents C1-8 alkyl,

$R^{55}$-$R^{59}$ each represents hydrogen or C 1-4 alkyl,

ring 3 represents (1) C3-8 mono-carbocyclic aryl which may be partially or fully saturated, or (2) 3-8 membered mono-cyclic hetero aryl containing 1-4 nitrogen atoms, 1-2 oxygen atoms and/or 1-2 sulfur atoms which may be partially or fully saturated,

ring 3 may be substituted with 1-3 of=O or =S,

$R^2$ represents (1) hydrogen, (2) C1-8 alkyl, (3) C2-8 alkenyl, (4) C2-8 alkynyl, (5) ring 4, or (6) C1-8 alkyl, C2-8 alkenyl or C2-8 alkynyl optionally substituted with 1-5 substituents selected from the following (a)-(i): (a) hydrogen, (b) $-OR^{60}$, (c) $-NR^{61}R^{62}$, (d) - $CONR^{63}R^{64}$, (e) $-NR^{65}COR^{66}$, (f) $-NR^{67}SO_2R^{68}$, (g) $NR^{69}COOR^{70}$, (h) ring 4, (i) cyano,

$R^{60}$-$R^{67}$ and $R^{69}$ each represents hydrogen, C1-8 alkyl, C2-8 alkenyl, or C2-8 alkynyl,

$R^{68}$ and $R^{70}$ each represents C1-4 alkyl, C2-4 alkenyl or C2-4 alkynyl,

ring 4 represents phenyl, pyridinyl, or C3-8 cycloalkyl,

ring 4 may be substituted with 1-5 of C1-4 alkyl,

$R^3$ and $R^4$ together with a carbon atom to which they are attached, form C3-8 cycloalkyl, or $R^3$ and $R^4$ each represents (1) hydrogen, (2) C1-8 alkyl, (3) C2-8 alkenyl, (4) C2-8 alkynyl, (5) C1-8 alkyl, C2-8 alkenyl or C2-8 alkynyl optionally substituted with 1-5 substituents selected from the following (a)-(c): (a) ring 5, (b) hydroxyl,

(c) 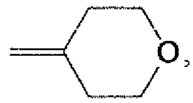

ring 5 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 5 may be substituted with 1-5 of $-OR^{71}$, C1-4 alkyl or oxo,

$R^{71}$ represents hydrogen or C1-4 alkyl,

a quaternary ammonium salt thereof, an N-oxide thereof, or a salt thereof.

2. The compound according to the above 1, which is selected from the group consisting of

(1) (3R)-1-butyl-2,5-dioxo-3-((1R)-1-hydroxy-1-cyclohexylmethyl)-9-(4-(4-methylaminocarbonyl-2-chlorophenoxy)phenylmethyl)-1,4,9-triazaspiro[5.5]undecane,
(2) (3R)-1-butyl-2,5-dioxo-3-((1R)-1-hydroxy-1-cyclohexylmethyl)-9-(4-(4-(5-oxo-4,5-dihydro-1,2,4-thiadiazol-3-yl)phenoxy)phenylmethyl)-1,4,9-triazaspiro[5.5]undecane,
(3) (3R)-1-butyl-2,5-dioxo-3-((1R)-1-hydroxy-2-ethylbutyl)-9-(4-(4-(2-methoxyethylaminocarbonyl)-2-methoxyphenoxy)phenylmethyl)-1,4,9-triazaspiro [5.5]undecane,
(4) (3R)-1-butyl-2,5-dioxo-3-((1R)-1-hydroxy-2-methylpropyl)-9-(4-(4-methylsulfonylamino-2-methoxyphenoxy)phenylmethyl)-1,4,9-triazaspiro[5.5]undecane,
(5) (3R)-1-butyl-2,5-dioxo-3-((1R)-1-hydroxy-1-(tetrahydropyran-4-yl)methyl)-9-(4-(4-(pyrrolidin-1-yl)carbonyl-2-methoxyphenoxy)phenylmethyl)-1,4,9-triazaspiro[5.5]undecane,
(6) (3R)-1-butyl-2,5-dioxo-3-((1R)-1-hydroxy-2-ethylbutyl)-9-(4-(2-methoxy-4-methylsulfonylaminophenoxy)phenylmethyl)-1,4,9-triazaspiro[5.5]undecane,
(7) (3S)-1-butyl-2,5-dioxo-3-(2-methylpropyl)-9-(4-(2-methoxy-4-methylsulfonylaminophenoxy)phenylmethyl)-1,4,9-triazaspiro[5.5]undecane,
(8) (3R)-1-butyl-2,5-dioxo-3-((1R)-1-hydroxy-1-(tetrahydropyran-4-yl)methyl)-9-(4-(4-(2-methylpropyl)carbonylaminophenylmethyl)phenylmethyl)-1,4,9-triazaspiro[5.5]undecane,
(9) (3R)-1-butyl-2,5-dioxo-3((1R)-1-hydroxy-1-(tetrahydropyran-4-yl)methyl)-9-(4-(4-(2-methylpropyl)carbonylamino-2-methoxyphenoxy)phenylmethyl)-1,4,9-triazaspiro[5.5]undecane,
(10) (3R)-1-butyl-2,5-dioxo-3-((1R)-1-hydroxy-1-cyclohexylmethyl)-9-(2-(4-methylaminocarbonylphenoxy)ethyl)-1,4,9-triazaspiro[5.5]undecane,
(11) (3R)-1-butyl-2,5-dioxo-3-((1R)-1-hydroxy-1-cyclohexylmethyl)-9-(5-(4-methylaminocarbonylphenoxy)pentyl)-1,4,9-triazaspiro[5.5]undecane,
(12) (3R)-1-butyl-2,5-dioxo-3-((1R)-1-hydroxy-1-cyclohexylmethyl)-9-(4-(4-methylaminocarbonylphenoxy)butyl)-1,4,9-triazaspiro[5.5]undecane,
(13) (3R)-1-butyl-2,5-dioxo-3-((1R)-1-hydroxy-1-(cyclohexen-4-yl)methyl)-9-(4-(4-methylaminocarbonylphenoxy)phenylmethyl)-1,4,9-triazaspiro[5.5]undecane,
(14) (3R)-1-butyl-2,5-dioxo-3-((1R)-1-hydroxy-1-cyclohexylmethyl)-9-(4-(4-methylsulfonylaminophenoxy)butyl)-1,4,9-triazaspiro[5.5]undecane, and
(15) (3R)-1-pentyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane.

3. The compound according to the above 1, which is selected from the group consisting of

(1) (3R)-1-butyl-2,5-dioxo-3-((1R)-1-hydroxy-1-cyclohexylmethyl)-9-(4-(4-cyclopropylmethylaminocarbonylphenoxy)phenylmethyl)-1,4,9-triazaspiro[5.5]undecane,
(2) (3R)-1-butyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(3) (3R)-1-butyl-2,5-dioxo-3-((1R)-1-hydroxy-1-cyclohexylmethyl)-9-(4-(4-methylaminocarbonyl-2-methoxyphenoxy)phenylmethyl)-1,4,9-triazaspiro[5.5]undecane,
(4) (3R)-1-butyl-2,5-dioxo-3-((1R)-1-hydroxy-1-cyclohexylmethyl)-9-(4-(4-carboxy-2-methoxyphenylmethyl)phenylmethyl)-1,4,9-triazaspiro[5.5]undecane,
(5) (3R)-1-butyl-2,5-dioxo-3-((1R)-1-hydroxy-1-cyclohexylmethyl)-9-(4-(4-(N,N,-dimethylaminocarbonyl)-2-methoxyphenoxy)phenylmethyl)-1,4,9-triazaspiro[5.5]undecane,
(6) (3R)-1-butyl-2,5-dioxo-3-((1R)-1-hydroxy-1-cyclohexylmethyl)-9-(4-(4-carboxy-2-ethoxyphenoxy)phenylmethyl)-1,4,9-triazaspiro[5.5]undecane,

(7) (3R)-1-butyl-2,5-dioxo-3-((1R)-1-hydroxy-1-cyclohexylmethyl)-9-(4-(4-carboxy-2,6-dimethylphenoxy)phenylmethyl)-1,4,9-triazaspiro[5.5]undecane,
(8) (3R)-1-pentyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(9) (3R)-1-butyl-2,5-dioxo-3-((1R)-1-hydroxy-2-ethylbutyl)-9-(4-(4-carboxy-2-methoxyphenoxy)phenylmethyl)-1,4,9-triazaspiro[5.5]undecane,
(10) (3R)-1-butyl-2,5-dioxo-3-((1R)-1-hydroxy-1-(tetrahydropyran-4-yl)methyl)-9-(4-(4-cyclopropylmethylaminocarbonylphenoxy)phenylmethyl)-1,4,9-triazaspiro[5.5]undecane,
(11) (3R)-1-propyl-2,5-dioxo-3-((1R)-1-hydroxy-1-cyclohexylmethyl)-9-(4-(4-dimethylaminocarbonyl-2-methoxyphenoxy)phenylmethyl)-1,4,9-triazaspiro[5.5]undecane,
(12) (3R)-1-butyl-2,5-dioxo-3-((1R)-1-hydroxy-1-cycloheptylmethyl)-9-(4-(4-carboxy-2-methoxyphenoxy)phenylmethyl)-1,4,9-triazaspiro[5.5]undecane,
(13) (3R)-1-butyl-2,5-dioxo-3-((1R)-1-hydroxy-1-cyclopentylmethyl)-9-(4-(4-carboxy-2-methoxyphenoxy)phenylmethyl)-1,4,9-triazaspiro[5.5]undecane,
(14) (3R)-1-butyl-2,5-dioxo-3-((1R)-1-hydroxy-1-cyclopentylmethyl)-9-(4-(4-carboxy-2-ethoxyphenoxy)phenylmethyl)-1,4,9-triazaspiro[5.5]undecane,
(15) (3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenoxy)phenylmethyl)-1,4,9-triazaspiro[5.5]undecane,
(16) (3R)-1-butyl-2,5-dioxo-3-((1R)-1-hydroxy-1-(tetrahydropyran-4-yl)methyl)-9-(4-(4-methylaminocarbonyl-2-methoxyphenylmethyl)phenylmethyl)-1,4,9-triazaspiro[5,5]undecane,
(17) (3R)-1-butyl-2,5-dioxo-3-((1R)-1-hydroxy-1-(tetrahydropyran-4-yl)methyl)-9-(4-(4-isopropylaminocarbonyl-2-methoxyphenoxy)phenylmethyl)-1,4,9-triazaspiro[5.5]undecane,
(18) (3R)-1-butyl-2,5-dioxo-3-((1R)-1-hydroxy-1-(tetrahydropyran-4-yl)methyl)-9-(4-(4-(2-methylpropyl)aminocarbonyl-2-methoxyphenoxy)phenylmethyl)-1,4,9-triazaspiro[5.5]undecane,
(19) (3R)-1-butyl-2,5-dioxo-3-((1R)-1-hydroxy-1-(tetrahydropyran-4-yl)methyl)-9-(4-(4-(2,2-dimethylpropylaminocarbonyl)-2-methoxyphenoxy)phenylmethyl)-1,4,9-triazaspiro[5.5]undecane,
(20) (3R)-1-butyl-2,5-dioxo-3-((1R)-1-hydroxy-1-(tetrahydropyran-4-yl)methyl)-9-(4-(4-isopropylcarbonylaminophenoxy)phenylmethyl)-1,4,9-triazaspiro[5.5]undecane,
(21) (3R)-1-butyl-2,5-dioxo-3-((1R)-1-hydroxy-1-(tetrahydropyran-4-yl)methyl)-9-(4-(4-(2-methylpropyl)carbonylaminophenoxy)phenylmethyl)-1,4,9-triazaspiro[5.5]undecane,
(22) (3R)-1-butyl-2,5-dioxo-3-((1R)-1-hydroxy-1-(tetrahydropyran-4-yl)methyl)-9-(4-(4-isopropylcarbonylamino-2-methoxyphenoxy)phenylmethyl)-1,4,9-triazaspiro[5.5]undecane,
(23) (3R)-1-butyl-2,5-dioxo-3-((1R)-1-hydroxy-1-(cyclopenten-4-yl)methyl)-9-(4-(4-carboxy-2-methylphenoxy)phenylmethyl)-1,4,9-triazaspiro[5.5]undecane,
(24) (3R)-1-butyl-2,5-dioxo-3-((1R)-1-hydroxy-1-(cyclopenten-4-yl)methyl)-9-(4-(4-carboxy-2-ethoxyphenoxy)phenylmethyl)-1,4,9-triazaspiro[5.5]undecane,
(25) (3R)-1-(2-butynyl)-2,5-dioxo-3-((1R)-1-hydroxy-1-cyclohexylmethyl)-9-(4-(4-methylaminocarbonylphenoxy)butyl)-1,4,9-triazaspiro[5.5]undecane,
(26) (3R)-1-butyl-2,5-dioxo-3-((1R)-1-hydroxy-1-cyclohexylmethyl)-9-(1-(4-(4-methylaminocarbonylphenoxy)phenyl)ethyl)-1,4,9-triazaspiro[5.5]undecane, and
(27) (3R)-1-butyl-2,5-dioxo-3-((1R)-1-hydroxy-1-cyclohexylmethyl)-9-(1-(4-(4-carboxyphenoxy)phenyl)ethyl)-1,4,9-triazaspiro[5.5]undecane.

4. The compound according to the above 1, wherein $R^1$ is C1-8 alkyl, C2-4 alkenyl, or C2-4 alkynyl substituted with $-COR^6$, $=NR^{15}$, or $=NOR^{16}$, in which $R^6$, $R^{15}$ and $R^{16}$ have the same meanings as defined in the above 1.
5. The compound according to the above 1, wherein at least one of substituents of ring 1 in $R^1$ is $-COR^{12}$, oxo, or $=NOR^{35}$, in which $R^{12}$ and $R^{35}$ have the same meanings as defined in the above 1.
6. The compound according to the above 1, wherein at least one of substituents of ring 2 in $R^1$ is $-COR^{44}$ or $-C(NH_2)=NOR^{54}$, in which $R^{44}$ and $R^{54}$ have the same meanings as defined in the above 1.
7. The compound according to the above 1, wherein at least one of substituents of ring 3 in $R^1$ is =O or =S.
8. A pharmaceutical composition which comprises, as an active ingredient, the compound represented by formula (I) according to the above 1, a quaternary ammonium salt thereof, an N-oxide thereof, or a salt thereof.
9. A regulator of a chemokine/chemokine receptor, which comprises, as an active ingredient, the compound represented by formula (I) according to the above 1, a quaternary ammonium salt thereof, an N-oxide thereof, or a salt thereof.
10. The regulator of a chemokine/chemokine receptor according to the above 9, which is a CCR5 antagonist.
11. A pharmaceutical composition for prevention and/or treatment for inflammatory diseases, immunologic diseases, human immunodeficiency virus, allergic diseases, ischemia-reperfusion injury, acute respiratory distress syndrome, shock accompanied by bacterial infection, diabetes mellitus, or metastasis, which comprises, as an active ingredient, the compound represented by formula (I) according to the above 1, a quaternary ammonium salt thereof, an N-oxide thereof, or a salt thereof.

12. A method for antagonizing CCR5 in a mammal, which comprises administering to a mammal an effective amount of the compound of formula (I) according to the above 1, a quaternary ammonium salt thereof, an N-oxide thereof, or a salt thereof.

13. Use of the compound of formula (I) according to the above 1, a quaternary ammonium salt thereof, an N-oxide thereof, or a salt thereof for the manufacture of a CCR5 antagonist.

Detailed description of the invention

In the specification, C1-4 alkyl means methyl, ethyl, propyl, butyl or isomer thereof

In the specification, C1-8 alkyl means methyl, ethyl, propyl, butyl, pentyl, hexyl, heptyl, octyl or isomer thereof

In the specification, C2-4 alkenyl means etenyl, propenyl, butenyl or isomer thereof

In the specification, C2-8 alkenyl means etenyl, propenyl, butenyl, pentenyl, hexenyl, heptenyl, octenyl or isomer thereof.

In the specification, C2-4 alkynyl means ethynyl, propynyl, butynyl or isomer thereof.

In the specification, C2-8 alkynyl means ethynyl, propynyl, butynyl, pentynyl, hexynyl, heptynyl, octynyl or isomer thereof

In the specification, C3-8 cycloalkyl means cyclopropyl, cyclobutyl, cyclopentyl, cyclohexyl, cycloheptyl or cyclooctyl.

In the specification, C3-15 mono-, bi- or tri-carbocyclic aryl, which may be partially or fully saturated, represented by ring 1, ring 2 or ring 5 means, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, or noradamantane *etc*.

In the specification, 3- to 15-membered mono-, bi- or tri-cyclic hetero aryl containing 1-4 nitrogen atoms, 1-2 oxygen atoms and/or 1-2 sulfur atoms in 3- to 15-membered mono-, bi- or tri-cyclic hetero aryl containing 1-4 nitrogen atoms, 1-2 oxygen atoms and/or 1-2 sulfur atoms, which may be partially or fully saturated, represented by ring 1, ring 2 or ring 5 means, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, beta-carboline, acridine, phenazine, dibenzofuran, xanthene, dibenzothiophene, phenothiazine, phenoxazine, phenoxathiin, thianthrene, phenanthridine, phenanthroline, perimidine *etc*.

Furthermore, partially or fully saturated 3- to 15-membered mono-, bi- or tri-cyclic hetero aryl containing 1-4 nitrogen atoms, 1-2 oxygen atoms and/or 1-2 sulfur atoms means, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiophene, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane *etc*.

In the specification, C3-8 mono-carbocyclic aryl, which may be partially or fully saturated, represented by ring 3 means, for example, cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene *etc*.

In the specification, 3-8 membered mono-cyclic hetero aryl containing 1-4 nitrogen atoms, 1-2 oxygen atoms and/or 1-2 sulfur atoms in 3-8 membered mono-cyclic hetero aryl containing 1-4 nitrogen atoms, 1-2 oxygen atoms and/or 1-2 sulfur atoms, which may be partially or fully saturated, represented by ring 3 means, for example, pyrrole, imidazole, triazole, tetrazol, pyrazole, pyridine, pyrazine, pyrimidine, pyridazine, azepine, diazepine, furan, pyran, oxepine, thiophene, thiaine (thiopyran), thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine *etc*.

Furthermore, partially or fully saturated 3-8 membered mono-cyclic hetero aryl containing 1-4 nitrogen atoms, 1-2 oxygen atoms and/or 1-2 sulfur atoms means, for example, pyrroline, pyrrolidine, imidazoline, imidazolidine, pyrazoline, pyrazolidine, triazoline, triazolidine, tetrazoline, tetrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiaine (dihydrothiopyran), tetrahydrothaine (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, dihydrooxadiazole, tetrahydrooxadiazole, dihydrothiadiazole, tetrahydrothiadiazole, tetrahydrooxadiazine, tetrahydrothiadiazine, tetrahydrooxazepine, tetrahydrooxadiazepine, perhydrooxazepine, perhydrooxadiazepine, tetrahydrothiazepine, tetrahydrothiadiazepine, perhydrothiazepine, perhydrothiadiazepine, morpholine, thiomorpholine, dioxolane, dioxane, dithiolane, dithiane *etc*.

In the present invention, each group represented by $R^1$, $R^2$, $R^3$ or $R^4$ is all preferable.

In the group represented by $R^1$, C1-8 alkyl, C2-4 alkenyl or C2-4 alkynyl alkyl substituted with ring 1 is preferable specially.

In the group represented by $R^2$, C1-6 alkyl, C2-6 alkenyl, C2-6 alkynyl alkyl, C1-6 alkyl, C2-6 alkenyl or C2-6 alkynyl substituted with $-OR^{60}$, or benzyl is preferable specially.

In the group represented by $R^3$ and $R^4$, one is hydrogen and the other is C1-6 alkyl, C2-6 alkenyl or C2-6 alkynyl substituted with hydroxy and/or ring 5 is preferable specially.

Furthermore, compounds described in example, specifically. More preferred compounds are

(1) (3R)-1-butyl-2,5-dioxo-3-((1R)-1-hydroxy-1-cyclohexylmethyl)-9-(4-(4-cyclopropylmethylaminocarbonylphenoxy)phenylmethyl)-1,4,9-triazaspiro[5.5]undecane,

(2) (3R)-1-butyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,

(3) (3R)-1-butyl-2,5-dioxo-3-((1R)-1-hydroxy-1-cyclohexylmethyl)-9-(4-(4-methylaminocarbonyl-2-methoxyphenoxy)phenylmethyl)-1,4,9-triazaspiro[5.5]undecane,

(4) (3R)-1-butyl-2,5-dioxo-3-((1R)-1-hydroxy-1-cyclohexylmethyl)-9-(4-(4-methylaminocarbonyl-2-chlorophenoxy)phenylmethyl)-1,4,9-triazaspiro[5.5]undecane,

(5) (3R)-1-butyl-2,5-dioxo-3-((1R)-1-hydroxy-1-cyclohexylmethyl)-9-(4-(4-(5-oxo-4,5-dihydro-1,2,4-thiadiazol-3-yl)phenoxy)phenylmethyl)-1,4,9-triazaspiro[5.5]undecane,

(6) (3R)-1-butyl-2,5-dioxo-3-((1R)-1-hydroxy-1-cyclohexylmethyl)-9-(4-(4-carboxy-2-methoxyphenylmethyl)phenylmethyl)-1,4,9-triazaspiro[5.5]undecane,

(7) (3R)-1-butyl-2,5-dioxo-3-((1R)-1-hydroxy-1-cyclohexylmethyl)-9-(4-(4-(N,N,-dimethylaminocarbonyl)-2-methoxyphenoxy)phenylmethyl)-1,4,9-triazaspiro[5.5]undecane,

(8) (3R)-1-butyl-2,5-dioxo-3-((1R)-1-hydroxy-1-cyclohexylmethyl)-9-(4-(4-carboxy-2-ethoxyphenoxy)phenylmethyl)-1,4,9-triazaspiro[5.5]undecane,
(9) (3R)-1-butyl-2,5-dioxo-3-((1R)-1-hydroxy-1-cyclohexylmethyl)-9-(4-(4-carboxy-2,6-dimethylphenoxy)phenylmethyl)-1,4,9-triazaspiro[5.5]undecane,
(10) (3R)-1-pentyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(11) (3R)-1-butyl-2,5-dioxo-3-((1R)-1-hydroxy-2-ethylbutyl)-9-(4-(4-carboxy-2-methoxyphenoxy)phenylmethyl)-1,4,9-triazaspiro[5.5]undecane,
(12) (3R)-1-butyl-2,5-dioxo-3-((1R)-1-hydroxy-1-(tetrahydropyran-4-yl)methyl)-9-(4-(4-cyclopropylmethylaminocarbonylphenoxy)phenylmethyl)-1,4,9-triazaspiro[5.5]undecane,
(13) (3R)-1-propyl-2,5-dioxo-3-((1R)-1-hydroxy-1-cyclohexylmethyl)-9-(4-(4-dimethylaminocarbonyl-2-methoxyphenoxy)phenylmethyl)-1,4,9-triazaspiro[5.5]undecane,
(14) (3R)-1-butyl-2,5-dioxo-3-((1R)-1-hydroxy-1-cycloheptylmethyl)-9-(4-(4-carboxy-2-methoxyphenoxy)phenylmethyl)-1,4,9-triazaspiro[5.5]undecane,
(15) (3R)-1-butyl-2,5-dioxo-3-((1R)-1-hydroxy-1-cyclopentylmethyl)-9-(4-(4-carboxy-2-methoxyphenoxy)phenylmethyl)-1,4,9-triazaspiro[5.5]undecane,
(16) (3R)-1-butyl-2,5-dioxo-3-((1R)-1-hydroxy-1-cyclopentylmethyl)-9-(4-(4-carboxy-2-ethoxyphenoxy)phenylmethyl)-1,4,9-triazaspiro[5.5]undecane,
(17) (3R)-1-butyl-2,5-dioxo-3-((1R)-1-hydroxy-2-ethylbutyl)-9-(4-(4-(2-methoxyethylaminocarbonyl)-2-methoxyphenoxy)phenylmethyl)-1,4,9-triazaspiro[5.5]undecane,
(18) (3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenoxy)phenylmethyl)-1,4,9-triazaspiro[5.5]undecane,
(19) (3R)-1-butyl-2,5-dioxo-3-((1R)-1-hydroxy-1-(tetrahydropyran-4-yl)methyl)-9-(4-(4-methylaminocarbonyl-2-methoxyphenylmethyl)phenylmethyl)-1,4,9-triazaspiro[5.5]undecane,
(20) (3R)-1-butyl-2,5-dioxo-3-((1R)-1-hydroxy-1-(tetrahydropyran-4-yl)methyl)-9-(4-(4-isopropylaminocarbonyl-2-methoxyphenoxy)phenylmethyl)-1,4,9-triazaspiro[5.5]undecane,
(21) (3R)-1-butyl-2,5-dioxo-3-((1R)-1-hydroxy-1-(tetrahydropyran-4-yl)methyl)-9-(4-(4-(2-methylpropyl)aminocarbonyl-2-methoxyphenoxy)phenylmethyl)-1,4,9-triazaspiro[5.5]undecane,
(22)(3R)-1-butyl-2,5-dioxo-3-((1R)-1-hydroxy-1-(tetrahydropyran-4-yl)methyl)-9-(4-(4-(2,2-dimethylpropylaminocarbonyl)-2-methoxyphenoxy)phenylmethyl)-1,4,9-triazaspiro[5.5]undecane,
(23) (3R)-1-butyl-2,5-dioxo-3-((1R)-1-hydroxy-2-methylpropyl)-9-(4-(4-methylsulfonylamino-2-methoxyphenoxy)phenylmethyl)-1,4,9-triazaspiro[5.5]undecane,
(24) (3R)-1-butyl-2,5-dioxo-3-((1R)-1-hydroxy-1-(tetrahydropyran-4-yl)methyl)-9-(4-(4-(pyrrolidin-1-yl)carbonyl-2-methoxyphenoxy)phenylmethyl)-1,4,9-triazaspiro[5.5]undecane,
(25) (3R)-1-butyl-2,5-dioxo-3-((1R)-1-hydroxy-2-ethylbutyl)-9-(4-(2-methoxy-4-methylsulfonylaminophenoxy)phenylmethyl)-1,4,9-triazaspiro[5.5]undecane,
(26) (3S)-1-butyl-2,5-dioxo-3-(2-methylpropyl)-9-(4-(2-methoxy-4-methylsulfonylaminophenoxy)phenylmethyl)-1,4,9-triazaspiro[5.5]undecane,
(27) (3R)-1-butyl-2,5-dioxo-3-((1R)-1-hydroxy-1-(tetrahydropyran-4-yl)methyl)-9-(4-(4-(2-methylpropyl)carbonylaminophenylmethyl)phenylmethyl)-1,4,9-triazaspiro[5.5]undecane,
(28) (3R)-1-butyl-2,5-dioxo-3-((1R)-1-hydroxy-1-(tetrahydropyran-4-yl)methyl)-9-(4-(4-isopropylcarbonylaminophenoxy)phenylmethyl)-1,4,9-triazaspiro[5.5]undecane,
(29) (3R)-1-butyl-2,5-dioxo-3-((1R)-1-hydroxy-1-(tetrahydropyran-4-yl)methyl)-9-(4-(4-(2-methylpropyl)carbonylaminophenoxy)phenylmethyl)-1,4,9-triazaspiro[5.5]undecane,
(30) (3R)-1-butyl-2,5-dioxo-3-((1R)-1-hydroxy-1-(tetrahydropyran-4-yl)methyl)-9-(4-(4-isopropylcarbonylamino-2-methoxyphenoxy)phenylmethyl)-1,4,9-triazaspiro[5.5]undecane,
(31) (3R)-1-butyl-2,5-dioxo-3-((1R)-1-hydroxy-1-(tetrahydropyran-4-yl)methyl)-9-(4-(4-(2-methylpropyl)carbonylamino-2-methoxyphenoxy)phenylmethyl)-1,4,9-triazaspiro[5.5]undecane,
(32) (3R)-1-butyl-2,5-dioxo-3-((1R)-1-hydroxy-1-cyclohexylmethyl)-9-(2-(4-methylaminocarbonylphenoxy)ethyl)-1,4,9-triazaspiro[5.5]undecane,
(33) (3R)-1-butyl-2,5-dioxo-3-((1R)-1-hydroxy-1-cyclohexylmethyl)-9-(5-(4-methylaminocarbonylphenoxy)pentyl)-1,4,9-triazaspiro[5.5]undecane,
(34) (3R)-1-butyl-2,5-dioxo-3-((1R)-1-hydroxy-1-cyclohexylmethyl)-9-(4-(4-methylaminocarbonylphenoxy)butyl)-1,4,9-triazaspiro[5.5]undecane,
(35) (3R)-1-butyl-2,5-dioxo-3-((1R)-1-hydroxy-1-(cyclohexen-4-yl)methyl)-9-(4-(4-methylaminocarbonylphenoxy)phenylmethyl)-1,4,9-triazaspiro[5.5]undecane,
(36) (3R)-1-butyl-2,5-dioxo-3-((1R)-1-hydroxy-1-(cyclopenten-4-yl)methyl)-9-(4-(4-carboxy-2-methylphenoxy)phenylmethyl)-1,4,9-triazaspiro[5.5]undecane,

(37) (3R)-1-butyl-2,5-dioxo-3-((1R)-1-hydroxy-1-(cyclopenten-4-yl)methyl)-9-(4-(4-carboxy-2-ethoxyphenoxy)phenylmethyl)-1,4,9-triazaspiro[5.5]undecane,
(38) (3R)-1-(2-butynyl)-2,5-dioxo-3-((1R)-1-hydroxy-1-cyclohexylmethyl)-9-(4-(4-methylaminocarbonylphenoxy)butyl)-1,4,9-triazaspiro[5.5]undecane,
(39) (3R)-1-butyl-2,5-dioxo-3-((1R)-1-hydroxy-1-cyclohexylmethyl)-9-(1-(4-(4-methylaminocarbonylphenoxy)phenyl)ethyl)-1,4,9-triazaspiro[5.5]undecane,
(40) (3R)-1-butyl-2,5-dioxo-3-((1R)-1-hydroxy-1-cyclohexylmethyl)-9-(4-(4-methylsulfonylaminophenoxy)butyl)-1,4,9-triazaspiro[5.5]undecane,
(41) (3R)-1-pentyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane,
(42) (3R)-1-butyl-2,5-dioxo-3-((1R)-1-hydroxy-1-cyclohexymethyl)-9-(1-(4-(4-carboxyphenoxy)phenyl)ethyl)-1,4,9-triazaspiro[5.5]undecane, or salts thereof.

Specific preferred compounds are

(1) (3R)-1-butyl-2,5-dioxo-3-((1R)-1-hydroxy-1-cyclohexylmethyl)-9-(4-(4-cyclopropylmethylaminocarbonylphenoxy)phenylmethyl)-1,4,9-triazaspiro[5.5]undecane,
(2) (3R)-1-butyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(3) (3R)-1-butyl-2,5-dioxo-3-((1R)-1-hydroxy-1-cyclohexylmethyl)-9-(4-(4-methylaminocarbonyl-2-methoxyphenoxy)phenylmethyl)-1,4,9-triazaspiro[5.5]undecane,
(4) (3R)-1-butyl-2,5-dioxo-3-((1R)-1-hydroxy-1-cyclohexylmethyl)-9-(4-(4-carboxy-2-methoxyphenylmethyl)phenylmethyl)-1,4,9-triazaspiro[5.5]undecane,
(5) (3R)-1-butyl-2,5-dioxo-3-((1R)-1-hydroxy-1-cyclohexylmethyl)-9-(4-(4-(N,N,-dimethylaminocarbonyl)-2-methoxyphenoxy)phenylmethyl)-1,4,9-triazaspiro[5.5]undecane,
(6) (3R)-1-butyl-2,5-dioxo-3-((1R)-1-hydroxy-1-cyclohexylmethyl)-9-(4-(4-carboxy-2-ethoxyphenoxy)phenylmethyl)-1,4,9-triazaspiro[5.5]undecane,
(7) (3R)-1-butyl-2,5-dioxo-3-((1R)-1-hydroxy-1-cyclohexylmethyl)-9-(4-(4-carboxy-2,6-dimethylphenoxy)phenylmethyl)-1,4,9-triazaspiro[5.5]undecane,
(8) (3R)-1-pentyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(9) (3R)-1-butyl-2,5-dioxo-3-((1R)-1-hydroxy-2-ethylbutyl)-9-(4-(4-carboxy-2-methoxyphenoxy)phenylmethyl)-1,4,9-triazaspiro[5.5]undecane,
(10) (3R)-1-butyl-2,5-dioxo-3-((1R)-1-hydroxy-1-(tetrahydropyran-4-yl)methyl)-9-(4-(4-cyclopropylmethylaminocarbonylphenoxy)phenylmethyl)-1,4,9-triazaspiro[5.5]undecane,
(11) (3R)-1-propyl-2,5-dioxo-3-((1R)-1-hydroxy-1-cyclohexylmethyl)-9-(4-(4-dimethylaminocarbonyl-2-methoxyphenoxy)phenylmethyl)-1,4,9-triazaspiro[5.5]undecane,
(12) (3R)-1-butyl-2,5-dioxo-3-((1R)-1-hydroxy-1-cycloheptylmethyl)-9-(4-(4-carboxy-2-methoxyphenoxy)phenylmethyl)-1,4,9-triazaspiro[5.5]undecane,
(13) (3R)-1-butyl-2,5-dioxo-3-((1R)-1-hydroxy-1-cyclopentylmethyl)-9-(4-(4-carboxy-2-methoxyphenoxy)phenylmethyl)-1,4,9-triazaspiro[5.5]undecane,
(14) (3R)-1-butyl-2,5-dioxo-3-((1R)-1-hydroxy-1-cyclopentylmethyl)-9-(4-(4-carboxy-2-ethoxyphenoxy)phenylmethyl)-1,4,9-triazaspiro[5.5]undecane,
(15) (3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenoxy)phenylmethyl)-1,4,9-triazaspiro[5.5]undecane,
(16) (3R)-1-butyl-2,5-dioxo-3-((1R)-1-hydroxy-1-(tetrahydropyran-4-yl)methyl)-9-(4-(4-methylaminocarbonyl-2-methoxyphenylmethyl)phenylmethyl)-1,4,9-triazaspiro[5.5]undecane,
(17) (3R)-1-butyl-2,5-dioxo-3-((1R)-1-hydroxy-1-(tetrahydropyran-4-yl)methyl)-9-(4-(4-isopropylaminocarbonyl-2-methoxyphenoxy)phenylmethyl)-1,4,9-triazaspiro[5.5]undecane,
(18) (3R)-1-butyl-2,5-dioxo-3-((1R)-1-hydroxy-1-(tetrahydropyran-4-yl)methyl)-9-(4-(4-(2-methylpropyl)aminocarbonyl-2-methoxyphenoxy)phenylmethyl)-1,4,9-triazaspiro[5.5]undecane,
(19)(3R)-1-butyl-2,5-dioxo-3-((1R)-1-hydroxy-1-(tetrahydropyran-4-yl)methyl)-9-(4-(4-(2,2-dimethylpropylaminocarbonyl)-2-methoxyphenoxy)phenylmethyl)-1,4,9-triazaspiro[5.5]undecane,
(20) (3R)-1-butyl-2,5-dioxo-3-((1R)-1-hydroxy-1-(tetrahydropyran-4-yl)methyl)-9-(4-(4-isopropylcarbonylaminophenoxy)phenylmethyl)-1,4,9-triazaspiro[5.5]undecane,
(21) (3R)-1-butyl-2,5-dioxo-3-((1R)-1-hydroxy-1-(tetrahydropyran-4-yl)methyl)-9-(4-(4-(2-methylpropyl)carbonylaminophenoxy)phenylmethyl)-1,4,9-triazaspiro[5.5]undecane,
(22) (3R)-1-butyl-2,5-dioxo-3-((1R)-1-hydroxy-1-(tetrahydropyran-4-yl)methyl)-9-(4-(4-isopropylcarbonylamino-2-methoxyphenoxy)phenylmethyl)-1,4,9-triazaspiro[5.5]undecane,

(23) (3R)-1-butyl-2,5-dioxo-3-((1R)-1-hydroxy-1-(cyclopenten-4-yl)methyl)-9-(4-(4-carboxy-2-methylphenoxy) phenylmethyl)-1,4,9-triazaspiro[5.5]undecane,

(24) (3R)-1-butyl-2,5-dioxo-3-((1R)-1-hydroxy-1-(cyclopenten-4-yl)methyl)-9-(4-(4-carboxy-2-ethoxyphenoxy) phenylmethyl)-1,4,9-triazaspiro[5.5]undecane,

(25) (3R)-1-(2-butynyl)-2,5-dioxo-3-((1R)-1-hydroxy-1-cyclohexylmethyl)-9-(4-(4-methylaminocarbonylphenoxy) butyl)-1,4,9-triazaspiro[5.5]undecane,

(26) (3R)-1-butyl-2,5-dioxo-3-((1R)-1-hydroxy-1-cyclohexylmethyl)-9-(1-(4-(4-methylaminocarbonylphenoxy)phenyl)ethyl)-1,4,9-triazaspiro[5.5]undecane,

(27) (3R)-1-butyl-2,5-dioxo-3-((1R)-1-hydroxy-1-cyclohexylmethyl)-9-(1-(4-(4-carboxyphenoxy)phenyl)ethyl)-1,4,9-triazaspiro[5.5]undecane, or salts thereof.

Unless otherwise specified, all isomers are included in the present invention. For example, alkyl, alkenyl, alkynyl, alkoxy alkylthio, alkylene, alkenylene and alkynylene groups include straight or branched ones. In addition, isomers on double bond, ring, fused ring (E-, Z-, cis-, trans-isomer), isomers generated from asymmetric carbon atoms (R-, S-isomer, α-, β-configration, enantiomer, diastereomer), optically active isomer (D-, L-, d-, 1-isomer), polar compounds generated by chromatographic separation (more polar compound, less polar compound), equilibrium compounds, rotational isomer, mixtures thereof at voluntary ratios and racemic mixtures are also included in the present invention.

In the present invention, unless otherwise specified, as will be apparent to those skilled in the art, a symbol 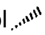 represents bonding to back of the paper (that is, α-configuration), 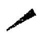 represents bonding to front of the paper (that is, β-configuration), 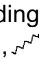 represents α-configuration, β-configuration or mixture thereof and 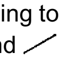 represents mixture of α-configuration and β-configuration.

The compounds represented by formula (I) may be converted into the corresponding salts by conventional means.

As the salt, alkali metal salt, alkali earth metal salt, ammonium salt, amine salt and acid addition salt *etc.* are included.

Water-soluble salts are preferred. Suitable salts, for example, include: salts of alkali metals (e.g. potassium and sodium), salts of alkaline earth metals (e.g. calcium and magnesium), ammonium salts, and salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl) aminnomethane, lysine, arginine and N-methyl-D-glucamine).

Preferred acid addition salts are water-soluble salts. Suitable acid addtion salts, for example, include: salts of inorganic acids e.g. hydrochloride, hydrobromide, hydroiodide, sulfate, phosphate, nitrate, and salts of organic acids e.g. acetate, lactate, tartrate, benzoate, citrate, methanesulfonate, benzenesulfonate, toluenesulfonate, isethionate, glucuronate and gluconate.

The compounds represented by formula (1) and salts thereof may be converted into the corresponding solvates.

Preferred solvates are water-soluble salts. Suitable solvates, for example, include: solvates of water and alcohol (e.g. ethanol) and the like

All of the compounds represented by formula (I) or salts thereof are preferable, concretely, the compounds described in the example or salts thereof.

Quaternary ammonium salts of the compounds represented by formula (I) are the compounds where nitrogen of the compounds represented by formula (I) is quarternalized by $R^0$.

$R^0$ is C1-8 alkyl or C1-8 alkyl substituted with phenyl.

N-oxides of the compounds represented by formula (I) are the compounds where nitrogen of the compounds represented by formula (I) is oxidized.

Methods for preparation of the compounds in the present invention:

The compounds of the present invention of formula (I) may be prepared by the following methods or the methods described in examples.

Among the compounds of the present invention of formula (I), the compounds where nitrogens are not quaternary ammonium salts or N-oxides, i.e., the compounds of formula (I-1)

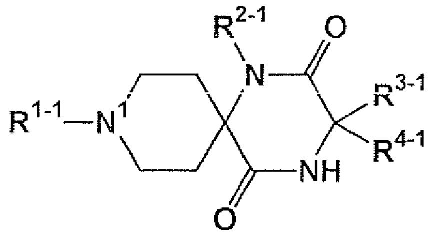

(I-1)

(wherein $R^{1-1}$, $R^{2-1}$, $R^{3-1}$ and $R^{4-1}$ have the same meanings as $R^1$, $R^2$, $R^3$ and $R^4$, respectively, and $N^1$ is nitrogen, and wherein any nitrogen are not quaternary ammonium salts or N-oxides.) may be prepared by the following methods.

Among the compounds of formula (I-1), the compounds in which any $R^{1-1}$, $R^{2-1}$, $R^{3-1}$ and $R^{4-1}$ are not a group containing carboxyl, hydroxy, amino or thiol, i.e., the compounds of formula (I-1A)

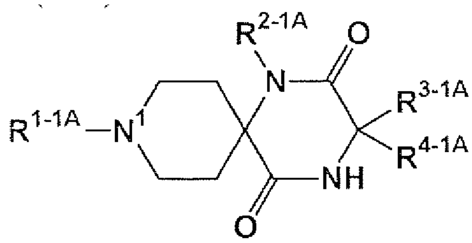

(I-1A)

(wherein $R^{1-1A}$, $R^{2-1A}$, $R^{3-1A}$, and $R^{4-1A}$ have the same meanings as $R^{1-1}$, $R^{2-1}$, $R^{3-1}$ and $R^{4-1}$, respectively, and wherein all of them are not a group containing carboxyl, hydroxy, amino or thiol, and the other symbols have the same meanings as defined hereinbefore.) may be prepared by cyclization of the compounds of formula (II)

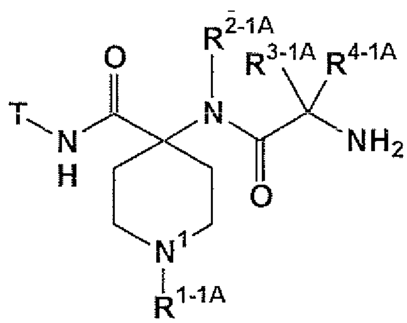

(II)

(wherein T is C1-4 alkyl, C5-6 mono-carbocyclic ring or C1-4 alkyl substituted with C5-6 mono-carbocyclic ring or 5-6 membered mono-cyclic hetero ring containing 1-2 nitrogen atoms and/or one oxygen atom.).

The cyclization is known *per se* and can be carried out by, for example, heating in an organic solvent (dichloroethane or toluene *etc.*), with tertiary amine (triethylamine or diisopropylethylamine *etc.*) or acid (acetic acid or trifluoroacetic acid *etc.*), or without tertiary amine or acid at 60-120°C. This cyclization reaction is carried out with the cleavage of T group.

Moreover, the cyclization may be carried out with the compounds wherein $R^3$ or $R^4$ includes hydroxy.

Moreover, the cyclization may be carried out with the compounds wherein nitrogen atom in $R^1$, $R^2$, $R^3$ or $R^4$ is oxidized to N-oxide.

Furthermore, if necessary, following the reaction, the compounds may be converted into the desired salts.

Furthermore, the compounds of formula (I-1A) may be prepared by the reductive amination of the compounds of formula (III)

$$R^{1-1A}—CHO \qquad (III)$$

(wherein all of the symbols have the same meanings as defined hereinbefore.)

with the compounds of formula (IV)

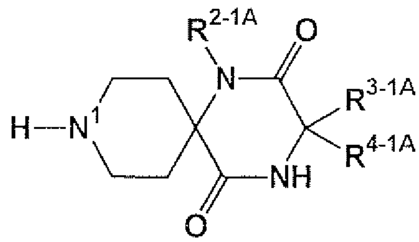

(IV)

(wherein all of the symbols have the same meanings as defined hereinbefore.).

The reductive amination is known *per se* and can be carried out, for example, in an organic solvent (dichloroethane, dichloromethane, dimethylformamide or acetic acid, or a mixture thereof *etc.*) in the presence of a reducing agent (sodium triacetoxyborohydride, sodium cyanoborohydride or sodium borohydride *etc.*) at 0-40°C.

Moreover, the reductive amination may be carried out with the compounds in which nitrogen of $R^1$ is oxidized to N-oxide.

Moreover, the reductive amination may be carried out with the compounds wherein $R^3$ or $R^4$ includes hydroxy.

Furthermore, the compounds of formula (I-1A) may be prepared by reacting the compounds of formula (V)

$$R^{1\text{-}1A}—X \qquad (V)$$

(wherein X is halogen or methanesulfonate, and the other symbols have the same meanings as defined hereinbefore.) with the compounds of formula (IV).

The reaction is known *per se* and can be carried out, for example, in an organic solvent (potassium carbonate or sodium carbonate *etc.*) in the presence of sodium iodide or potassium iodide at 100-150°C.

Moreover, the reaction may be carried with the compounds wherein nitrogen atom in $R^1$, $R^2$, $R^3$ or $R^4$ is oxidized to N-oxide.

Among the compounds of formula (I-IA), the compounds where $R^{1\text{-}1A}$ is ring 1 which is not 3-15 mono-, bi- or tri-carbocyclic aryl nor 3- to 15-membered mono-, bi- or tri-cyclic hetero aryl containing 1-4 nitrogen atoms, 1-2 oxygen atoms and/or 1-2 sulfur atoms, i.e., the compounds of formula (I-1A-1a)

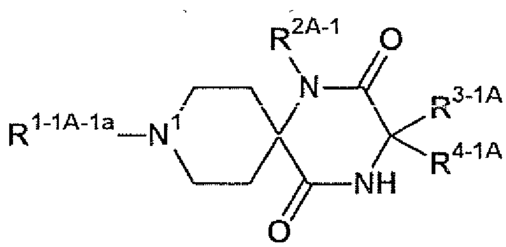

(I-1A-1a)

(wherein $R^{1\text{-}1A}$ is ring 1 which is not 3-15 mono-, bi- or tri-carbocyclic aryl nor 3- to 15-membered mono-, bi- or tricyclic hetero aryl containing 1-4 nitrogen atoms, 1-2 oxygen atoms and/or 1-2 sulfur atoms, and the other symbols have the same meanings as defined hereinbefore.) may be prepared by the reductive animation of the compounds of formula (VI)

$$R^{1\text{-}1A\text{-}1a}{=}O \qquad (VI)$$

(wherein all of the symbols have the same meanings as defined hereinbefore.)
with the compounds of formula (IV).

The reductive amination is known *per se* and can be carried out, for example, in an organic solvent (dichloroethane, dichloromethane, dimethylformamide or acetic acid, or a mixture thereof *etc.*) in the presence of a reducing agent (sodium triacetoxyborohydride, sodium cyanoborohydride or sodium borohydride *etc.*) at 0-40°C.

Among the compounds of formula (I-1), the compounds where at least one group of $R^1$, $R^2$, $R^3$ or $R^4$ represents

a group containing carboxyl, hydroxy, amino or thiol, i.e., the compounds of formula (I-1B)

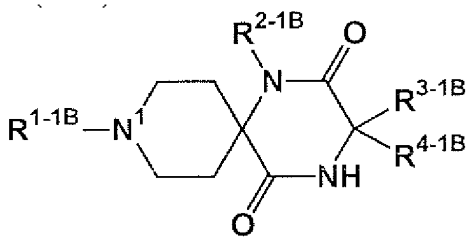

(I-1B)

(wherein $R^{1\text{-}1B}$, $R^{2\text{-}1\ B}$, $R^{3\text{-}1B}$ and $R^{4\text{-}1B}$ have the same meanings as $R^{1\text{-}1}$, $R^{2\text{-}1}$, $R^{3\text{-}1}$ and $R^{4\text{-}1}$, respectively, and wherein at least one group represents a group containing carboxyl, hydroxy, amino or thiol, and the other symbols have the same meanings as defined hereinbefore.) may be prepared by the removal of protecting groups containing carboxyl, hydroxy, amino and thiol protected by protecting groups, i.e., the compounds of formula (I-1A-1)

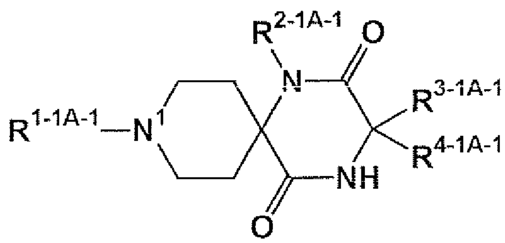

(I-1A-1)

(wherein $R^{1\text{-}1A\text{-}1}$, $R^{2\text{-}1A\text{-}1}$, $R^{3\text{-}1A\text{-}1}$ and $R^{4\text{-}1A\text{-}1}$ have the same meanings as $R^{1\text{-}1}$, $R^{2\text{-}1}$, $R^{3\text{-}1}$ or $R^{4\text{-}1}$, respectively, and wherein at least one group represents a group containing carboxyl, hydroxy, amino or thiol, and the other symbols have the same meanings as defined hereinbefore.).

A protecting group of carboxyl includes, for example, methyl, ethyl, allyl, t-butyl, trichloroethyl, benzyl (Bn) or phenacyl *etc*.

A protecting group of hydroxy includes, for example, methyl trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac) pivaloyl, benzoyl, benzyl (Bn), p-methoxybenzyl, allyloxycarbonyl (Alloc) or 2,2,2-trichloroethoxycarbonyl (Troc) *etc*.

A protecting group of amino includes, for example, benzyloxycarbonyl, t-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl, benzyl (Bn), p-methoxybenzyl, benzyloxymethyl (BOM) or 2-(trimethylsilyl)ethoxymethyl (SEM) *etc*.

A protecting group of thiol includes, for example, benzyl, methoxybenzyl, methoxymethyl (MOM), 2-tetrahydropyranyl (THP), diphenylmethyl or acetyl (Ac).

The protecting group of carboxyl, hydroxy, amino or thiol includes the above one, and in addition to the other protecting group which is removable selectively and easily, for example, one described in T. W. Greene *et al.*, *Protective Groups in Organic Synthesis*, Third Edition, Wiley-Interscience, New York, 1999.

The removal of a protecting group of carboxyl, hydroxy, amino or thiol is known *per se*. For example, it is

(1) the alkaline hydrolysis,
(2) the removal of a protecting group in an acidic condition,
(3) the removal of a protecting group by hydrogenolysis,
(4) the removal of a protecting group containing silyl,
(5) the removal of a protecting group using metal or
(6) the removal of a protecting group using metal complex *etc.*

The concrete descriptions of these methods are as follows:

(1) The removal of a protecting group by alkaline hydrolysis condition may be carried out, for example, in an organic solvent (methanol, tetrahydrofuran or dioxane *etc.*) with hydroxide of alkaline metal (sodium hydroxide, potassium hydroxide or lithium hydroxide *etc.*), hydroxide of alkaline earth metal (barium hydroxide or calcium hydroxide *etc.*) or carbonate (sodium carbonate or potassium carbonate *etc.*), or an aqueous solution thereof or a mixture thereof at 0-100°C.

(2) The removal of a protecting group in an acidic condition may be carried out, for example, in an organic solvent (dichloromethane, chloroform, dioxane, ethyl acetate or anisole *etc.*), organic acid (acetic acid, trifluoroacetic acid, methanesulfonic acid or p-toluenesulfonic acid *etc.*) or inorganic acid (hydrochloric acid or sulfuric acid *etc.*), or a mixture thereof (hydrogen bromide/acetic acid *etc.*) at 0-100°C.

(3) The removal of a protecting group by hydrogenolysis may be carried out, for example, in a solvent (ether (tetrahydrofuran, dioxane, dimethoxyethane or diethylether *etc.*), alcohol (methanol or ethanol *etc.*), benzene (benzene or toluene *etc.*), ketone (acetone or methylethylketone *etc.*), nitrile (acetonitrile *etc.*), amide (dimethylformamide *etc.*), water, ethyl acetate, acetic acid, or a mixture thereof *etc.*) in the presence of a catalyst (palladium on carbon, palladium black, palladium hydroxide, platinum oxide, or Raney nickel *etc.*), at an atmospheric or positive pressure under atmosphere of hydrogen or in the presence of ammonium formate at 0-200°C.

(4) The removal of a protecting group containing silyl may be carried out, for example, in an organic solvent which can be mixed with water (tetrahydrofuran or acetonitrile *etc.*), with tetrabutylammoniumfluoride at 0-40°C.

(5) The removal of a protecting group using metal may be carried out, for example, in an acidic solvent (acetic acid or buffer solution of pH 4.2-7.2, or mixture of the above solution and an organic solvent such as tetrahydrofuran *etc.*), in the presence of zinc powder, if necessary, with sonication at 0-40°C.

(6) The removal of a protecting group using metal complex may be carried out, for example, in an organic solvent (dichloromethane, dimethylformamide, tetrahydrofuran, ethyl acetate, acetonitrile, dioxane or ethanol *etc.*) or water, or mixture thereof in the presence of a trap reagent (tributyltin hydride, triethylsilane, dimedone, morpholine, diethylamine or pyrrolidine *etc.*), and an organic acid (acetic acid, formic acid, 2-ethylhexanoic acid *etc.*) and/or an organic acid salt (sodium 2-ethylhexanoate or potassium 2-ethylhexanoate *etc.*) in the presence or absence of phosphine reagent (triphenylphosphine *etc.*) with metal complex (tetrakis(triphenylphosphine)palladium(0), bis (triphenylphosphine)palladium(II) dichloride, palladium(II) acetate, tris(triphenylphosphine)chlororhodium(I) *etc.*) at 0-40°C.

Furthermore, as methods other than the above methods, the removal of a protecting group may be prepared by, for example, the methods described in T. W. Greene, *Protective Groups in Organic Synthesis*, Wiley, New York, 1999.

As well known to the person in the art, the aimed compounds of the present invention may be prepared easily by choice of these methods.

Among the compounds of formula (I), the compounds where at least one nitrogen represents quaternary ammonium salt, i.e., the compounds of formula (I-2)

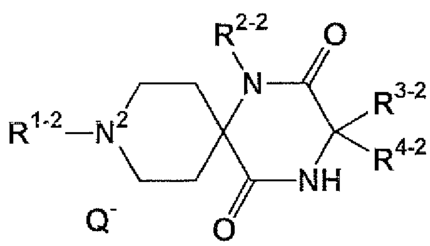

**(I-2)**

(wherein $R^{1-2}$, $R^{2-2}$, $R^{3-2}$ and $R^{4-2}$ have the same meanings as $R^1$, $R^2$, $R^3$ and $R^4$ respectively and $N^2$ is nitrogen atom, and wherein at least one nitrogen represents quaternary ammonium salt, and wherein Q is halogen.)
may be prepared by reacting the compound of formula (I-1) with the compounds of formula (VII)

$$\mathbf{R^0—Q} \qquad \text{(VII)}$$

(wherein R° is C1-4 alkyl substituted with C1-4 alkyl or phenyl and Q is halogen.).

The reaction is known *per se* and can be carried out, for example, in an organic solvent (acetone, dimethylformamide or methyl ethyl ketone *etc.*) at 0-40°C.

Among the compounds of formula (I), the compounds where at least one nitrogen represents N-oxide, i.e., the compounds of formula (I-3)

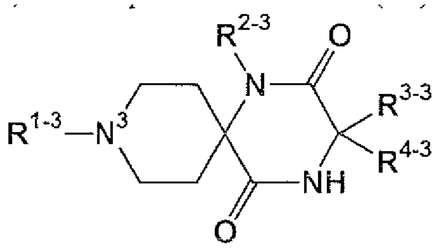

(I-3)

(wherein $R^{1-3}$, $R^{2-3}$, $R^{3-3}$ and $R^{4-3}$ have the same meanings as $R^1$, $R^2$, $R^3$ and $R^4$ respectively and $N^3$ is nitrogen atom, and wherein at least one nitrogen represents N-oxide.)
may be prepared by the oxidation of the compounds of formula (I-1).

The oxidation is known *per se* and can be carried out, for example, in a suitable organic solvent (dichloromethane, chloroform, benzene, hexane or t-butylalcohol *etc.*) in the presence of an excessive oxidizing reagent (hydrogen peroxide, sodium periodate, acyl nitrite, sodium perborate, peroxidized acid (for example, 3-chloroperbenzoic acid or peracetic acid *etc.*), OXONE (brand name, OXONE is an abbreviation for potassium peroxymonosulfate.), potassium permanganate or chromic acid *etc.*) at 20-60°C.

The compounds of formula (II) and formula (IV) may be prepared by the method according to the following Reaction Scheme 1.

Reaction Scheme (1)

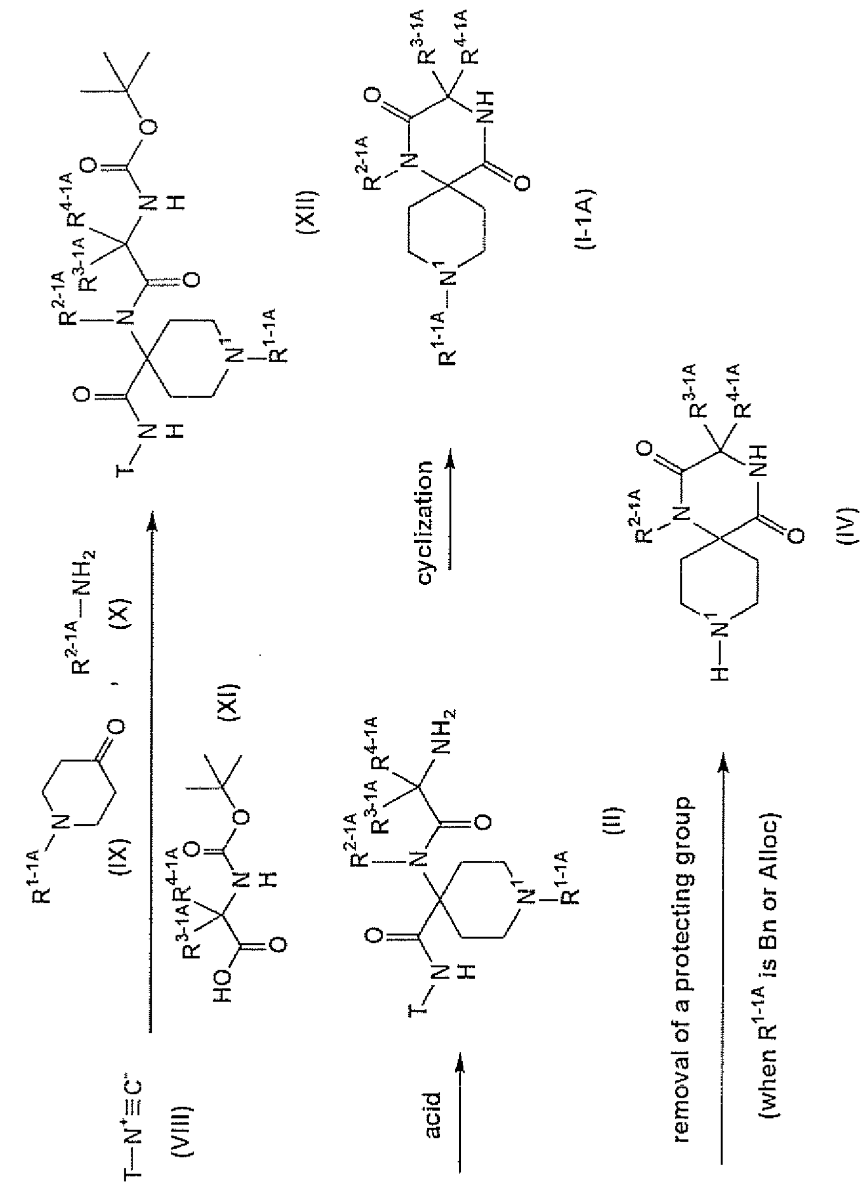


(VIII) (IX) (X) (XI) (XII) (II) (I-1A) (IV)

In above Reaction Scheme, each reaction may be carried out by known methods. Moreover in above Reaction Scheme, the starting materials of the compounds of formula (VIII), formula (IX), formula (X) and formula (XI) may be known *per se* or may be prepared by known methods.

In each reaction of the specification, the reactions with heating, as will be apparent to those skilled in the art, it may be carried with water bath, oil bath, sand bath or microwave.

In each reaction of the specification, it may be used a solid phase reagent which is supported by polymer (for example, polystyrene, polyacrylamide, polypropylene or polyethyleneglycol *etc.*).

In each reaction of the specification, the obtained products may be purified by conventional techniques. For example, the purification may be carried out by distillation at atmospheric or reduced pressure, by high performance liquid chromatography with silica gel or magnesium silicate, by thin layer chromatography, by ion-exchange resin, by scavenger resin, by column chromatography, by washing or by recrystallization. The purification may be done each reaction or after several reactions.

The other starting materials and each reagent in the present invention may be known *per se* or may be prepared by known methods.

Effect of the invention

Efficacy of the compounds of the invention of formula (I) was confirmed, e.g., by the following tests. However, the present invention is not limited thereto.

Test methods:

<Pharmaceutical activity of the compounds of the present invention>

(1) Isolation of human CCR5 gene

Human placental cDNA was prepared using Marathon cDNA amplification kit (Clontech). PCR primers hCCR5Xbal-F1:

5'-AGCTAGTCTAGATCCGTTCCCCTACAAGAAACTCTCC-3' (SEQ ID NO:1)

and hCCR5Xbal-R1:

5'-AGCTAGTCTAGAGTGCACAACTCTGACTGGGTCACCA-3' (SEQ ID NO:2)

were designed based on the sequence of GenBank U54994.

Using the human placental cDNA as the template and using Ex Taq (Takara), PCR reaction (2 minutes at 95°C → (30 seconds at 95°C, 45 seconds at 60°C, 1 minute at 72°C) × 35 times) was carried out. The thus amplified PCR product was subjected to a 1% agarose gel electrophoresis, purified using QIAquick Gel Extraction Kit (QUIAGEN) and then digested with a restriction enzyme *Xba*I. The digested fragments were ligated to an expression vector pEF-BOS-bsr using DNA Ligation Kit Ver. 2 (Takara) and transformed into *Escherichia coli* DH5a. By preparing the resulting plasmid pEF-BOS-bsr/hCCR5, its DNA sequence was verified.

(2) Culturing of CHO cell

CHO-dhfr(-) was cultured using Ham's F-12 (containing fetal bovine serum (10%), penicillin (50 U/ml) and streptomycin (50 mg/ml)). Also, the transduced cell was cultured by adding blasticidin (5 mg/ml) to the above medium.

(3) Transduction into CHO cell

The plasmid pEF-BOS-bsr/hCCR5 was transduced into the CHO-dhfr(-) cell using DMRIE-C reagent (Gibco BRL). After 48 hours, the medium was replaced with a medium containing 5 mg/ml of blasticidin to carry out the selection, thereby establishing a stably over-expressing cell.

(4) Inhibition test on the binding of RANTES to CCR5 (activity of RANTES to induce transient increase of Ca ion).

The thus established human CCR5 stably over-expressing CHO cell (CCR5/CHO cell) was suspended in Ham's F-12 medium containing FBS (10%) and dispensed in $3.0 \times 10^6$ cells/well portions into a 96 well plate. One day after culturing at 37°C, the culture supernatant was discarded, and Ham's F-12 medium (containing Fura-2AM (5 μM), Probenecid (2.5 mM) and HEPES (20 mM; pH 7.4)) was dispensed in 80 μl/well portions to carry out 1 hour of incubation at 37°C under shaded condition. After washing twice with 1× Hanks/HEPES (20 mM; pH 7.4) solution, the same solution was dispensed in 100 μl/well portions. Each of the test compounds was added to the thus Fura-2AM-incorporated CCR5/CHO cell, and 3 minutes thereafter, a recombinant human RANTES (PeproTach) diluted with 1× Hanks/HEPES (20 mM; pH 7.4) solution was added thereto to a final concentration of 10 nM. Transient increase in the intracellular $Ca^{2+}$ concentration induced by the human RANTES was measured using a $Ca^{2+}$ detector for 96 well use (Hamamatsu Photonics), and inhibition ratio (%) of the test compound was calculated by the following calculation formula.

$$\text{Inhibition ratio} = (Ec - Ea)/Ec \times 100$$

Ec: measured value of $Ca^{2+}$ transient increase by RANTES
Ea: measured value of $Ca^{2+}$ transient increase by RANTES when a test compound was added.

As a result, the compounds of the present invention showed an inhibition ratio of 50% or more at 10 μM. For example, the compound of Example 3(179) showed an $IC_{50}$ value of 0.01μM.

<Pharmacodynamics of the Inventive Compound>

(5) Stability test in liver microsome

To a mixture solution of a phosphate buffer (2 g/L KCl, 2 g/L $KH_2PO_4$, 80 g/L NaCl, 11.5 g/L $Na_2HPO_4$) (50 μL), 50 mM magnesium chloride ($MgCl_2$; 63 μL), 10 mM EDTA (63 μL), 20 mg/mL liver microsome (30 μL) and distilled water (328 μL), 6 μL of the inventive compound prepared to 1 mM in 50% acetonitrile solution was added. The resulting mixture was pre-incubated at 37°C for 3 minutes. To the preliminary incubate, 60 μL of 20 mM NADPH or distilled water was added to start the reaction. While the mixture was incubated at 37°C, 100 μL of the reaction solution was taken out 0, 5, 10, 15, and 30 minutes after the start, and was then added to a solution containing acetonitrile (3 mL) and 10 μg/mL internal standard solution (nonyl paraben-50% acetonitrile solution; 50 μL) to terminate the reaction. After agitation, the reaction mixture was centrifuged at 3,000 rpm for 10 minutes. The resulting supernatant was transferred to another tube and dried with a centrifuge concentrator. To the dried product, 100 μL of 10 mM SDS (0.1% TFA-50% acetonitrile solution) was added, followed by agitation, to assay the concentration of the unmetabolite by HPLC.
The HPLC data was analyzed by automatically computerizing the peak areas for the compound and the internal standard (nonyl paraben). For correction, the peak area for the compound was divided by the peak area for the internal standard. The residual ratio of the unmetabolite was calculated at the individual times, while the residual ratio thereof at time 0 was defined as 100%. The resulting values were plotted in a graph on the axis of abscissa expressing time and the axis of ordinate expressing the logarithm of the residual ratio of the unmetabolite, for linear approximation to calculate the slope. According to the following calculation formula, the half life was calculated on the basis of the slope.

$$\text{Half life (t1/2)} = 0.693/(-2.303 \times \text{slope})$$

Compared with the compound described in the publication of International Publication No. 01/40227, the inventive compound has improved metabolic stability.

(6) Assaying plasma protein binding ratio

To 990 μL of plasma, 10 μL of a solution of the compound (a solution of the compound at 1 mg/mL in 50% acetonitrile) was added. To a solution containing acetonitrile (3 mL) and 10 μg/mL internal standard solution (nonyl paraben-50% acetonitrile solution; 50 μL), 50 μL of the resulting solution was added. The remaining plasma to which the compound had been added was ultra-centrifuged at 100,000 rpm and 10°C for 3 hours. After 50 μL of the resulting supernatant was taken out (n = 2), it was added to a solution containing acetonitrile (3 mL) and 10 μg/mL internal standard solution (nonyl paraben-50% acetonitrile solution; 50 μL). The resulting individual acetonitrile solutions were agitated and centrifuged at 3,000 rpm for 10 minutes. The resulting supernatants were transferred to other tubes and dried with a centrifuge concentrator. To the dried products, 100 μL of SDS (0.1% TFA-50% acetonitrile solution) was added, followed by agitation. The concentration of the compound was subsequently assayed by HPLC.
HPLC data were analyzed by automatically computerizing the peak areas for the compound and the internal standard (nonyl paraben). For correction, the peak area for the compound was divided by the peak area for the internal standard. According to the following calculation formula and based on the individual data, the protein binding ratio was calculated.

$$\text{Protein binding ratio (\%)}$$

$$= [1- \text{(corrected value for the compound in the supernatant)}$$

$$/\text{(corrected value for the compound before centrifugation)}] \times 100$$

According to the following calculation formula and based on the results from the experiments (5) and (6), liver

clearance and the extent of liver availability were calculated (see "Dispersion model", *J. Pharm. Pharmacol.*, 38(3), 177-81 (1986); *Biopharm. Drug Dispos.,* 17, 273-310 (1996)).

$$Fh= \frac{4a}{(1+a)^2 exp\{(a-1)/2D_N\}-(1-a)^2 exp\{-(a+1)/2D_N\}}$$

$CLh = Qh(1-Fh)$
$a = (1+4R_N \bullet D_N)^{1/2}$
$R_N = (f_u/R_B) \bullet CLint/Qh$
CLint = Ke/(mg MS protein/mL) · (mg MS protein/g liver) · (g liver/kg)
Ke = 0.693/t1/2

CLh: Liver clearance
Qh: Liver blood flow
Fh: Extent of liver availability
$D_N$: Dispersion number
$f_u$: Plasma non-binding fraction (1 - protein binding ratio)
$R_B$: Plasma to blood concentration ratio
CLint: Metabolism-intrinsic clearance

Compared with the compound described in International Publication No. 01/40227, the inventive compound has improved liver clearance and extent of liver availability.

(7) Systemic clearance and plasma concentration assay of the compound intravenously administered in rat

The inventive compound (in a solvent 30% HP-β CD/d$H_2O$) was intravenously administered to a male Crj:CD (SD) IGS rat (aged 8 to 9 weeks). About 400 µL of blood was collected from the jugular vein, using a heparinized syringe 2, 10, 30, 60, 120 and 240 minutes after the administration. The collected blood was centrifuged (12,000 rpm at 4°C for 3 minutes). The concentration of the unmetabolite in the recovered plasma was analyzed by HPLC. The clearance (CL) and the area under the curve (curve of blood concentration vs. time) [AUC (i.v.) in µg·min/mL] were estimated, using a commercially available software (Winnonln).
Compared with the compound described in International Publication No. 01/40227, the inventive compound has improved systemic clearance.

(8) Plasma concentration assay of the compound orally administered to rat

The inventive compound (in a solvent 1% DK ester) was orally administered to a male Crj:CD (SD) IGS rat (aged 8 to 9 weeks). About 400 µL of blood was collected from the jugular vein, using a heparinized syringe, 15, 30, 60, 120, 240 and 360 minutes after administration. The collected blood was centrifuged (12,000 rpm at 4°C for 3 minutes). The concentration of the unmetabolite in the recovered plasma was analyzed by HPLC. The AUC (p.o.), µg·min/mL) was estimated, using a commercially available software (Winnonln).
According to the following calculation formula and based on the results from the experiments (7) and (8), the extent of bioavailability (BA) was calculated.

$$\text{Extent of bioavailability (BA)} = [\text{AUC (p.o.)/AUC (i.v.)}] \times 100$$

When the concentration of the inventive compound administered in (7) was different from the concentration thereof in (8), their concentrations were corrected for BA calculation.
Compared with the compound described in International Publication No. 01/40227, the inventive compound has an improved extent of bioavailability.

Toxicity:

The toxicity of the compounds of the present invention is very low and therefore the compounds may be considered safe for pharmaceutical use.

INDUSTRIAL APPLICABILITY

Application to pharmaceuticals:

The compounds of the present invention of formula (I) regulate the effect of chemokine/chemokine receptor in animal included human, especially human, so they are used for prevention and/or treatment of various inflammatory diseases (asthma, nephritis, nephropathy, hepatitis, arthritis, chronic rheumatoid arthritis, rhinitis, conjunctivitis, ulcerative colitis and the like), immunologic diseases (autoimmune diseases, transplant rejection, immunosuppression, psoriasis, multiple sclerosis and the like), human immunodeficiency virus (acquired immunodeficiency syndrome and the like), allergic diseases (atopic dermatitis, nettle rash, allergic bronchopulmonary aspergillosis, allergic eosinophilic gastroenteritis and the like), ischemia-reperfusion injury, acute respiratory distress syndrome, shock accompanied by bacterial infection, diabetes mellitus, or metastasis and the like.

For the purpose above described, the compounds of formula (I), salts thereof, acid addition salts or hydrates thereof may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered for example, in the form of solid for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.

In such solid forms, one or more of the active compound(s) may be admixed with vehicles (such as lactose, mannitol, glucose, microcrystalline cellulose or starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable solutions, suspensions, emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, saline, vegetable oil, propylene glycol, polyethylene glycol, alcohol, e.g. ethanol, or a mixture thereof. Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known *per se*.

Sprays may comprise additional substances other than diluents, such as stabilizing agents, such as sodium sulfate, isotonic buffers, such as sodium chloride, sodium citrate or citric acid. For preparation of such sprays, for example, the method described in the United States Patent No. 2,868,691 or 3,095,355 may be used.

The compound of the present invention represented by formula (I), a quaternary ammonium salt thereof or an N-oxide thereof, or a salt thereof may be used together with other drugs, for example, preventive and/or treating agent(s) for HIV infection (particularly an agent for prevention and/or treatment for AIDS). In that case, the drug as such may be mixed with pharmacologically acceptable excipient, binder, disintegrating agent, lubricant, stabilizer, solubilizer, diluent, *etc*. either separately or simultaneously to make into a pharmaceutical preparation and that can be

administered either orally or parenterally as a pharmaceutical composition for prevention and/or treatment of HIV infection.

The compound of the present invention represented by formula (I), a quaternary ammonium salt thereof or an N-oxide thereof, or a salt thereof has an infection inhibiting activity to HIV-1 which acquired resistance to other agent for prevention and/or treatment for HIV infection (particularly an agent for prevention and/or treatment for AIDS). Therefore, it is also able to be used for HIV-infected patients to whom other agent for prevention and/or treatment for HIV infection is no longer effective. In that case, although the compound of the present invention may be used solely, it may be also used together with an agent for prevention and/or treatment HIV infection where infected HIV-1 strain acquired resistance or with other drugs.

The present invention covers the case where the compound represented by formula (I), a quaternary ammonium salt thereof or an N-oxide thereof, or a salt thereof is combined with a drug which does not inhibit the HIV infection whereby preventive and/or treating effect for HIV infection is enhanced as compared with a single preparation.

Examples of other agent for preventive and/or treating HIV infection used for a combination with the compound of the present invention represented by formula (I), a quaternary ammonium salt thereof or an N-oxide thereof, or a salt thereof are reverse transcriptase inhibitor, protease inhibitor, chemokine antagonist (such as CCR2 antagonist, CCR3 antagonist, CCR4 antagonist, CCR5 antagonist and CXCR4 antagonist), fusion inhibitor, antibody to surface antigen of HIV-1 and vaccine of HIV-1.

Reverse transcriptase inhibitors are concretely (1) nucleoside/nucleotide reverse transcriptase inhibitors: zidovudine (brand name: Retrovir), didanosine (brand name: Videx), zalcitabine (brand name: HIVID), stavudine (brand name: Zerit), lamivudine (brand name: Epivir), abacavir (brand name: Ziagen), adefovir, adefovir dipivoxil, emtricitabine (brand name: Coviracil) or PMPA (brand name: Tenofovir) *etc*. and (2) nonnucleoside reverse transcriptase inhibitors: nevirapine (brand name: Viramune), delavirdine (brand name: Rescriptor), efavirenz (brand name: Sustiva, Stocklin) or capravirine (AG1549) *etc*.

Protease inhibitors are concretely indinavir (brand name: Crixivan), ritonavir (brand name: Norvir), nelfinavir (brand name: Viracept), saquinavir (brand name: Invirase, Fortovase), amprenavir (brand name: Agenerase), lopinavir (brand name: Kaletra) or tipranavir *etc*.

As chemokine antagonists, internal ligand of chemokine receptor, its derivatives, non-peptide low molecular compound or antibody of chemokine receptor are included.

The examples of internal ligand of chemokine receptor are concretely, MIP-1$\alpha$, MIP-1$\beta$, RANTES, SDF-1$\alpha$, SDF-1$\beta$, MCP-1, MCP-2, MCP-4, Eotaxin and MDC *etc*.

The derivatives of internal ligand are concretely, AOP-RANTES, Met-SDF-1$\alpha$, Met- SDF-1$\beta$ *etc*.

Antibodies of chemokine receptor are concretely, Pro-140 *etc*.

CCR2 antagonists are concretely written in specification of WO99/07351, WO99/40913, WO00/46195, WO00/46196, WO00/46197, WO00/46198, WO00/46199, WO00/69432 or WO00/69815 or in *Bioorg. Med. Chem. Lett*., 10, 1803 (2000) *etc.*

CCR3 antagonists are concretely written in specification of DE19837386, WO99/55324, WO99/55330, WO00/04003, WO00/27800, WO00/27835, WO00/27843, WO00/29377, WO00/31032, WO00/31033, WO00/34278, WO00/35449, WO00/35451, WO00/35452, WO00/35453, WO00/35454, WO00/35876, WO00/35877, WO00/41685, WO00/51607, WO00/51608, WO00/51609, WO00/51610, WO00/53172, WO00/53600, WO00/58305, WO00/59497, WO00/59498, WO00/59502, WO00/59503, WO00/62814, WO00/73327 or WO01/09088 *etc*.

CCR5 antagonists are concretely written in specification of WO99/17773, WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00/66558, WO00/66559, WO00/66141, WO00/68203, JP2000309598, WO00/51607, WO00/51608, WO00/51609, WO00/51610, WO00/56729, WO00/59497, WO00/59498, WO00/59502, WO00/59503, WO00/76933, WO98/25605 or WO99/04794, WO99/38514 or in *Bioorg. Med. Chem. Lett*., 10, 1803 (2000) *etc*.

CXCR4 antagonists are concretely AMD-3100, T-22, KRH-1120 or the compounds written in specification of WO00/66112 *etc*.

Fusion Inhibitors are concretely, T-20(Pentafuside) and T-1249 *etc*.

The examples of combination agents written above are intended to illustrate the present invention, but do not limit them.

The typical examples of the usual the dosage level in clinical trials of reverse transcriptase inhibitors or protease inhibitors written below are intended to illustrate the present invention, but do not limit them.

| | |
|---|---|
| zidovudine | 100 mg capsule, 200 mg per dose, 3 times per day; 300 mg tablet, 300 mg per dose, twice per day; |
| didanosine | 25-200 mg tablet, 125-200 mg per dose, twice per day; |
| zalcitabine | 0.375-0.75 mg tablet, 0.75 mg per dose, 3 times per day; |

(continued)

| | |
|---|---|
| stavudine | 15-40 mg capsule, 30-40 mg per dose, twice per day; |
| lamivudine | 150 mg tablet, 150 mg per dose, twice per day; |
| abacavir | 300 mg tablet, 300 mg per dose, twice per day; |
| nevirapine | 200 mg tablet, 200 mg per dose, once per day for 14 days and then twice per day; |
| delavirdine | 100 mg tablet, 400 mg per dose, 3 times per day; |
| efavirenz | 50-200 mg capsule, 600 mg per dose, once per day; |
| indinavir | 200-400 mg capsule, 800 mg per dose, 3 times per day; |
| ritonavir | 100 mg capsule, 600 mg per dose, twice per day; |
| nelfinavir | 250 mg tablet, 750 mg per dose, 3 times per day; |
| saquinavir | 200 mg capsule, 100 or 200 mg per dose, 3 times per day; |
| amprenavir | 50-150 mg tablet, 100 or 200 mg per dose, twice per day. |

## BEST MODE FOR CARRYING OUT THE INVENTION

The following Reference Examples and Examples are intended to illustrate the present invention, but do not limit them.
In chromatographic separations and TLC, the solvents in parenthesis show the eluting and developing solvents and the ratios of the solvents used are by volume.
Unless otherwise specified, NMR data are H-NMR data.
The solvents in parenthesis in NMR show the solvents used for measurement.
Name of the compounds were named according to IUPAC nomenclature system.

### Reference Example 1

1-benzyl-4-(2-(morpholin-4-yl)ethylaminocarbonyl)-4-(N-butyl-N-((2R,3R)-2-amino-3-hydroxy-3-cyclohexylpropanoyl)amino)piperidine

To a solution of benzylpiperidone (49.4 g) in methanol (1L) was added (2R,3R)-2-(t-butoxycarbonylamino)-3-cyclohexyl-3-hydroxypropanoic acid (75 g) and n-butylamine (258 mL). The mixture was stirred at room temperature for few minutes and then 2-(morpholin-4-yl)ethylisocyanide (36 mL) was added thereto. The reaction mixture was stirred at 50°C overnight. To the reaction mixture was added concentrated hydrochloric acid (261 mL) at room temperature. Further, the reaction mixture was heated to 50°C and stirred. The reaction mixture was concentrated. To the residue were added water (500 mL), ethyl acetate (1 L), and sodium carbonate, and extracted. Further, water layer was extracted with ethyl acetate (2 L). All of the organic layer was washed with brine, dried over sodium sulfate and concentrated to give the title compound (151g: include residual solvent) having the following physical data. The obtained residue was used in the next reaction without further purification.
TLC: Rf 0.39 (chloroform:methanol=10:1).

Example 7

(3R)-1-butyl-2,5-dioxo-3-((1R)-1-hydroxy-1-cyclohexylmethyl)-9-phenylmethyl-1,4,9-triazaspiro[5.5]undecane

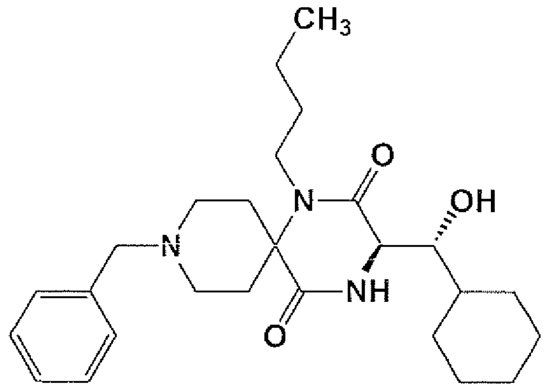

To a solution of the compound prepared in Reference Example 1 (151 g) in toluene (1 L) was added acetic acid (71.6 mL). The reaction mixture was stirred with heating for 1 hour at 70°C. After cooling to room temperature, the reaction mixture was diluted with ethyl acetate (1.5 L) and washed with water (200 mL) twice. The organic layer was neutralized with saturated aqueous solution of sodium bicarbonate (800 mL), washed with brine, dried over anhydrous magnesium sulfate and concentrated to give the compound of the present invention (103 g) having the following physical data. Further, the compound was converted to the corresponding acid addition salt by using a conventional method.
Free form :
TLC: Rf 0.34 (chloroform:methanol=10:1);
NMR($CD_3OD$): δ 7.51-7.24, 4.13, 3.88, 3.60-2.95, 2.42-2.11, 2.10-1.56, 1.54-1.08, 1.08-0.79.
hydrochloride salt :
TLC: Rf 0.76 (ethyl acetate:methanol=4:1);
NMR($CD_3OD$): δ 7.60-7.47, 4.35, 4.15, 3.99, 3.74, 3.55-3.42, 3.33-3.20, 2.54-2.35, 2.12-1.90, 1.80-1.65, 1.44-1.15, 1.01-0.87. Methanesulfonate :
TLC: Rf 0.42 (chloroform:methanol=10:1);
NMR($CD_3OD$): δ 7.51-7.22, 4.36, 4.14, 4.00, 3.76, 3.59-3.38, 3.30-3.03, 2.70, 2.54-1.83, 1.82-1.56, 1.53-1.08, 1.06-0.77.

Reference Example 2

(3R)-1-butyl-2,5-dioxo-3-((1R)-1-hydroxy-1-cyclohexylmethyl)-1,4,9-triazaspiro[5.5]undecane hydrochloride

To 20% palladium hydroxide on carbon (20 g, wet) was added a solution of the compound prepared in Example 1 (102.5 g) in ethanol (1 L). The reaction mixture was stirred at 50°C for 3 hours under an atmosphere of hydrogen. The reaction mixture was filtrated through CELITE (brand name). To the filtrate was added 4N hydrogen chloride /ethyl acetate solution on ice bath. The reaction mixture was concentrated to give the title compound (83 g) having the following physical data.
TLC: Rf 0.32 (butanol:acetic acid:water=4:2:1);
NMR($CD_3OD$): δ 4.16, 3.95, 3.70, 3.52, 3.37, 3.28, 3.22-3.13, 2.46-1.93, 1.80-1.64, 1.48-1.15, 1.02-0.87.
specific optical rotation:$[\alpha]_D$ -37.5 (c 1.04, methanol, 18°C).

Example 2

(3R)-1-butyl-2,5-dioxo-3-((1R)-1-hydroxy-1-cyclohexylmethyl)-9-(4-methylsulfonylaminophenylmethyl)-1,4,9-triazaspiro[5.5]undecane hydrochloride

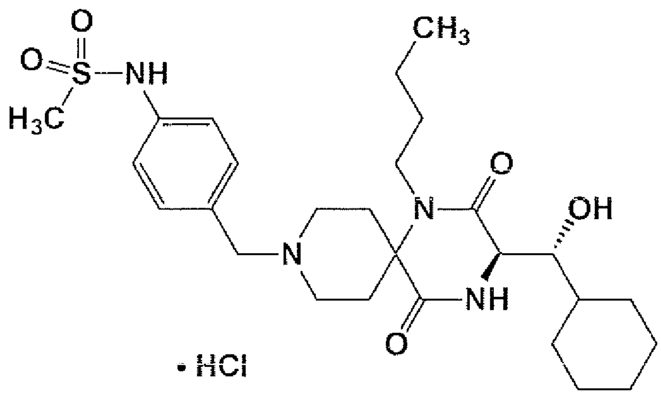

To a solution of the compound prepared in Reference Example 2 (100 mg) in dimethyl formamide (3 mL) was added acetic acid (16 μL) (or triethylamine (40 μL)). To the reaction mixture was added N-(4-formylphenyl)methanesulfonamide (56 mg) and sodium triacetoxyborohydride (82 mg). The reaction mixture was stirred at room temperature overnight. The reaction mixture was concentrated, the obtained residue was purified by column chromatography on silica gel (ethyl acetate : methanol = 20 : 1-10 : 1) and 4N hydrogen chloride / ethyl acetate solution was added thereto. The reaction mixture was concentrated to give the compound of the present invention (72 mg) having the following physical data.
TLC: Rf 0.67 (dichloromethane:methanol=5:1);
NMR($CD_3OD$): δ 7.53, 7.34, 4.32, 4.15, 3.99, 3.74, 3.54-3.42, 3.33-3.16, 3.01, 2.49-2.24, 2.14- 1.91, 1.80-1.60, 1.40-1.15, 1.00-0.87.

Example 2(1) - Example 2(165)

By the same procedure as described in Example 2 using the corresponding aldehyde derivatives respectively instead of N-(4-formylphenyl)methanesulfonamide, the following compounds of the present invention were obtained.

Example 2(1)

(3R)-1-butyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride TLC: Rf 0.43 (chloroform:methanol=5:1);

NMR($CD_3OD$): δ 8.05, 7.61, 7.19, 7.08, 4.38, 4.17, 4.02, 3.78, 3.60-3.40, 3.30-3.10, 2.56-1.86, 1.82-1.60, 1.52-1.16, 1.06-0.82, 0.97.

Example 2(2)

(3R)-1-butyl-2,5-dioxo-3-((1R)-1-hydroxy-1-cyclohexylmethyl)-9-(5-(4-methylaminocarbonylphenoxy)pyridin-2-ylmethyl)-1,4,9-triazaspiro[5.5]undecane hydrochloride

TLC: Rf 0.51 (chloroform:methanol=9:1);
NMR($CD_3OD$): δ 8.51, 7.89, 7.66, 7.60, 7.16, 4.52, 4.16, 4.11, 3.85, 3.61-3.50, 3.33-3.20, 2.92, 2.62-2.35, 2.16-1.91, 1.78-1.65, 1.45-1.15, 1.05-0.86, 0.97.

Example 2(3)

(3R)-1-butyl-2,5-dioxo-3-((1R)-1-hydroxy-1-cyclohexylmethyl)-9-(5-(4-methylaminocarbonylphenoxy-1-oxido)pyridin-2-ylmethyl)-1,4,9-triazaspiro[5.5]undecane hydrochloride

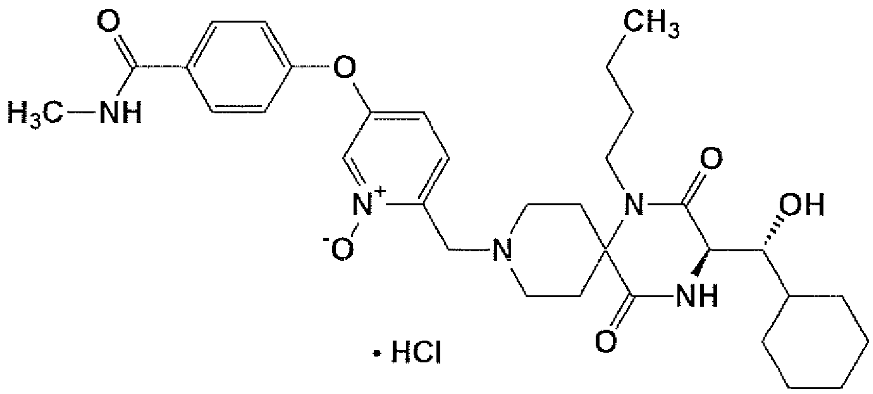

TLC: Rf 0.45 (chloroform:methanol=9:1);
NMR($CD_3OD$): δ 8.31, 7.93, 7.75, 7.25, 7.24, 4.58, 4.16, 4.13, 3.87, 3.52-3.48, 3.30-3.19, 2.92, 2.51-2.30, 2.16-1.92, 1.80-1.62, 1.45-1.18, 1.05-0.88, 0.96.

Example 2(4)

(3R)-1-butyl-2,5-dioxo-3-((1R)-1-hydroxy-1-cyclohexylmethyl)-9-(2,6-dimethyl-4-(4-methylaminocarbonylphenoxy) phenylmethyl)-1,4,9-triazaspiro[5.5]undecane hydrochloride

TLC: Rf 0.42 (chloroform:methanol=10:1);
NMR($CD_3OD$): δ 7.83, 7.04, 6.87, 4.49, 4.20, 4.17, 3.96, 3.62-3.42, 3.38-3.10, 2.91, 2.62-1.58, 2.47, 1.54-0.80, 0.95.

Example 2(5)

(3R)-1-butyl-2,5-dioxo-3-((1R)-1-hydroxy-1-cyclohexylmethyl)-9-(5-(4-methoxyphenylmethylaminocarbonyl)pyridin-2-ylmethyl)-1,4,9-triazaspiro[5.5]undecane hydrochloride

TLC: Rf 0.43 (chloroform:methanol=9:1);
NMR($CD_3OD$): δ 9.12, 8.33, 7.70, 7.29, 6.88, 4.61, 4.52, 4.16, 4.10, 3.85, 3.76, 3.61-3.50, 3.30-3.26, 2.62-2.42, 2.13, 2.04-1.92, 1.81-1.61, 1.50-1.15, 1.00-0.86, 0.97.

Example 2(6)

(3R)-1-butyl-2,5-dioxo-3-((1R)-1-hydroxy-1-cyclohexylmethyl)-9-(2-methylpyridin-5-ylmethyl)-1,4,9-triazaspiro[5.5] undecane dihydrochloride

TLC: Rf 0.50 (chloroform:methanol=10:1);
NMR($CD_3OD$): δ 9.09, 8.76, 8.01, 4.58, 4.15, 4.07, 3.82, 3.62-3.48, 3.36, 3.28, 2.83, 2.75-2.42, 2.13-1.91, 1.80-1.63, 1.45-1.15, 1.00-0.87.

Example 2(7)

(3R)-1-butyl-2,5-dioxo-3-((1R)-1-hydroxy-1-cyclohexylmethyl)-9-(2-chloropyridin-5-ylmethyl)-1,4,9-triazaspiro[5.5] undecane dihydrochloride

TLC: Rf 0.53 (chloroform:methanol=10:1);
NMR($CD_3OD$): δ 8.58, 8.10, 7.59, 4.43, 4.15, 4.01, 3.77, 3.56-3.45, 3.33-3.19, 2.59-2.35, 2.14-1.92, 1.80-1.62,

1.45-1.15, 1.00-0.87.

Example 2(8)

(3R)-1-butyl-2,5-dioxo-3-((1R)-1-hydroxy-1-cyclohexylmethyl)-9-(2-aminopyridin-5-ylmethyl)-1,4,9-triazaspiro[5.5] undecane dihydrochloride

TLC: Rf 0.48 (chloroform:methanol=4:1);
NMR($CD_3OD$): δ 8.17-8.14, 7.07, 4.30, 4.15, 3.97, 3.74, 3.60-3.45, 3.37-3.26, 2.70-2.40, 2.13-1.91, 1.80-1.60, 1.45-1.10, 1.02-0.87, 0.95.

Example 2(9)

(3R)-1-butyl-2,5-dioxo-3-((1R)-1-hydroxy-1-cyclohexylmethyl)-9-(4-methoxyphenylmethyl)-1,4,9-triazaspiro[5.5] undecane hydrochloride

TLC: Rf 0.44 (chloroform:methanol=10:1);
NMR($CD_3OD$): δ 7.47, 7.02, 4.28, 4.15, 3.96, 3.82, 3.70, 3.58-3.36, 3.30-3.10, 2.56-2.20, 2.18-1.86, 1.82-1.56, 1.50-1.06, 1.04-0.80, 0.95.

Example 2(10)

(3R)-1-butyl-2,5-dioxo-3-((1R)-1-hydroxy-1-cyclohexylmethyl)-9-(4-methylphenylmethyl)-1,4,9-triazaspiro[5.5] undecane hydrochloride

TLC: Rf 0.47 (chloroform:methanol=10:1);
NMR($CD_3OD$): δ 7.42, 7.31, 4.30, 4.14, 3.98, 3.70, 3.60-3.08, 2.56-2.20, 2.37, 2.18-1.84, 1.82-1.58, 1,54-1.06, 1.04-0.80, 0.95.

Example 2(11)

(3R)-1-butyl-2,5-dioxo-3-((1R)-1-hydroxy-1-cyclohexylmethyl)-9-(4-hydroxyphenylmethyl)-1,4,9-triazaspiro[5.5] undecane hydrochloride

TLC: Rf 0.49 (chloroform:methanol=5:1);
NMR($CD_3OD$): δ 7.35, 6.87, 4.24, 4.15, 3.96, 3.68, 3.58-3.36, 3.30-3.06, 2.54-1.82, 1.80-1.58, 1.48-1.06, 1.04-0.80, 0.95.

Example 2(12)

(3R)-1-butyl-2,5-dioxo-3-((1R)-1-hydroxy-1-cyclohexylmethyl)-9-(4-carboxyphenylmethyl)-1,4,9-triazaspiro[5.5] undecane hydrochloride

TLC: Rf 0.53 (chloroform:methanol=5:1);
NMR($CD_3OD$): δ 8.13, 7.67, 4.44, 4.15, 4.02, 3.78, 3.62-3.40, 3.38-3.02, 2.56-1.84, 1.82-1.56, 1.54-1.06, 1.04-0.80, 0.95.

Example 2(13)

(3R)-1-butyl-2,5-dioxo-3-((1R)-1-hydroxy-1-cyclohexylmethyl)-9-(4-(N,N-dimethylamino)phenylmethyl)-1,4,9-triazaspiro[5.5]undecane dihydrochloride

TLC: Rf 0.41 (chloroform:methanol=10:1);
NMR($CD_3OD$): δ 7.81, 7.63, 4.40, 4.15, 4.00, 3.76, 3.60-3.38, 3.36-3.18, 3.27, 2.70-2.38, 2.16-1.84, 1.82-1.56, 1.50-1.06, 1.04-0.80, 0.94.

Example 2(14)

(3R)-1-butyl-2,5-dioxo-3-((1R)-1-hydroxy-1-cyclohexylmethyl)-9-(4-(N,N-dimethylaminosulfonyl)phenylmethyl)-1,4,9-triazaspiro[5.5]undecane hydrochloride

TLC: Rf 0.45 (chloroform:methanol=10:1);
NMR($CD_3OD$): δ 7.91-7.84, 4.47, 4.15, 4.02, 3.78, 3.58-3.40, 3.30-3.18, 2.71, 2.64-2.36, 2.18-1.84, 1.82-1.56, 1.52-1.06, 1.04-0.80, 0.95.

Example 2(15)

(3R)-1-butyl-2,5-dioxo-3-((1R)-1-hydroxy-1-cyclohexylmethyl)-9-(4-(2-hydroxyethoxy)phenylmethyl)-1,4,9-triazaspiro[5.5]undecane hydrochloride

TLC: Rf 0.31 (chloroform:methanol=10:1);
NMR($CD_3OD$): δ 7.47, 7.05, 4.28, 4.14, 4.08, 3.96, 3.87, 3.70, 3.58-3.36, 3.30-3.08, 2.58-2.20, 2.18-1.84, 1.82-1.58, 1.54-1.06, 1.04-0.80, 0.95.

Example 2(16)

(3R)-1-butyl-2,5-dioxo-3-((1R)-1-hydroxy-1-cyclohexylmethyl)-9-(pyridin-3-ylmethyl)-1,4,9-triazaspiro[5.5]undecane dihydrochloride

TLC: Rf 0.35 (chloroform:methanol=10:1);
NMR($CD_3OD$): δ 9.24, 8.97-8.89, 8.16, 4.63, 4.15, 4.04, 3.84, 3.66-3.18, 2.84-2.38, 2.20-1.84, 1.82-1.56, 1.52-1.06, 1.04-0.80, 0.94.

Example 2(17)

(3R)-1-butyl-2,5-dioxo-3-((1R)-1-hydroxy-1-cyclohexylmethyl)-9-(pyridin-2-ylmethyl)-1,4,9-triazaspiro[5.5]undecane dihydrochloride

TLC: Rf 0.34 (chloroform:methanol=10:1);
NMR($CD_3OD$): δ 8.81, 8.20, 7.84, 7.72, 4.62, 4.16, 4.14, 3.86, 3.66-3.42, 3.40-3.20, 2.72-2.26, 2.20-1.86, 1.84-1.58, 1.56-1.06, 1.04-0.80, 0.96.

Example 2(18)

(3R)-1-butyl-2,5-dioxo-3-((1R)-1-hydroxy-1-cyclohexylmethyl)-9-(4-chlorophenylmethyl)-1,4,9-triazaspiro[5.5]undecane hydrochloride

TLC: Rf 0.44 (ethyl acetate:methanol=20:1);
NMR($CD_3OD$): δ 7.58, 7.50, 4.35, 4.14, 3.98, 3.74, 3.58-3.36, 3.30-3.12, 2.58-2.22, 2.20-1.84, 1.82-1.56, 1.52-1.06, 1.04-0.78, 0.95.

Example 2(19)

(3R)-1-butyl-2,5-dioxo-3-((1R)-1-hydroxy-1-cyclohexylmethyl)-9-(4-cyanophenylmethyl)-1,4,9-triazaspiro[5.5]undecane hydrochloride

TLC: Rf 0.43 (ethyl acetate:methanol=20:1);
NMR($CD_3OD$): δ 7.87, 7.79, 4.45, 4.15, 4.02, 3.78, 3.60-3.36, 3.30-3.16, 2.60-2.24, 2.18-1.82, 1.80-1.56, 1.54-1.06, 1.04-0.80, 0.95.

Example 2(20)

(3R)-1-butyl-2,5-dioxo-3-((1R)-1-hydroxy-1-cyclohexylmethyl)-9-cyclohexylmethyl-1,4,9-triazaspiro[5.5]undecane hydrochloride

TLC: Rf 0.28 (ethyl acetate:methanol=10:1);
NMR($CD_3OD$): δ 4.16, 3.96, 3.78-3.40, 3.38-3.20, 3.00, 2.64-2.26, 2.20-1.60, 1.58-0.80, 0.97.

Example 2(21)

(3R)-1-butyl-2,5-dioxo-3-((1R)-1-hydroxy-1-cyclohexylmethyl)-9-(tetrahydropyran-4-ylmethyl)-1,4,9-triazaspiro[5.5] undecane hydrochloride

TLC: Rf 0.38 (chloroform:methanol=10:1);
NMR($CD_3OD$): δ 4.16, 4.06-3.86, 3.80-3.40, 3.38-3.16, 3.06, 2.66-2.32, 2.28-1.58, 1.56-1.06, 1.04-0.80, 0.97.

Example 2(22)

(3R)-1-butyl-2,5-dioxo-3-((1R)-1-hydroxy-1-cyclohexylmethyl)-9-(pyridin-4-ylmethyl)-1,4,9-triazaspiro[5.5]undecane dihydrochloride

TLC: Rf 0.43 (chloroform:methanol=10:1);
NMR($CD_3OD$): δ 8.98, 8.44, 4.70, 4.15, 4.10, 3.84, 3.66-3.16, 2.84-2.36, 2.20-1.84, 1.82-1.56, 1.54-1.06, 1.04-0.80, 0.95.

Example 2(23)

(3R)-1-butyl-2,5-dioxo-3-((1R)-1-hydroxy-1-cyclohexylmethyl)-9-(2-methoxypyridin-5-ylmethyl)-1,4,9-triazaspiro[5.5] undecane dihydrochloride

TLC: Rf 0.52 (ethyl acetate:methanol=10:1);
NMR($CD_3OD$): δ 8.50, 8.34, 7.06, 4.41, 4.15, 4.11, 4.00, 3.78, 3.60-3.40, 3.38-3.16, 2.66-2.40, 2.18-1.84, 1.82-1.60, 1.52-1.04, 1.04-0.80, 0.94.

Example 2(24)

(3R)-1-butyl-2,5-dioxo-3-((1R)-1-hydroxy-1-cyclohexylmethyl)-9-(2-methylcarbonylaminopyridin-5-ylmethyl)-1,4,9-triazaspiro[5.5]undecane dihydrochloride

TLC: Rf 0.46 (chloroform:methanol=10:1);
NMR($CD_3OD$): δ 8.60, 8.48, 7.73, 4.47, 4.15, 4.02, 3.76, 3.64-3.40, 3.38-3.18, 2.66-2.38, 2.30, 2.18-1.84, 1.82-1.56, 1.50-1.06, 1.04-0.80, 0.94.

Example 2(25)

(3R)-1-butyl-2,5-dioxo-3-((1R)-1-hydroxy-1-cyclohexylmethyl)-9-(2-methylsulfonylaminopyridin-5-ylmethyl)-1,4,9-triazaspiro[5.5]undecane dihydrochloride

TLC: Rf 0.35 (chloroform:methanol=10:1);
NMR($CD_3OD$): δ 8.42, 7.99, 7.12, 4.36, 4.15, 4.00, 3.76, 3.60-3.40, 3.38-3.14, 3.30, 2.60-2.24, 2.22-1.84, 1.82-1.58, 1.54-1.06, 1.04-0.80, 0.95.

Example 2(26)

(3R)-1-butyl-2,5-dioxo-3-((1R)-1-hydroxy-1-cyclohexylmethyl)-9-(4-methylaminocarbonylphenylmethyl)-1,4,9-triazaspiro[5.5]undecane hydrochloride

TLC: Rf 0.37 (chloroform:methanol=10:1);

NMR($CD_3OD$): δ 7.92, 7.68, 4.42, 4.15, 4.02, 3.76, 3.58-3.38, 3.32-3.10, 2.92, 2.58-2.24, 2.18-1.84, 1.82-1.56, 1.50-1.06, 1.04-0.80, 0.95.

Example 2(27)

(3R)-1-butyl-2,5-dioxo-3-((1R)-1-hydroxy-1-cyclohexylmethyl)-9-(2-methyl-1-oxidopyridin-5-ylmethyl)-1,4,9-triazaspiro[5.5]undecane hydrochloride

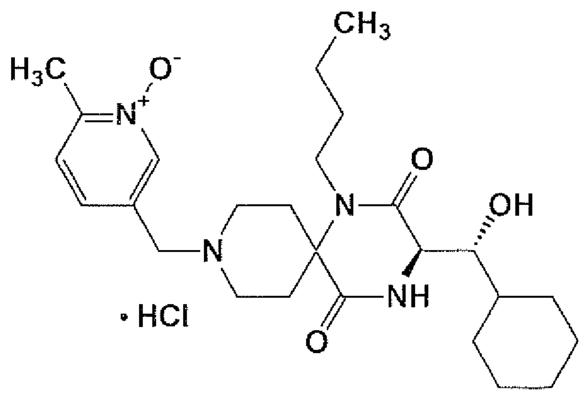

TLC: Rf 0.34 (chloroform:methanol=10:1);
NMR($CD_3OD$): δ 9.00, 8.2-3, 7.89, 451, 4.15, 4.04, 3.80, 3.62-3.42, 3.40-3.20, 2.80-2.40, 2.70, 2.20-1.84, 1.82-1.56, 1.52-1.06, 1.04-0.80, 0.94.

Example 2(28)

(3R)-1-butyl-2,5-dioxo-3-((1R)-1-hydroxy-1-cyclohexylmethyl)-9-(5-carboxypyridin-2-ylmethyl)-1,4,9-triazaspiro[5.5]undecane dihydrochloride

TLC: Rf 0.47 (chloroform:methanol:acetic acid=10:1:1);
NMR($CD_3OD$): δ 9.25, 8.44, 7.63, 4.63, 4.20-4.16, 3.94-3.82, 3.64-3.50, 3.30-3.16, 2.61-2.36, 2.24-2.10, 2.06-1.90, 1.82-1.62, 1.52-1.14, 1.12-0.85.

Example 2(29)

(3R)-1-butyl-2,5-dioxo-3-((1R)-1-hydroxy-1-cyclohexylmethyl)-9-(5-methylpyridin-2-ylmethyl)-1,4,9-triazaspiro[5.5]undecane dihydrochloride

TLC: Rf 0.59 (ethyl acetate:methanol=4:1);
NMR($CD_3OD$): δ 8.77, 8.29, 8.06, 4.64, 4.15, 4.10, 3.87, 3.55-3.52, 3.33, 3.27, 2,65, 2.54-2.48, 2.1 5-1.91, 1.76-1.65, 1.45-1.14, 1.00-0.86.

Example 2(30)

(3R)-1-butyl-2,5-dioxo-3-((1R)-1-hydroxy-1-cyclohexylmethyl)-9-(1-oxidopyridin-3-ylmethyl)-1,4,9-triazaspiro[5.5] undecane hydrochloride

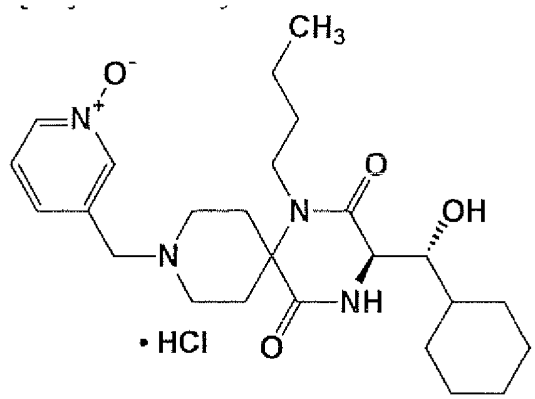

TLC: Rf 0.44 (dichloromethane:methanol=5:1);
NMR($CD_3OD$): δ 8.98, 8.70, 8.26, 7.87, 4.53, 4.16, 4.11, 3.82, 3.60-3.49, 3.34, 3.28, 2.61-2.46, 2.15-1.91, 1.75-1.65, 1.39-1.14, 1.00-0.92.

Example 2(31)

(3R)-1-butyl-2,5-dioxo-3-((1R)-1-hydroxy-1-cyclohexylmethyl)-9-(1-oxidopyridin-4-ylmethyl)-1,4,9-triazaspiro[5.5] undecane hydrochloride

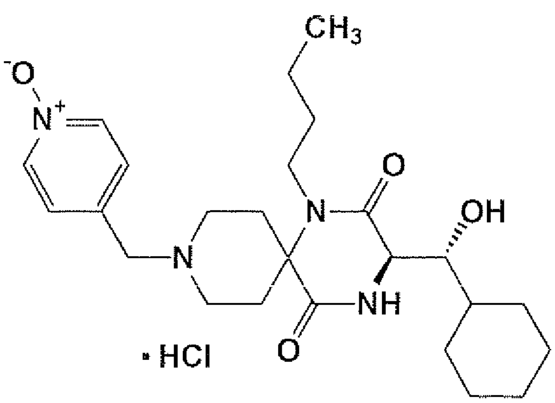

TLC: Rf 0.41 (dichloromethane:methanol=5:1);
NMR($CD_3OD$): δ 8.74, 8.14, 4.55, 4.15, 4.06, 3.82, 3.59-3.47, 3.36, 3.28, 2.73-2.42, 2.13-1.91, 1.75-1.64, 1.40-1.14, 1.00-0.86.

Example 2(32)

(3R)-1-butyl-2,5-dioxo-3-((1R)-1-hydroxy-1-cyclohexylmethyl)-9-(4-(4-(N-ethyl-N-methylsulfonylamino)phenoxy) phenylmethyl)-1,4,9-triazaspiro[5.5]undecane hydrochloride

TLC: Rf 0.42 (chloroform:methanol=10:1);
NMR($CD_3OD$): δ 7.57, 7.41, 7.12, 7.08, 4.34, 4.15, 4.00, 3.76, 3.72, 3.58-3.38, 3.32-3.10, 2.94, 2.58-2.24, 2.20-1.86, 1.84-1.60, 1.52-1.06, 1.11, 1.04-0.80, 0.95.

Example 2(33)

(3R)-1-butyl-2,5-dioxo-3-((1R)-1-hydroxy-1-cyclohexylmethyl)-9-(2-methylaminocarbonylpyridin-5-ylmethyl)-1,4,9-triazaspiro[5.5]undecane dihydrochloride

TLC: Rf 0.41 (chloroform:methanol=10:1);
NMR($CD_3OD$): δ 8.82, 8.19, 4.51, 4.15, 4.06, 3.80, 3.58-3.40, 3.32-3.08, 2.97, 2.58-2.24, 2.20-1.82, 1.80-1.58, 1.50-1.06, 1.04-0.78, 0.94.

Example 2(34)

(3R)-1-butyl-2,5-dioxo-3-((1R)-1-hydroxy-1-cyclohexylmethyl)-9-(5-(4-methylaminocarbonylphenyl)pentyl)-1,4,9-triazaspiro[5.5]undecane hydrochloride

TLC: Rf 0.25 (chloroform:methanol=10:1);
NMR($CD_3OD$): δ 7.72, 7.29, 4.16, 3.92, 3.76-3.42, 3.36-3.02, 2.90, 2.78-2.64, 2.58-2.22, 2.20-1.58, 1.56-1.06, 1.04-0.80, 0.97.

Example 2(35)

(3R)-1-butyl-2,5-dioxo-3-((1R)-1-hydroxy-1-cyclohexylmethyl)-9-(2-amino-1-oxidopyridin-5-ylmethyl)-1,4,9-triazaspiro[5.5]undecane dihydrochloride

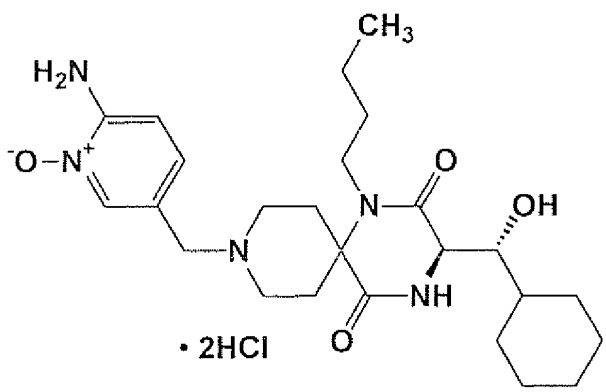

TLC: Rf 0.51 (chloroform:methanol=5:1);
NMR($CD_3OD$): δ 8.53, 8.04, 7.19, 4.31, 4.15, 3.98, 3.76, 3.64-3.42, 3.40-3.20, 2.76-2.30, 2.20-1.84, 1.82-1.58, 1.52-1.06, 1.04-0.80, 0.95.

Example 2(36)

(3R)-1-butyl-2,5-dioxo-3-((1R)-1-hydroxy-1-cyclohexylmethyl)-9-(2-methylaminocarbonyl-1-oxidopyridin-5-ylmethyl)-1,4,9-triazaspiro[5.5]undecane hydrochloride

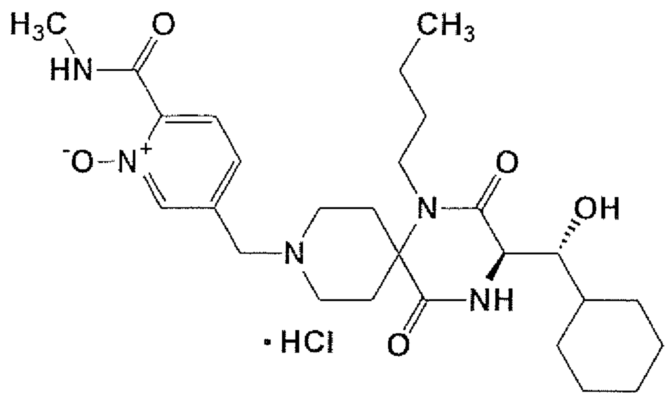

TLC: Rf 0.26 (chloroform:methanol=10:1);
NMR($CD_3OD$): δ 8.67, 8.39, 7.85, 4.46, 4.15, 4.06, 3.80, 3.60-3.42, 3.32-3.14, 3.01, 2.60-2.28, 2.20-1.84, 1.82-1.58, 1.50-1.06, 1.04-0.80, 0.95.

Example 2(37)

(3R)-1-butyl-2,5-dioxo-3-((1R)-1-hydroxy-1-cyclohexylmethyl)-9-(5-(4-carboxyphenyl)pentyl)-1,4,9-triazaspiro[5.5] undecane hydrochloride

TLC: Rf 0.58 (chloroform:methanol=5:1);
NMR($CD_3OD$): δ 7.93, 7.31, 4.16, 3.94, 3.78-3.40, 3.32-3.02, 2.80-2.64, 2.56-2.22, 2.20-1.60, 1.56-1.06, 1.04-0.80, 0.97.

Example 2(38)

(3R)-1-butyl-2,5-dioxo-3-((1R)-1-hydroxy-1-cyclohexylmethyl)-9-(4-(4-methylaminocarbonylphenoxy) cyclohexylmethyl)-1,4,9-triazaspiro[5.5]undecane hydrochloride

TLC: Rf 0.12 (ethyl acetate:methanol=10:1);
NMR($CD_3OD$): δ 7.75, 6.96, 4.37, 4.17, 3.98, 3.72, 3.64-3.55, 3.33-3.20, 3.08-3.06, 2.89, 2.60- 2.41, 2.24-1.9-3, 1.80-1.63, 1.57-1.19, 1.00-0.91.

Example 2(39)

(3R)-1-butyl-2,5-dioxo-3-((1R)-1-hydroxy-1-cyclohexylmethyl)-9-(4-cyclohexyloxyphenylmethyl)-1,4,9-triazaspiro[5.5] undecane hydrochloride

TLC: Rf 0.60 (ethyl acetate:methanol=10:1);
NMR($CD_3OD$): δ 7.42, 7.00, 4.37, 4.27, 4.15, 3.97, 3.71, 3.53-3.40, 3.33-3.15, 2.49-1.95 , 1.85-1.18, 1.00-0.87.

Example 2(40)

(3R)-1-butyl-2,5-dioxo-3-((1R)-1-hydroxy-1-cyclohexylmethyl)-9-(4-benzylphenylmethyl)-1,4,9-triazaspiro[5.5] undecane hydrochloride

TLC: Rf 0.72 (ethyl acetate:methanol=10:1);

NMR($CD_3OD$): δ 7.45, 7.34, 7.29-7.17, 4.31, 4.14, 4.00, 3.98, 3.73, 3.52-3.41, 3.33-3.14, 2.47-2.20, 2.13-1.91, 1.80-1.63, 1.45-1.15, 1.00-0.87.

Example 2(41)

(3R)-1-butyl-2,5-dioxo-3-((1R)-1-hydroxy-1-cyclohexylmethyl)-9-(2-phenylethenylphenylmethyl)-1,4,9-thazaspiro[5.5] undecane hydrochloride

TLC: Rf 0.38 (chloroform:methanol=10:1);
NMR($CD_3OD$): δ 7.76-7.64, 7.62-7.46, 7.42-7.16, 4.36, 4.15, 4.00 3.78, 3.60-3.40, 3.38-3.10, 2.60-1.86, 1.84-1.58, 1.54-1.08, 1.06-0.80, 0.95.

Example 2(42)

(3R)-1-butyl-2,5-dioxo-3-((1R)-1-hydroxy-1-cyclohexylmethyl)-9-(3-phenoxyphenylmethyl)-1,4,9-triazaspiro[5.5] undecane hydrochloride

TLC: Rf 0.44 (chloroform:methanol=10:1);
NMR($CD_3OD$): δ 7.50-6.96, 4.33, 4.15, 4.00, 3.74, 3.58-3.36, 3.30-3.08, 2.54-1.84, 1.82-1.58, 1.54-1.06, 1.04-0.80, 0.95.

Example 2(43)

(3R)-1-butyl-2,5-dioxo-3-((1R)-1-hydroxy-1-cyclohexylmethyl)-9-(2-chloro-1-oxidopyridin-5-ylmethyl)-1,4,9-triazaspiro[5.5]undecane hydrochloride

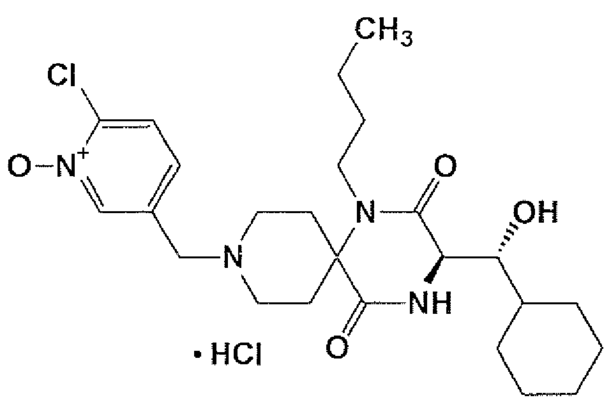

TLC: Rf 0.31 (chloroform:methanol=10:1);
NMR($CD_3OD$): δ 8.76, 7.88, 7.78, 4.43, 4.15, 4.04, 3.80, 3.64-3.42, 3.32-3.16, 2.64-2.32, 2.20-1.84, 1.82-1.60, 1.50-1.06, 1.04-0.80, 0.95.

Example 2(44)

(3R)-1-butyl-2,5-dioxo-3-((1R)-1-hydroxy-1-cyclohexylmethyl)-9-(6-(4-methylaminocarbonylphenoxy)hexyl)-1,4,9-triazaspiro[5.5]undecane hydrochloride

TLC: Rf 0.19 (chloroform:methanol=10:1);
NMR($CD_3OD$): δ 7.76, 6.95, 4.16, 4.05, 3.94, 3.78-3.44, 3.32-3.08, 2.89, 2.60-2.24, 2.20-1.08, 1.06-0.80, 0.97.

Example 2(45)

(3R)-1-butyl-2,5-dioxo-3-((1R)-1-hydroxy-1-cyclohexylmethyl)-9-(1-(4-methylaminocarbonylphenylmethyl)piperidin-4-ylmethyl)-1,4,9-triazaspiro[5.5]undecane dihydrochloride

TLC: Rf 0.14 (chloroform:methanol=10:1);
NMR($CD_3OD$): δ 7.92, 7.65, 4.39, 4.15, 3.96, 3.78-3.22, 3.18-2.96, 2.93, 2.80-2.38, 2.36-1.86, 1.82-1.10, 1.06-0.80, 0.96.

Example 2(46)

(3R)-1-butyl-2,5-dioxo-3-((1R)-1-hydroxy-1-cyclohexylmethyl)-9-(4-(2-(N,N-dimethylamino)ethylaminocarbonyl)phenylmethyl)-1,4,9-triazaspiro[5.5]undecane dihydrochloride

TLC: Rf 0.11 (chloroform:methanol=5:1);
NMR($CD_3OD$): δ 8.00, 7.74, 4.42, 4.14, 4.00, 3.84-3.66, 3.60-3.20, 2.99, 2.64-2.36, 2.18-1.84, 1.82-1.58, 1.50-1.06, 1.04-0.80, 0.95.

Example 2(47)

(3R)-1-butyl-2,5-dioxo-3-((1R)-1-hydroxy-1-cyclohexylmethyl)-9-(6-(4-methylsulfonylaminophenoxy)hexyl)-1,4,9-triazaspiro[5.5]undecane hydrochloride

TLC: Rf 0.20 (dichloromethane:methanol=10:1);
NMR($CD_3OD$): δ 7.17, 6.89, 4.16, 3.97, 3.93, 3.68, 3.60-3.50, 3.35-3.10, 2.87, 2.60-2.30, 2.20-1.10, 1.00-0.80, 0.97.

Example 2(48)

(3R)-1-butyl-2,5-dioxo-3-((1R)-1-hydroxy-1-cyclohexylmethyl)-9-(4-(4-ethylsulfonylaminophenoxy)phenylmethyl)-1,4,9-triazaspiro[5.5]undecane hydrochloride

TLC: Rf 0.27 (chloroform:methanol=10:1);
NMR($CD_3OD$): δ 7.53, 7.29, 7.06, 7.02, 4.32, 4.15, 3.98, 3.74, 3.58-3.38, 3.30-3.10, 3.08, 2.56-2.20, 2.18-1.84, 1.82-1.58, 1.50-1.06, 1.32, 1.04-0.80, 0.95.

Example 2(49)

(3S)-1-butyl-2,5-dioxo-3-cyclohexylmethyl-9-(4-(4-methylsulfonylaminophenoxy)butyl)-1,4,9-triazaspiro[5.5]undecane hydrochloride

TLC: Rf 0.35 (dichloromethane:methanol=10:1);
NMR($CD_3OD$): δ 7.18, 6.92, 4.07-4.02, 3.83-3.69, 3.61-3.57, 3.45, 3.26-3.20, 2.87, 2.60-2.45, 2.25-1.15, 1.05-0.90, 0.96.

Example 2(50)

(3R)-1-butyl-2,5-dioxo-3-((I R)-1-hydroxy-1-cyclohexylmethyl)-9-(4-(4-methylcarbonylaminophenoxy)phenylmethyl)-1,4,9-triazaspiro[5.5]undecane hydrochloride

TLC: Rf 0.56 (chloroform:methanol=9:1);
NMR($CD_3OD$): δ 7.59-7.49, 7.06-6.96, 4.32, 4.15, 3.95, 3.70, 3.59-3.38, 3.30-3.10, 2.52-2.22, 2.12, 2.12-1.88 , 1.80-1.60, 1.48-1.12, 1.03-0.80, 0.95.

Example 2(51)

(3R)-1-butyl-2,5-dioxo-3-((1R)-1-hydroxy-1-cyclohexylmethyl)-9-(1-benzylpiperidin-4-ylmethyl)-1,4,9-triazaspiro[5.5]undecane dihydrochloride

TLC: Rf 0.52 (chloroform:methanol=9:1);
NMR($CD_3OD$): δ 7.60-7.42, 4.33, 4.15, 3.95, 3.78-3.46, 3.46-3.22 3.16-3.00, 2.75, 2.60, 2.42, 2.36-2.15, 2.15-1.86 , 1.82-1.50, 1.50-1.05, 1.04-0.80, 0.96.

Example 2(52)

(3R)-1-butyl-2,5-dioxo-3-((1R)-1-hydroxy-1-cyclohexylmethyl)-9-(3-phenylaminocarbonylpropyl)-1,4,9-triazaspiro[5.5]undecane hydrochloride

TLC: Rf 0.46 (chloroform:methanol:acetic acid=20:4:1);
NMR($CD_3OD$): δ 7.59-7.52, 7.35-7.26, 7.10, 4.17, 3.96, 3.71, 3.65-3.48, 3.32-3.12, 2.65, 2.57-2.27, 2.24-1.88, 1.85-1.61, 1.55-1.08, 1.06-0.80, 0.96.

Example 2(53)

(3R)-1-butyl-2,5-dioxo-3-((1R)-1-hydroxy-1-cyclohexylmethyl)-9-(cis-2-phenoxymethylcyclopropylmethyl)-1,4,9-triazaspiro[5.5]undecane hydrochloride

TLC: Rf 0.25 (ethyl acetate:methanol=9:1);
NMR($CD_3OD$): δ 7.26, 6.95-6.90, 4.34, 4.16, 3.99, 3.81, 3.74-3.45, 3.34-3.11, 2.51-2.31, 2.17-1.91, 1.77-1.64, 1.50-1.11, 1.00-0.85, 0.98, 0.64.

Example 2(54)

(3R)-1-butyl-2,5-dioxo-3-((1R)-1-hydroxy-1-cyclohexylmethyl)-9-(trans-2-phenoxymethylcyclopropylmethyl)-1,4,9-triazaspiro[5.5]undecane hydrochloride

TLC: Rf 0.19 (ethyl acetate:methanol=9: 1);
NMR($CD_3OD$): δ 7.25, 6.94-6.88, 4.18, 4.17, 3.98, 3.85-3.50, 3.34-3.16, 3.02, 2.56-2.25, 2.19-1.90, 1.82-1.65, 1.51-1.15, 1.00-0.87, 0.98, 0.81.

Example 2(55)

(3R)-1-butyl-2,5-dioxo-3-((1R)-1-hydroxy-1-cyclohexylmethyl)-9-(5-(4-methylsulfonylaminophenyl)pentyl)-1,4,9-triazaspiro[5.5]undecane hydrochloride

TLC: Rf 0.26 (ethyl acetate:methanol=9:1);
NMR($CD_3OD$): δ 7.16, 4.16, 3.92, 3.67, 3.58-3.48, 3.30-3.20, 3.14-3.08, 2.91, 2.63, 2.52-2.40, 2.41, 2.15-1.90, 1.84-1.62, 1.48-1.15, 1.00-0.85, 0.97.

Example 2(56)

(3R)-1-butyl-2,5-dioxo-3-((1R)-1-hydroxy-1-cyclohexylmethyl)-9-(4-(2-phenylethyl)phenylmethyl)-1,4,9-triazaspiro[5.5]undecane hydrochloride

TLC: Rf 0.67 (ethyl acetate:methanol=9:1);
NMR($CD_3OD$): δ 7.43, 7.29, 7.25-7.19, 7.15-7.12, 4.30, 4.15, 3.98, 3.72, 3.55-3.35, 3.30-3.12, 2.98-2.91, 2.50-2.20, 2.14-1.90, 1.80-1.60, 1.45-1.15, 1.00-0.86, 0.95.

Example 2(57)

(3R)-1-butyl-2,5-dioxo-3-((1R)-1-hydroxy-1-cyclohexylmethyl)-9-(3-diphenyl-2-propenyl)-1,4,9-triazaspiro[5.5] undecane hydrochloride

TLC: Rf 0.78 (ethyl acetate:methanol=9:1);
NMR($CD_3OD$): δ 7.50-7.42, 7.32, 7.19, 6.27, 4.14, 3.85, 3.84, 3.62-3.45, 3.30-3.15, 2.52-2.25, 2.15-1.91, 1.80-1.62, 1.45-1.16, 0.97, 0.96-0.85.

Example 2(58)

(3R)-1-butyl-2,5-dioxo-3-((1R)-1-hydroxy-1-cyclohexylmethyl)-9-(4-(4-(N,N-bis(2-ethoxyethyl)aminocarbonyl) phenoxy)butyl)-1,4,9-triazaspiro[5.5]undecane hydrochloride

TLC: Rf 0.19 (chloroform:methanol=9:1);
NMR($CD_3OD$): δ 7.38, 6.98, 4.16, 4.10, 3.97, 3.79-3.32, 3.30-3.12, 2.56-2.39, 2.31, 2.12, 2.06-1.82, 1.78-1.60, 1.49-1.03, 1.02-0.80, 0.97.

Example 2(59)

(3R)-1-butyl-2,5-dioxo-3-((1R)-1-hydroxy-1-cyclohexylmethyl)-9-(3-(1,3-benzodioxol-4-yl)propyl)-1,4,9-triazaspiro [5.5]undecane hydrochloride

TLC: Rf 0.53 (dichloromethane:methanol=10:1);
NMR($CD_3OD$): δ 6.76-6.68, 5.90, 4.15, 3.91, 3.66, 3.60-3.45, 3.33-3.09, 2.64, 2.49-2.26, 2.15-1.92, 1.80-1.6 5, 1.40-1.14, 1.00-0.87.

Example 2(60)

(3R)-1-butyl-2,5-dioxo-3-((1R)-1-hydroxy-1-cyclohexylmethyl)-9-(4-(4-methylaminocarbonylphenylmethyl) phenylmethyl)-1,4,9-triazaspiro[5.5]undecane hydrochloride

TLC: Rf 0.45 (dichloromethane:methanol=10:1);
NMR($CD_3OD$): δ 7.73, 7.47, 7.36, 7.31, 4.32, 4.14, 4.07, 3.99, 3.74, 3.52-3.41, 3.25-3.14, 2.89, 2.47-2.24, 2.13-1.91, 1.80-1.65, 1.40-1.15, 1.00-0.86.

Example 2(61)

(3R)-1-butyl-2,5-dioxo-3-((1R)-1-hydroxy-1-cyclohexylmethyl)-9-(4-(4-carboxyphenylmethyl)phenylmethyl)-1,4,9-triazaspiro[5.5]undecane hydrochloride

TLC: Rf 0.20 (dichloromethane:methanol=10:1);
NMR($CD_3OD$): δ 7.93, 7.47, 7.37, 7.32, 4.32, 4.14, 4.09, 3.98, 3.74, 3.53-3.36, 3.29-3.13, 2.49-2.24, 2.13-1.91, 1.80-1.65, 1.40-1.18, 1.00-0.91.

Example 2(62)

(3R)-1-butyl-2,5-dioxo-3-((1R)-1-hydroxy-1-cyclohexylmethyl)-9-(4-(cis-2-phenylcyclopropyl)phenylmethyl)-1,4,9-triazaspiro[5.5]undecane hydrochloride

TLC: Rf 0.68 (ethyl acetate:methanol=19:1);
NMR($CD_3OD$): δ 7.23, 7.08, 7.03-6.96, 4.18, 4.14, 3.90, 3.63, 3.47, 3.33-3.08, 2.60-2.52, 2.43, 2.34, 2.21, 2.11-1.92, 1.84-1.60, 1.54, 1.46, 1.45-1.10, 0.99-0.85, 0.95.

Example 2(63)

(3R)-1-butyl-2,5-dioxo-3-((1R)-1-hydroxy-1-cyclohexylmethyl)-9-(4-(trans-2-phenylcyclopropyl)phenylmethyl)-1,4,9-triazaspiro[5.5]undecane hydrochloride

TLC: Rf 0.62 (ethyl acetate:methanol=19:1);
NMR($CD_3OD$): δ 7.45, 7.30-7.23, 7.17-7.12, 4.32, 4.15, 3.99, 3.74, 3.53-3.40, 3.30-3.10, 2.51-1.85, 2.20, 1.84-1.60, 1.54-1.45, 1.45-1.18, 0.99-0.83, 0.95.

Example 2(64)

(3R)-1-butyl-2,5-dioxo-3-((1R)-1-hydroxy-1-cyclohexylmethyl)-9-(4-(4-methylphenylsulfonylamino)phenylmethyl)-1,4,9-triazaspiro[5.5]undecane hydrochloride

TLC: Rf 0.76 (chloroform:methanol=5:1);
NMR($CD_3OD$): δ 7.69, 7.41, 7.29, 7.21, 4.24, 4.14, 3.94, 3.69, 3.49, 3.42-3.33, 3.25, 3.17, 2.48-2.18, 2.36, 2.16-1.88, 1.80-1.62, 1.48-1.10, 1.02-0.80, 0.94.

Example 2(65)

(3R)-1-butyl-2,5-dioxo-3-((1R)-1-hydroxy-1-cyclohexylmethyl)-9-(2-phenylimidazol-4-ylmethyl)-1,4,9-triazaspiro[5.5]undecane dihydrochloride

TLC: Rf 0.27 (dichloromethane:methanol=10:1);
NMR(CD30D): δ 8.00-7.95, 7.72-7.65, 4.56, 4.16, 4.08, 3.83, 3.63-3.51, 3.35, 3.27, 2.73-2.51, 2.17-1.92, 1.80-1.65, 1.45-1.14, 1.00-0.85.

Example 2(66)

(3R)-1-butyl-2,5-dioxo-3-((1R)-1-hydroxy-1-cyclohexylmethyl)-9-(2,3-diphenylpropyl)-1,4,9-triazaspiro[5.5]undecane hydrochloride

TLC: Rf 0.68 (ethyl acetate:methanol=19:1);
NMR($CD_3OD$): δ 7.37-7.24, 7.24-7.10, 7.07-7.03, 4.12, 3.91, 3.75, 3.62-3.35, 3.30-3.18, 3.18-2.95, 2.87, 2.44-1.89, 1.80-1.60, 1.45-1.10, 1.00-0.80.

Example 2(67)

(3R)-1-butyl-2,5-dioxo-3-((1R)-1-hydroxy-1-cyclohexylmethyl)-9-(4-(4-methoxycarbonylphenoxy)phenylmethyl)-1,4,9-triazaspiro[5.5]undecane hydrochloride

TLC: Rf 0.45 (chloroform:methanol=10:1);
NMR($CD_3OD$): δ 8.02, 7.63, 7.16, 7.06, 4.36, 4.15, 3.98, 3.88, 3.74, 3.62-3.37, 3.33-3.15, 2.65-2.28, 2.18-1.84, 1.83-1.55, 1.53-1.07, 1.07-0.77.

Example 2(68)

(3R)-1-butyl-2,5-dioxo-3-((1R)-1-hydroxy-1-cyclohexylmethyl)-9-(4-(4-methylbenzylsulfonylamino)phenylmethyl)-1,4,9-triazaspiro[5.5]undecane hydrochloride

TLC: Rf 0.52 (chloroform:methanol=9:1);
NMR($CD_3OD$): δ 7.48, 7.24, 7.14, 4.40, 4.31, 4.15, 3.98, 3.73, 3.58-3.38, 3.25, 3.19, 2.53-2.38, 2.38-2.22, 2.21, 2.18-1.88, 1.81-1.60, 1.50-1.12, 1.04-0.80, 0.95.

Example 2(69)

(3R)-1-butyl-2,5-dioxo-3-((1R)-1-hydroxy-1-cyclohexylmethyl)-9-(4-(4-carboxyphenylcarbonyl)phenylmethyl)-1,4,9-triazaspiro[5.5]undecane hydrochloride

TLC: Rf 0.36 (chloroform:methanol:acetic acid=20:2:1);
NMR($CD_3OD$): δ 8.17, 7.90, 7.87, 7.76, 4.49, 4.16, 4.06, 3.81, 3.61-3.43, 3.30-3.12, 2.62-2.28, 2.20-1.87, 1.84-1.60, 1.55-1.08, 1.08-0.80, 0.95.

Example 2(70)

(3R)-1-butyl-2,5-dioxo-3-((1R)-1-hydroxy-1-cyclohexylmethyl)-9-(4-(4-methylaminocarbonylphenylcarbonyl)phenylmethyl)-1,4,9-triazaspiro[5.5]undecane hydrochloride

TLC: Rf 0.51 (dichloromethane:methanol=10:1);
NMR($CD_3OD$): δ 7.95, 7.89, 7.86, 7.77, 4.48, 4.16, 4.06, 3.80, 3.61-3.40, 3.30-3.10, 2.95, 2.60-2.28, 2.19-1.87, 1.84-1.60, 1.54-1.08, 1.05-0.79, 0.95.

Example 2(71)

(3R)-1-butyl-2,5-dioxo-3-((1R)-1-hydroxy-1-cyclohexylmethyl)-9-(2-(pyridin-4-yl)-1,3-thiazol-4-ylmethyl)-1,4,9-triazaspiro[5.5]undecane dihydrochloride

TLC: Rf 0.42 (chloroform:methanol=5:1);
NMR($CD_3OD$): δ 8.95, 8.65, 8.34, 4.65, 4.20-4.06, 4.15, 3.94-3.80, 3.66-3.48, 3.38-3.22, 3.26, 2.65, 2.53-2.42, 2.18-1.88, 1.81-1.60, 1.50-1.08, 1.02-0.80, 0.95.

Example 2(72)

(3R)-1-butyl-2,5-dioxo-3-((1R)-1-hydroxy-1-cyclohexylmethyl)-9-(2-phenyl-1,3-thiazol-4-ylmethyl)-1,4,9-triazaspiro[5.5]undecane hydrochloride

TLC: Rf 0.45 (chloroform:methanol=5:1);
NMR($CD_3OD$): δ 8.04-7.98, 7.86, 7.52-7.46, 4.54, 4.15, 4.12, 3.87, 3.66-3.46, 3.33-3.13, 3.25, 2.60-2.43, 2.35, 2.16, 2.08-1.90, 1.82-1.60, 1.50-1.10, 1.06-0.80, 0.95.

Example 2(73)

(3R)-1-butyl-2,5-dioxo-3-((1R)-1-hydroxy-1-cyclohexylmethyl)-9-(4-(4-carboxyphenyl)phenylmethyl)-1,4,9-triazaspiro[5.5]undecane hydrochloride

TLC: Rf 0.21 (chloroform:methanol=5:1);
NMR($CD_3OD$): δ 8.12, 7.83, 7.77, 7.68, 4.43, 4.15, 4.04, 3.78, 3.60-3.42, 3.3 0-3.10, 3.26, 2.55-2.24, 2.13, 2.08-1.86, 1.82-1.60, 1.50-1.08, 1.02-0.80, 0.95.

Example 2(74)

(3R)-1-butyl-2,5-dioxo-3-((1R)-1-hydroxy-1-cyclohexylmethyl)-9-(4-(4-carboxyphenylmethyloxy)phenylmethyl)-1,4,9-triazaspiro[5.5]undecane hydrochloride

TLC: Rf 0.55 (chloroform:methanol:acetic acid=20:2:1);
NMR($CD_3OD$): δ 8.02, 7.54, 7.47, 7.11, 5.22, 4.28, 4.15, 3.97, 3.71, 3.57-3.37, 3.30-3.08, 2.51-2.18, 2.17-1.87, 1.82-1.58, 1.50-1.09, 1.04-0.80, 0.95.

Example 2(75)

(3R)-1-butyl-2,5-dioxo-3-((1R)-1-hydroxy-1-cyclohexylmethyl)-9-(4-(4-methylaminocarbonylphenylmethyloxy) phenylmethyl)-1,4,9-triazaspiro[5.5]undecane hydrochloride

TLC: Rf 0.47 (dichloromethane:methanol=10:1);
NMR($CD_3OD$): δ 7.82, 7.53, 7.48, 7.10, 5.20, 4.28, 4.14, 3.95, 3.70, 3.59-3.37, 3.30-3.12, 2.91, 2.54-2.20, 2.16-1.86, 1.83-1.59, 1.54-1.12, 1.04-0.80, 0.94.

Example 2(76)

(3R)-1-butyl-2,5-dioxo-3-((1R)-1-hydroxy-1-cyclohexylmethyl)-9-(4-(imidazol-1-yl)phenylmethyl)-1,4,9-triazaspiro [5.5]undecane dihydrochloride

TLC: Rf 0.75 (chloroform:methanol=5:1);
NMR($CD_3OD$): δ 9.56, 8.15, 8.00-7.84, 7.81, 4.48, 4.15, 4.03, 3.77, 3.64-3.41, 3.40-3.20, 3.26, 2.7 2-2.36, 2.16-1.88, 1.80-1.60, 1.48-1.10, 1.02-0.80, 0.95.

Example 2(77)

(3R)-1-butyl-2,5-dioxo-3-((1R)-1-hydroxy-1-cyclohexylmethyl)-9-(dibenzo[b,d]furan-2-yl)methyl-1,4,9-triazaspiro[5.5] undecane hydrochloride

TLC: Rf 0.83 (chloroform:methanol=5:1);
NMR($CD_3OD$): δ 8.29, 8.10, 7.72-7.36, 4.53 , 4.15, 4.06, 3.80, 3.60-3.44, 3.34-3.10, 3.26, 2.60-2.22, 2.18-1.88, 1.82-1.60, 1.50-1.06, 1.04-0.88.

Example 2(78)

(3R)-1-butyl-2,5-dioxo-3-((1R)-1-hydroxy-1-cyclohexylmethyl)-9-(5-(4-methoxyphenyl)thiophen-2-ylmethyl)-1,4,9-triazaspiro[5.5]undecane hydrochloride

TLC: Rf 0.88 (chloroform:methanol=5:1);
NMR($CD_3OD$): δ 7.58, 7.29, 6.97, 4.59, 4.15, 4.03, 3.82, 3.78, 3.62-3.40, 3.38-3.09, 3.27, 2.58-2.20, 2.20-2.10, 2.08-1.88, 1.80-1.60, 1.48-1.12, 1.02-0.86, 0.96.

Example 2(79)

(3R)-1-butyl-2,5-dioxo-3-((1R)-1-hydroxy-1-cyclohexylmethyl)-9-(3-(4-hydroxyphenyl)phenylmethyl)-1,4,9-triazaspiro [5.5]undecane hydrochloride

TLC: Rf 0.80 (chloroform:methanol=5:1);
NMR($CD_3OD$): δ 7.86-7.64, 7.58-7.36, 6.94-6.80, 4.40, 4.15, 4.03, 3.77, 3.58-3.42, 3.38-3.00, 2.56-2.16, 2.16-1.84, 1.82-1.59, 1.50-1.08, 1.08-0.80, 0.94.

Example 2(80)

(3R)-1-butyl-2,5-dioxo-3-((1R)-1-hydroxy-1-cyclohexylmethyl)-9-(2-phenylpyridin-5-ylmethyl)-1,4,9-triazaspiro[5.5] undecane dihydrochloride

TLC: Rf 0.93 (chloroform:methanol=5:1);
NMR($CD_3OD$): δ 9.13, 8.73, 8.35, 8.08-7.90, 7.76-7.60, 4.62, 4.16, 4.10, 3.84, 3.60-3.26, 3.40-3.20, 3.26, 2.68, 2.58-2.40, 2.18-1.86, 1.82-1.60, 1.48-1.08, 1.06-0.80, 0.94.

Example 2(81)

(3R)-1-butyl-2,5-dioxo-3-((1R)-1-hydroxy-1-cyclohexylmethyl)-9-(4-(4-carboxyphenoxymethyl)phenylmethyl)-1,4,9-triazaspiro[5.5]undecane hydrochloride

TLC: Rf 0.57 (chloroform:methanol:acetic acid=20:2:1);
NMR($CD_3OD$): δ 7.96, 7.60, 7.05, 5.23, 4.37, 4.15, 4.01, 3.75, 3.59-3.39, 3.30-3.10, 2.56-2.22, 2.17-1.86, 1.85-1.60, 1.52-1.10, 1.05-0.79, 0.94.

Example 2(82)

(3R)-1-butyl-2,5-dioxo-3-((1R)-1-hydroxy-1-cyclohexylmethyl)-9-(4-(4-methylaminocarbonylphenoxymethyl) phenylmethyl)-1,4,9-triazaspiro[5.5]undecane hydrochloride

TLC: Rf 0.51 (chloroform:methanol:acetic acid=20:2:1);
NMR($CD_3OD$): δ 7.77, 7.59, 7.04, 5.21, 4.37, 4.15, 4.00, 3.75, 3.58-3.39, 3.30-3.10, 2.89, 2.56-2.24, 2.17-1.86, 1.85-1.60, 1.55-1.12, 1.06-0.80, 0.94.

Example 2(83)

(3R)-1-butyl-2,5-dioxo-3-((1R)-1-hydroxy-1-cyclohexylmethyl)-9-(4-(4-carboxyphenylmethylaminocarbonyl) phenylmethyl)-1,4,9-triazaspiro[5.5]undecane hydrochloride

TLC: Rf 0.15 (chloroform:methanol=5:1);
NMR($CD_3OD$): δ 8.04-7.95, 7.72-7.64, 7.45, 4.65, 4.43, 4.15, 4.02, 3.77, 3.58-3.40, 3.38-3.22, 3.25, 2.54-2.26, 2.18-1.88, 1.82-1.60, 1.48-1.10, 1.04-0.80, 0.94.

Example 2(84)

(3R)-1-butyl-2,5-dioxo-3-((1R)-1-hydroxy-1-cyclohexylmethyl)-9-(5-methyl-1-phenylpyrazol-4-ylmethyl)-1,4,9-triazaspiro[5.5]undecane dihydrochloride

TLC: Rf 0.56 (chloroform:methanol=5:1)
NMR($CD_3OD$): δ 7.87, 7.60-7.44, 4.32, 4.16, 4.00, 3.77, 3.62-3.48, 3.38-3.18, 3.27, 2.60-2.32, 2.39, 2.21-1.90, 1.84-1.64, 1.52-1.10, 1.08-0.80, 0.96.

Example 2(85)

(3R)-1-butyl-2,5-dioxo-3-((1R)-1-hydroxy-1-cyclohexylmethyl)-9-(4-(4-methylcarbonylaminophenylmethyl) phenylmethyl)-1,4,9-triazaspiro[5.5]undecane hydrochloride

TLC: Rf 0.46(dichloromethane:methanol=10:1);
NMR($CD_3OD$): δ 7.45-7.42, 7.34, 7.15, 4.31, 4.14, 3.98, 3.97, 3.73, 3.53-3.42, 3.26-3.11, 2.47-1.92, 2.09, 1.80-1.65, 1.45-1.14, 1.00-0.91, 0.94.

Example 2(86)

(3R)-1-butyl-2,5-dioxo-3-((1R)-1-hydroxy-1-cyclohexylmethyl)-9-(5-(4-chlorophenyl)furan-2-ylmethyl)-1,4,9-triazaspiro[5.5]undecane hydrochloride

TLC: Rf 0.90 (chloroform:methanol=5:1);
NMR($CD_3OD$): δ 7.75, 7.43, 6.90, 6.85, 4.52, 4.15, 4.07, 3.80, 3.60-3.44, 3.38-3.10, 3.25, 2.58-2.44, 2.36, 2.17, 2.08-1.86, 1.82-1.60, 1.50-1.12, 1.08-0.88, 0.95.

Example 2(87)

(3R)-1-butyl-2,5-dioxo-3-((1R)-1-hydroxy-1-cyclohexylmethyl)-9-(4-(4-methylaminocarbonylphenylmethylaminocarbonyl)phenylmethyl)-1,4,9-triazaspiro[5.5]undecane hydrochloride

TLC: Rf 0.69 (chloroform:methanol=5:1);
NMR($CD_3OD$): δ 7.93, 7.78, 7.60, 7.43, 4.63, 4.14, 4.13, 3.63, 3.55-3.10, 2.90, 2.41-2.15, 2.04-1.83, 1.80-1.60, 1.48-1.10, 1.02-0.80, 0.95.

Example 2(88)

(3R)-1-butyl-2,5-dioxo-3-((1R)-1-hydroxy-1-cyclohexylmethyl)-9-(1-methylbenzimidazol-2-ylmethyl)-1,4,9-triazaspiro[5.5]undecane dihydrochloride

TLC: Rf 0.79 (chloroform:methanol=5:1);
NMR($CD_3OD$): δ 8.00-7.86, 7.74-7.60, 4.96, 4.23, 4.16, 3.96, 3.76-3.62, 3.56, 3.42-3.20, 3.27, 2.80-2.42, 2.18-1.86, 1.82-1.62, 1.46-1.08, 1.02-0.80, 0.94.

Example 2(89)

(3R)-1-butyl-2,5-dioxo-3-((1R)-1-hydroxy-1-cyclohexylmethyl)-9-(4-(4-cyclopropylmethylaminocarbonylphenoxy)phenylmethyl)-1,4,9-triazaspiro[5.5]undecane hydrochloride

TLC: Rf 0.75 (chloroform:methanol=5:1);
NMR($CD_3OD$): δ 7.86, 7.60, 7.14, 7.08, 4.36, 4.15, 4.00, 3.75, 3.60-3.37, 3.34- 3.12, 2.56-2.24, 2.18-1.86, 1.80-1.60, 1.50-0.80, 0.95, 0.56-0.48, 0.33-0.24.

Example 2(90)

(3R)-1-butyl-2,5-dioxo-3-((1R)-1-hydroxy-1-cyclohexylmethyl)-9-(4-(4-(2-butynylaminocarbonyl)phenoxy)phenylmethyl)-1,4,9-triazaspiro[5.5]undecane hydrochloride

TLC: Rf 0.77 (chloroform:methanol=5:1);
NMR($CD_3OD$): δ 7.86, 7.60, 7.15, 7.07, 4.36, 4.15, 4.08, 3.98, 3.76 , 3.60-3.36, 3.34-3.10, 3.26, 2.52-2.24, 2.18-1.88, 1.82-1.62, 1.79, 1.50-1.12, 1.02-0.80, 0.95.

Example 2(91)

(3R)-1-butyl-2,5-dioxo-3-((1R)-1-hydroxy-1-cyclohexylmethyl)-9-(3-(4-carboxyphenylmethyloxy)phenylmethyl)-1,4,9-triazaspiro[5.5]undecane hydrochloride

TLC: Rf 0.51 (chloroform:methanol:acetic acid=20:2:1);
NMR($CD_3OD$): δ 8.02, 7.56, 7.41, 7.25, 7.28-7.18, 5.25, 4.30, 4.15, 3.93, 3.71, 3.60-3.14, 2.55-2.25, 2.14-1.87, 1.84-1.60, 1.52-1.08, 1.08-0.80, 0.95.

Example 2(92)

(3R)-1-butyl-2,5-dioxo-3-((1R)-1-hydroxy-1-cyclohexylmethyl)-9-(3-(4-methylaminocarbonylphenylmethyloxy)phenylmethyl)-1,4,9-triazaspiro[5.5]undecane hydrochloride

TLC: Rf 0.42 (chloroform:methanol:acetic acid=20:2:1);
NMR($CD_3OD$): δ 7.81, 7.54, 7.41, 7.22, 7.18-7.09, 5.23, 4.30, 4.15, 3.94, 3.70, 3.57-3.10, 2.91, 2.52-2.18, 2.14-1.88, 1.84-1.61, 1.52-1.08, 1.08-0.80, 0.95.

Example 2(93)

(3R)-1-butyl-2,5-dioxo-3-((1R)-1-hydroxy-1-cyclohexylmethyl)-9-(4-(4-dimethylaminocarbonylphenoxy) phenylmethyl)-1,4,9-triazaspiro[5.5]undecane hydrochloride

TLC: Rf 0.71 (chloroform:methanol=9:1);
NMR($CD_3OD$): δ 7.60, 7.47, 7.20-7.03, 4.35, 4.15, 3.99, 3.74, 3.60-3.40, 3.26, 3.22, 3.09, 3.04, 2.59-2.26, 2.18-1.86, 1.82-1.62, 1.48-1.08, 1.04-0.80, 0.95.

Example 2(94)

(3R)-1-butyl-2,5-dioxo-3-((1R)-1-hydroxy-1-cyclohexylmethyl)-9-(1-phenyl-3,5-bis(trifluoromethyl)-1H-pyrazol-4-ylmethyl)-1,4,9-triazaspiro[5.5]undecane dihydrochloride

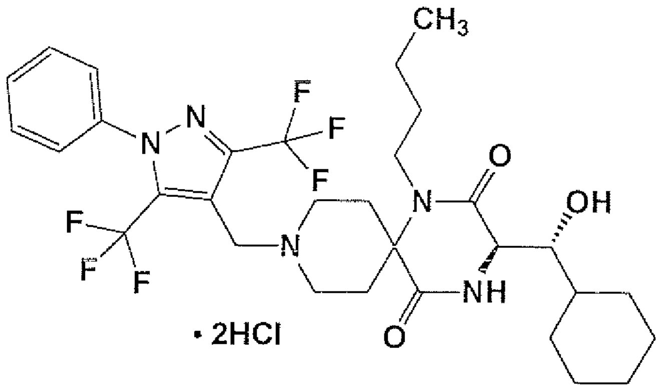

TLC: Rf 0.86 (chloroform:methanol=9:1);
NMR($CD_3OD$): δ 7.70-7.50, 4.59, 4.18-, 4.17, 3.90, 3.68-3.40, 3.26, 3.17, 2.60-2.12, 2.08-1.90, 1.82-1.62, 1.50-1.10, 1.02-0.80, 0.96.

Example 2(95)

(3R)-1-butyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane hydrochloride

TLC: Rf 0.45 (chloroform:methanol=5:1);
NMR($CD_3OD$): δ 7.74, 7.68, 7.52, 7.08, 6.99, 4.32, 4.15, 3.99, 3.8 2, 3.73, 3.58-3.38, 3.26, 3.19, 2.58-2.20, 2.19-1.88, 1.82-1.58, 1.46-1.12, 1.02-0.80, 0.95.

Example 2(96)

(3R)-1-butyl-2,5-dioxo-3-((1R)-1-hydroxy-1-cyclohexylmethyl)-9-(4-(4-cyclopentylaminocarbonylphenoxy) phenylmethyl)-1,4,9-triazaspiro[5.5]undecane hydrochloride

TLC: Rf 0.81 (chloroform:methanol=5:1);
NMR($CD_3OD$): δ 7.84, 7.59, 7.14, 7.06, 4.36, 4.31, 4.15, 4.00, 3.75, 3.56-3.34, 3.26, 3.20, 2.54-2.24, 2.18-1.88, 1.84-1.50, 1.50-1.10, 1.04-0.80, 0.95.

Example 2(97)

(3R)-1-butyl-2,5-dioxo-3-((1R)-1-hydroxy-1-cyclohexylmethyl)-9-(3-(4-carboxyphenoxymethyl)phenylmethyl)-1,4,9-triazaspiro[5.5]undecane hydrochloride

TLC: Rf 0.58 (chloroform:methanol=5:1);

NMR($CD_3OD$): δ 7.97, 7.68, 7.62-7.50, 7.07, 5.24, 4.38, 4.15, 3.97, 3.74, 3.56-3.34, 3.26, 3.19, 2.50-2.22, 2.14-1.86, 1.80-1.60, 1.48-1.10, 1.04-0.80, 0.95.

Example 2(98)

(3R)-1-butyl-2,5-dioxo-3-((1R)-1-hydroxy-1-cyclohexylmethyl)-9-(4-(4-carboxymethylaminocarbonylphenoxy) phenylmethyl)-1,4,9-triazaspiro[5.5]undecane hydrochloride

TLC: Rf 0.47 (chloroform:methanol:acetic acid=20:4:1);
NMR($CD_3OD$): δ 7.90, 7.59, 7.16, 7.09, 4.36, 4.16, 4.09, 4.00, 3.76, 3.58-3.40, 3.26, 3.19, 2.54-2.20, 2.20-1.88, 1.80-1.62, 1.44-1.08, 1.06-0.80, 0.95.

Example 2(99)

(3R)-1-butyl-2,5-dioxo-3-((1R)-1-hydroxy-1-cyclohexylmethyl)-9-(4-(4-carboxy-2-methylphenoxy)phenylmethyl)-1,4,9-triazaspiro[5.5]undecane hydrochloride

TLC: Rf 0.58 (chloroform:methanol=5:1);
NMR($CD_3OD$): δ 7.97, 7.84, 7.59, 7.05, 6.92, 4.34, 4.15, 3.97, 3.7 2, 3.60-3.38, 3.32-3.16, 3.26, 2.61-2.30, 2.28, 2.14-1.86, 1.80-1.58, 1.48-1.10, 1.02-0.80, 0.95.

Example 2(100)

(3R)-1-butyl-2,5-dioxo-3-((1R)-1-hydroxy-1-cyclohexylmethyl)-9-(4-(4-methylaminocarbonyl-2-methoxyphenoxy) phenylmethyl)-1,4,9-triazaspiro[5.5]undecane hydrochloride

TLC: Rf 0.57 (chloroform:methanol=5:1);
NMR($CD_3OD$): δ 7.59, 7.51, 7.45, 7.09, 6.96, 4.31, 4.15, 3.97, 3.8 2, 3.72, 3.60-3.34, 3.31-3.10, 2.93, 2.54-2.26, 2.18-1.86, 1.80-1.58, 1.48-1.08, 1.02-0.80, 0.95.

Example 2(101)

(3R)-1-butyl-2,5-dioxo-3-((1R)-1-hydroxy-1-cyclohexylmethyl)-9-(4-(4-carboxy-2-chlorophenoxy)phenylmethyl)-1,4,9-triazaspiro[5.5]undecane hydrochloride

TLC: Rf 0.22 (chloroform:methanol=5:1);
NMR($CD_3OD$): δ 8.13, 7.94, 7.64, 7.13-7.08, 4.36, 4.15, 3.98, 3.72, 3.58-3.39, 3.32-3.18, 3.26, 2.6 2-2.28, 2.18-1.86, 1.80-1.56, 1.50-1.08, 1.04-0.80, 0.95.

Example 2(102)

(3R)-1-butyl-2,5-dioxo-3-((1R)-1-hydroxy-1-cyclohexylmethyl)-9-(4-(4-carboxyphenoxy)-3-chlorophenylmethyl)-1,4,9-triazaspiro[5.5]undecane hydrochloride

TLC: Rf 0.43 (chloroform:methanol=5:1);
NMR($CD_3OD$): δ 8.03, 7.85, 7.58, 7.25, 7.01, 4.39, 4.16, 4.02, 3.77, 3.60-3.42, 3.38-3.18, 3.26, 2.62-2.30, 2.19-1.88, 1.82-1.60, 1.52-1.16, 1.04-0.80, 0.96.

Example 2(103)

(3R)-1-butyl-2,5-dioxo-3-((1R)-1-hydroxy-1-cyclohexylmethyl)-9-(4-(3,5-dicarboxyphenoxy)phenylmethyl)-1,4,9-triazaspiro[5.5]undecane hydrochloride

TLC: Rf 0.54 (chloroform:methanol:acetic acid=20:4:1);
NMR($CD_3OD$): δ 8.42, 7.82, 7.66, 7.15, 4.36, 4.15, 3.98, 3.72, 3.62-3.42, 3.40-3.18, 2.68 - 2.38, 2.18-1.86, 1.84-1.60, 1.52-1.12, 1.08-0.80, 0.95.

Example 2(104)

(3R)-1-butyl-2,5-dioxo-3-((1R)-1-hydroxy-1-cyclohexylmethyl)-9-(4-(4-bromophenylmethyloxy)phenylmethyl)-1,4,9-triazaspiro[5.5]undecane hydrochloride

TLC: Rf 0.73 (chloroform:methanol=5:1);
NMR($CD_3OD$): δ 7.53, 7.47, 7.36, 7.09, 5.10, 4.28, 4.14, 3.96, 3.70, 3.5 6-3.34, 3.25, 3.17, 2.52-2.19, 2.18-1.88, 1.82-1.62, 1.48-1.10, 1.06-0.90, 0.95.

Example 2(105)

(3R)-1-butyl-2,5-dioxo-3-((1R)-1-hydroxy-1-cyclohexylmethyl)-9-(4-(4-fluorophenylmethylaminocarbonyl) phenylmethyl)-1,4,9-triazaspiro[5.5]undecane hydrochloride

TLC: Rf 0.62 (chloroform:methanol=5:1);
NMR($CD_3OD$): δ 7.96, 7.68, 7.40-7.32, 7.08-6.98, 4.55, 4.42, 4.15, 4.01, 3.88-3.58, 3.58-3.08, 3.25, 2.52-2.19, 2.18-1.84, 1.83-1.60, 1.50-1.10, 1.04-0.80, 0.95.

Example 2(106)

(3R)-1-butyl-2,5-dioxo-3-((1R)-1-hydroxy-1-cyclohexylmethyl)-9-(4-(4-methylaminocarbonyl-2-methylphenoxy) phenylmethyl)-1,4,9-triazaspiro[5.5]undecane hydrochloride

TLC: Rf 0.64 (chloroform:methanol=5:1);
NMR($CD_3OD$): δ 7.78, 7.66, 7.55, 7.03, 6.94, 4.34, 4.15, 3.99, 3.74 , 3.59-3.41, 3.26, 3.18, 2.91, 2.52-2.22, 2.27, 2.20-1.88, 1.83-1.60, 1.50-1.12, 1.06-0.80, 0.95.

Example 2(107)

(3R)-1-butyl-2,5-dioxo-3-((1R)-1-hydroxy-1-cyclohexylmethyl)-9-(4-(4-methylaminocarbonyl-2-chlorophenoxy) phenylmethyl)-1,4,9-triazaspiro[5.5]undecane hydrochloride

TLC: Rf 0.69 (chloroform:methanol=5:1);
NMR($CD_3OD$): δ 8.00, 7.78, 7.59, 7.13, 7.08, 4.35, 4.15, 4.00, 3.7 4, 3.58-3.42, 3.26, 3.21, 2.91, 2.58-2.28, 2.19-1.88, 1.82-1.62, 1.49-1.14, 1.08-0.80, 0.95.

Example 2(108)

(3R)-1-butyl-2,5-dioxo-3-((1R)-1-hydroxy-1-cyclohexylmethyl)-9-(4-(4-bromophenylmethylcarbonylamino) phenylmethyl)-1,4,9-triazaspiro[5.5]undecane hydrochloride

TLC: Rf 0.74 (dichloromethane:methanol=5:1);
NMR($CD_3OD$): δ 7.69, 7.51-7.46, 7.27, 4.30, 4.14, 3.97, 3.71, 3.67, 3.56-3.41, 3.28-3.15, 2.48-1.65, 1.39-1.15, 1.00-0.87, 0.94.

Example 2(109)

(3R)-1-butyl-2,5-dioxo-3-((1R)-1-hydroxy-1-cyclohexylmethyl)-9-(4-(4-methylaminocarbonylphenoxy)-3-chlorophenylmethyl)-1,4,9-triazaspiro[5.5]undecane hydrochloride

TLC: Rf 0.67 (chloroform:methanol=5:1);
NMR($CD_3OD$): δ 7.87-7.79, 7.55, 7.21, 7.01, 4.38, 4.16, 4.02, 3.77, 3.60-3.42, 3.26, 3.20, 2.91, 2.59-2.25, 2.20-1.85, 1.83-1.60, 1.51-1.08, 1.06-0.80, 0.96.

Example 2(110)

(3R)-1-butyl-2,5-dioxo-3-((1R)-1-hydroxy-1-cyclohexylmethyl)-9-(4-(pyridin-2-ylmethylaminocarbonyl)phenylmethyl)-1,4,9-triazaspiro[5.5]undecane dihydrochloride

TLC: Rf 0.62 (chloroform:methanol=5:1);
NMR($CD_3OD$): δ 8.78, 8.59, 8.10-7.94, 7.78, 4.92, 4.43, 4.15, 4.01, 3.75, 3.61-3.38, 3.38-3.19, 3.26, 2.60, 2.49-2.38, 2.16-1.87, 1.84-1.58, 1.50-1.08, 1.06-0.80, 0.94.

Example 2(111)

(3R)-1-butyl-2,5-dioxo-3-((1R)-1-hydroxy-1-cyclohexylmethyl)-9-(4-(4-carboxyphenoxy)-3-bromophenylmethyl)-1,4,9-triazaspiro[5.5]undecane hydrochloride

TLC: Rf 0.57 (chloroform:methanol=5:1);
NMR($CD_3OD$): δ 8.03, 7.99, 7.60, 7.20, 6.99, 4.35, 4.16, 3.98, 3.73, 3.59-3.40, 3.27, 3.22, 2.60-2.22, 2.08-1.88, 1.83-1.60, 1.50-1.08, 1.04-0.81, 0.95.

Example 2(112)

(3R)-1-butyl-2,5-dioxo-3-((1R)-1-hydroxy-1-cyclohexylmethyl)-9-(4-(1,2,4-triazol-1-yl)phenylmethyl)-1,4,9-triazaspiro[5.5]undecane dihydrochloride

TLC: Rf 0.70 (chloroform:methanol=5: 1);
NMR($CD_3OD$): δ 9.85, 8.63, 8.03, 7.87, 4.46, 4.15, 4.03, 3.78, 3.59-3.38, 3.38-3.20, 3.26, 2.60, 2.50-2.41, 2.16-1.84, 1.82-1.59, 1.50-1.08, 1.06-0.80, 0.94.

Example 2(113)

(3R)-1-butyl-2,5-dioxo-3-((1R)-1-hydroxy-1-cyclohexylmethyl)-9-(4-(4-carboxyphenoxy)-3-trifluoromethylphenylmethyl)-1,4,9-triazaspiro[5.5]undecane hydrochloride

TLC: Rf 0.49 (chloroform:methanol=5:1);
NMR($CD_3OD$): δ 8.08, 8.04, 7.83, 7.19, 7.12, 4.44, 4.16, 4.03, 3.7 8, 3.60-3.40, 3.26, 3.20, 2.58-2.26, 2.20-1.88, 1.82-1.60, 1.49-1.12, 1.06-0.80, 0.95.

Example 2(114)

(3R)-1-butyl-2,5-dioxo-3-((1R)-1-hydroxy-1-cyclohexylmethyl)-9-(1,3-dimethyl-5-chloropyrazol-4-ylmethyl)-1,4,9-triazaspiro[5.5]undecane dihydrochloride

TLC: Rf 0.59 (chloroform:methanol=9:1);
NMR($CD_3OD$): δ 4.22, 4.15, 4.02, 3.82, 3.75, 3.52-3.48, 3.25, 3.17, 2.52-2.38, 2.38-2.19, 2.32, 2.1 8-1.84, 1.82-1.60, 1.50-1.10, 1.05-0.80, 0.96.

Example 2(115)

(3R)-1-butyl-2,5-dioxo-3-((1R)-1-hydroxy-1-cyclohexylmethyl)-9-(4-((1-(4-chlorophenyl)-1-iminomethyl)aminocarbonyl)phenylmethyl)-1,4,9-triazaspiro[5.5]undecane hydrochloride

TLC: Rf 0.57 (chloroform:methanol=9:1);
NMR($CD_3OD$): δ 8.17, 7.97, 7.89, 7.74, 4.48, 4.15, 4.03, 3.78, 3.60-3.38 , 3.38-3.29, 3.25, 2.63, 2.53-2.38, 2.14-1.83, 1.81-1.55, 1.50-1.07, 1.06-0.80, 0.94.

Example 2(116)

(3R)-1-butyl-2,5-dioxo-3-((1R)-1-hydroxy-1-cyclohexylmethyl)-9-(4-(4-chlorophenylmethyloxy)-3-methoxyphenylmethyl)-1,4,9-triazaspiro[5.5]undecane hydrochloride

TLC: Rf 0.68 (chloroform:methanol=9:1);
NMR($CD_3OD$): δ 7.43, 7.36, 7.21, 7.04, 5.12, 4.27, 4.15, 3.97, 3.91, 3.71, 3.58- 3.34, 3.26, 3.19, 2.52-2.24, 2.16-1.84, 1.84-1.60, 1.50-1.08, 1.04-0.80, 0.95.

Example 2(117)

(3R)-1-butyl-2,5-dioxo-3-((1R)-1-hydroxy-1-cyclohexylmethyl)-9-(4-(4-methylaminocarbonylphenoxy)-3-trifluoromethylphenylmethyl)-1,4,9-triazaspiro[5.5]undecane hydrochloride

TLC: Rf 0.62 (chloroform:methanol=9:1);
NMR($CD_3OD$): δ 8.02, 7.89, 7.80, 7.18-7.08, 4.44, 4.16, 4.03, 3.78, 3.58-3.40, 3.26, 3.18, 2.92, 2.54-2.22, 2.20-1.88, 1.80-1.60, 1.49-1.08, 1.02-0.80, 0.95.

Example 2(118)

(3R)-1-butyl-2,5-dioxo-3-((1R)-1-hydroxy-1-cyclohexylmethyl)-9-(4-(4-carboxyphenylcarbonylaminomethyl) phenylmethyl)-1,4,9-triazaspiro[5.5]undecane hydrochloride

TLC: Rf 0.60 (n-butanol:acetic acid:water=4:1:1);
NMR($CD_3OD$): δ 8.10, 7.93, 7.56, 7.49, 4.62, 4.33, 4.14, 3.97, 3.72, 3.59-3.38, 3.30-3.16, 2.57-2.27, 2.14-1.91, 1.79-1.62, 1.43-1.13, 1.01-0.82, 0.93.

Example 2(119)

(3R)-1-butyl-2,5-dioxo-3-((1R)-1-hydroxy-1-cyclohexylmethyl)-9-(4-(4-methylaminocarbonylphenylcarbonylaminomethyl)phenylmethyl)-1,4,9-triazaspiro[5.5]undecane hydrochloride

TLC: Rf 0.52 (ethyl acetate:methanol=2:1);
NMR($CD_3OD$): δ 7.93, 7.89, 7.53, 7.49, 4.61, 4.34, 4.14, 4.00, 3.73, 3.54-3.38, 3.30-3.05, 2.92, 2.49-1.88, 1.78-1.61, 1.46-1.12, 1.00-0.81, 0.94.

Example 2(120)

(3R)-1-butyl-2,5-dioxo-3-((1R)-1-hydroxy-1-cyclohexylmethyl)-9-(1-(4-carboxyphenyl)-2,5-dimethylpyrrol-3-ylmethyl)-1,4,9-triazaspiro[5.5]undecane hydrochloride

TLC: Rf 0.27 (chloroform:methanol=5:1);
NMR($CD_3OD$): δ 8.18, 7.36, 6.09, 4.19, 4.16, 3.95, 3.70, 3.59-3.42, 3.32-3.08, 2.56-2.20, 2.20-1.88, 2.06, 2.02, 1.82-1.60, 1.50-1.06, 1.04-0.80, 0.97.

Example 2(121)

(3R)-1-butyl-2,5-dioxo-3-((1R)-1-hydroxy-1-cyclohexylmethyl)-9-(4-(2-fluoro-6-chlorophenylmethyloxy) phenylmethyl)-1,4,9-triazaspiro[5.5]undecane hydrochloride

TLC: Rf 0.76 (chloroform:methanol=5:1);
NMR($CD_3OD$): δ 7.50, 7.41, 7.33, 7.16-7.14, 5.23, 4.30, 4.15, 3.97, 3.71, 3.57-3.36, 3.34-3.10, 2.52-2.20, 2.16-1.88, 1,82-1.60, 1.50-1.06, 1.04-0.80,0.95.

Example 2(122)

(3R)-1-butyl-2,5-dioxo-3-((1R)-1-hydroxy-1-cyclohexylmethyl)-9-(3-(3-carboxyphenyl)phenylmethyl)-1,4,9-triazaspiro[5.5]undecane hydrochloride

TLC: Rf 0.39 (chloroform:methanol=5:1);
NMR($CD_3OD$): 5 8.33, 8.05, 7.98-7.86, 7.82, 7.65-7.54, 4.46, 4.15, 4.06, 3.80, 3.58-3.38, 3.32-3.10, 2.54-2.24, 2.18-1.88, 1.80-1.58, 1.50-1.08, 1.04-0.80, 0.94.

Example 2(123)

(3R)-1-butyl-2,5-dioxo-3-((1R)-1-hydroxy-1-cyclohexylmethyl)-9-(2-oxo-1,2-dihydropyridin-3-ylmethyl)-1,4,9-triazaspiro[5.5]undecane hydrochloride

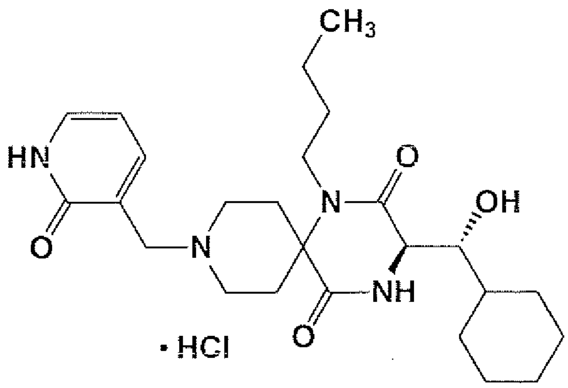

TLC: Rf 0.33 (chloroform:methanol:acetic acid=20:4:1);
NMR($CD_3OD$): δ 7.85, 7.58, 6.46, 4.23, 4.15, 4.05, 3.78, 3.60-3.42, 3.25, 3.18, 2.58-2.38, 2.30, 2.18-1.88, 1.82-1.60, 1.50-1.06, 1.04-0.80,0.96.

Example 2(124)

(3R)-1-butyl-2,5-dioxo-3-((1R)-1-hydroxy-1-cyclohexylmethyl)-9-(1-(4-carboxyphenyl)-3,5-dimethylpyrazol-4-ylmethyl)-1,4,9-triazaspiro[5.5]undecane dihydrochloride

TLC: Rf 0.74 (chloroform:methanol:acetic acid=20:4:1);
NMR($CD_3OD$): δ 8.19, 7.62, 4.32, 4.17, 4.04, 3.88-3.46, 3.27, 3.21, 2.60-2.28, 2.44, 2.39, 2.21-1.61, 1.60-1.08, 1.08-0.80, 0.96.

Example 2(125)

(3R)-1-butyl-2,5-dioxo-3-((1R)-1-hydroxy-1-cyclohexylmethyl)-9-(3-(4-carboxyphenyl)phenylmethyl)-1,4,9-triazaspiro[5.5]undecane hydrochloride

TLC: Rf 0.54 (chloroform:methanol=5:1);
NMR($CD_3OD$): δ 8.12, 7.94, 7.86-7.78, 7.64-7.56, 4.45, 4.15, 4.05, 3.80, 3.60-3.42, 3.26, 3.21, 2.56-2.26, 2.18-1.88, 1.84-1.60, 1.50-1.08, 1.04-0.80, 0.94.

Example 2(126)

(3R)-1-butyl-2,5-dioxo-3-((1R)-1-hydroxy-1-cyclohexylmethyl)-9-(4-(4-carboxy-2,6-dimethoxyphenoxy)phenylmethyl)-1,4,9-triazaspiro[5.5]undecane hydrochloride

TLC: Rf 0.54 (chloroform:methanol=5:1);

NMR($CD_3OD$): δ 7.44, 7.43, 6.88, 4.29, 4.15, 3.98, 3.80, 3.71, 3.56-3.32, 3.25, 3.13, 2.50-1.88, 1.80-1.60, 1.46-1.08, 1.02-0.79, 0.95.

Example 2(127)

(3R)-1-butyl-2,5-dioxo-3-((1R)-1-hydroxy-1-cyclohexylmethyl)-9-(4-(3-carboxyphenoxy)phenylmethyl)-1,4,9-triazaspiro[5.5]undecane hydrochloride

TLC: Rf 0.64 (dichloromethane:methanol=5:1);
NMR($CD_3OD$): δ 7.83, 7.60, 7.56, 7.51, 7.19, 7.12, 4.35, 4.16, 4.01, 3.75, 3.55-3.10, 2.50-1.18, 1.10-0.87, 0.95.

Example 2(128)

(3R)-1-butyl-2,5-dioxo-3-((1R)-1-hydroxy-1-cyclohexylmethyl)-9-(4-(4-(1-(hydroxyimino)-1-aminomethyl)phenoxy) phenylmethyl)-1,4,9-triazaspiro[5.5]undecane dihydrochloride

TLC: Rf 0.53 (dichloromethane:methanol=5:1);
NMR($CD_3OD$): δ 7.67, 7.56, 7.12, 7.07, 4.30, 4.15, 3.93, 3.69, 3.51-3.17, 2.46-1.15, 1.00-0.87, 0.95.

Example 2(129)

(3R)-1-butyl-2,5-dioxo-3-((1R)-1-hydroxy-1-cyclohexylmethyl)-9-(4-(4-carboxy-3-hydroxyphenoxy)phenylmethyl)-1,4,9-triazaspiro[5.5]undecane hydrochloride

TLC: Rf 0.80 (chloroform:methanol:acetic acid=20:4:1);
NMR($CD_3OD$): δ 7.86, 7.63, 7.19, 6.54, 6.44, 4.37, 4.16, 4.01, 3.76 , 3.60-3.41, 3.26, 3.23, 2.60-2.28, 2.18-1.88, 1.83-1.60, 1.49-1.06, 1.06-0.80, 0.95.

Example 2(130)

(3R)-1-butyl-2,5-dioxo-3-((1R)-1-hydroxy-1-cyclohexylmethyl)-9-(4-(4-(5-thioxo-4,5-dihydro-1,2,4-oxadiazol-3-yl) phenoxy)phenylmethyl)-1,4,9-triazaspiro[5.5]undecane hydrochloride

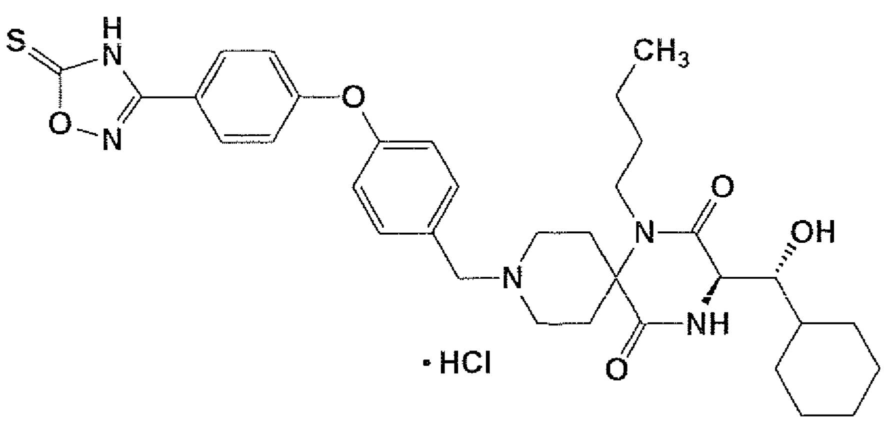

TLC: Rf 0.55 (chloroform:methanol=5:1);
NMR($CD_3OD$): δ 7.85, 7.64, 7.22-7.16, 4.37, 4.16, 4.01, 3.76, 3.58-3.38, 3.26, 3.22, 2.58-2.26, 2.18-1.84, 1.82-1.58, 1.52-1.06, 1.04-0.80, 0.95.

Example 2(131)

(3R)-1-butyl-2,5-dioxo-3-((1R)-1-hydroxy-1-cyclohexylmethyl)-9-(4-(4-carboxyphenylcarbonylamino)phenylmethyl)-1,4,9-triazaspiro[5.5]undecane hydrochloride

TLC: Rf 0.23 (ethyl acetate:methanol=2:1);
NMR($CD_3OD$): δ 8.15, 8.01, 7.88, 7.58, 4.36, 4.15, 4.01, 3.75, 3.56-3.45, 3.30- 3.17, 2.48-1.14, 1.00-0.87, 0.95.

Example 2(132)

(3R)-1-butyl-2,5-dioxo-3-((1R)-1-hydroxy-1-cyclohexylmethyl)-9-(4-(4-carboxyphenylaminocarbonyl)phenylmethyl)-1,4,9-triazaspiro[5.5]undecane hydrochloride

TLC: Rf 0.26 (dichloromethane:methanol-5:1);
NMR($CD_3OD$): δ 8.07, 8.03, 7.85, 7.72, 4.46, 4.16, 4.04, 3.79, 3.55-3.14, 2.51-1.15, 1.00-0.89, 0.95.

Example 2(133)

(3R)-1-butyl-2,5-dioxo-3-((1R)-1-hydroxy-1-cyclohexylmethyl)-9-(4-(4-carboxy-2-methoxyphenylaminocarbonyl) pbenylmethyl)-1,4,9-triazaspiro[5.5]undecane hydrochloride

TLC: Rf 0.54 (dichloromethane:methanol=5:1);
NMR($CD_3OD$): δ 8.27, 8.07, 7.74-7.69, 4.47, 4.16, 4.06, 3.99, 3.80, 3.50-3.07, 2.52-1.92, 1.76-1.18, 1.00-0.90, 0.96.

Example 2(134)

(3R)-1-butyl-2,5-dioxo-3-((1R)-1-hydroxy-1-cyclohexylmethyl)-9-(4-(4-(tetrazol-5-yl)phenoxy)phenylmethyl)-1,4,9-triazaspiro[5.5]undecane dihydrochloride

TLC: Rf 0.25 (chloroform:methanol=5:1);
NMR($CD_3OD$): δ 8.04, 7.66, 7.25-7.15, 4,36, 4.15, 3.98, 3.75, 3.60-3.40, 3.40-3.18, 2.66-2.34, 2.18-1.88, 1.84-1.60, 1.50-1.06, 1.06-0.80, 0.95.

Example 2(135)

(3R)-1-butyl-2,5-dioxo-3-((1R)-1-hydroxy-1-cyclohexylmethyl)-9-(4-(4-(5-oxo-4,5-dihydro-1,2,4-thiadiazol-3-yl) phenoxy)phenylmethyl)-1,4,9-triazaspiro[5.5]undecane hydrochloride

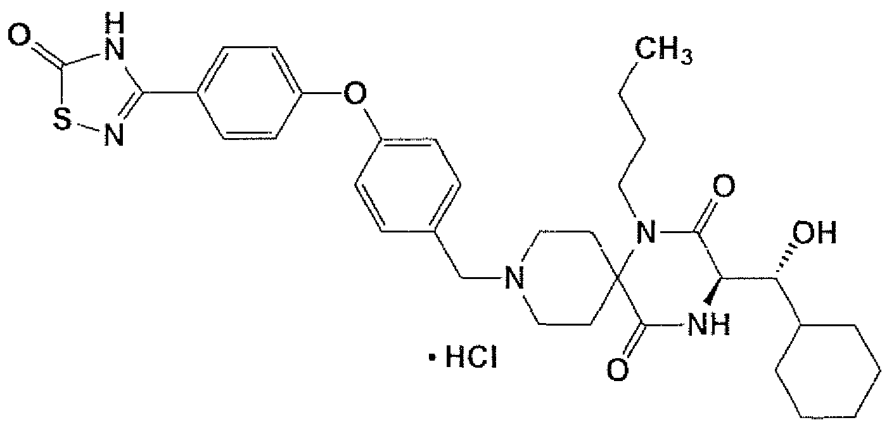

TLC: Rf 0.74 (chloroform:methanol=5:1);
NMR($CD_3OD$): δ 7.91, 7.61, 7.22-7.10, 4.37, 4.16, 3.99, 3.74, 3.58-3.38, 3.38-3.08, 3.24, 2.54-1.84, 1.82-1.58, 1.50-1.10, 1.06-0.80, 0.96.

Example 2(136)

(3R)-1-butyl-2,5-dioxo-3-((1R)-1-hydroxy-1-cyclohexylmethyl)-9-(4-(4-(5-oxo-4,5-dihydro-1,2,4-oxadiazol-3-yl) phenoxy)phenylmethyl)-1,4,9-triazaspiro[5.5]undecane hydrochloride

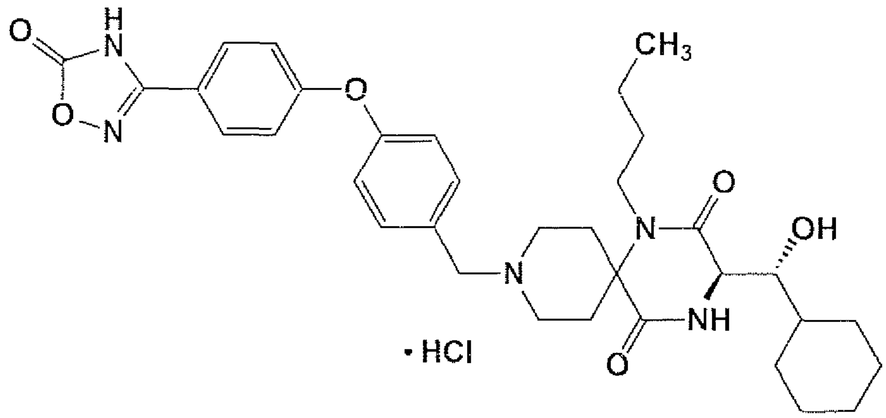

TLC: Rf 0.57 (chloroform:methanol=5:1);
NMR($CD_3OD$): δ 7.80, 7.63, 7.24-7.14, 4.37, 4.16, 4.00, 3.75, 3.58-3.40, 3.26, 3.19, 2.56-2.20, 2.19-1.88, 1.82-1.60, 1.50-1.08, 1.04-0.80, 0.96.

Example 2(137)

(3R)-1-butyl-2,5-dioxo-3-((1R)-1-hydroxy-1-cyclohexylmethyl)-9-(3-(4-carboxyphenylaminocarbonyl)phenylmethyl)-1,4,9-triazaspiro[5.5]undecane hydrochloride

TLC: Rf 0.23 (dichloromethane:methanol=5:1);
NMR($CD_3OD$): δ 8.19, 8.08, 8.03, 7.88, 7.78, 7.67, 4.48, 4.15, 4.06, 3.82, 3.60-3.45, 3.30-3.15, 2.51-1.14, 1.00-0.87, 0.95.

Example 2(138)

(3R)-1-butyl-2,5-dioxo-3-((1R)-1-hydroxy-1-cyclohexylmethyl)-9-(4-(3-carboxyphenylaminocarbonyl)phenylmethyl)-1,4,9-triazaspiro[5.5]undecane hydrochloride

TLC: Rf 0.15 (dichloromethane:methanol=5:1);
NMR($CD_3OD$): δ 8.38, 8.07, 7.97, 7.83, 7.74, 7.48, 4.46, 4.15, 4.05, 3.80, 3.60-3.45, 3.30-3.17, 2.50-1.14, 1.00-0.86, 0.95.

Example 2(139)

(3R)-1-butyl-2,5-dioxo-3-((1R)-1-hydroxy-1-cyclohexylmethyl)-9-(4-(4-carboxyphenylmethylcarbonylamino) phenylmethyl)-1,4,9-triazaspiro[5.5]undecane hydrochloride

TLC: Rf 0.15 (dichloromethane:methanol=5:1);
NMR($CD_3OD$): δ 7.98, 7.71, 7.48, 7.45, 4.31, 4.14, 3.99, 3.78, 3.63, 3.53-3.07, 2.48-1.14, 1.00-0.87, 0.94.

Example 2(140)

(3R)-1-butyl-2,5-dioxo-3-((1R)-1-hydroxy-1-cyclohexylmethyl)-9-(4-(2-carboxy-4-methoxyphenylaminocarbonyl) phenylmethyl)-1,4,9-triazaspiro[5.5]undecane hydrochloride

TLC: Rf 0.21 (dichloromethane:methanol=5:1);

NMR($CD_3OD$): δ 8.69, 8.10, 7.76, 7.66, 7.23, 4.46, 4.15, 4.06, 3.84, 3.80, 3.60-3.10, 2.50-1.14, 1.00-0.85, 0.95.

Example 2(141)

(3R)-1-butyl-2,5-dioxo-3-((1R)-1-hydroxy-1-cyclohexylmethyl)-9-(4-(4-carboxy-2-methoxyphenylmethyl) phenylmethyl)-1,4,9-triazaspiro[5.5]undecane hydrochloride

TLC: Rf 0.53 (chloroform:methanol:acetic acid=10:1:1);
NMR($CD_3OD$): δ 7.57-7.54, 7.44, 7.34, 7.22, 4.29, 4.14, 4.04, 3.96, 3.85, 3.71, 3.54-3.37, 3.27, 3.15, 2.46-2.20, 2.12-1.90, 1.79-1.62, 1.45-1.11, 0.99-0.81, 0.93.
amorphous
softening point: about 190-191°C

Example 2(142)

(3R)-1-butyl-2,5-dioxo-3-((1R)-1-hydroxy-1-cyclohexylmethyl)-9-(4-(4-carboxy-2-methoxyphenylcarbonylaminomethyl)phenylmethyl)-1,4,9-triazaspiro[5.5]undecane hydrochloride

TLC: Rf 0.24 (chloroform:methanol:acetic acid=10:1:1);
NMR($CD_3OD$): δ 8.98, 7.89, 7.74, 7.69, 7.55-7.49, 4.65, 4.35, 4.14, 4.01, 3.97, 3.74, 3.59-3.36, 3.30-3.10, 2.50-2.20, 2.16-1.89, 1.81-1.62, 1.43-1.13, 1.00-0.82, 0.94.

Example 2(143)

(3R)-1-butyl-2,5-dioxo-3-((1R)-1-hydroxy-1-cyclohexylmethyl)-9-(4-(2,4-dimethoxyphenoxy)phenylmethyl)-1,4,9-triazaspiro[5.5]undecane hydrochloride

TLC: Rf 0.42 (dichloromethane:methanol=10:1);
NMR($CD_3OD$): δ 7.43, 6.96, 6.87, 6.68, 6.54, 4.28, 4.15, 3.96, 3.81, 3.72, 3.71, 3.60-3.10, 2.50-1.85, 1.80-1.60, 1.50-1.10, 0.95, 0.94.

Example 2(144)

(3R)-1-butyl-2,5-dioxo-3-((1R)-1-hydroxy-1-cyclohexylmethyl)-9-(4-(4-carboxy-2-methoxyphenylmethyloxy) phenylmethyl)-1,4,9-triazaspiro[5.5]undecane hydrochloride

TLC: Rf 0.67 (chloroform:methanol:acetic acid=10:1:1);
NMR($CD_3OD$): δ 7.64, 7.62, 7.50, 7.49, 7.09, 5.20, 4.28, 4.14, 3.95, 3.94, 3.70, 3.56-3.38, 3.30-3.15, 2.52-2.26, 2.13-1.88, 1.79-1.62, 1.45-1.12, 1.01-0.82, 0.95.

Example 2(145)

(3R)-1-butyl-2,5-dioxo-3-((1R)-1-hydroxy-1-cyclohexylmethyl)-9-(4-(N-(4-carboxyphenyl)methyl-N-methylaminocarbonyl)phenylmethyl)-1,4,9-triazaspiro[5.5]undecane hydrochloride

TLC: Rf 0.34 (chloroform:methanol=5:1);
NMR($CD_3OD$): δ 8.12-7.96, 7.74-7.20, 4.70-4.24, 4.14, 3.92, 3.66, 3.60-2.80, 2.60-2.16, 2.16-1.84, 1.82-1.56, 1.50-1.04, 1.04-0.80.

Example 2(146)

(3R)-1-butyl-2,5-dioxo-3-((1R)-1-hydroxy-1-cyclohexylmethyl)-9-(4-(4-cyano-2-methoxyphenoxy)phenylmethyl)-1,4,9-triazaspiro[5.5]undecane hydrochloride

TLC: Rf 0.69 (chloroform:methanol=5:1);
NMR($CD_3OD$): δ 7.53, 7.50, 7.36, 7.13, 7.02, 4.33, 4.15, 3.98, 3.8 3, 3.73, 3.60-3.38, 3.26, 3.18, 2.52-2.22, 2.18-1.88, 1.80-1.60, 1.50-1.08, 1.06-0.80, 0.95.

Example 2(147)

(3R)-1-butyl-2,5-dioxo-3-((1R)-1-hydroxy-1-cyclohexylmethyl)-9-(4-(4-(2-oxido-3H-1,2,3,5-oxathiadiazol-4-yl)phenoxy)phenylmethyl)-1,4,9-triazaspiro[5.5]undecane hydrochloride

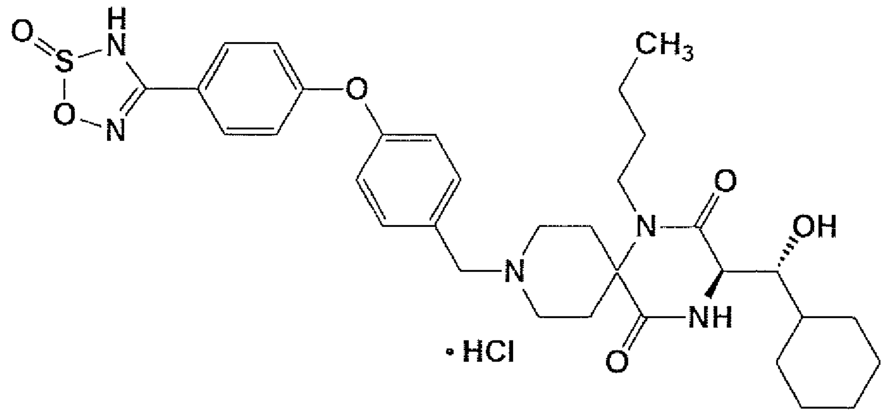

TLC: Rf 0.55 (chloroform:methanol=5:1);
NMR($CD_3OD$): δ 7.85, 7.62, 7.24-7.12, 4.37, 4.16, 4.01, 3.76, 3.58-3.42, 3.26, 3.21, 2.56-2.26, 2.20-1.88, 1.86-1.60, 1.48-1.12, 1.08-0.80, 0.96.

Example 2(148)

(3R)-1-butyl-2,5-dioxo-3-((1R)-1-hydroxy-1-cyclohexylmethyl)-9-(4-(4-carboxy-2-methoxyphenylmethylaminocarbonyl)phenylmethyl)-1,4,9-triazaspiro[5.5]undecane hydrochloride

TLC: Rf 0.47 (chloroform:methanol:acetic acid=10:1:1);
NMR($CD_3OD$): δ 8.97, 7.98, 7.70, 7.62-7.59, 7.33, 4.63, 4.43, 4.15, 4.02, 3.93, 3.76, 3.57-3.39, 3.30-3.16, 2.56-2.29, 2.16-1.88, 1.80-1.61, 1.47-1.10, 1.01-0.81, 0.95.

Example 2(149)

(3R)-1-butyl-2,5-dioxo-3-((1R)-1-hydroxy-1-cyclohexylmethyl)-9-(4-(4-hydroxy-2-methoxyphenoxy)phenylmethyl)-1,4,9-triazaspiro[5.5]undecane hydrochloride

TLC: Rf 0.29 (dichloromethane:methanol=10:1);
NMR($CD_3OD$): δ 7.42, 6.87, 6.85, 6.56, 6.39, 4.27, 4.15, 3.96, 3.71, 3.69, 3.60-3.10, 2.50-1.96, 1.80-1.60, 1.50-1.10, 1.05-0.90, 0.95.

Example 2(150)

(3R)-1-butyl-2,5-dioxo-3-((1R)-1-hydroxy-1-cyclohexylmethyl)-9-(4-(4-(N,N,-dimethylaminocarbonyl)-2-methoxyphenoxy)phenylmethyl)-1,4,9-triazaspiro[5.5]undecane hydrochloride

TLC: Rf 0.63 (chloroform:methanol=5:1);
NMR($CD_3OD$): δ 7.48, 7.20, 7.16, 7.04, 6.96, 4.31, 4.15, 3.99, 3.79, 3.72, 3.62-3.38, 3.25, 3.18, 3.11, 3.06, 2.52-1.88, 1.86-1.62, 1.54-1.08, 1.06-0.80, 0.95.

Example 2(151)

(3R)-1-butyl-2,5-dioxo-3-((1R)-1-hydroxy-1-cyclohexylmethyl)-9-(4-(4-carboxy-3-methylphenoxy)phenylmethyl)-1,4,9-triazaspiro[5.5]undecane hydrochloride

TLC: Rf 0.60 (chloroform:methanol=5:1);
NMR($CD_3OD$): δ 7.97, 7.60, 7.15, 6.91, 6.87, 4.36, 4.15, 4.01, 3.7 6, 3.60-3.40, 3.26, 3.20, 2.56, 2.56-2.25, 2.18-1.88, 1.86-1.60, 1.56-1.13, 1.06-0.80, 0.95.

Example 2(152)

(3R)-1-butyl-2,5-dioxo-3-((1R)-1-hydroxy-1-cyclohexylmethyl)-9-(4-(4-methylsulfonylamino-2-methoxyphenoxy) phenylmethyl)-1,4,9-triazaspiro[5.5]undecane hydrochloride

TLC: Rf 0.45 (chloroform: methanol=10:1);
NMR($CD_3OD$): δ 7.45, 7.04, 7.03, 6.92, 6.87, 4.29, 4.15, 3.98, 3.74, 3.70, 3.55-3.39, 3.30-3.09, 2.99, 2.50-1.88, 1.81-1.61, 1.46-1.14, 1.03-0.82, 0.95.

Example 2(153)

(3R)-1-butyl-2,5-dioxo-3-((1R)-1-hydroxy-1-cyclohexylmethyl)-9-(4-(4-carboxy-2-ethoxyphenoxy)phenylmethyl)-1,4,9-triazaspiro[5.5]undecane hydrochloride

TLC: Rf 0.65 (dichloromethane:methanol=5:1);
NMR($CD_3OD$): δ 7.72, 7.66, 7.49, 7.09, 7.01, 4.33, 4.15, 4.07, 4.00, 3.74, 3.54-3.10, 2.51-1.15, 1.24, 1.03-0.90, 0.95.
amorphous
softening point: about 180-198°C

Example 2(154)

(3R)-1-butyl-2,5-dioxo-3-((1R)-1-hydroxy-1-cyclohexylmethyl)-9-(4-(4-carboxy-3-methoxyphenoxy)phenylmethyl)-1,4,9-triazaspiro[5.5]undecane hydrochloride

TLC: Rf 0.71 (chloroform:methanol=5:1);
NMR($CD_3OD$): δ 7.88, 7.62, 7.19, 6.80, 6.57, 4.37, 4.15, 4.00, 3.8 6, 3.75, 3.60-3.40, 3.26, 3.24, 2.59-2.24, 2.18-1.87, 1.82-1.62, 1.50-1.08, 1.06-0.80, 0.95.

Example 2(155)

(3R)-1-butyl-2,5-dioxo-3-((1R)-1-hydroxy-1-cyclohexylmethyl)-9-(4-(4-methylaminocarbonyl-3-hydroxyphenoxy) phenylmethyl)-1,4,9-triazaspiro[5.5]undecane hydrochloride

TLC: Rf 0.82 (chloroform:methanol=5:1);
NMR($CD_3OD$): δ 7.72, 7.61, 7.17, 6.52, 6.44, 4.36, 4.15, 4.00, 3.7 5, 3.60-3.40, 3.26, 3.20, 2.90, 2.56-2.24, 2.18-1.88, 1.82-1.60, 1.52-1.07, 1.06-0.80, 0.95.

Example 2(156)

(3R)-1-butyl-2,5-dioxo-3-((1R)-1-hydroxy-1-cyclohexylmethyl)-9-(4-(3,5-dimethoxyphenoxy)phenylmethyl)-1,4,9-triazaspiro[5.5]undecane hydrochloride

TLC: Rf 0.64 (dichloromethane:methanol=10:1);
NMR($CD_3OD$): δ 7.53, 7.08, 6.31, 6.17, 4.33, 4.15, 3.99, 3.74, 3.74, 3.58-3.32, 3.30-3.08, 2.54-2.20, 2.18-1.83, 1.82-1.55, 1.50-1.05, 1.05-0.80, 0.95.

Example 2(157)

(3R)-1-butyl-2,5-dioxo-3-((1R)-1-hydroxy-1-cyclohexylmethyl)-9-(4-(4-methylcarbonylamino-2-methoxyphenoxy) phenylmethyl)-1,4,9-triazaspiro[5.5]undecane hydrochloride

TLC: Rf 0.36 (chloroform:methanol=10:1);
NMR($CD_3OD$): δ 9.93, 7.51, 7.44, 7.08, 6.98, 6.91, 4.29, 4.15, 3.98, 3.73, 3.71, 3.55-3.40, 3.27, 3.15, 2.49-1.90, 2.13, 1.80-1.62, 1.47-1.16, 1.01-0.85, 0.95.

Example 2(158)

(3R)-1-butyl-2,5-dioxo-3-((1R)-1-hydroxy-1-cyclohexylmethyl)-9-(4-(4-(t-butyl)phenoxy)phenylmethyl)-1,4,9-triazaspiro[5.5]undecane hydrochloride

TLC: Rf 0.79 (ethyl acetate:methanol=10:1);
NMR($CD_3OD$): δ 7.49-7.45, 7.43, 7.03, 6.96, 4.31, 4.15, 3.98, 3.71, 3.56-3.40, 3.32-3.08, 2.55-2.30, 2.15-1.90, 1.82-1.60, 1.50-1.04, 1.33, 0.95, 1.00-0.82.

Example 2(159)

(3R)-1-butyl-2,5-dioxo-3-((1R)-1-hydroxy-1-cyclohexylmethyl)-9-(4-(3-carboxy-2-propynyloxy)phenylmethyl) phenylmethyl)-1,4,9-triazaspiro[5.5]undecane hydrochloride

TLC: Rf 0.40 (n-butanol:acetic acid:water=4:2:1);
NMR($CD_3OD$): δ 7.51, 7.11, 4.97, 4.30, 4.15, 3.98, 3.73, 3.56-3.38, 3.30-3.09, 2.52-2.18, 2.18-1.88, 1.86-1.60, 1.50-1.06, 1.05-0.80, 0.95.

Example 2(160)

(3R)-1-butyl-2,5-dioxo-3-((1R)-1-hydroxy-1-cyclohexylmethyl)-9-(4-(2-methoxyphenoxy)phenylmethyl)-1,4,9-triazaspiro[5.5]undecane hydrochloride

TLC: Rf 0.67 (dichloromethane:methanol=10:1);
NMR($CD_3OD$): δ 7.45, 7.24, 7.13, 7.08-6.94, 6.90, 4.29, 4.15, 3.97, 3.75, 3.73, 3.58-3.39, 3.30-3.10, 2.52-2.20, 2.17-1.87, 1.82-1.60, 1.50-1.06, 1.04-0.78, 0.95.

Example 2(161)

(3R)-1-butyl-2,5-dioxo-3-((1R)-1-hydroxy-1-cyclohexylmethyl)-9-(4-(4-trifluoromethoxyphenoxy)phenylmethyl)-1,4,9-triazaspiro[5.5]undecane hydrochloride

TLC: Rf 0.77 (ethyl acetate:methanol=10:1);
NMR($CD_3OD$): δ 7.53, 7.31, 7.11, 4.30, 4.15, 3.94, 3.70, 3.53-3.34, 3.25-3.05, 2.49-1.91, 1.84-1.60, 1.50-1.21, 0.96, 1.05-0.80.

Example 2(162)

(3R)-1-butyl-2,5-dioxo-3-((1R)-1-hydroxy-1-cyclohexylmethyl)-9-(4-(4-carboxy-2,6-dimethylphenoxy)phenylmethyl)-1,4,9-triazaspiro[5.5]undecane hydrochloride

TLC: Rf 0.68 (ethyl acetate:methanol=4:1);
NMR($CD_3OD$): δ 7.82, 7.55, 6.84, 4.29, 4.15, 4.05-3.85, 3.75-3.60, 3.58-3.38, 3.35-3.20, 2.68-2.50, 2.48-2.32 2.17. 2.20-2.02, 1.71, 1.50-1.20, 0.94, 1.05-0.80.
amorphous
softening point: about 180-194°C

Example 2(163)

(3R)-1-butyl-2,5-dioxo-3-((1R)-1-hydroxy-1-cyclohexylmethyl)-9-(4-(4-bromophenoxy)phenylmethyl)-1,4,9-triazaspiro[5.5]undecane hydrochloride

TLC: Rf 0.85 (chloroform:methanol=5:1);
NMR($CD_3OD$): δ 7.60-7.50, 7.09, 6.97, 4.34, 4.15, 3.99, 3.74, 3.58-3.38, 3.26, 3.19, 2.52-2.22, 2.18-1.88, 1.82-1.62, 1.50-1.06, 1.04-0.80, 0.95.

Example 2(164)

(3R)-1-butyl-2,5-dioxo-3-((1R)-1-hydroxy-1-cyclohexylmethyl)-9-(4-(2-carboxy-4-methoxyphenoxy)phenylmethyl)-1,4,9-triazaspiro[5.5]undecane hydrochloride

TLC: Rf 0.62 (chloroform:methanol=5:1);
NMR($CD_3OD$): δ 7.48-7.40, 7.17, 7.05, 6.91, 4.29, 4.15, 3.97, 3.85, 3.72, 3.58-3.38, 3.26, 3.18, 2.52-2.19, 2.18-1.84, 1.84-1.58, 1.50-1.08, 1.04-0.80, 0.95.

Example 2(165)

(3R)-1-butyl-2,5-dioxo-3-((1R)-1-hydroxy-1-cyclohexylmethyl)-9-(4-(2-carboxyphenoxy)phenylmethyl)-1,4,9-triazaspiro[5.5]undecane hydrochloride

TLC: Rf 0.41 (dichloromethane:methanol=5:1);
NMR($CD_3OD$): δ 7.95, 7.58, 7.48, 7.31, 7.07, 6.99, 4.31, 4.15, 3.99, 3.74, 3.54-3.09, 2.50-1.15, 1.00-0.87, 0.95.

Example 3(1) - Example 3(207)

By the same procedure as described in Example 2 using the corresponding aldehyde derivatives respectively instead of N-(4-formylphenyl)methanesulfonamide and using the corresponding amine derivatives respectively instead of the compound prepared in Reference Example 2, the following compounds of the present invention were obtained.

Example 3(1)

(3R)-1-ethyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

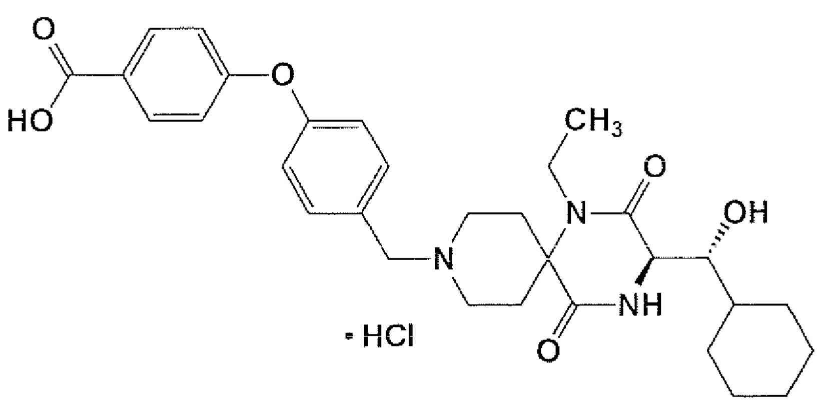

TLC: Rf 0.66 (dichloromethane:methanol=4: 1);
NMR($CD_3OD$): δ 8.03, 7.61, 7.17, 7.07, 4.37, 4.15, 4.00, 3.76, 3.63, 3.49-3.45, 3.33-3.26, 2.50-2.38, 2.17-1.93, 1.80-1.65, 1.34-1.17, 1.00-0.87.

Example 3(2)

7-butyl-8,16-dioxo-3-(4-(4-methylaminocarbonylphenoxy)phenylmethyl-3,7,15-triazadispiro[5.2.5.2]hexadecane hydrochloride

TLC: Rf 0.31 (ethyl acetate:methanol=10:1);
NMR($CD_3OD$): δ 7.84, 7.61, 7.14, 7.07, 4.36, 3.95-3.80, 3.55-3.35, 2.91, 2.52-2.41, 2.14-1.94, 1.80-1.30, 0.95.

Example 3(3)

(3S)-1-butyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

TLC: Rf 0.38 (chloroform:methanol=10:1);
NMR($CD_3OD$): δ 8.04, 7.61, 7.18, 7.07, 4.37, 4.09, 4.08, 3.71, 3.64-3.41, 3.22, 2.58-2.41, 2.33, 2.21-1.97, 1.83-1.60, 1.54-1.10, 1.05-0.84, 0.95.

Example 3(4)

(3R)-1-butyl-2,5-dioxo-3-((1S)-1-hydroxy-1-cyclohexylmethyl)-9-(4-(4-carboxyphenoxy)phenylmethyl)-1,4,9-triazaspiro[5.5]undecane hydrochloride

TLC: Rf 0.38 (chloroform:methanol=10:1);
NMR($CD_3OD$): δ 8.04, 7.61, 7.18, 7.07, 4.37, 4.09, 4.08, 3.71, 3.64-3.41, 3.22, 2.58-2.41, 2.33, 2.21-1.97, 1.83-1.60, 1.54-1.10, 1.05-0.84, 0.95.

Example 3(5)

(3R)-1-methyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

TLC: Rf 0.55 (dichloromethane:methanol=4:1);
NMR($CD_3OD$): δ 8.03, 7.60, 7.18, 7.07, 4.38, 4.17, 3.99, 3.79, 3.50-3.46, 3.26, 2.95, 2.60-2.36, 2.12-1.94, 1.80-1.64, 1.38-1.18, 1.00-0.88.

Example 3(6)

(3R)-1-(2-methylsulfonylaminoethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.35 (chloroform:methanol:acetic acid=20:2:1);
NMR($CD_3OD$): δ 7.84, 7.57, 7.15, 7.07, 4.36, 4.18, 4.02, 3.83-3.58, 3.55-3.10, 2.95, 2.91, 2.56-2.36, 2.34-2.10, 2.10-1.88, 1.82-1.60, 1.40-1.10, 1.05-0.80.

Example 3(7)

(3R)-1-methyl-2,5-dioxo-3-((1R)-1-hydroxy-1-cyclohexylmethyl)-9-(4-methylsulfonylaminophenylmethyl)-1,4,9-triazaspiro[5.5]undecane hydrochloride

TLC: Rf 0.22 (ethyl acetate:methanol=4:1);
NMR($CD_3OD$): δ 7.49, 7.33, 4.22, 4.16, 3.82, 3.64, 3.37-3.33, 3.26, 3.00, 2.95, 2.49, 2.36-2.31, 2.05-1.92, 1.80-1.66, 1.33-1.14, 1.00-0.88.

Example 3(8)

(3R)-1-ethyl-2,5-dioxo-3-((1R)-1-hydroxy-1-cyclohexylmethyl)-9-(4-methylsulfonylaminophenylmethyl)-1,4,9-triazaspiro[5.5]undecane hydrochloride

TLC: Rf 0.28 (ethyl acetate:methanol=4:1);
NMR($CD_3OD$): δ 7.53, 7.34, 4.32, 4.15, 3.98, 3.77-3.59, 3.45-3.41, 3.33, 3.27, 3.01, 2.49-2.26, 2.15-1.91, 1.80-1.65, 1.34-1.16, 1.00-0.86.

Example 3(9)

(3R)-1-propyl-2,5-dioxo-3-((1R)-1-hydroxy-1-cyclohexylmethyl)-9-(4-methylsulfonylaminophenylmethyl)-1,4,9-triazaspiro[5.5]undecane hydrochloride

TLC: Rf 0.50 (ethyl acetate:methanol=4:1);
NMR($CD_3OD$): δ 7.52, 7.35, 4.32, 4.15, 3.99, 3.74, 3.46-3.42, 3.28, 3.11, 3.01, 2.49-2.28, 2.14-1.91, 1.80-1.64, 1.47-1.18, 1.00-0.87.

Example 3(10)

(3R)-1-methyl-2,5-dioxo-3-((1R)-1-hydroxy-1-cyclohexylmethyl)-9-(4-(4-methylsulfonylaminophenoxy)phenylmethyl)-1,4,9-triazaspiro[5.5]undecane hydrochloride

TLC: Rf 0.41 (ethyl acetate:methanol=4:1);
NMR($CD_3OD$): δ 7.48, 7.28, 7.04, 7.02, 4.18, 4.16, 3.75, 3.58, 3.34-3.33, 3.26, 2.95, 2.94, 2.52-2.23, 2.04-1.92, 1.80-1.64, 1.34-1.14, 1.01-0.87.

Example 3(11)

(3R)-1-ethyl-2,5-dioxo-3-((1R)-1-hydroxy-1-cyclohexylmethyl)-9-(4-(4-methylsulfonylaminophenoxy)phenylmethyl)-1,4,9-triazaspiro[5.5]undecane hydrochloride

TLC: Rf 0.62 (ethyl acetate:methanol=4:1);
NMR($CD_3OD$): δ 7.53, 7.29, 7.06, 7.03, 4.32, 4.15, 3.98, 3.76-3.60, 3.46-3.42, 3.33, 3.29, 2.95, 2.48-2.25, 2.16-1.92, 1.80-1.64, 1.39-1.16, 1.00-0.87.

Example 3(12)

(3R)-1-propyl-2,5-dioxo-3-((1R)-1-hydroxy-1-cyclohexylmethyl)-9-(4-(4-methylsulfonylaminophenoxy)phenylmethyl)-1,4,9-triazaspiro[5.5]undecane hydrochloride

TLC: Rf 0.74 (ethyl acetate:methanol=4:1);
NMR($CD_3OD$): δ 7.52, 7.29, 7.06, 7.03, 4.30, 4.15, 3.94, 3.70, 3.49-3.41, 3.29, 3.14, 2.95, 2.48-2.24, 2.14-1.91, 1.80-1.64, 1.50-1.14, 1.01-0.87.

Example 3(13)

(3R)-1-butyl-2,5-dioxo-3-((1R)-1-hydroxy-2,2-dimethylpropyl)-9-(4-(4-carboxyphenoxy)phenylmethyl)-1,4,9-triazaspiro[5.5]undecane hydrochloride

TLC: Rf 0.53 (chloroform:methanol:acetic acid=20:2:1);
NMR($CD_3OD$): δ 8.00, 7.55, 7.07, 6.99, 4.14, 3.68, 3.50, 3.60-3.20, 2.60-2.35, 2.00, 1.70-1.30, 0.99, 0.92.

Example 3(14)

(3R)-1-butyl-2,5-dioxo-3-((1R)-1-hydroxy-2,2-dimethylpropyl)-9-(4-(4-methylaminocarbonylphenoxy)phenylmethyl)-1,4,9-triazaspiro[5.5]undecane hydrochloride

TLC: Rf 0.56 (chloroform:methanol:acetic acid=20:2:1);
NMR($CD_3OD$): δ 7.84, 7.62, 7.14, 7.07, 4.35, 4.14, 3.96, 3.77, 3.50-3.30, 3.46, 2.91, 2.60-2.05, 1.70-1.30, 0.99, 0.94.

Example 3(15)

(3R)-1-(3-hydroxybutyl)-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

TLC: Rf 0.61 (dichloromethane:methanol=5:1);
NMR($CD_3OD$): δ 8.03, 7.60, 7.18, 7.07, 4.38, 4.16, 3.98, 3.84-3.75, 3.61 -3.45, 3.34, 3.26, 2.55-2.35, 2.16-1.92, 1.80-1.63, 1.50-1.17, 1.00-0.87.

Example 3(16)

(3R)-1-butyl-2,5-dioxo-3-((1R)-1-hydroxy-1-cycloheptylmethyl)-9-(4-(4-carboxyphenoxy)phenylmethyl)-1,4,9-triazaspiro[5.5]undecane hydrochloride

TLC: Rf 0.28 (dichloromethane:methanol=10:1);
NMR($CD_3OD$): δ 8.03, 7.59, 7.18, 7.07, 4.37, 4.15, 4.02, 3.76, 3.58-3.44, 3.30-3.07, 2.51-1.17,0.96.

Example 3(17)

(3R)-1-butyl-2,5-dioxo-3-((1R)-1-hydroxy-1-cycloheptylmethyl)-9-(4-(4-methylaminocarbonylphenoxy)phenylmethyl)-1,4,9-triazaspiro[5.5]undecane hydrochloride

TLC: Rf 0.49 (dichloromethane:methanol=10:1);
NMR($CD_3OD$): δ 7.84, 7.58, 7.15, 7.07, 4.36, 4.15, 4.02, 3.75, 3.55-3.44, 3.30-3.10, 2.91, 2.50-1.21, 0.95.

Example 3(18)

(3R)-1-butyl-2,5-dioxo-3-((1R)-1-hydroxy-1-(cis-4-hydroxycyclohexyl)methyl)-9-(4-(4-carboxyphenoxy)phenylmethyl)-1,4,9-triazaspiro[5.5]undecane hydrochloride

TLC: Rf 0.17 (ethyl acetate:methanol=2:1);
NMR($CD_3OD$): δ 8.02, 7.59, 7.15, 7.05, 4.32, 4.17, 4.00-3.91, 3.70, 3.52-3.37, 3.30-3.17, 2.50-2.33, 2.10, 1.80-1.18, 0.94.

Example 3(19)

(3R)-1-butyl-2,5-dioxo-3-((1R)-1-hydroxy-1-(cis-4-hydroxycyclohexyl)methyl)-9-(4-(4-methylaminocarbonylphenoxy)phenylmethyl)-1,4,9-triazaspiro[5.5]undecane hydrochloride

TLC: Rf 0.43 (ethyl acetate:methanol=2:1);
NMR($CD_3OD$): δ 7.84, 7.61, 7.14, 7.07, 4.35, 4.17, 4.01-3.69, 3.53-3.37, 3.30-3.20, 2.91, 2.51-1.30, 0.95.

Example 3(20)

(3R)-1-butyl-2,5-dioxo-3-((1R)-1-hydroxy-1-adamantylmethyl)-9-(4-(4-carboxyphenoxy)phenylmethyl)-1,4,9-triazaspiro[5.5]undecane hydrochloride

TLC: Rf 0.40 (ethyl acetate:methanol=2:1);
NMR($CD_3OD$): δ 8.04, 7.60, 7.17, 7.07, 4.37, 4.18, 3.98, 3.78, 3.54-3.40, 3.30-3.24, 2.55-2.10, 1.98-1.33, 0.95.

Example 3(21)

(3R)-1-butyl-2,5-dioxo-3-((1R)-1-hydroxy-1-adamantylmethyl)-9-(4-(4-methylaminocarbonylphenoxy)phenylmethyl)-1,4,9-triazaspiro[5.5]undecane hydrochloride

TLC: Rf 0.74 (ethyl acetate:methanol=2:1);
NMR($CD_3OD$): δ 7.84, 7.59, 7.15, 7.07, 4.36, 4.18, 3.98, 3.77, 3.53-3.40, 3.30-3.25, 2.91, 2.54-2.10, 1.95-1.33, 0.95.

Example 3(22)

(3R)-1-butyl-2,5-dioxo-3-((1R)-1-hydroxy-1-phenylmethyl)-9-(4-(4-methylaminocarbonylphenoxy)phenylmethyl)-1,4,9-triazaspiro[5.5]undecane hydrochloride

TLC: Rf 0.17 (dichloromethane:methanol=10:1);
NMR($CD_3OD$): δ 7.84, 7.54, 7.36-7.24, 7.11, 7.06, 5.23, 4.41, 4.25, 3.80, 3.49-3.05, 2.91, 2.30, 2.01, 1.74, 1.50-1.25, 0.93, -0.02.

Example 3(23)

(3R)-1-butyl-2,5-dioxo-3-((1R)-1-hydroxy-1-(trans-4-hydroxycyclohexyl)methyl)-9-(4-(4-carboxyphenoxy)phenylmethyl)-1,4,9-triazaspiro[5.5]undecane hydrochloride

TLC: Rf 0.27 (dichloromethane:methanol=3:1);
NMR($CD_3OD$): δ 8.03, 7.62, 7.15, 7.07, 4.36, 4.14, 3.97, 3.71, 3.57-3.41, 3.30-3.24, 2.58-2.43, 2.12-1.68, 1.40-0.93, 0.95.

Example 3(24)

(3R)-1-butyl-2,5-dioxo-3-((1R)-1-hydroxy-1-(trans-4-hydroxycyclohexyl)methyl)-9-(4-(4-methylaminocarbonylphenoxy)phenylmethyl)-1,4,9-triazaspiro[5.5]undecane hydrochloride

TLC: Rf 0.77 (dichloromethane:methanol=3:1);
NMR($CD_3OD$): δ 7.82, 7.50, 7.09, 7.04, 4.13, 4.05, 3.62-3.36, 3.30-3.24, 2.90, 2.41-2.25, 2.09-1.93, 1.70-0.91, 0.95.

Example 3 (25)

(3R)-1-butyl-2,5-dioxo-3-((1R)-1-hydroxy-1-cyclopropylmethyl)-9-(4-(4-carboxyphenoxy)phenylmethyl)-1,4,9-triazaspiro[5.5]undecane hydrochloride

TLC: Rf 0.29 (ethyl acetate:methanol=2:1);
NMR($CD_3OD$): δ 8.03, 7.64, 7.17, 7.07, 4.37, 4.11, 3.92, 3.80, 3.54-3.34, 3.14, 2.61-2.43, 2.35, 2.15, 1.70-1.49, 1.49-1.33, 1.09, 0.96, 0.60-0.43, 0.33, 0.24.

Example 3(26)

(3R)-1-butyl-2,5-dioxo-3-((1R)-1-hydroxy-1-cyclopropylmethyl)-9-(4-(4-methylaminocarbonylphenoxy)phenylmethyl)-1,4,9-triazaspiro[5.5]undecane hydrochloride

TLC: Rf 0.62 (ethyl acetate:methanol=2:1);
NMR($CD_3OD$): δ 7.84, 7.61, 7.14, 7.07, 4.36, 4.11, 3.92, 3.81, 3.54-3.40, 3.37, 3.13, 2.91, 2.55-2.30, 2.16, 1.71-1.48, 1.48-1.32, 1.10, 0.96, 0.60-0.43, 0.32, 0.24.

Example 3(27)

(3R)-1-butyl-2,5-dioxo-3-((1R)-1-hydroxy-2-ethylbutyl)-9-(4-(4-methylaminocarbonylphenoxy)phenylmethyl)-1,4,9-triazaspiro[5.5]undecane hydrochloride

TLC: Rf 0.38 (ethyl acetate:methanol=5:1);

NMR($CD_3OD$): δ 7.84, 7.60, 7.15, 7.07, 4.36, 4.14, 4.01, 3.76, 3.60-3.40, 3.20, 2.91, 2.55-2.10, 1.80-1.30, 0.95, 0.88, 0.85.

Example 3(28)

(3R)-1-butyl-2,5-dioxo-3-((1R)-1-hydroxy-2-ethylbutyl)-9-(4-(4-carboxyphenoxy)phenylmethyl)-1,4,9-triazaspiro[5.5] undecane hydrochloride

TLC: Rf 0.70 (dichloromethane:methanol:water=8:2:0.1);
NMR($CD_3OD$): δ 8.04, 7.61, 7.18, 7.07, 4.37, 4.14, 4.01, 3.76, 3.60-3.40, 3.20, 2.60-2.10, 1.80-1.30, 0.95, 0.88, 0.85.

Example 3(29)

(3R)-1-butyl-2,5-dioxo-3-((1R)-1-hydroxy-1-(furan-3-yl)methyl)-9-(4-(4-methylaminocarbonylphenoxy)phenylmethyl)-1,4,9-triazaspiro[5.5]undecane hydrochloride

TLC: Rf 0.31 (ethyl acetate:methanol=5:1);
NMR($CD_3OD$): δ 7.84, 7.57, 7.44, 7.39, 7.13, 7.07, 6.38, 5.18, 4.34, 4.32, 3.85-3.70, 3.50-3.20, 2.91, 2.45-2.00, 1.50-1.20, 1.05, 0.93.

Example 3(30)

(3R)-1-butyl-2,5-dioxo-3-((1R)-1-hydroxy-1-(furan-3-yl)methyl)-9-(4-(4-carboxyphenoxy)phenylmethyl)-1,4,9-triazaspiro[5.5]undecane hydrochloride

TLC: Rf 0.59 (dichloromethane:methanol:water=8:2:0.1);
NMR($CD_3OD$): δ 8.04, 7.59, 7.44, 7.39, 7.16, 7.07, 6.38, 5.18, 4.34, 4.33, 3.89-3.70, 3.50-3.20, 2.45-2.00, 1.50-1.25, 1.10, 0.93.

Example 3(31)

(3R)-1-butyl-2,5-dioxo-3-((1R)-1-hydroxy-1-(trans-4-methylcyclohexyl)methyl)-9-(4-(4-methylaminocarbonylphenoxy) phenylmethyl)-1,4,9-triazaspiro[5.5]undecane hydrochloride

TLC: Rf 0.48 (ethyl acetate:methanol=4:1);
NMR($CD_3OD$): δ 7.84, 7.60, 7.14, 7.07, 4.36, 4.15, 4.00, 3.75, 3.57-3.36, 3.30-3.11, 2.91, 2.57-2.41, 2.33, 2.19-1.83, 1.78-1.59, 1.46-1.22, 1.04-0.89, 0.95, 0.88.

Example 3(32)

(3R)-1-butyl-2,5-dioxo-3-((1R)-1-hydroxy-1-(trans-4-methylcyclohexyl)methyl)-9-(4-(4-carboxyphenoxy) phenylmethyl)-1,4,9-triazaspiro[5.5]undecane hydrochloride

TLC: Rf 0.66 (chloroform:methanol:acetic acid=10:1:1);
NMR($CD_3OD$): δ 8.03, 7.62, 7.17, 7.07, 4.36, 4.15, 3.99, 3.75, 3.59-3.40, 3.30-3.18, 2.58-2.30, 2.15-1.92, 1.78-1.62, 1.46-1.21, 1.02-0.89, 0.95, 0.88.

Example 3(33)

(3R)-1-butyl-2,5-dioxo-3-((1R)-1-hydroxy-1-(1,4-benzodioxan-6-yl)methyl)-9-(4-(4-methylaminocarbonylphenoxy) phenylmethyl)-1,4,9-triazaspiro[5.5]undecane hydrochloride

TLC: Rf 0.32 (dichloromethane:methanol=10:1);
NMR($CD_3OD$): δ 7.84, 7.57, 7.12, 7.07, 6.78-6.76, 5.10, 4.34, 4.31, 4.25, 4.18, 3.81, 3.57, 3.40-3.15, 2.91, 2.34, 2.02, 1.91, 1.55-1.20, 0.93, 0.34.

Example 3(34)

(3R)-1-butyl-2,5-dioxo-3-((1R)-1-hydroxy-1-(1,4-benzodioxan-6-yl)methyl)-9-(4-(4-carboxyphenoxy)phenylmethyl)-1,4,9-triazaspiro[5.5]undecane hydrochloride

TLC: Rf 0.11 (dichloromethane:methanol=10:1);
NMR($CD_3OD$): δ 8.03, 7.58, 7.15, 7.06, 6.78-6.76, 5.10, 4.34, 4.33, 4.27, 4.18, 3.81, 3.58, 3.40-3.10, 2.33, 2.03, 1.90, 1.55-1.20, 0.93, 0.35.

Example 3(35)

(3R)-1-butyl-2,5-dioxo-3-((1R)-1-hydroxy-2-cyclohexylethyl)-9-(4-(4-carboxyphenoxy)phenylmethyl)-1,4,9-triazaspiro [5.5]undecane hydrochloride

TLC: Rf 0.45 (dichloromethane:methanol=5:1);
NMR($CD_3OD$): δ 8.03, 7.60, 7.18, 7.07, 4.37, 3.97-3.79, 3.50-3.34, 2.49-2.36, 2.15, 1.82, 1.71-1.15, 1.02-0.82, 0.95.

Example 3(36)

(3R)-1-butyl-2,5-dioxo-3-((1R)-1-hydroxy-2-cyclohexylethyl)-9-(4-(4-methylaminocarbonylphenoxy)phenylmethyl)-1,4,9-triazaspiro[5.5]undecane hydrochloride

TLC: Rf 0.79 (dichloromethane:methanol=5:1);
NMR($CD_3OD$): δ 7.84, 7.60, 7.15, 7.07, 4.36, 3.98-3.80, 3.50-3.34, 2.91, 2.50-2.36, 2.13, 1.81, 1.71-1.15, 1.01-0.82, 0.95.

Example 3(37)

(3R)-1-benzyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

TLC: Rf 0.25 (dichloromethane:methanol=10:1);
NMR($CD_3OD$): δ 5.03, 7.56, 7.32-7.19, 7.15, 7.05, 5.00, 4.58, 4.33, 3.94 , 3.74, 3.48-3.32, 2.62-2.34, 2.06-1.69. 1.31-1.14, 1.00-0.90.

Example 3(38)

(3R)-1-butyl-2,5-dioxo-3-((1R)-1-hydroxy-3-phenylpropyl)-9-(4-(4-carboxyphenoxy)phenylmethyl)-1,4,9-triazaspiro [5.5]undecane hydrochloride

TLC: Rf 0.29 (dichloromethane:methanol=10:1);
NMR($CD_3OD$): δ 8.04, 7.59, 7.27-7.12, 7.07, 4.37, 3.99, 3.98-3.79, 3.49-3.42, 3.20, 2.80, 2.65, 2.47-2.13, 1.93-1.18, 0.95.

Example 3(39)

(3R)-1-butyl-2,5-dioxo-3-((1R)-1-hydroxy-3-phenylpropyl)-9-(4-(4-methylaminocarbonylphenoxy)phenylmethyl)-1,4,9-triazaspiro[5.5]undecane hydrochloride

TLC: Rf 0.53 (dichloromethane:methanol=10:1);
NMR($CD_3OD$): δ 7.84, 7.58, 7.26-7.13, 7.07, 4.36, 3.98, 3.97-3.75, 3.49-3.34, 3.27, 2.91, 2.78, 2.64, 2.49-2.12, 1.95-1.31, 0.95.

Example 3(40)

(3R)-1-butyl-2,5-dioxo-3-((1R)-1-hydroxy-2-(tetrahydropyran-4-yl)ethyl)-9-(4-(4-carboxyphenoxy)phenylmethyl)-1,4,9-triazaspiro[5.5]undecane hydrochloride

TLC: Rf 0.58 (dichloromethane:methanol:water=8:2:0.1);
NMR($CD_3OD$): δ 8.04, 7.61, 7.18, 7.07, 4.37, 3.98-3.80, 3.60-3.30, 2.55-2.30, 2.16, 1.85-1.10, 0.96.

Example 3(41)

(3R)-1-butyl-2,5-dioxo-3-((1R)-1-hydroxy-2-(tetrahydropyran-4-yl)ethyl)-9-(4-(4-methylaminocarbonylphenoxy) phenylmethyl)-1,4,9-triazaspiro[5.5]undecane hydrochloride

TLC: Rf 0.24 (dichloromethane:methanol=10:1);
NMR($CD_3OD$): δ 7.84, 7.60, 7.15, 7.07, 4.36, 4.10-3.80, 3.60-3.30, 2.91, 2.55-2.30, 2.16, 1.90-1.10, 0.95.

Example 3(42)

(3R)-1-butyl-2,5-dioxo-3-((1R)-1-hydroxy-3-methylbutyl)-9-(4-(4-carboxyphenoxy)phenylmethyl)-1,4,9-triazaspiro [5.5]undecane hydrochloride

TLC: Rf 0.48 (chloroform:methanol:acetic acid=10:1:1);
NMR($CD_3OD$): δ 8.04, 7.62, 7.17, 7.07, 4.37, 3.98-3.78, 3.52-3.34, 2.55-2.30, 2.14, 1.82-1.31, 1.20, 0.95, 0.93, 0.90.

Example 3(43)

(3R)-1-butyl-2,5-dioxo-3-((1R)-1-hydroxy-1-cyclobutylmethyl)-9-(4-(4-carboxyphenoxy)phenylmethyl)-1,4,9-triazaspiro[5.5]undecane hydrochloride

TLC: Rf 0.68 (butanol:acetic acid:water=4:2:1);
NMR($CD_3OD$): δ 8.02, 7.61, 7.14, 7.05, 4.32, 3.90, 3.90, 3.73, 3.68, 3.53-3.40, 3.26, 2.67, 2.51, 2.43-2.37, 2.15-1.29, 0.94.

Example 3(44)

(3R)-1-butyl-2,5-dioxo-3-((1R)-1-hydroxy-1-(cis-4-methylcyclohexyl)methyl)-9-(4-(4-methylaminocarbonylphenoxy) phenylmethyl)-1,4,9-triazaspiro[5.5]undecane hydrochloride

TLC: Rf 0.36 (chloroform:methanol=10:1);
NMR($CD_3OD$): δ 7.84, 7.60, 7.14, 7.07, 4.36, 4.16, 4.00, 3.75, 3.57, 3.53-3.42, 3.22, 2.91, 2.55-2.42, 2.35, 2.12, 1.93-1.26, 0.95, 0.94.

Example 3(45)

(3R)-1-butyl-2,5-dioxo-3-((1R)-1-hydroxy-3-methylbutyl)-9-(4-(4-methylaminocarbonylphenoxy)phenylmethyl)-1,4,9-triazaspiro[5.5]undecane hydrochloride

TLC: Rf 0.29 (chloroform:methanol=10:1);
NMR($CD_3OD$): δ 7.84, 7.61, 7.14, 7.07, 4.36, 3.97, 3.97-3.77, 3.51-3.34, 2.91, 2.54-2.31, 2.14, 1.77, 1.67-1.28, 1.20, 0.95, 0.93, 0.90.

Example 3(46)

(3R)-1-butyl-2,5-dioxo-3-((1R)-1-hydroxy-1-(trans-4-methoxycyclohexyl)methyl)-9-(4-(4-methylaminocarbonylphenoxy)phenylmethyl)-1,4,9-triazaspiro[5.5]undecane hydrochloride

TLC: Rf 0.68 (dichloromethane:methanol=5:1);
NMR($CD_3OD$): δ 7.84, 7.58, 7.15, 7.07, 4.36, 4.14, 4.01, 3.74, 3.57-3.44, 3.33, 3.30-3.12, 2.91, 2.50-1.98, 1.72-1.10,

0.96.

Example 3(47)

(3R)-1-butyl-2,5-dioxo-3-((1R)-1-hydroxy-1-(trans-4-methoxycyclohexyl)methyl)-9-(4-(4-carboxyphenoxy) phenylmethyl)-1,4,9-triazaspiro[5.5]undecane hydrochloride

TLC: Rf 0.24 (dichloromethane:methanol=5:1);
NMR($CD_3OD$): δ 8.04, 7.60, 7.17, 7.07, 4.36, 4.14, 4.02, 3.74, 3.54-3.44, 3.33, 3.30-3.12, 2.52-1.98, 1.71-1.00, 0.96.

Example 3(48)

(3R)-1-butyl-2,5-dioxo-3-((1R)-1-hydroxy-1-cyclobutylmethyl)-9-(4-(4-methylaminocarbonylphenoxy)phenylmethyl)-1,4,9-triazaspiro[5.5]undecane hydrochloride

TLC: Rf 0.43 (chloroform:methanol:acetic acid=10:1:1),
NMR($CD_3OD$): δ 7.84, 7.62, 7.14, 7.07, 4.35, 3.95, 3.90, 3.75, 3.68, 3.52-3.42, 3.28, 2.91, 2.75-2.38, 2.14-1.31, 0.95.

Example 3(49)

(3R)-1-butyl-2,5-dioxo-3-((1R)-1-hydroxy-1-(cis-4-ethoxycyclohexyl)methyl)-9-(4-(4-carboxyphenoxy)phenylmethyl)-1,4,9-triazaspiro[5.5]undecane hydrochloride

TLC: Rf 0.35 (dichloromethane:methanol=5:1);
NMR($CD_3OD$): δ 8.04, 7.60, 7.18, 7.07, 4.37, 4.16, 4.02, 3.75, 3.54-3.15, 2.51-1.31, 1.17, 0.95.

Example 3(50)

(3R)-1-butyl-2,5-dioxo-3-((1R)-1-hydroxy-1-(trans-4-ethoxycyclohexyl)methyl)-9-(4-(4-carboxyphenoxy) phenylmethyl)-1,4,9-triazaspiro[5.5]undecane hydrochloride

TLC: Rf 0.35 (dichloromethane:methanol=5:1);
NMR($CD_3OD$): δ 8.03, 7.58, 7.18, 7.07, 4.37, 4.14, 4.02, 3.75, 3.57-3.15 , 2.50-0.93, 1.16, 0.96.

Example 3(51)

(3R)-1-butyl-2,5-dioxo-3-((1R)-1-hydroxy-1-(cis-4-ethoxycyclohexyl)methyl)-9-(4-(4-methylaminocarbonylphenoxy) phenylmethyl)-1,4,9-triazaspiro[5.5]undecane hydrochloride

TLC: Rf 0.68 (dichloromethane:methanol=5:1);
NMR($CD_3OD$): δ 7.84, 7.59, 7.14, 7.07, 4.36, 4.16, 4.00, 3.75, 3.55-3.20, 2.91 (s, 3H), 2.49-1.31, 1.17, 0.95.

Example 3(52)

(3R)-1-butyl-2,5-dioxo-3-((1R)-1-hydroxy-1-(trans-4-ethoxycyclohexyl)methyl)-9-(4-(4-methylaminocarbonylphenoxy) phenylmethyl)-1,4,9-triazaspiro[5.5]undecane hydrochloride

TLC: Rf 0.68 (dichloromethane:methanol=5:1);
NMR($CD_3OD$): δ 7.84, 7.59, 7.15, 7.07, 4.36, 4.14, 4.00, 3.76, 3.59-3.20, 2.91, 2.49-0.94, 1.16, 0.95.

Example 3(53)

(3R)-1-butyl-2,5-dioxo-3-((1R)-1-hydroxy-2-methyl-2-phenylpropyl)-9-(4-(4-carboxyphenoxy)phenylmethyl)-1,4,9-triazaspiro[5.5]undecane hydrochloride

TLC: Rf 0.35 (dichloromethane:methanol=10:1);
NMR($CD_3OD$): δ 8.04, 7.60, 7.47, 7.30, 7.20-7.16, 7.07, 4.35, 3.89, 3.89, 3.84, 3.65, 3.50-3.15, 2.55-2.20, 2.03, 1.70-1.30, 1.44, 1.37, 0.94.

Example 3(54)

(3R)-1-butyl-2,5-dioxo-3-((1R)-1-hydroxy-2-methyl-2-phenylpropyl)-9-(4-(4-methylaminocarbonylphenoxy) phenylmethyl)-1,4,9-triazaspiro[5.5]undecane hydrochloride

TLC: Rf 0.40 (dichloromethane:methanol=10:1);
NMR($CD_3OD$): δ 7.84, 7.59, 7.47, 7.30, 7.20-7.13, 7.07, 4.34, 3.92, 3.92, 3.84, 3.64, 3.50-3.15, 2.91, 2.50-2.25, 2.02, 1.70-1.30, 1.43, 1.37, 0.94.

Example 3(55)

(3R)-1-butyl-2,5-dioxo-3-((1R)-1-hydroxy-1-(1,1-dioxidotetrahydro-2H-thiopyran-4-yl)methyl)-9-(4-(4-methylaminocarbonylphenoxy)phenylmethyl)-1,4,9-triazaspiro[5.5]undecane hydrochloride

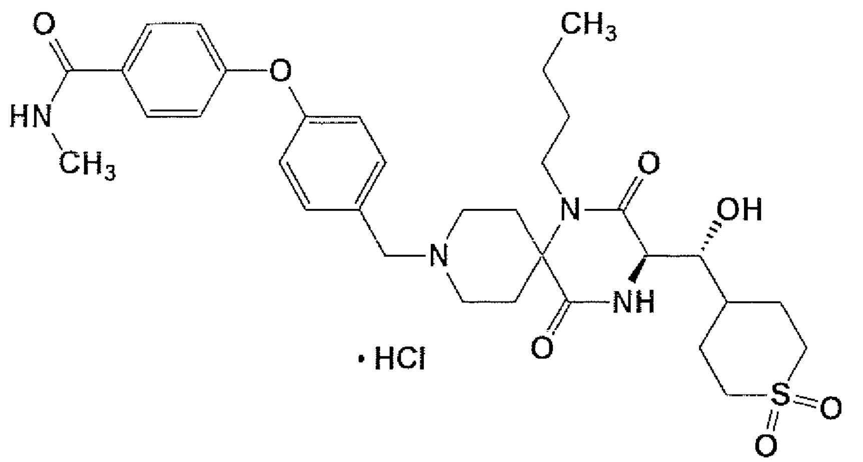

TLC: Rf 0.68 (dichloromethane:methanol:acetic acid=10:2:1);
NMR($CD_3OD$): δ 7.84, 7.60, 7.14, 7.07, 4.36, 4.19, 4.00, 3.74, 3.60-3.00, 2.91, 2.60-2.30, 2.20-2.00, 1.90-1.60, 1.50-1.30, 0.95.

Example 3(56)

(3R)-1-pentyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane hydrochloride

TLC: Rf 0.61 (dichloromethane:methanol=5:1);
NMR($CD_3OD$): δ 7.75, 7.68, 7.49, 7.09, 6.99, 4.32, 4.15, 3.99, 3.82, 3.73, 3.53-3.07, 2.50-1.14, 1.00-0.85,0.92.

Example 3(57)

(3R)-1-pentyl-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

TLC: Rf 0.60 (dichloromethane:methanol=5:1);
NMR($CD_3OD$): δ 8.03, 7.58, 7.18, 7.07, 4.37, 4.16, 4.02, 3.77, 3.55-3.05, 2.52-1.15, 1.00-0.85, 0.92.

Example 3(58)

(3R)-1-butyl-2,5-dioxo-3-((1R)-1-hydroxy-2-ethylbutyl)-9-(4-(4-carboxy-2-methoxyphenoxy)phenylmethyl)-1,4,9-triazaspiro[5.5]undecane hydrochloride

TLC: Rf 0.32 (dichloromethane:methanol:water=9:1:0.1);
NMR($CD_3OD$): δ 7.75, 7.68, 7.51, 7.09, 6.99, 4.32, 4.13, 3.99, 3.82, 3.74, 3.60-3.40, 3.19, 2.50-2.20, 2.12, 1.80-1.30, 0.95, 0.88, 0.85.

Example 3(59)

(3R)-1-butyl-2,5-dioxo-3-((1R)-1-hydroxy-1-(1,1-dioxidotetrahydro-2H-thiopyran-4-yl)methyl)-9-(4-(4-chlorophenoxy)phenylmethyl)-1,4,9-triazaspiro[5.5]undecane hydrochloride

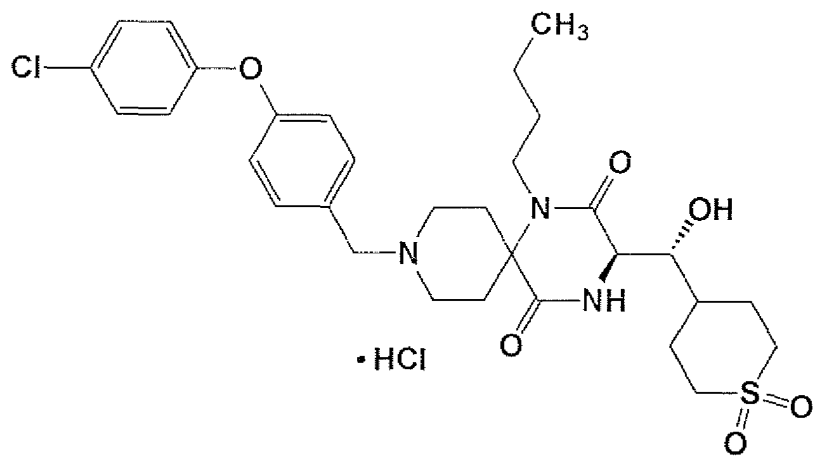

TLC: Rf 0.48 (dichloromethane:methanol:water=9:1:0.1);
NMR($CD_3OD$): δ 7.56, 7.38, 7.08, 7.02, 4.34, 4.19, 4.00, 3.73, 3.60-3.00, 2.55-2.20, 2.20-2.00, 1.85-1.60, 1.50-1.30, 0.95.

Example 3(60)

(3R)-1-butyl-2,5-dioxo-3-((1R)-1-hydroxy-1-(1,1-dioxidotetrahydro-2H-thiopyran-4-yl)methyl)-9-(4-(4-methoxyphenoxy)phenylmethyl)-1,4,9-triazaspiro[5.5]undecane hydrochloride

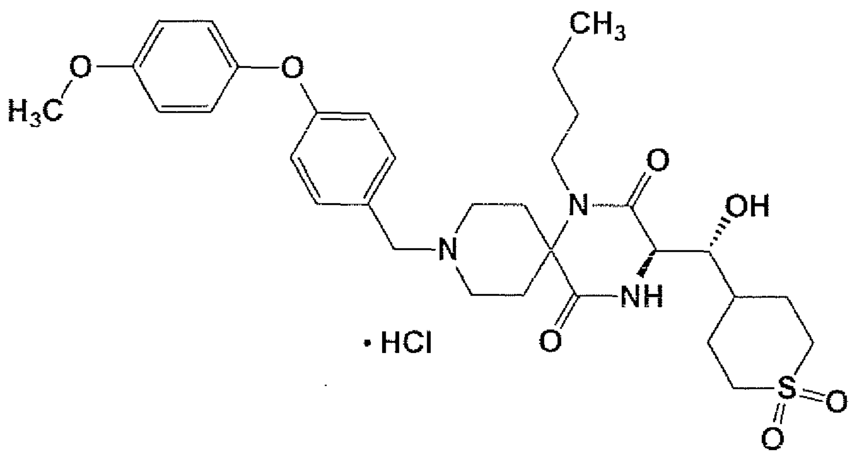

TLC: Rf 0.43 (dichloromethane:methanol:water=9:1:0.1);
NMR($CD_3OD$): δ 7.49, 7.01-6.93, 4.31, 4.19, 3.98, 3.79, 3.72, 3.60-3.00, 2.55-2.20, 2.20-2.00, 1.90-1.60, 1.50-1.30, 0.95.

Example 3(61)

(3R)-1-butyl-2,5-dioxo-3-((1R)-1-hydroxy-1-(tetrahydropyran-4-yl)methyl)-9-(4-(4-cyclopropylmethylaminocarbonylphenoxy)phenylmethyl)-1,4,9-triazaspiro[5.5]undecane hydrochloride

TLC: Rf 0.35 (ethyl acetate:methanol=5:1);
NMR($CD_3OD$): δ 7.86, 7.59, 7.15, 7.08, 4.36, 4.12, 4.06-3.90, 3.75, 3.59-3.16, 2.52-1.07, 0.96, 0.56-0.48, 0.30-0.25.

Example 3(62)

(3R)-1-butyl-2,5-dioxo-3-((1R)-1-hydroxy-1-(tetrahydropyran-4-yl)methyl)-9-(4-(4-methoxyphenoxy)phenylmethyl)-1,4,9-triazaspiro[5.5]undecane hydrochloride

TLC: Rf 0.37 (ethyl acetate:methanol=5:1);
NMR($CD_3OD$): δ 7.49, 7.00-6.96, 4.30, 4.12, 3.97-3.90, 3.79, 3.75-3.20, 2.50-1.18, 0.95.

Example 3(63)

(3R)-1-butyl-2,5-dioxo-3-((1R)-1-hydroxy-2-ethylbutyl)-9-(4-(4-carboxy-3-methylphenoxy)phenylmethyl)-1,4,9-triazaspiro[5.5]undecane hydrochloride

TLC: Rf 0.34 (dichloromethane:methanol:water=9:1:0.1);
NMR($CD_3OD$): δ 7.97, 7.60, 7.15, 6.91, 6.87, 4.36, 4.14, 4.01, 3.76, 3.60-3.40, 3.22, 2.56, 2.50-2.25, 2.13, 1.80-1.30, 0.95, 0.88, 0.85.

Example 3(64)

(3R)-1-butyl-2,5-dioxo-3-((1R)-1-hydroxy-2-ethylbutyl)-9-(4-(4-carboxy-2-methoxyphenylmethyl)phenylmethyl)-1,4,9-triazaspiro[5.5]undecane hydrochloride

TLC: Rf 0.32 (dichloromethane:methanol:water=9:1:0.1);

NMR($CD_3OD$): δ 7.58-7.55, 7.44, 7.35, 7.22, 4.30, 4.12, 4.04, 3.97, 3.86, 3.73, 3.60-3.35, 3.15, 2.50-2.20, 2.10, 1.80-1.30, 0.93, 0.87, 0.85.

Example 3(65)

(3R)-1-butyl-2,5-dioxo-3-((1R)-1-hydroxy-2-ethylbutyl)-9-(4-methylsulfonylaminophenylmethyl)-1,4,9-triazaspiro[5.5] undecane hydrochloride

TLC: Rf 0.41 (dichloromethane:methanol:water=9:1:0.1);
NMR($CD_3OD$): δ 7.53, 7.34, 4.32, 4.14, 3.99, 3.75, 3.60-3.40, 3.20, 3.01, 2.50-2.20, 2.11, 1.80-1.30, 0.95, 0.88, 0.85.

Example 3(66)

(3R)-1-butyl-2,5-dioxo-3-((1R)-1-hydroxy-1-(tetrahydropyran-4-yl)methyl)-9-(4-phenoxyphenylmethyl)-1,4,9-triazaspiro[5.5]undecane hydrochloride

TLC: Rf 0.60 (dichloromethane:methanol=10:1);
NMR($CD_3OD$): δ 7.53, 7.43-7.35, 7.18, 7.09-7.00, 4.33, 4.12, 4.08-3.87, 3.73, 3.60-3.30, 3.30-3.10, 2.56-2.22, 2.20-1.92, 1.92-1.78, 1.70, 1.55-1.16, 0.95.

Example 3(67)

(3R)-1-butyl-2,5-dioxo-3-((1R)-1-hydroxy-1-(tetrahydropyran-4-yl)methyl)-9-(4-(4-hydroxyphenoxy)phenylmethyl)-1,4,9-triazaspiro[5.5]undecane hydrochloride

TLC: Rf 0.36 (dichloromethane:methanol=10:1);
NMR($CD_3OD$): δ 7.47, 6.97, 6.89, 6.80, 4.30, 4.12, 4.08-3.88, 3.72, 3.60-3.30, 3.30-3.10, 2.54-2.20, 2.20-1.94, 1.94-1.79, 1.68, 1.55-1.15, 0.95.

Example 3(68)

(3R)-1-butyl-2,5-dioxo-3-((1R)-1-hydroxy-1-(tetrahydropyran-4-yl)methyl)-9-(4-(4-methylcarbonylaminophenylmethyl)phenylmethyl)-1,4,9-triazaspiro[5.5]undecane hydrochloride

TLC: Rf 0.37 (dichloromethane:methanol=10:1);
NMR($CD_3OD$): δ 7.48-7.40, 7.34, 7.15, 4.31, 4.11, 4.07-3.87, 3.97, 3.73, 3.60-3.30, 3.30-3.04, 2.54-2.32, 2.24, 2.17-1.91, 2.09, 1.91-1.78, 1.69, 1.50-1.10, 0.94.

Example 3(69)

(3R)-1-butyl-2,5-dioxo-3-((1R)-1-hydroxy-2-ethylbutyl)-9-(4-(4-carboxyphenoxymethyl)phenylmethyl)-1,4,9-triazaspiro[5.5]undecane hydrochloride

TLC: Rf 0.39 (dichloromethane:methanol=10:1);
NMR($CD_3OD$): δ 7.96, 7.60, 7.05, 5.23 4.37, 4.13, 4.01, 3.76, 3.57-3.35, 3.20, 2.55-2.22, 2.11, 1.82-1.50, 1.50-1.24, 0.94, 0.87, 0.85.

Example 3(70)

(3R)-1-butyl-2,5-dioxo-3-((1R)-1-hydroxy-2-ethylbutyl)-9-(4-(4-aminosulfonylphenoxy)phenylmethyl)-1,4,9-triazaspiro[5.5]undecane hydrochloride

TLC: Rf 0.33 (dichloromethane:methanol:water=9:1:0.1);
NMR($CD_3OD$): δ 7.90, 7.61, 7.18, 7.13, 4.37, 4.14, 4.01, 3.76, 3.60-3.30, 3.20, 2.50-2.10, 1.80-1.30, 0.95, 0.88, 0.86.

Example 3(71)

(3R)-1-butyl-2,5-dioxo-3-((1R)-1-hydroxy-2-ethylbutyl)-9-(4-(4-fluorophenylmethylcarbonylamino)phenylmethyl)-1,4,9-triazaspiro[5.5]undecane hydrochloride

TLC: Rf 0.33 (dichloromethane:methanol:water=9:1:0.1);
NMR($CD_3OD$): δ 7.70, 7.49, 7.37-7.33, 7.08-7.02, 4.31, 4.13, 3.99, 3.74, 3.68, 3.60-3.30, 3.15, 2.50-2.10, 1.80-1.30, 0.94, 0.87, 0.85.

Example 3(72)

(3R)-1-butyl-2,5-dioxo-3-((1R)-1-hydroxy-1-(tetrahydropyran-4-yl)methyl)-9-(4-(4-cyanophenoxy)phenylmethyl)-1,4,9-triazaspiro[5.5]undecane hydrochloride

TLC: Rf 0.85 (chloroform:methanol=5:1);
NMR($CD_3OD$): δ 7.74, 7.64, 7.20, 7.13, 4.37, 4.12, 4.08-3.86, 3.74, 3.64-3.16, 2.56-2.24, 2.20-1.92, 1.86, 1.71, 1.60-1.16, 0.96.

Example 3(73)

(3R)-1-butyl-2,5-dioxo-3-((1R)-1-hydroxy-1-(tetrahydropyran-4-yl)methyl)-9-(4-(4-cyano-2-methoxyphenoxy) phenylmethyl)-1,4,9-triazaspiro[5.5]undecane hydrochloride

TLC: Rf 0.85 (chloroform:methanol=5:1);
NMR($CD_3OD$): δ 7.52, 7.44, 7.36, 7.13, 7.02, 4.31, 4.12, 4.01-3.86, 3.83, 3.71, 3.64-3.10, 2.54-2.20, 2.20-1.92, 1.85, 1.71, 1.50-1.16, 0.95.

Example 3(74)

(3R)-1-butyl-2,5-dioxo-3-((1R)-1-hydroxy-2-ethylbutyl)-9-(4-(4-methylaminocarbonylphenoxymethyl)phenylmethyl)-1,4,9-triazaspiro[5.5]undecane hydrochloride

TLC: Rf 0.76 (chloroform:methanol=5:1);
NMR($CD_3OD$): δ 7.77, 7.60, 7.04, 5.21, 4.37, 4.13, 4.02, 3.77, 3.60-3.36, 3.16, 2.89, 2.52-2.08, 1.82-1.26, 0.94, 0.88, 0.85.

Example 3(75)

(3R)-1-butyl-2,5-dioxo-3-((1R)-1-hydroxy-2-ethylbutyl)-9-(4-(4-carboxy-3-hydroxyphenoxy)phenylmethyl)-1,4,9-triazaspiro[5.5]undecane hydrochloride

TLC: Rf 0.74 (chloroform:methanol=5:1);
NMR($CD_3OD$): δ 7.86, 7.61, 7.20, 6.54, 6.44, 4.38, 4.14, 4.02, 3.77, 3.60-3.38, 3.20, 2.54-2.26, 2.14, 1.82-1.26, 0.95, 0.88, 0.86.

Example 3(76)

(3R)-1-butyl-2,5-dioxo-3-((1R)-1-hydroxy-1-(tetrahydropyran-4-yl)methyl)-9-(4-(4-chlorophenoxy)phenylmethyl)-1,4,9-triazaspiro[5.5]undecane hydrochloride

TLC: Rf 0.36 (ethyl acetate:methanol=10:1);
NMR($CD_3OD$): δ 7.54, 7.38, 7.08, 7.02, 4.32, 4.12, 3.98-3.90, 3.72, 3.56-3.15, 2.5 0-1.68, 1.40-1.18, 0.95.

Example 3 (77)

(3R)-1-butyl-2,5-dioxo-3-((1R)-1-hydroxy-2-ethylbutyl)-9-(4-(4-methylaminocarbonyl-2-methoxyphenoxy) phenylmethyl)-1,4,9-triazaspiro[5.5]undecane hydrochloride

TLC: Rf 0.39 (ethyl acetate:methanol=10:1);
NMR($CD_3OD$): δ 7.59, 7.52, 7.44, 7.08, 6.95, 4.31, 4.13, 3.97, 3.82, 3.72, 3.54-3.43, 3.24, 2.93, 2.52-2.36, 2.11, 1.76-1.33, 0.94, 0.87, 0.85.

Example 3(78)

(3R)-1-butyl-2,5-dioxo-3-((1R)-1-hydroxy-2-ethylbutyl)-9-(4-(4-dimethylaminocarbonyl-2-methoxyphenoxy) phenylmethyl)-1,4,9-triazaspiro[5.5]undecane hydrochloride

TLC: Rf 0.25 (ethyl acetate:methanol=10:1);
NMR($CD_3OD$): δ 7.48, 7.20, 7.10, 7.04, 6.96, 4.31, 4.12, 3.98, 3.78, 3.74-3.40, 3.17, 3.10, 3.06, 2.48-2.09, 1.73-1.28, 0.94, 0.87, 0.84.

Example 3(79)

(3R)-1-butyl-2,5-dioxo-3-((1R)-1-hydroxy-2-ethylbutyl)-9-(4-(4-methylaminocarbonylphenylmethyl)phenylmethyl)-1,4,9-triazaspiro[5.5]undecane hydrochloride

TLC: Rf 0.40 (dichloromethane:methanol:water=9:1:0.1);
NMR($CD_3OD$): δ 7.73, 7.47, 7.36, 7.31, 4.32, 4.13, 4.07, 3.99, 3.74, 3.60-3.40, 3.15, 2.89, 2.50-2.15, 1.80-1.30, 0.94, 0.87, 0.85.

Example 3(80)

(3R)-1-butyl-2,5-dioxo-3-((1R)-1-hydroxy-2-ethylbutyl)-9-(4-methoxyphenylmethyl)-1,4,9-triazaspiro[5.5]undecane hydrochloride

TLC: Rf 0.38 (dichloromethane:methanol:water=9:1:0.1);
NMR($CD_3OD$): δ 7.46, 7.03, 4.28, 4.13, 3.96, 3.83, 3.72, 3.60-3.40, 3.18, 2.50-2.10, 1.80-1.30, 0.95, 0.88, 0.85.

Example 3(81)

(3R)-1-butyl-2,5-dioxo-3-((1R)-1-hydroxy-2-ethylbutyl)-9-(4-(4-methylaminocarbonylphenylmethyloxy)phenylmethyl)-1,4,9-triazaspiro[5.5]undecane hydrochloride

TLC: Rf 0.38 (dichloromethane:methanol:water=9:1:0.1);
NMR($CD_3OD$): δ 7.82, 7.53, 7.46, 7.11, 5.21, 4.29, 4.13, 3.97, 3.73, 3.55-3.35, 3.15, 2.91, 2.50-2.10, 1.80-1.30, 0.94, 0.87, 0.85.

Example 3(82)

(3R)-1-butyl-2,5-dioxo-3-((1R)-1-hydroxy-2-ethylbutyl)-9-(4-(4-methylcarbonylaminophenylmethyl)phenylmethyl)-1,4,9-triazaspiro[5.5]undecane hydrochloride

TLC: Rf 0.64 (chloroform:methanol=5:1);
NMR($CD_3OD$): δ 7.50-7.42, 7.34, 7.15, 4.31, 4.13, 4.08-3.90, 3.97, 3.73, 3.54-3.36, 3.15, 2.50-2.18, 2.16-2.04, 2.09, 1.82-1.26, 0.94, 0.87, 0.85.

Example 3(83)

(3R)-1-butyl-2,5-dioxo-3-((1R)-1-hydroxy-1-(tetrahydropyran-4-yl)methyl)-9-(4-(4-methylaminocarbonyl-2-methoxyphenoxy)phenylmethyl)-1,4,9-triazaspiro[5.5]undecane hydrochloride

TLC: Rf 0.29 (dichloromethane:methanol=10:1);
NMR($CD_3OD$): δ 7.59, 7.50, 7.45, 7.09, 6.96, 4.31, 4.12, 4.07-3.87, 3.82, 3.72, 3.60-3.35, 3.30-3.12, 2.93, 2.56-2.23, 2.20-1.92, 1.92-1.78, 1.68, 1.55-1.13, 0.95.

Example 3(84)

(3R)-1-butyl-2,5-dioxo-3-((1R)-1-hydroxy-2-ethylbutyl)-9-(4-(4-carboxyphenylcarbonyl)phenylmethyl)-1,4,9-triazaspiro[5.5]undecane hydrochloride

TLC: Rf 0.22 (dichloromethane:methanol:water=9:1:0.1);
NMR($CD_3OD$): δ 8.17, 7.91, 7.87, 7.76, 4.49, 4.14, 4.07, 3.82, 3.60-3.40, 3.20, 2.60-2.10, 1.80-1.30, 0.95, 0.88, 0.86.

Example 3(85)

(3R)-1-butyl-2,5-dioxo-3-((1R)-1-hydroxy-2-ethylbutyl)-9-(4-(4-carboxy-2-chlorophenoxy)phenylmethyl)-1,4,9-triazaspiro[5.5]undecane hydrochloride

TLC: Rf 0.26 (dichloromethane:methanol:water=9:1:0.1);
NMR($CD_3OD$): δ 8.14, 7.96, 7.59, 7.14-7.09, 4.37, 4.14, 4.02, 3.77, 3.60-3.40, 3.15, 2.50-2.10, 1.80-1.30, 0.95, 0.88, 0.85.

Example 3(86)

(3R)-1-butyl-2,5-dioxo-3-((1R)-1-hydroxy-1-(tetrahydropyran-4-yl)methyl)-9-(4-(4-methylcarbonylaminophenoxy) phenylmethyl)-1,4,9-triazaspiro[5.5]undecane hydrochloride

TLC: Rf 0.49 (dichloromethane:methanol:water=9:1:0.1);
NMR($CD_3OD$): δ 7.57, 7.51, 7.05, 7.00, 4.32, 4.12, 4.10-3.90, 3.73, 3.60-3.10, 2.55-1.60, 2.12, 1.50-1.15, 0.95.

Example 3(87)

(3R)-1-propyl-2,5-dioxo-3-((1R)-1-hydroxy-1-cyclohexylmethyl)-9-(4-(4-methylaminocarbonylphenoxymethyl) phenylmethyl)-1,4,9-triazaspiro[5.5]undecane hydrochloride

TLC: Rf 0.42 (dichloromethane:methanol:water=9:1:0.1);
NMR($CD_3OD$): δ 7.77, 7.59, 7.04, 5.21, 4.37, 4.15, 4.00, 3.76, 3.50-3.40, 3.30, 3.15, 2.89, 2.50-1.90, 1.85-1.60, 1.55-1.10, 1.10-0.80, 0.92.

Example 3(88)

(3R)-1-propyl-2,5-dioxo-3-((1R)-1-hydroxy-1-cyclohexylmethyl)-9-(4-(4-cyano-2-methoxyphenoxy)phenylmethyl)-1,4,9-triazaspiro[5.5]undecane hydrochloride

TLC: Rf 0.42 (dichloromethane:methanol:water=9:1:0.1);
NMR($CD_3OD$): δ 7.53, 7.50, 7.36, 7.13, 7.02, 4.33, 4.15, 3.98, 3.83, 3.74, 3.55-3.40, 3.30, 3.15, 2.50-1.85, 1.85-1.60, 1.55-1.10, 1.10-0.80, 0.93.

Example 3(89)

(3R)-1-benzyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane hydrochloride

TLC: Rf 0.17 (ethyl acetate:methanol=10:1);

NMR($CD_3OD$): δ 7.73, 7.66, 7.48, 7.30-7.18, 7.07, 6.96, 4.97, 4.60, 4.32, 4.27, 3.90, 3.80, 3.70, 3.58-3.34, 2.60-2.30, 2.10-1.90, 1.80-1.60, 1.40-1.05, 1.00-0.80.

Example 3(90)

(3R)-1-benzyl-2,5-dioxo-3-((1R)-1-hydroxy-1-cyclohexylmethyl)-9-(4-(4-carboxy-2-methoxyphenylmethyl) phenylmethyl)-1,4,9-triazaspiro[5.5]undecane hydrochloride

TLC: Rf 0.21 (chloroform:methanol=10:1);
NMR($CD_3OD$): δ 7.56-7.53, 7.41, 7.32-7.18, 4.55, 4.31, 4.27, 4.02, 3.84, 3.90, 3.69, 3.45-3.30, 2.54, 2.48-2.20, 2.10-1.90, 1.80-1.60, 1.40-1.05, 1.00-0.80.

Example 3(91)

(3R)-1-butyl-2,5-dioxo-3-((1R)-1-hydroxy-1-(tetrahydropyran-4-yl)methyl)-9-(4-(2,4-dimethoxyphenoxy) phenylmethyl)-1,4,9-triazaspiro[5.5]undecane hydrochloride

TLC: Rf 0.48 (dichloromethane:methanol=10:1);
NMR($CD_3OD$): δ 7.41, 6.96, 6.88, 6.69, 6.53, 4.28, 4.12, 4.01-3.90, 3.81, 3.72, 3.66, 3.54-3.07, 2.50-1.18, 0.95.

Example 3(92)

(3R)-1-butyl-2,5-dioxo-3-((1R)-1-hydroxy-1-(tetrahydropyran-4-yl)methyl)-9-(4-(4-hydroxy-2-methoxyphenoxy) phenylmethyl)-1,4,9-triazaspiro[5.5]undecane hydrochloride

TLC: Rf 0.36 (dichloromethane:methanol=10:1);
NMR($CD_3OD$): δ 7.41, 6.88, 6.85, 6.56, 6.38, 4.27, 4.12, 4.01-3.90, 3.69, 3.69, 3.54-3.16, 2.50-1.18, 0.95.

Example 3(93)

(3R)-1-butyl-2,5-dioxo-3-((1R)-1-hydroxy-1-(tetrahydropyran-4-yl)methyl)-9-(4-(4-methylaminocarbonyl-2-chlorophenoxy)phenylmethyl)-1,4,9-triazaspiro[5.5]undecane hydrochloride

TLC: Rf 0.42 (dichloromethane:methanol=10:1);
NMR($CD_3OD$): δ 8.01, 7.78, 7.58, 7.13, 7.09, 4.36, 4.12, 4.06-3.91, 3.76, 3.48-3.08, 2.91, 2.51-1.18, 0.96.

Example 3(94)

(3R)-1-benzyl-2,5-dioxo-3-((1R)-1-hydroxy-1-cyclohexylmethyl)-9-(4-methylaminocarbonylphenylmethyl)-1,4,9-triazaspiro[5.5]undecane hydrochloride

TLC: Rf 0.45 (dichloromethane:methanol=10:1);
NMR($CD_3OD$): δ 7.86, 7.56, 7.31-7.18, 4.94, 4.58, 4.31, 4.21, 3.73, 3.57, 3.38-3.20, 2.91, 2.50, 2.33-2.24, 2.07-1.91, 1.77-1.69, 1.30-1.18, 0.98-0.90.

Example 3(95)

(3R)-1-propyl-2,5-dioxo-3-((1R)-1-hydroxy-1-cyclohexylmethyl)-9-(4-(4-dimethylaminocarbonyl-2-methoxyphenoxy) phenylmethyl)-1,4,9-triazaspiro[5.5]undecane hydrochloride

TLC: Rf 0.49 (dichloromethane:methanol=10:1);
NMR($CD_3OD$): δ 8.50, 7.20, 7.10, 7.04, 6.96, 4.30, 4.14, 3.97, 3.79, 3.72, 3.51-3.16, 3.11, 3.06, 2.51-1.14, 1.00-0.87, 0.92.

Example 3(96)

(3R)-1-butyl-2,5-dioxo-3-((1R)-1-hydroxy-1-(tetrahydropyran-4-yl)methyl)-9-(4-(4-methoxyphenylcarbonyl) phenylmethyl)-1,4,9-triazaspiro[5.5]undecane hydrochloride

TLC: Rf 0.53 (dichloromethane:methanol=10:1);
NMR($CD_3OD$): δ 7.87-7.78, 7.74, 7.06, 4.47, 4.12, 4.07, 4.00-3.86, 3.90, 3.80, 3.64-3.34, 3.30-3.15, 2.60-2.26, 2.15, 2.00, 1.92-1.78, 1.70, 1.55-1.10, 0.95.

Example 3(97)

(3R)-1-butyl-2,5-dioxo-3-((1R)-1-hydroxy-1-(tetrahydropyran-4-yl)methyl)-9-(4-(4-(2-(N,N-dimethylamino)ethyl) aminocarbonylphenoxy)phenylmethyl)-1,4,9-triazaspiro[5.5]undecane dihydrochloride

TLC: Rf 0.13 (n-butanol:acetic acid:water=4:2:1);
NMR($CD_3OD$): δ 7.93, 7.62, 7.15, 7.10, 4.36, 4.12, 4.08-3.87, 3.80-3.66, 3.60-3.33, 3.30-3.18, 2.98, 2.60-2.25, 2.19-1.92, 1.92-1.78, 1.72, 1.50-1.10, 0.95.

Example 3(98)

(3R)-1-butyl-2,5-dioxo-3-((1R)-1-hydroxy-1-(tetrahydropyran-4-yl)methyl)-9-(4-(4-methylaminocarbonylphenylmethyl)phenylmethyl)-1,4,9-triazaspiro[5.5]undecane hydrochloride

TLC: Rf 0.60 (chloroform:methanol=5:1);
NMR($CD_3OD$): δ 7.73, 7.49, 7.35, 7.30, 4.31, 4.11, 4.07, 4.00-3.86, 3.71, 3.60-3.10, 3.28, 2.89, 2.52-2.20, 2.16-1.52, 1.50-1.06, 0.94.

Example 3(99)

(3R)-1-butyl-2,5-dioxo-3-((1R)-1-hydroxy-2-ethylbutyl)-9-(4-(4-methylaminocarbonylphenylmethylaminocarbonyl) phenylmethyl)-1,4,9-triazaspiro[5.5]undecane hydrochloride

TLC: Rf 0.62 (chloroform:methanol=5:1);
NMR($CD_3OD$): δ 7.98, 7.78, 7.69, 7.44, 4.63, 4.43, 4.13, 4.02, 3.77, 3.58-3.38, 3.20, 2.90, 2.58-2.26, 2.12, 1.82-1.26, 0.94, 0.87, 0.85.

Example 3(100)

(3R)-1-pentyl-2,5-dioxo-3-((1R)-1-hydroxy-1-cyclohexylmethyl)-9-(4-methylaminocarbonylphenylmethyl)-1,4,9-triazaspiro[5.5]undecane hydrochloride

TLC: Rf 0.74 (chloroform:methanol=5:1);
NMR($CD_3OD$): δ 7.92, 7.67, 4.42, 4.15, 4.02, 3.77, 3.56-3.30, 3.30-3.08, 3.26, 2.92, 2.54-2.18, 2.16-1.85, 1.80-1.60, 1.50-1.06, 1.02-0.80, 0.91.

Example 3(101)

(3R)-1-pentyl-2,5-dioxo-3-((1R)-1-hydroxy-1-cyclohexylmethyl)-9-(4-(4-carboxyphenylmethylaminocarbonyl) phenylmethyl)-1,4,9-triazaspiro[5.5]undecane hydrochloride

TLC: Rf 0.61 (chloroform:methanol=5:1);
NMR($CD_3OD$): δ 8.02-7.96, 7.69, 7.45, 4.65, 4.43, 4.15, 4.02, 3.77, 3.58-3.36, 3.26, 3.19, 2.54-2.24, 2.08-1.86, 1.82-1.60, 1.50-1.06, 1.02-0.80, 0.91.

Example 3(102)

(3R)-1-pentyl-2,5-dioxo-3-((1R)-1-hydroxy-1-cyclohexylmethyl)-9-(4-(4-carboxymethylaminocarbonylphenoxy)phenylmethyl)-1,4,9-triazaspiro[5.5]undecane hydrochloride

TLC: Rf 0.29 (chloroform:methanol=5:1);
NMR($CD_3OD$): δ 7.90, 7.60, 7.15, 7.09, 4.34, 4.15, 4.09, 4.01, 3.75, 3.58-3.41, 3.27, 3.20, 2.56-2.26, 2.18-1.88, 1.82-1.62, 1.52-1.08, 1.04-0.80, 0.92.

Example 3.(103)

(3R)-1-butyl-2,5-dioxo-3-((1R)-1-hydroxy-1-cyclopentylmethyl)-9-(4-(4-fluorophenylmethylcarbonylamino)phenylmethyl)-1,4,9-triazaspiro[5.5]undecane hydrochloride

TLC: Rf 0.38 (dichloromethane:methanol:water=9:1:0.1);
NMR($CD_3OD$): δ 7.70, 7.49, 7.36, 7.34, 7.06, 7.03, 4.30, 3.98, 3.97, 3.73, 3.68, 3.60-3.30, 3.38, 3.20, 2.50-2.05, 1.95-1.80, 1.80-1.50, 1.50-1.10, 0.94.

Example 3(104)

(3R)-1-butyl-2,5-dioxo-3-((1R)-1-hydroxy-1-cyclopentylmethyl)-9-(4-methylsulfonylaminophenylmethyl)-1,4,9-triazaspiro[5.5]undecane hydrochloride

TLC: Rf 0.33 (dichloromethane:methanol:water=9:1:0.1);
NMR($CD_3OD$): δ 7.53, 7.34, 4.32, 3.99, 3.99, 3.73, 3.60-3.40, 3.38, 3.20, 3.01, 2.50-2.05, 1.95-1.80, 1.80-1.50, 1.50-1.10, 0.95.

Example 3(105)

(3R)-1-butyl-2,5-dioxo-3-((1R)-1-hydroxy-1-cyclopentylmethyl)-9-(4-(4-methylaminocarbonylphenylmethyloxy)phenylmethyl)-1,4,9-triazaspiro[5.5]undecane hydrochloride

TLC: Rf 0.28 (dichloromethane:methanol:water=9:1:0.1);
NMR($CD_3OD$): δ 7.82, 7.53, 7.47, 7.11, 5.20, 4.28, 3.99, 3.96, 3.71, 3.60-3.35, 3.38, 3.20, 2.91, 2.50-2.05, 1.95-1.80, 1.80-1.50, 1.50-1.10, 0.94.

Example 3(106)

(3R)-1-butyl-2,5-dioxo-3-((1R)-1-hydroxy-1-cyclopentylmethyl)-9-(4-(4-carboxyphenoxymethyl)phenylmethyl)-1,4,9-triazaspiro[5.5]undecane hydrochloride

TLC: Rf 0.29 (dichloromethane:methanol:water=9:1:0.1);
NMR($CD_3OD$): δ 7.96, 7.70-7.55, 7.05, 5.23, 4.37, 3.99, 3.99, 3.76, 3.60-3.35, 3.38, 3.20, 2.50-2.05, 1.95-1.80, 1.80-1.50, 1.50-1.10, 0.94.

Example 3(107)

(3R)-1-butyl-2,5-dioxo-3-((1R)-1-hydroxy-1-cyclopentylmethyl)-9-(4-(4-carboxy-2-methoxyphenylmethyl)phenylmethyl)-1,4,9-triazaspiro[5.5]undecane hydrochloride

TLC: Rf 0.55 (chloroform:methanol:acetic acid=10:1:1);
NMR($CD_3OD$): δ 7.58-7.55, 7.43, 7.35, 7.22, 4.30, 4.04, 3.98, 3.96, 3.86, 3.72, 3.55-3.05, 2.50-2.05, 1.94-1.12, 0.94.

Example 3(108)

(3R)-1-butyl-2,5-dioxo-3-((1R)-1-hydroxy-1-cyclopentylmethyl)-9-(4-(4-carboxy-2-methylphenoxy)phenylmethyl)-1,4,9-triazaspiro[5.5]undecane hydrochloride

TLC: Rf 0.60 (chloroform:methanol:acetic acid=10:1:1);
NMR($CD_3OD$): δ 7.98, 7.86, 7.56, 7.07, 6.92, 4.35, 4.00, 4.00, 3.76, 3.56-3.42, 3.38, 3.26-3.13, 2.54-2.09, 2.28, 1.94-1.11, 0.95.

Example 3(109)

(3R)-1-butyl-2,5-dioxo-3-((1R)-1-hydroxy-1-cyclopentylmethyl)-9-(4-(4-carboxy-3-methylphenoxy)phenylmethyl)-1,4,9-triazaspiro[5.5]undecane hydrochloride

TLC: Rf 0.71 (chloroform:methanol:acetic acid=20:1:1);
NMR($CD_3OD$): δ 7.97, 7.58, 7.16, 6.91, 6.86, 4.36, 4.00, 4.00, 3.76, 3.55-3.42, 3.38, 3.18, 2.56, 2.55-2.10, 1.94-1.14, 0.95.

Example 3(110)

(3R)-1-butyl-2,5-dioxo-3-((1R)-1-hydroxy-1-cycloheptylmethyl)-9-(4-(4-carboxyphenoxymethyl)phenylmethyl)-1,4,9-triazaspiro[5.5]undecane hydrochloride

TLC: Rf 0.23 (chloroform:methanol:acetic acid=20:1:1);
NMR($CD_3OD$): δ 7.96, 7.61, 7.57, 7.05, 5.23, 4.38, 4.15, 4.03, 3.77, 3.54-3.41, 3.34, 3.13, 2.51-2.07, 2.04-1.11, 0.95.

Example 3(111)

(3R)-1-butyl-2,5-dioxo-3-((1R)-1-hydroxy-1-cycloheptylmethyl)-9-(4-(4-carboxy-2-methylphenoxy)phenylmethyl)-1,4,9-triazaspiro[5.5]undecane hydrochloride

TLC: Rf 0.39 (chloroform:methanol:acetic acid=20:1:1);
NMR($CD_3OD$): δ 7.98, 7.86, 7.55, 7.07, 6.92, 4.35, 4.15, 4.01, 3.76, 3.54-3.41, 3.32, 3.15, 2.52-2.09, 2.28, 2.03-1.13, 0.95.

Example 3(112)

(3R)-1-butyl-2,5-dioxo-3-((1R)-1-hydroxy-1-cycloheptylmethyl)-9-(4-(4-carboxy-2-chlorophenoxy)phenylmethyl)-1,4,9-triazaspiro[5.5]undecane hydrochloride

TLC: Rf 0.32 (chloroform:methanol:acetic acid=20:1:1);
NMR($CD_3OD$): δ 8.14, 7.95, 7.60, 7.12, 7.11, 4.37, 4.15, 4.01, 3.76, 3.57-3.42, 3.32, 3.18, 2.51-2.08, 2.02-1.13, 0.95.

Example 3(113)

(3R)-1-butyl-2,5-dioxo-3-((1R)-1-hydroxy-1-cycloheptylmethyl)-9-(4-(4-carboxy-2-methoxyphenoxy)phenylmethyl)-1,4,9-triazaspiro[5.5]undecane hydrochloride

TLC: Rf 0.46 (dichloromethane:methanol:acetic acid=20:2:1);
NMR($CD_3OD$): δ 7.75, 7.68, 7.51, 7.09, 6.99, 4.32, 4.15, 3.99, 3.82, 3.73, 3.58-3.38, 3.35-3.08, 2.51-2.36, 2.27, 2.12, 2.04-1.79, 1.78-1.10, 0.95.

Example 3(114)

(3R)-1-butyl-2,5-dioxo-3-((1R)-1-hydroxy-1-cyclopentylmethyl)-9-(4-methoxyphenylmethyl)-1,4,9-triazaspiro[5.5]undecane hydrochloride

TLC: Rf 0.29 (dichloromethane:methanol:water=9:1:0.1);

NMR($CD_3OD$): δ 7.46, 7.02, 4.28, 3.99, 3.96, 3.82, 3.71, 3.60-3.35, 3.38, 3.20, 2.50-2.05, 1.95-1.80, 1.80-1.50, 1.50-1.10, 0.95.

Example 3(115)

(3R)-1-butyl-2,5-dioxo-3-((1R)-1-hydroxy-1-cyclopentylmethyl)-9-(4-(4-carboxy-2-methoxyphenoxy)phenylmethyl)-1,4,9-triazaspiro[5.5]undecane hydrochloride

TLC: Rf 0.24 (dichloromethane:methanol:water=9:1:0.1);
NMR($CD_3OD$): δ 7.75, 7.68, 7.50, 7.09, 7.00, 4.32, 3.99, 3.99, 3.82, 3.74, 3.60-3.35, 3.38, 3.20, 2.50-2.05, 1.95-1.80, 1.80-1.50, 1.50-1.10, 0.95.

Example 3(116)

(3R)-1-butyl-2,5-dioxo-3-((1R)-1-hydroxy-1-cycloheptylmethyl)-9-(4-methylsulfonylaminophenylmethyl)-1,4,9-triazaspiro[5.5]undecane hydrochloride

TLC: Rf 0.53 (chloroform:methanol=10:1);
NMR($CD_3OD$): δ 7.52, 7.34, 4.31, 4.14, 3.95, 3.70, 3.60-3.35, 3.30-3.10, 3.00, 2.60-2.20, 2.10-1.90, 1.80-1.05, 0.95.

Example 3(117)

(3R)-1-butyl-2,5-dioxo-3-((1R)-1-hydroxy-1-cycloheptylmethyl)-9-(4-methylaminocarbonylphenylmethyl)-1,4,9-triazaspiro[5.5]undecane hydrochloride

TLC: Rf 0.35 (chloroform:methanol=10:1);
NMR($CD_3OD$): δ 7.92, 7.65, 4.42, 4.15, 4.03, 3.79, 3.60-3.40, 3.31-3.05, 2.93 2.50-2.20, 2.10, 2.04-1.80, 1.80-1.05, 0.95.

Example 3(118)

(3R)-1-butyl-2,5-dioxo-3-((1R)-1-hydroxy-1-cycloheptylmethyl)-9-(4-(4-carboxyphenylcarbonyl)phenylmethyl)-1,4,9-triazaspiro[5.5]undecane hydrochloride

TLC: Rf 0.23 (chloroform:methanol=10:1);
NMR($CD_3OD$): δ 8.17, 7.90-7.78, 4.47, 4.15, 4.05, 3.77, 3.60-3.40, 3.35-3.20, 2.60, 2.50-2.35, 2.10, 2.00-1.75, 1.70-1.10, 0.94.

Example 3(119)

(3R)-1-butyl-2,5-dioxo-3-((1R)-1-hydroxy-1-cycloheptylmethyl)-9-(4-(4-carboxyphenylaminocarbonyl)phenylmethyl)-1,4,9-triazaspiro[5.5]undecane hydrochloride

TLC: Rf 0.23 (chloroform:methanol=10:1);
NMR($CD_3OD$): δ 8.08-8.01, 7.86-7.83, 7.74, 4.45, 4.15, 4.03, 3.75, 3.60-3.40, 3.35-3.15, 2.60-2.30, 2.15, 2.00-1.80, 1.70-1.10, 0.95.

Example 3(120)

(3R)-1-butyl-2,5-dioxo-3-((1R)-1-hydroxy-1-cycloheptylmethyl)-9-(4-(4-carboxy-2-methoxyphenylmethyl) phenylmethyl)-1,4,9-triazaspiro[5.5]undecane hydrochloride

TLC: Rf 0.36 (chloroform:methanol=10:1);
NMR($CD_3OD$): δ 7.57-7.54, 7.43, 7.34, 7.21, 4.28, 4.13, 4.03, 3.95, 3.70, 3.50-3.35, 3.33, 3.15, 2.50-2.35, 2.30, 2.10, 2.00-1.80, 1.75-1.10, 0.93.

Example 3(121)

(3R)-1-butyl-2,5-dioxo-3-((1R)-1-hydroxy-1-cycloheptylmethyl)-9-(4-(N-(4-carboxyphenylmethyl)-N-methylaminocarbonyl)phenylmethyl)-1,4,9-triazaspiro[5.5]undecane hydrochloride

TLC: Rf 0.21 (chloroform:methanol=10:1);
NMR($CD_3OD$): δ 8.05-8.03, 7.67-7.46, 7.29, 4.61, 4.39, 4.35, 4.14, 3.98, 3.70, 3.50-3.20, 3.07, 2.94, 2.50-2.20, 2.07-1.82, 1.75-1.15, 0.93.

Example 3(122)

(3R)-1-butyl-2,5-dioxo-3-((1R)-1-hydroxy-1-cyclopentylmethyl)-9-(4-(4-methylaminocarbonylphenoxymethyl) phenylmethyl)-1,4,9-triazaspiro[5.5]undecane hydrochloride

TLC: Rf 0.47 (dichloromethane:methanol=10:1);
NMR($CD_3OD$): δ 7.77, 7.59, 7.04, 5.21, 4.37, 4.00, 3.99, 3.76, 3.57-3.35, 3.38 3.20, 2.89, 2.52-2.18, 2.11, 1.95-1.77, 1.76-1.50, 1.50-1.08, 0.94.

Example 3(123)

(3R)-1-butyl-2,5-dioxo-3-((1R)-1-hydroxy-1-cyclopentylmethyl)-9-(4-(4-methylcarbonylaminophenylmethyl) phenylmethyl)-1,4,9-triazaspiro[5.5]undecane hydrochloride

TLC: Rf 0.44 (dichloromethane:methanol=10:1);
NMR($CD_3OD$): δ 7.46, 7.44, 7.33, 7.14, 4.30, 3.98, 3.97, 3.97, 3.72, 3.58-3.35, 3.37 3.18, 2.50-2.00, 2.09, 1.95-1.78, 1.77-1.50, 1.50-1.05, 0.94.

Example 3(124)

(3R)-1-butyl-2,5-dioxo-3-((1R)-1-hydroxy-1-cycloheptylmethyl)-9-(4-(4-carboxyphenylmethylaminocarbonyl) phenylmethyl)-1,4,9-triazaspiro[5.5]undecane hydrochloride

TLC: Rf 0.19 (dichloromethane:methanol:acetic acid=20:2:1);
NMR($CD_3OD$): δ 7.99, 7.68, 7.45, 4.65, 4.43, 4.15, 4.04, 3.78, 3.58-3.40, 3.35-3.06, 2.52-2.36, 2.28, 2.12, 2.04-1.78, 1.78-1.05, 0.95.

Example 3(125)

(3R)-1-butyl-2,5-dioxo-3-((1R)-1-hydroxy-1-cyclopentylmethyl)-9-(4-(4-carboxy-2-ethoxyphenoxy)phenylmethyl)-1,4,9-triazaspiro[5.5]undecane hydrochloride

TLC: Rf 0.37 (dichloromethane:methanol:water=9:1:0.1);
NMR($CD_3OD$): δ 7.72, 7.67, 7.50, 7.09, 7.01, 4.33, 4.08, 3.99, 3.99, 3.74, 3.60-3.35, 3.38, 3.18, 2.55-2.10, 1.95-1.80, 1.80-1.50, 1.50-1.10, 1.24, 0.95.

Example 3(126)

(3R)-1-butyl-2,5-dioxo-3-((1R)-1-hydroxy-1-cyclopentylmethyl)-9-(4-(4-carboxy-3-hydroxyphenoxy)phenylmethyl)-1,4,9-triazaspiro[5.5]undecane hydrochloride

TLC: Rf 0.74 (chloroform:methanol=5:1);
NMR($CD_3OD$): δ 7.86, 7.63, 7.19, 6.54, 6.44, 4.37, 4.01, 4.00, 3.76, 3.60-3.42, 3.39, 3.23, 2.58-2.05, 1.94-1.79, 1.78-1.08,0.95.

Example 3(127)

(3R)-1-butyl-2,5-dioxo-3-((1R)-1-hydroxy-2-ethylbutyl)-9-(4-(4-carboxyphenylthio)phenylmethyl)-1,4,9-triazaspiro [5.5]undecane hydrochloride

TLC: Rf 0.29 (dichloromethane:methanol:water=9:1:0.1);
NMR($CD_3OD$): δ 7.95, 7.58, 7.52, 7.37, 4.37, 4.14, 4.01, 3.77, 3.60-3.40, 3.20, 2.50-2.10, 1.80-1.30, 0.95, 0.88, 0.85.

Example 3(128)

(3R)-1-butyl-2,5-dioxo-3-((1R)-1-hydroxy-1-cycloheptylmethyl)-9-(4-(4-carboxymethylaminocarbonylphenoxy) phenylmethyl)-1,4,9-triazaspiro[5.5]undecane hydrochloride

TLC: Rf 0.47 (chloroform:methanol:acetic acid=10:1:1);
NMR($CD_3OD$): δ 7.90, 7.58, 7.16, 7.09, 4.36, 4.15, 4.07, 4.01, 3.75, 3.55-3.42, 3.37-3.00, 2.51-1.10, 0.95.

Example 3(129)

(3R)-1-butyl-2,5-dioxo-3-((1R)-1-hydroxy-1-cycloheptylmethyl)-9-(4-(4-carboxyphenylcarbonylaminomethyl) phenylmethyl)-1,4,9-triazaspiro[5.5]undecane hydrochloride

TLC: Rf 0.23 (chloroform:methanol=10:1);
NMR($CD_3OD$): δ 7.91, 7.84, 7.46, 7.34, 4.60, 4.11, 3.66, 3.41, 3.40, 3.20, 3.00-2.80, 2.30-2.05, 1.90-1.80, 1.75-1.20, 0.95.

Example 3(130)

(3R)-1-butyl-2,5-dioxo-3-((1R)-1-hydroxy-1-cyclopentylmethyl)-9-(4-(4-dimethylaminocarbonyl-2-methoxyphenoxy) phenylmethyl)-1,4,9-triazaspiro[5.5]undecane hydrochloride

TLC: Rf 0.32 (ethyl acetate:methanol=5:1);
NMR($CD_3OD$): δ 7.48, 7.20, 7.11, 7.05, 6.97, 4.31, 3.99, 3.98, 3.79, 3.74, 3.47-3.16, 3.11, 3.06, 2.51-1.16, 0.95.

Example 3(131)

(3R)-1-butyl-2,5-dioxo-3-((1R)-1-hydroxy-1-cyclopentylmethyl)-9-(4-(4-methylaminocarbonyl-2-chlorophenoxy) phenylmethyl)-1,4,9-triazaspiro[5.5]undecane hydrochloride

TLC: Rf 0.50 (dichloromethane:methanol=10:1);
NMR($CD_3OD$): δ 8.00, 7.77, 7.60, 7.13, 7.08, 4.35, 4.00, 3.99, 3.75, 3.59-3.41, 3.38, 3.22, 2.91, 2.56-2.04, 1.96-1.78, 1.78-1.08, 0.95.

Example 3(132)

(3R)-1-butyl-2,5-dioxo-3-((1R)-1-hydroxy-2-ethylbutyl)-9-(4-(4-(morpholin-4-ylcarbonyl)phenoxy)phenylmethyl)-1,4,9-triazaspiro[5.5]undecane hydrochloride

TLC: Rf 0.37 (dichloromethane:methanol:water=9:1:0.1);
NMR($CD_3OD$): δ 7.59, 7.48, 7.14, 7.10, 4.36, 4.14, 4.00, 3.85-3.40, 3.20, 2.55-2.25, 2.13, 1.80-1.30, 0.95, 0.88, 0.85.

Example 3(133)

(3R)-1-butyl-2,5-dioxo-3-((1R)-1-hydroxy-2-ethylbutyl)-9-(4-(4-(pyrrolidin-1-ylcarbonyl)phenoxy)phenylmethyl)-1,4,9-triazaspiro[5.5]undecane hydrochloride

TLC: Rf 0.30 (dichloromethane:methanol:water=9:1:0.1);
NMR($CD_3OD$): δ 7.58, 7.15, 7.08, 4.36, 4.14, 4.00, 3.76, 3.59, 3.55-3.40, 3.20, 2.55-2.20, 2.13, 2.05-1.85, 1.80-1.30, 0.95, 0.88, 0.85.

Example 3(134)

(3R)-1-butyl-2,5-dioxo-3-((1R)-1-hydroxy-2-ethylbutyl)-9-(4-(4-isopropylaminocarbonylphenoxy)phenylmethyl)-1,4,9-triazaspiro[5.5]undecane hydrochloride

TLC: Rf 0.27 (dichloromethane:methanol:water=9:1:0.1);
NMR($CD_3OD$): δ 7.85, 7.58, 7.15, 7.07, 4.36, 4.20, 4.14, 4.01, 3.76, 3.60-3.40, 3.20, 2.50-2.20, 2.14, 1.80-1.30, 1.25, 0.96, 0.88, 0.86.

Example 3(135)

(3R)-1-butyl-2,5-dioxo-3-((1R)-1-hydroxy-2-ethylbutyl)-9-(4-(4-(2-hydroxyethylaminocarbonyl)phenoxy)phenylmethyl)-1,4,9-triazaspiro[5.5]undecane hydrochloride

TLC: Rf 0.25 (dichloromethane:methanol:water=9:1:0.1);
NMR($CD_3OD$): δ 7.88, 7.59, 7.16, 7.08, 4.37, 4.14, 4.01, 3.77, 3.71, 3.60-3.40, 3.50, 3.17, 2.55-2.40, 2.14, 1.80-1.30, 0.95, 0.88, 0.86.

Example 3(136)

(3R)-1-butyl-2,5-dioxo-3-((1R)-1-hydroxy-2-ethylbutyl)-9-(4-(4-(2-methoxyethylaminocarbonyl)phenoxy)phenylmethyl)-1,4,9-triazaspiro[5.5]undecane hydrochloride

TLC: Rf 0.37 (dichloromethane:methanol:water=9:1:0.1);
NMR($CD_3OD$): δ 7.86, 7.58, 7.15, 7.07, 4.36, 4.14, 4.01, 3.76, 3.56, 3.56-3.40, 3.37, 3.19, 2.50-2.20, 2.14, 1.80-1.30, 0.95, 0.88, 0.86.

Example 3(137)

(3R)-1-butyl-2,5-dioxo-3-((1R)-1-hydroxy-2-ethylbutyl)-9-(4-(4-(morpholin-4-yl)carbonyl-2-methoxyphenoxy)phenylmethyl)-1,4,9-triazaspiro[5.5]undecane hydrochloride

TLC: Rf 0.40 (dichloromethane:methanol:water=9:1:0.1);
NMR($CD_3OD$): δ 7.48, 7.21, 7.12, 7.05, 6.97, 4.31, 4.13, 3.99, 3.90-3.40, 3.79, 3.17, 2.50-2.20, 2.13, 1.80-1.30, 0.95, 0.88, 0.85.

Example 3(138)

(3R)-1-butyl-2, 5-dioxo-3-((1R)-1-hydroxy-2-ethylbutyl)-9-(4-(4-(pyrrolidin-1-yl)carbonyl-2-methoxyphenoxy)phenylmethyl)-1,4,9-triazaspiro[5.5]undecane hydrochloride

TLC: Rf 0.52 (dichloromethane:methanol:water=9:1:0.1);
NMR($CD_3OD$): δ 7.48, 7.29, 7.16, 7.10, 6.97, 4.31, 4.13, 3.98, 3.79, 3.74, 3.60, 3.55-3.40, 3.17, 2.50-2.20, 2.13, 2.05-1.85, 1.80-1.30, 0.95, 0.88, 0.85.

Example 3(139)

(3R)-1-butyl-2,5-dioxo-3-((1R)-1-hydroxy-2-ethylbutyl)-9-(4-(4-isopropylaminocarbonyl-2-methoxyphenoxy)phenylmethyl)-1,4,9-triazaspiro[5.5]undecane hydrochloride

TLC: Rf 0.45 (dichloromethane:methanol:water=9:1:0.1);
NMR($CD_3OD$): δ 7.59, 7.49-7.44, 7.08, 6.97, 4.31, 4.22, 4.14, 3.99, 3.82, 3.75, 3.55-3.40, 3.16, 2.50-2.20, 2.13, 1.80-1.30, 1.26, 0.95, 0.88, 0.85.

Example 3(140)

(3R)-1-butyl-2,5-dioxo-3-((1R)-1-hydroxy-2-ethylbutyl)-9-(4-(4-(2-hydroxyethylaminocarbonyl)-2-methoxyphenoxy) phenylmethyl)-1,4,9-triazaspiro[5.5]undecane hydrochloride

TLC: Rf 0.45 (dichloromethane:methanol:water=9:1:0.1);
NMR($CD_3OD$): δ 7.63, 7.59, 7.58, 7.09, 6.97, 4.32, 4.13, 3.99, 3.83, 3.74, 3.72, 3.55-3.40, 3.52, 3.18, 2.50-2.20, 2.13, 1.80-1.30, 0.95, 0.88, 0.85.

Example 3(141)

(3R)-1-butyl-2,5-dioxo-3-((1R)-1-hydroxy-2-ethylbutyl)-9-(4-(4-(2-methoxyethylaminocarbonyl)-2-methoxyphenoxy) phenylmethyl)-1,4,9-triazaspiro[5.5]undecane hydrochloride

TLC: Rf 0.51 (dichloromethane:methanol:water=9:1:0.1);
NMR($CD_3OD$): δ 7.61, 7.50-7.45, 7.09, 6.97, 4.31, 4.13, 3.99, 3.82, 3.74, 3.57, 3.57-3.40, 3.38, 3.18, 2.50-2.20, 2.13, 1.80-1.30, 0.95, 0.88, 0.85.

Example 3(142)

(3R)-1-butyl-2,5-dioxo-3-((1R)-1-hydroxy-1-(tetrahydropyran-4-yl)methyl)-9-(4-(4-methylaminocarbonylphenylmethyloxy)phenylmethyl)-1,4,9-triazaspiro[5.5]undecane hydrochloride

TLC: Rf 0.55 (dichloromethane:methanol=5:1);
NMR($CD_3OD$): δ 7.82, 7.53, 7.45, 7.11, 5.20, 4.28, 4.11, 4.01-3.94, 3.71, 3.54-3.07, 2.91, 2.50-1.70, 1.39-1.18, 0.95.

Example 3(143)

(3R)-1-butyl-2,5-dioxo-3-((1R)-1-hydroxy-1-(tetrahydropyran-4-yl)methyl)-9-(4-(4-methylaminocarbonylphenoxymethyl)phenylmethyl)-1,4,9-triazaspiro[5.5]undecane hydrochloride

TLC: Rf 0.61 (dichloromethane:methanol=5:1);
NMR($CD_3OD$): δ 7.77, 7.59, 7.57, 7.04, 5.21, 4.37, 4.11, 4.08-3.90, 3.76, 3.54-3.12, 2.89, 2.51-1.70, 1.39-1.18, 0.95.

Example 3(144)

(3R)-1-butyl-2,5-dioxo-3-((1R)-1-hydroxy-1-(tetrahydropyran-4-yl)methyl)-9-(4-(4-carboxy-2-chlorophenoxy) phenylmethyl)-1,4,9-triazaspiro[5.5]undecane hydrochloride

TLC: Rf 0.43 (dichloromethane:methanol=5:1);
NMR($CD_3OD$): δ 8.14, 7.95, 7.60, 7.12, 7.11, 4.37, 4.12, 4.06-3.92, 3.75, 3.57-3.20, 2.51-1.71, 1.40-1.17, 0.95.

Example 3(145)

(3R)-1-butyl-2,5-dioxo-3-((1R)-1-hydroxy-1-(tetrahydropyran-4-yl)methyl)-9-(4-(4-methylcarbonylamino-2-methoxyphenoxy)phenylmethyl)-1,4,9-triazaspiro[5.5]undecane hydrochloride

TLC: Rf 0.53 (dichloromethane:methanol=5:1);
NMR($CD_3OD$): δ 7.51, 7.43, 7.07, 6.98, 6.91, 4.29, 4.11, 4.02-3.90, 3.72, 3.67, 3.53-3.16, 2.50-1.68, 1.40-1.17, 0.95.

Example 3(146)

(3R)-1-butyl-2,5-dioxo-3-((1R)-1-hydroxy-1-(tetrahydropyran-4-yl)methyl)-9-(4-(4-bromophenoxy)phenylmethyl)-1,4,9-triazaspiro[5.5]undecane hydrochloride

TLC: Rf 0.63 (dichloromethane:methanol=5:1);
NMR($CD_3OD$): δ 7.54, 7.52, 7.09, 6.97, 4.34, 4.12, 4.04-3.94, 3.73, 3.47-3.16, 2.51-1.18, 0.95.

Example 3(147)

(3R)-1-butyl-2,5-dioxo-3-((1R)-1-hydroxy-1-(tetrahydropyran-4-yl)methyl)-9-(4-(4-(morpholin-4-yl)carbonylphenoxy) phenylmethyl)-1,4,9-triazaspiro[5.5]undecane hydrochloride

TLC: Rf 0.64 (dichloromethane:methanol=5:1);
NMR($CD_3OD$): δ 7.56, 7.48, 7.14, 7.10, 4.32, 4.12, 4.00-3.90, 3.69-3.14, 2.51-1.18, 0.95.

Example 3(148)

(3R)-1-butyl-2,5-dioxo-3-((1R)-1-hydroxy-1-(tetrahydropyran-4-yl)methyl)-9-(4-(4-(pyrrolidin-1-yl)carbonylphenoxy) phenylmethyl)-1,4,9-triazaspiro[5.5]undecane hydrochloride

TLC: Rf 0.69 (dichloromethane:methanol=5:1);
NMR($CD_3OD$): δ 7.59, 7.58, 7.14, 7.08, 4.36, 4.12, 4.06-3.91, 3.75, 3.61-3.17, 2.51-1.69, 1.41-1.17, 0.95.

Example 3(149)

(3R)-1-butyl-2,5-dioxo-3-((1R)-1-hydroxy-1-(tetrahydropyran-4-yl)methyl)-9-(4-(4-methylcarbonylamino-2-methoxy-5-chlorophenylmethyl)phenylmethyl)-1,4,9-triazaspiro[5.5]undecane hydrochloride

TLC: Rf 0.73 (dichloromethane:methanol=5:1);
NMR($CD_3OD$): δ 7.44, 7.43, 7.34, 7.16, 4.30, 4.11, 4.05-3.90, 3.79, 3.73, 3.53-3.14, 2.49-1.69, 2.17, 1.38-1.21, 0.94.

Example 3(150)

(3R)-1-butyl-2,5-dioxo-3-((1R)-1-hydroxy-1-cyclopentylmethyl)-9-(4-(4-methylaminocarbonyl-2-methoxyphenoxy) phenylmethyl)-1,4,9-triazaspiro[5.5]undecane hydrochloride

TLC: Rf 0.59 (dichloromethane:methanol=5:1);
NMR($CD_3OD$): δ 7.59, 7.46, 7.45, 7.09, 6.97, 4.32, 3.99, 3.99, 3.82, 3.75, 3.53-3.07, 2.93, 2.52-1.17, 0.95.

Example 3(151)

(3R)-1-butyl-2,5-dioxo-3-((1R)-1-hydroxy-1-(tetrahydropyran-4-yl)methyl)-9-(4-(4-methylaminocarbonylphenylcarbonyl)phenylmethyl)-1,4,9-triazaspiro[5.5]undecane hydrochloride

TLC: Rf 0.44 (chloroform:methanol=10:1);
NMR($CD_3OD$): δ 7.97-7.84, 7.75, 4.49, 4.12, 4.10, 4.00-3.90, 3.80, 3.60-3.10, 2.95, 2.55-2.40, 2.30, 2.15, 2.00, 1.90-1.80, 1.70, 1.50-1.20, 0.96.

Example 3(152)

(3R)-1-butyl-2,5-dioxo-3-((1R)-1-hydroxy-1-cyclopentylmethyl)-9-(4-(4-methylaminocarbonylphenylcarbonyl) phenylmethyl)-1,4,9-triazaspiro[5.5]undecane hydrochloride

TLC: Rf 0.35 (chloroform:methanol=10:1);
NMR($CD_3OD$): δ 7.97-7.84, 7.76, 4.48, 4.15, 4.00, 3.80, 3.60-3.45, 3.39, 3.25, 2.95, 2.60-2.10, 1.95-1.80, 1.75-1.10, 0.95.

Example 3(153)

(3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenoxy)phenylmethyl)-1,4,9-triazaspiro[5.5]undecane hydrochloride

TLC: Rf 0.58 (chloroform:methanol=10:1);
NMR($CD_3OD$): δ 7.61, 7.52-7.46, 7.09, 6.97, 4.31, 4.12, 4.00-3.85, 3.83, 3.70, 3.60-3.20, 2.60-2.30, 2.20-2.00, 1.90-1.80, 1.70, 1.50-1.05, 0.95, 0.55-0.50, 0.30-0.27.

Example 3(154)

(3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenylmethyl)phenylmethyl)-1,4,9-triazaspiro[5.5]undecane hydrochloride

TLC: Rf 0.63 (chloroform:methanol=10:1);
NMR($CD_3OD$): δ 8.02, 7.47, 7.41, 7.37-7.33, 7.20, 4.28, 4.10, 4.02, 4.00-3.85, 3.86, 3.70, 3.60-3.35, 3.30-3.20, 3.07, 2.60-2.30, 2.10-1.95, 1.90-1.75, 1.65, 1.50-1.05, 0.93, 0.52-0.48, 0.29-0.21.

Example 3(155)

(3R)-1-butyl-2,5-dioxo-3-((1R)-1-hydroxy-1-(tetrahydropyran-4-yl)methyl)-9-(4-(4-methylaminocarbonyl-2-methoxyphenylmethyl)phenylmethyl)-1,4,9-triazaspiro[5.5]undecane hydrochloride

TLC: Rf 0.58 (chloroform:methanol=10:1);
NMR($CD_3OD$): δ 7.45-7.40, 7.35-7.31, 7.20, 4.30, 4.11, 4.02, 4.00-3.90, 3.86, 3.70, 3.50-3.10, 2.90, 2.50-2.15, 2.10-2.00, 1.90-1.80, 1.65, 1.50-1.10, 0.94.

Example 3(156)

(3R)-1-butyl-2,5-dioxo-3-((1R)-1-hydroxy-1-(tetrahydropyran-4-yl)methyl)-9-(4-(4-(morpholin-4-yl)carbonyl-2-methoxyphenoxy)phenylmethyl)-1,4,9-triazaspiro[5.5]undecane hydrochloride

TLC: Rf 0.72 (chloroform:methanol=5:1);
NMR($CD_3OD$): δ 7.52, 7.20, 7.11, 7.05, 6.96, 4.30, 4.11, 4.01-3.86 , 3.80, 3.80-3.18, 2.60-2.30, 2.16-1.92, 1.85, 1.68, 1.52-1.14, 0.95.

Example 3(157)

(3R)-1-butyl-2,5-dioxo-3-((1R)-1-hydroxy-1-(tetrahydropyran-4-yl)methyl)-9-(4-(4-isopropylaminocarbonyl-2-methoxyphenoxy)phenylmethyl)-1,4,9-triazaspiro[5.5]undecane hydrochloride

TLC: Rf 0.66 (chloroform:methanol=5:1);
NMR($CD_3OD$): δ 7.59, 7.52-7.42, 7.08, 6.96, 4.31, 4.22, 4.12, 4.06-3.85, 3.82, 3.72, 3.60-3.10, 2.52-2.20, 2.18-1.92, 1.84, 1.69, 1.50-1.10, 1.26, 0.95.
amorphous
softening point: about 178-183°C

Example 3(158)

(3R)-1-butyl-2,5-dioxo-3-((1R)-1-hydroxy-1-(tetrahydropyran-4-yl)methyl)-9-(4-(4-(2-methylpropyl)aminocarbonyl-2-methoxyphenoxy)phenylmethyl)-1,4,9-triazaspiro[5.5]undecane hydrochloride

TLC: Rf 0.67 (chloroform:methanol=5:1);
NMR($CD_3OD$): δ 7.60, 7.52-7.44, 7.10, 6.97, 4.31, 4.12, 4.06-3.86, 3.82, 3.71, 3.60-3.00, 2.52-2.36, 2.28, 2.18-1.62, 1.50-1.18, 0.97, 0.93.

Example 3(159)

(3R)-1-butyl-2,5-dioxo-3-((1R)-1-hydroxy-1-(tetrahydropyran-4-yl)methyl)-9-(4-(4-(2-methoxyethyl)aminocarbonyl-2-methoxyphenoxy)phenylmethyl)-1,4,9-triazaspiro[5.5]undecane hydrochloride

TLC: Rf 0.79 (chloroform:methanol=5:1);
NMR($CD_3OD$): δ 7.61, 7.52-7.44, 7.09, 6.97, 4.31, 4.12, 4.07-3.86, 3.82, 3.71, 3.57, 3.57-3.08, 2.52-2.36, 2.28, 2.20-1.92, 1.84, 1.69, 1.50-1.12, 0.95.

Example 3(160)

(3R)-1-butyl-2,5-dioxo-3-((1R)-1-hydroxy-1-(tetrahydropyran-4-yl)methyl)-9-(4-(4-isopropylaminocarbonylphenoxy) phenylmethyl)-1,4,9-triazaspiro[5.5]undecane hydrochloride

TLC: Rf 0.51 (chloroform:methanol=10:1);
NMR($CD_3OD$): δ 7.84, 7.59, 7.14, 7.07, 4.36, 4.19, 4.12, 4.06-3.90, 3.74, 3.57-3.15, 2.54-2.26, 2.16-1.16, 1.24, 0.95.

Example 3(161)

(3R)-1-butyl-2,5-dioxo-3-((1R)-1-hydroxy-1-(tetrahydropyran-4-yl)methyl)-9-(4-(4-(2-methylpropyl) aminocarbonylphenoxy)phenylmethyl)-1,4,9-triazaspiro[5.5]undecane hydrochloride

TLC: Rf 0.50 (chloroform:methanol=10:1);
NMR($CD_3OD$): δ 8.44, 7.85, 7.59, 7.15, 7.08, 4.36, 4.12, 4.06-3.90, 3.74, 3.58-3.17, 2.52-2.27, 2.16-1.15, 0.96, 0.96.

Example 3(162)

(3R)-1-butyl-2,5-dioxo-3-((1R)-1-hydroxy-1-(tetrahydropyran-4-yl)methyl)-9-(4-(4-(2-methoxyethylaminocarbonyl) phenoxy)phenylmethyl)-1,4,9-triazaspiro[5.5]undecane hydrochloride

TLC: Rf 0.50 (chloroform:methanol=10:1);
NMR($CD_3OD$): δ 8.43, 7.86, 7.61, 7.14, 7.07, 4.35, 4.12, 4.05-3.90, 3.73, 3.56-3.20, 3.37, 2.59-2.32, 2.15-1.16, 0.95.

Example 3(163)

(3R)-1-butyl-2,5-dioxo-3-((1R)-1-hydroxy-1-(tetrahydropyran-4-yl)methyl)-9-(4-(4-(2,2-dimethylpropylaminocarbonylphenoxy)phenylmethyl)-1,4,9-triazaspiro[5.5]undecane hydrochloride

TLC: Rf 0.50 (chloroform:methanol=10:1);
NMR($CD_3OD$): δ 7.85, 7.61, 7.14, 7.08, 4.36, 4.12, 4.05-3.90, 3.74, 3.57-3.23, 3.21, 2.58-2.31, 2.15-1.16, 0.96, 0.95.

Example 3(164)

(3R)-1-butyl-2,5-dioxo-3-((1R)-1-hydroxy-1-(tetrahydropyran-4-yl)methyl)-9-(4-(4-methylaminocarbonylphenylthio) phenylmethyl)-1,4,9-triazaspiro[5.5]undecane hydrochloride

TLC: Rf 0.32 (dichloromethane:methanol=10:1);
NMR($CD_3OD$): δ 7.77, 7.56, 7.47, 7.39, 4.36, 4.12, 4.08-3.88, 3.74, 3.60-3.10, 2.90, 2.55-2.25, 2.20-1.60, 1.50-1.15, 0.95.

Example 3(165)

(3R) - 1-butyl-2,5-dioxo-3-((1R)-1-hydroxy-1-cyclopentylmethyl)-9-(4-(4-carboxy-3-methoxyphenoxy)phenylmethyl)-1,4,9-triazaspiro[5.5]undecane hydrochloride

TLC: Rf 0.44 (chloroform:methanol=10:1);
NMR($CD_3OD$): δ 7.88, 7.64, 7.18, 6.80, 6.57, 4.36, 4,00, 3.99, 3.86, 3.70, 3.60-3.40, 3.38, 3.30, 2.65-2.20, 2.10, 2.00-1.75, 1.70-1.10, 0.95.

Example 3(166)

(3R)-1-butyl-2,5-dioxo-3-((1R)-1-hydroxy-1-cycloheptylmethyl)-9-(4-(4-carboxy-3-methoxyphenoxy)phenylmethyl)-1,4,9-triazaspiro[5.5]undecane hydrochloride

TLC: Rf 0.41 (chloroform:methanol=10:1);
NMR($CD_3OD$): δ 7.86, 7.62-7.59, 7.20-7.16, 6.80, 6.56, 4.36, 4.15, 4.00, 3.85, 3.70, 3.60-3.40, 3.30-3.10, 2.60-2.30, 2.20-1.80, 1.75-1.10, 0.95.

Example 3(167)

(3R)-1-butyl-2,5-dioxo-3-((1R)-1-hydroxy-1-(tetrahydropyran-4-yl)methyl)-9-(4-(4-(2,2-dimethylpropylaminocarbonyl)-2-methoxyphenoxy)phenylmethyl)-1,4,9-triazaspiro[5.5]undecane hydrochloride

TLC: Rf 0.62 (chloroform:methanol=10:1);
NMR($CD_3OD$): δ 7.59, 7.53, 7.47, 7.10, 6.96, 4.31, 4.11, 4.00-3.90, 3.82, 3.70, 3.60-3.20, 2.60-2.30, 2.20-2.00, 1.90-1.75, 1.65, 1.50-1.10, 0.97, 0.95.

Example 3(168)

(3R)-1-butyl-2,5-dioxo-3-((1R)-1-hydroxy-1-(tetrahydropyran-4-yl)methyl)-9-(4-(4-cyclopropylmethylaminocarbonylphenylmethyl)phenylmethyl)-1,4,9-triazaspiro[5.5]undecane hydrochloride

TLC: Rf 0.30 (chloroform:methanol=10:1);
NMR($CD_3OD$): δ 7.75, 7.46, 7.37, 7.31, 4.31, 4.11, 4.08, 4.04-3.89, 3.72, 3.54-3.07, 2.50-1.15, 1.08, 0.94, 0.54-0.48, 0.29-0.24.

Example 3(169)

(3R)-1-butyl-2,5-dioxo-3-((1R)-1-hydroxy-1-cyclopentylmethyl)-9-(4-(4-methylaminocarbonylphenylmethyl) phenylmethyl)-1,4,9-triazaspiro[5.5]undecane hydrochloride

TLC: Rf 0.51 (dichloromethane:methanol=10:1);
NMR($CD_3OD$): δ 7.73, 7.50-7.43, 7.37, 7.31, 4.32, 4.07, 3.98, 3.98, 3.73, 3.58-3.35, 3.37, 3.12, 2.89, 2.51-2.04, 1.95-1.76, 1.76-1.06, 0.94.

Example 3(170)

(3R)-1-butyl-2,5-dioxo-3-((1R)-1-hydroxy-1-(tetrahydropyran-4-yl)methyl)-9-(4-(4-methylsulfonylamino-2-methoxyphenoxy)phenylmethyl)-1,4,9-triazaspiro[5.5]undecane hydrochloride

TLC: Rf 0.44 (dichloromethane:methanol=10:1);
NMR($CD_3OD$): δ 7.47, 7.04, 7.02, 6.91, 6.87, 4.29, 4.11, 4.03-3.87, 3.74, 3.71, 3.60-3.30, 3.30-3.05, 2.99, 2.59-2.20, 2.17-1.55, 1.55-1.10, 0.95.

Example 3(171)

(3R)-1-butyl-2,5-dioxo-3-((1R)-1-hydroxy-2-methylpropyl)-9-(4-(4-methylsulfonylamino-2-methoxyphenoxy) phenylmethyl)-1,4,9-triazaspiro[5.5]undecane hydrochloride

TLC: Rf 0.52 (dichloromethane:methanol=10:1);
NMR($CD_3OD$): δ 7.46, 7.05, 7.03, 6.92, 6.87, 4.30, 4.14, 3.98, 3.75, 3.73, 3.58-3.35, 3.20, 3.18, 3.00, 2.52-2.18, 2.18-1.90, 1.68, 1.50-1.25, 1.04-0.88.

Example 3(172)

(3R)-1-butyl-2,5-dioxo-3-((1R)-1-hydroxy-1-(tetrahydropyran-4-yl)methyl)-9-(4-(4-(pyrrolidin-1-yl)carbonyl-2-methoxyphenoxy)phenylmethyl)-1,4,9-triazaspiro[5.5]undecane hydrochloride

TLC: Rf 0.38 (chloroform:methanol=5:1);
NMR($CD_3OD$): δ 7.49, 7.29, 7.16, 7.00, 6.97, 4.31, 4.12, 4.05-3.87 , 3.79, 3.72, 3.64-3.08, 2.52-2.24, 2.13, 2.06-1.62, 1.50-1.16, 0.95.

Example 3(173)

(3R)-1-butyl-2,5-dioxo-3-((1R)-1-hydroxy-1-(tetrahydropyran-4-yl)methyl)-9-(4-(4-methylaminocarbonyl-2-chlorophenylmethyl)phenylmethyl)-1,4,9-triazaspiro[5.5]undecane hydrochloride

TLC: Rf 0.14 (chloroform:methanol=10:1);
NMR($CD_3OD$): δ 7.85, 7.69, 7.49, 7.38, 7.34, 4.30, 4.20, 4.11, 4.00-3.90, 3.70, 3.60-3.10, 2.89, 2.50-2.20, 2.10-1.80, 1.70, 1.50-1.20, 0.94.

Example 3(174)

(3R)-1-butyl-2,5-dioxo-3-((1R)-1-hydroxy-2-ethylbutyl)-9-(4-(4-methylaminocarbonylphenylcarbonyl)phenylmethyl)-1,4,9-triazaspiro[5.5]undecane hydrochloride

TLC: Rf 0.40 (dichloromethane:methanol=10:1);
NMR($CD_3OD$): δ 7.96-7.84, 7.77, 4.48, 4.14, 4.05, 3.80, 3.60-3.40, 3.30, 2.95, 2.60-2.30, 2.10, 1.80-1.50, 1.45-1.25, 0.95, 0.88, 0.85.

Example 3(175)

(3R)-1-butyl-2,5-dioxo-3-((1R)-1-hydroxy-2-ethylbutyl)-9-(4-(4-carboxy-2-methylphenoxy)phenylmethyl)-1,4,9-triazaspiro[5.5]undecane hydrochloride

TLC: Rf 0.32 (dichloromethane: methanol= 10:1);
NMR($CD_3OD$): δ 7.97, 7.85, 7.60, 7.05, 6.92, 4.34, 4.13, 3.95, 3.85, 3.60-3.40, 3.49, 3.30, 2.60-2.30, 2.28, 2.10, 1.80-1.50, 1.45-1.25, 0.95, 0.88, 0.85.

Example 3(176)

(3R)-1-butyl-2,5-dioxo-3-((1R)-1-hydroxy-2-ethylbutyl)-9-(4-(4-carboxy-3-methoxyphenoxy)phenylmethyl)-1,4,9-triazaspiro[5.5]undecane hydrochloride

TLC: Rf 0.37 (dichloromethane:methanol=10:1);
NMR($CD_3OD$): δ 7.87, 7.65, 7.18, 6.79, 6.57, 4.36, 4.13, 4.00, 3.85, 3.70, 3.60-3.40, 3.49, 3.30, 2.70-2.40, 2.10, 1.80-1.50, 1.45-1.25, 0.95, 0.88, 0.85.

Example 3(177)

(3R)-1-butyl-2,5-dioxo-3-((1R)-1-hydroxy-1-(tetrahydropyran-4-yl)methyl)-9-(4-(4-carboxy-2-methoxyphenoxy) phenylmethyl)-1,4,9-triazaspiro[5.5]undecane hydrochloride

TLC: Rf 0.60 (dichloromethane:methanol=5:1);
NMR($CD_3OD$): δ 7.75, 7.67, 7.49, 7.09, 7.01, 4.33, 4.12, 4.06-3.90, 3.82, 3.74, 3.62-3.15, 2.52-1.18, 0.95.

Example 3(178)

(3R)-1-butyl-2,5-dioxo-3-((1R)-1-hydroxy-2-ethylbutyl)-9-(4-(4-carboxy-2-chlorophenylmethyl)phenylmethyl)-1,4,9-triazaspiro[5.5]undecane hydrochloride

TLC: Rf 0.40 (dichloromethane:methanol=10:1);
NMR($CD_3OD$): δ 8.00, 7.88, 7.49, 7.41, 7.35, 4.32, 4.22, 4.13, 4.00, 3.85, 3.60-3.40, 3.15, 2.50-2.00, 1.80-1.30, 0.94, 0.87, 0.85.

Example 3(179)

(3R)-1-butyl-2,5-dioxo-3-((1R)-1-hydroxy-2-ethylbutyl)-9-(4-(4-methylaminocarbonyl-2-chlorophenylmethyl) phenylmethyl)-1,4,9-triazaspiro[5.5]undecane hydrochloride

TLC: Rf 0.50 (dichloromethane:methanol=10:1);
NMR($CD_3OD$): δ 7.86, 7.69, 7.47, 7.39, 7.35, 4.32, 4.21, 4.13, 4.00, 3.85, 3.60-3.40, 3.15, 2.89, 2.50-2.00, 1.80-1.30, 0.94, 0.87, 0.85.

Example 3(180)

(3R)-1-butyl-2,5-dioxo-3-((1R)-1-hydroxy-2-ethylbutyl)-9-(4-(4-methylcarbonylamino-2-methoxy-5-chlorophenylmethyl)phenylmethyl)-1,4,9-triazaspiro[5.5]undecane hydrochloride

TLC: Rf 0.60 (dichloromethane:methanol=10:1);
NMR($CD_3OD$): δ 7.45, 7.43, 7.33, 7.16, 4.30, 4.12, 4.00, 3.95, 3.79, 3.75, 3.60-3.30, 3.48, 3.20, 2.50-2.00, 2.17, 1.80-1.20, 0.93, 0.87, 0.85.

Example 3(181)

(3R)-1-butyl-2,5-dioxo-3-((1R)-1-hydroxy-2-methylpropyl)-9-(4-(4-methylsulfonylamino-2-chlorophenylmethyl) phenylmethyl)-1,4,9-triazaspiro[5.5]undecane hydrochloride

TLC: Rf 0.55 (dichloromethane:methanol=10:1);
NMR($CD_3OD$): δ 7.49, 7.32-7.24, 7.14, 4.30, 4.13, 4.10, 3.95, 3.70, 3.60-3.40, 3.50-3.20, 2.96, 2.60-1.90, 1.65, 1.50-1.20, 0.98-0.90.

Example 3(182)

(3R)-1-butyl-2,5-dioxo-3-((1R)-1-hydroxy-1-(tetrahydropyran-4-yl)methyl)-9-(4-(4-carboxy-2-chlorophenylmethyl) phenylmethyl)-1,4,9-triazaspiro[5.5]undecane hydrochloride

TLC: Rf 0.40 (dichloromethane:methanol=10:1);
NMR($CD_3OD$): δ 8.00, 7.88, 7.47, 7.38, 7.34, 4.33, 4.23, 4.11, 4.05-3.90, 3.75, 3.60-3.10, 2.50-2.00, 1.90-1.80, 1.70, 1.50-1.20, 0.94.

Example 3 (183)

(3R)-1-butyl-2,5-dioxo-3-((1R)-1-hydroxy-2-ethylbutyl)-9-(4-(4-methylaminocarbonyl-2-methoxyphenylmethyl) phenylmethyl)-1,4,9-triazaspiro[5.5]undecane hydrochloride

TLC: Rf 0.37 (chloroform:methanol=10:1);
NMR($CD_3OD$): δ 0.85, 0.87, 0.93, 1.28-1.80, 2.09, 2.23-2.49, 2.90, 3.17, 3.38-3.53, 3.72, 3.86, 3.96, 4.02, 4.12, 4.29, 7.20, 7.32, 7.33, 7.40, 7.44.

Example 3(184)

(3R)-1-butyl-2,5-dioxo-3-((1R)-1-hydroxy-1-(tetrahydropyran-4-yl)methyl)-9-(1-(4-methylsulfonylaminophenyl)-3,5-dimethylpyrazol-4-ylmethyl)-1,4,9-triazaspiro[5.5]undecane dihydrochloride

TLC: Rf 0.33 (dichloromethane:methanol=10:1);
NMR($CD_3OD$): δ 7.45, 7.41, 4.30, 4.14, 4.08-3.91, 3.78, 3.63-3.53, 3.46-3.20, 3.03, 2.61-2.40, 2.40, 2.38, 2.18-1.72, 1.42-1.14, 0.96.

Example 3(185)

(3R)-1-butyl-2,5-dioxo-3-((1R)-1-hydroxy-2-ethylbutyl)-9-(4-(4-cyclopropylmethylaminocarbonylphenylmethyl) phenylmethyl)-1,4,9-triazaspiro[5.5]undecane hydrochloride

TLC: Rf 0.40 (chloroform:methanol=10:1);
NMR($CD_3OD$): δ 0.24-0.29, 0.48-0.54, 0.85, 0.87, 0.94, 1.08, 1.26-1.80, 2.07-2.49, 3.07-3.25, 3.39-3.53, 3.75, 3.99, 4.08, 4.13, 4.32, 7.31, 7.37, 7.46, 7.75, 8.45.

Example 3(186)

(3R)-1-butyl-2,5-dioxo-3-((1R)-1-hydroxy-2-ethylbutyl)-9-(4-(2-methoxy-4-methylsulfonylaminophenoxy) phenylmethyl)-1,4,9-triazaspiro[5.5]undecane hydrochloride

TLC: Rf 0.54 (dichloromethane:methanol=10:1);
NMR($CD_3OD$): δ 7.44, 7.04, 7.03, 6.92, 6.87, 4.30, 4.14, 3.98, 3.74, 3.73, 3.53-3.44, 3.16, 2.99, 2.49-2.10, 1.74-1.28, 0.95, 0.88, 0.85.

Example 3(187)

(3S)-1-butyl-2,5-dioxo-3-(2-methylpropyl)-9-(4-(2-methoxy-4-methylsulfonylaminophenoxy)phenylmethyl)-1,4,9-triazaspiro[5.5]undecane hydrochloride

TLC: Rf 0.57 (dichloromethane:methanol=10:1);
NMR($CD_3OD$): δ 7.45, 7.05, 7.03, 6.92, 6.87, 4.30, 4.00, 3.85-3.72, 3.74, 3.50-3.34, 2.99, 2.44-2.12, 1.84-1.32, 0.95, 0.94, 0.93.

Example 3(188)

(3R)-1-butyl-2,5-dioxo-3-((1R)-1-hydroxy-1-(tetrahydropyran-4-yl)methyl)-9-(4-(4-methylsulfonylamino-2,6-dimethylphenoxy)phenylmethyl)-1,4,9-triazaspiro[5.5]undecane hydrochloride

TLC: Rf 0.41 (dichloromethane:methanol=10:1);
NMR($CD_3OD$): δ 7.48, 7.04, 6.86, 4.30, 4.11, 4.03-3.90, 3.72, 3.55-3.13, 2.97, 2.50-1.15, 2.07, 0.95.

Example 3(189)

(3R)-1-butyl-2,5-dioxo-3-((1R)-1-hydroxy-2-methylpropyl)-9-(4-(4-carboxy-2,6-dimethylphenoxy)phenylmethyl)-1,4,9-triazaspiro[5.5]undecane hydrochloride

TLC: Rf 0.33 (chloroform:methanol=10:1);
NMR($CD_3OD$): δ 7.82, 7.50, 6.86, 4.30, 4.13, 4.00, 3.85, 3.60-3.40, 3.20, 3.19, 2.50-2.00, 2.14, 1.70, 1.50-1.30, 0.99-0.92.

Example 3(190)

(3R)-1-butyl-2,5-dioxo-3-((1R)-1-hydroxy-1-(tetrahydropyran-4-yl)methyl)-9-(4-(4-carboxy-2,6-dimethylphenoxy) phenylmethyl)-1,4,9-triazaspiro[5.5]undecane hydrochloride

TLC: Rf 0.20 (dichloromethane:methanol=10:1);
NMR($CD_3OD$): δ 7.82, 7.49, 6.87, 4.30, 4.11, 4.00-3.90, 3.70, 3.50-3.20, 2.50-2.00, 2.14, 1.90-1.80, 1.70, 1.50-1.20, 0.95.

Example 3(191)

(3R)-butyl-2,5-dioxo-3-((1R)-1-hydroxy-1-cyclopentylmethyl)-9-(4-(4-carboxy-2,6-dimethylphenoxy)phenylmethyl)-1,4,9-triazaspiro[5.5]undecane hydrochloride

TLC: Rf 0.34 (dichloromethane:methanol=10:1);
NMR($CD_3OD$): δ 7.83, 7.48, 6.87, 4.31, 4.00, 3.99, 3.70, 3.60-3.20, 3.38, 2.50-2.10, 2.14, 2.00-1.75, 1.70-1.10, 0.95.

Example 3(192)

(3R)-1-butyl-2,5-dioxo-3-((1R)-1-hydroxy-1-(tetrahydropyran-4-yl)methyl)-9-(4-(4-cyclopropylmethylcarbonylaminophenylmethyl)phenylmethyl)-1,4,9-triazaspiro[5.5]undecane hydrochloride

TLC: Rf 0.27 (dichloromethane:methanol=10:1);
NMR($CD_3OD$): δ 7.47-7.36, 7.35, 7.15, 4.31, 4.11, 4.10-3.80, 3.98, 3.70, 3.60-3.00, 2.60-1.60, 1.50-1.00, 0.94, 0.57-0.52, 0.24-0.21.

Example 3(193)

(3R)-1-butyl-2,5-dioxo-3-((1R)-1-hydroxy-1-(tetrahydropyran-4-yl)methyl)-9-(4-(4-isopropylcarbonylaminophenylmethyl)phenylmethyl)-1,4,9-triazaspiro[5.5]undecane hydrochloride

TLC: Rf 0.25 (dichloromethane:methanol=10:1);
NMR($CD_3OD$): δ 7.47-7.42, 7.34, 7.15, 4.31, 4.11, 4.00-3.90, 3.98, 3.70, 3.60-3.00, 2.60, 2.50-1.60, 1.50-1.20, 1.17, 0.94.

Example 3(194)

(3R)-1-butyl-2,5-dioxo-3-((1R)-1-hydroxy-1-(tetrahydropyran-4-yl)methyl)-9-(4-(4-(2-methylpropyl) carbonylaminophenylmethyl)phenylmethyl)-1,4,9-triazaspiro[5.5]undecane hydrochloride

TLC: Rf 0.27 (dichloromethane:methanol=10:1);
NMR($CD_3OD$): δ 7.47-7.42, 7.35, 7.15, 4.31, 4.11, 4.00-3.90, 3.98, 3.70, 3.50-3.00, 2.50-1.60, 1.50-1.20, 0.98, 0.94.

Example 3(195)

(3R)-1-butyl-2,5-dioxo-3-((1R)-1-hydroxy-1-(tetrahydropyran-4-yl)methyl)-9-(4-(4-cyclopropylmethylcarbonylaminophenoxy)phenylmethyl)-1,4,9-triazaspiro[5.5]undecane hydrochloride

TLC: Rf 0.25 (dichloromethane:methanol=10:1);
NMR($CD_3OD$): δ 7.59, 7.49, 7.08-6.99, 4.33, 4.12, 4.10-3.90, 3.70, 3.60-3.00, 2.26, 2.60-1.60, 1.50-1.10, 0.95, 0.59-0.55, 0.26-0.23.

Example 3(196)

(3R)-1-butyl-2,5-dioxo-3-((1R)-1-hydroxy-1-(tetrahydropyran-4-yl)methyl)-9-(4-(4-isopropylcarbonylaminophenoxy) phenylmethyl)-1,4,9-triazaspiro[5.5]undecane hydrochloride

TLC: Rf 0.27 (dichloromethane:methanol=10:1);
NMR($CD_3OD$): δ 7.58, 7.49, 7.07-6.98, 4.32, 4.12, 4.10-3.90, 3.70, 3.60-3.00, 2.62, 2.50-1.60, 1.50-1.20, 1.19, 0.96.

Example 3(197)

(3R)-1-butyl-2,5-dioxo-3-((1R)-1-hydroxy-1-(tetrahydropyran-4-yl)methyl)-9-(4-(4-(2-methylpropyl) carbonylaminophenoxy)phenylmethyl)-1,4,9-triazaspiro[5.5]undecane hydrochloride

TLC: Rf 0.25 (dichloromethane:methanol=10:1);
NMR($CD_3OD$): δ 7.58, 7.52, 7.04, 7.00, 4.32, 4.12, 4.10-3.90, 3.70, 3.60-3.00, 2.60-1.80, 1.50-1.10, 1.00, 0.95.

Example 3(198)

(3R)-1-butyl-2,5-dioxo-3-((1R)-1-hydroxy-1-(tetrahydropyran-4-yl)methyl)-9-(4-(4-cyclopropylmethylcarbonylamino-2-methoxyphenoxy)phenylmethyl)-1,4,9-triazaspiro[5.5]undecane hydrochloride

TLC: Rf 0.25 (dichloromethane:methanol=10:1);
NMR($CD_3OD$): δ 7.54, 7.45, 7.10, 6.99, 6.91, 4.29, 4.11, 4.10-3.90, 3.74, 3.70, 3.60-3.00, 2.27, 2.60-1.60, 1.50-1.10, 0.95, 0.59-0.56, 0.26-0.24.

Example 3(199)

(3R)-1-butyl-2,5-dioxo-3-((1R)-1-hydroxy-1-(tetrahydropyran-4-yl)methyl)-9-(4-(4-isopropylcarbonylamino-2-methoxyphenoxy)phenylmethyl)-1,4,9-triazaspiro[5.5]undecane hydrochloride

TLC: Rf 0.25 (dichloromethane:methanol=10:1);
NMR($CD_3OD$): δ 7.54, 7.46, 7.09, 6.98, 6.90, 4.29, 4.11, 4.00-3.80, 3.74, 3.70, 3.60-3.00, 2.63, 2.60-1.60, 1.50-1.10, 1.20, 0.95.

Example 3(200)

(3R)-1-butyl-2,5-dioxo-3-((1R)-1-hydroxy-1-(tetrahydropyran-4-yl)methyl)-9-(4-(4-(2-methylpropyl)carbonylamino-2-methoxyphenoxy)phenylmethyl)-1,4,9-triazaspiro[5.5]undecane hydrochloride

TLC: Rf 0.32 (dichloromethane:methanol=10:1);
NMR($CD_3OD$): δ 7.53, 7.45, 7.09, 6.98, 6.90, 4.25, 4.11, 4.00-3.80, 3.74, 3.70-3.00, 2.50-1.60, 1.50-1.10, 1.01, 0.95.

Example 3(201)

(3R)-1-(2-methoxyethyl)-2,5-dioxo-3-((1R)-1-hydroxy-1-cyclohexylmethyl)-9-(4-(2-methoxy-4-methylsulfonylaminophenoxy)butyl)-1,4,9-triazaspiro[5.5]undecane hydrochloride

TLC: Rf 0.29 (chloroform:methanol=5:1);
NMR($CD_3OD$): δ 6.93, 6.91, 6.79, 4.18, 4.04, 3.92, 3.84, 3.78-3.63, 3.62-3.42, 3.34-3.24, 2.90, 2.70-2.28, 2.18-1.82, 1.82-1.60, 1.40-1.08, 1.04-0.80.

Example 3(202)

(3R)-1-(2-methoxyethyl)-2,5-dioxo-3-((1R)-1-hydroxy-2-methylpropyl)-9-(4-(2-methoxy-4-methylsulfonylaminophenoxy)phenylmethyl)-1,4,9-triazaspiro[5.5]undecane hydrochloride

TLC: Rf 0.44 (chloroform:methanol=10:1);
NMR($CD_3OD$): δ 7.45, 7.03, 6.93-6.85, 4.29, 4.15, 3.80-3.60, 3.74, 3.60-3.40, 3.31, 3.20, 2.99, 2.60-2.20, 2.20-2.10, 0.98, 0.97.

Example 3(203)

(3R)-1-(2-methoxyethyl)-2,5-dioxo-3-((1R)-1-hydroxy-1-cyclohexylmethyl)-9-(4-(2-methoxy-4-methylsulfonylaminophenoxy)phenylmethyl)-1,4,9-triazaspiro[5.5]undecane hydrochloride

TLC: Rf 0.47 (chloroform:methanol=10:1);
NMR($CD_3OD$): δ 7.45, 7.03, 6.93-6.85, 4.23, 4.17, 3.90, 3.74, 3.70, 3.60-3.20, 3.31, 2.99, 2.60-2.40, 2.30, 2.10-1.80, 1.80-1.60, 1.40-1.10 1.00-0.80.

Example 3(204)

(3R)-1-ethyl-2,5-dioxo-3-((1R)-1-hydroxy-2-methylpropyl)-9-(4-(2-methoxy-4-methylsulfonylaminophenoxy) phenylmethyl)-1,4,9-triazaspiro[5.5]undecane hydrochloride

TLC: Rf 0.44 (chloroform:methanol=10:1);
NMR($CD_3OD$): δ 7.47, 7.03, 6.93-6.85, 4.30, 4.13, 3.95 3.90-3.60, 3.74, 3.50-3.20, 3.19, 2.99, 2.60-2.20, 2.20-2.10, 1.17, 0.99, 0.97.

Example 3(205)

(3R)-1-ethyl-2,5-dioxo-3-((1R)-1-hydroxy-2-methylpropyl)-9-(4-(2-methoxy-4-methylsulfonylaminophenoxy)butyl)-1,4,9-triazaspiro[5.5]undecane hydrochloride

TLC: Rf 0.44 (chloroform:methanol=5:1);
NMR($CD_3OD$): δ 6.93, 6.91, 6.79, 4.15, 4.05, 4.00-3.51, 3.84, 3.40-3.16, 2.90, 2.60-1.86, 1.40-1.14, 1.00, 0.98.

Example 3(206)

(3R)-1-ethyl-2,5-dioxo-3-((1R)-1-hydroxy-1-cyclohexyl)-9-(4-(2-methoxy-4-methylsulfonylaminophenoxy)butyl)-1,4,9-triazaspiro[5.5]undecane hydrochloride

TLC: Rf 0.42 (chloroform:methanol=5:1);
NMR($CD_3OD$): δ 6.93, 6.91, 6.79, 4.15, 4.12-3.90, 3.84, 3.82-3.50, 3.38-3.10, 2.90, 2.58-1.64, 1.46-1.08, 1.21, 1.06-0.80.

Example 3(207)

(3R)-1-(2-methoxyethyl)-2,5-dioxo-3-((1R)-1-hydroxy-2-methylpropyl)-9-(1-(4-methylsulfonylaminophenyl)-3,5-dimethylpyrazol-4-ylmethyl)-1,4,9-triazaspiro[5.5]undecane dihydrochloride

TLC: Rf 0.55 (chloroform:methanol=5:1);
NMR($CD_3OD$): δ 7.48-7.38, 4.30, 4.17, 4.01, 3.84-3.64, 3.64-3.40, 3.32-3.16, 3.03, 2.64-2.40, 2.37, 2.36, 2.20- 1.96, 1.02-0.95.

Example 4

(3R)-1-butyl-2,5-dioxo-3-((1R)-1-hydroxy-1-cyclohexylmethyl)-9-(4-phenoxybutyl)-1,4,9-triazaspiro[5.5]undecane hydrochloride

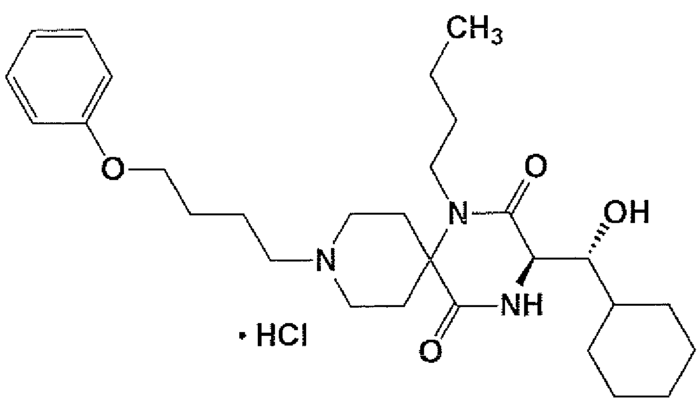

To a solution of the compound prepared in Reference Example 2 (100 mg) and 4-bromobutoxybenzene (71 mg) in dimethylformamide (2 mL) was added triethylamine (0.1 mL) and sodium iodide (58 mg). The mixture was stirred

at room temperature overnight. To the reaction mixture was added 1N hydrochloric acid and extracted with ethyl acetate. The organic layer was washed with saturated aqueous solution of sodium sulfite and brine, dried over anhydrous magnesium sulfate and concentrated. The obtained residue was purified by column chromatography on silica gel (ethyl acetate : methanol = 50 : 1) and 4N hydrogen chloride / ethyl acetate solution was added thereto. The reaction mixture was concentrated and washed with t-butylmethyl ether to give the compound of the present invention (35 mg) having the following physical data.
TLC: Rf 0.41 (dichloromethane:methanol=10:1);
NMR($CD_3OD$): δ 7.28-7.23, 6.93-6.89, 4.16, 4.05, 3.97, 3.73, 3.59-3.49, 3.33-3.17, 2.52-2.30, 2.18-1.83, 1.80-1.65, 1.42-1.18, 0.99-0.87.

Example 4(1) - 4(30)

By the same procedure as described in Example 4 using the corresponding halide compounds respectively instead of 4-bromobuthoxybenzene and using the corresponding amine derivatives respectively instead of the compound prepared in Reference Example 2, the following compounds of the present invention were obtained.

Example 4(1)

(3R)-1-butyl-2,5-dioxo-3-((1R)-1-hydroxy-1-cyclohexylmethyl)-9-(3-phenylsulfonylaminopropyl)-1,4,9-triazaspiro[5.5]undecane hydrochloride

TLC: Rf 0.42 (dichloromethane:methanol=10:1);
NMR($CD_3OD$): δ 7.90-7.83, 7.68-7.54, 4.16, 3.93, 3.68, 3.61-3.46, 3.30-3.14, 2.97, 2.59-2.28, 2.18-1.88, 1.84-1.61, 1.56-1.12, 1.08-0.80, 0.97.

Example 4(2)

(3R)-1-butyl-2,5-dioxo-3-((1R)-1-hydroxy-1-cyclohexylmethyl)-9-(3-benzyloxypropyl)-1 ,4,9-triazaspiro[5.5]undecane hydrochloride

TLC: Rf 0.50 (dichloromethane:methanol=10:1);
NMR($CD_3OD$): δ 7.36-7.28, 4.53, 4.15, 3.93, 3.69-3.45, 3.29, 3.07, 2.49-1.91, 1.80-1.63, 1.45-1.18, 1.00-0.87.

Example 4(3)

(3R)-1-butyl-2,5-dioxo-3-((1R)-1-hydroxy-1-cyclohexylmethyl)-9-(4-(4-chloromethylphenoxy)butyl)-1,4,9-triazaspiro[5.5]undecane hydrochloride

TLC: Rf 0.67 (dichloromethane:methanol=10:1);
NMR($CD_3OD$): δ 7.31, 6.91, 4.59, 4.17, 4.06, 3.98, 3.72, 3.58-3.48, 3.33-3.12, 2.52-1.88, 1.80-1.65, 1.45-1.14, 1.00-0.86.

Example 4(4)

(3R)-1-butyl-2,5-dioxo-3-((1R)-1-hydroxy-1-cyclohexylmethyl)-9-(3-phenylcarbonylaminopropyl)-1,4,9-triazaspiro[5.5]undecane hydrochloride

TLC: Rf 0.72 (dichloromethane:methanol=5:1);
NMR($CD_3OD$): δ 7.89-7.82, 7.60-7.43, 4.17, 3.96, 3.71, 3.63-3.57, 3.30-3.16, 2.62-2.31, 2.21-1.88, 1.84-1.61, 1.56-1.07, 1.07-0.80, 0.97.

Example 4(5)

(3R)-1-butyl-2,5-dioxo-3-((1R)-1-hydroxy-1-cyclohexylmethyl)-9-(1,4-benzodioxan-2-ylmethyl)-1,4,9-triazaspiro[5.5]undecane hydrochloride

TLC: Rf 0.60 (dichloromethane:methanol=10:1);
NMR($CD_3OD$): δ 7.00-6.80, 4.77, 4.32, 4.17, 4.07, 4.05, 3.82, 3.72-3.42, 3.47, 3.28-3.16, 2.65-2.30, 2.20-1.90,

1.85-1.60, 1.60-1.10, 1.00-0.80, 0.98.

Example 4(6)

(3R)-1-butyl-2,5-dioxo-3-((1R)-1-hydroxy-1-cyclohexylmethyl)-9-(4-(4-hydroxymethylphenoxy)butyl)-1,4,9-triazaspiro[5.5]undecane hydrochloride

TLC: Rf 0.52 (dichloromethane:methanol=10:1);
NMR($CD_3OD$): δ 7.25, 6.89, 4.51, 4.16, 4.04, 3.96, 3.72, 3.60-3.50, 3.33-3.17, 2.52-1.88, 1.80-1.65, 1.45-1.10, 1.00-0.87.

Example 4(7)

(3R)-1-butyl-2,5-dioxo-3-((1R)-1-hydroxy-1-cyclohexylmethyl)-9-(4-(4-hydroxyphenoxy)butyl)-1,4,9-triazaspiro[5.5]undecane hydrochloride

TLC: Rf 0.29(dichloromethane:methanol=10:1);
NMR($CD_3OD$): δ 6.76, 6.68, 4.15, 3.96, 3.77, 3.55-3.35, 3.33-3.06, 2.44-1.65, 1.40-1.18, 1.00-0.94.

Example 4(8)

(3R)-1-butyl-2,5-dioxo-3-((1R)-1-hydroxy-1-cyclohexylmethyl)-9-(4-(3-(4-methylaminocarbonylphenoxy)phenoxy)butyl)-1,4,9-triazaspiro[5.5]undecane hydrochloride

TLC: Rf 0.30 (ethyl acetate:methanol=5:1);
NMR($CD_3OD$): δ 7.81, 7.29, 7.00, 6.76, 6.63-6.61, 4.16, 4.03, 3.89, 3.64, 3.56- 3.45, 3.33-3.15, 2.90, 2.52-2.26, 2.13-1.84, 1.80-1.65, 1.45-1.18, 1.00-0.94.

Example 4(9)

(3R)-1-butyl-2,5-dioxo-3-((1R)-1-hydroxy-1-cyclohexylmethyl)-9-(4-(4-(4-methylaminocarbonylphenoxy)phenoxy)butyl)-1,4,9-triazaspiro[5.5]undecane hydrochloride

TLC: Rf 0.30 (ethyl acetate:methanol=5:1);
NMR($CD_3OD$): δ 7.76, 6.99-6.98, 6.92, 4.16, 4.06, 3.98, 3.72, 3.60-3.50, 3.33-3.20, 2.89, 2.52-2.26, 2.18-1.92, 1.80-1.65, 1.45-1.18, 1.00-0.87.

Example 4(10)

(3R)-1-butyl-2,5-dioxo-3-((1R)-1-hydroxy-1-cyclohexylmethyl)-9-(4-phenylaminobutyl)-1,4,9-triazaspiro[5.5]undecane dihydrochloride

TLC: Rf 0.45 (dichloromethane:methanol=10:1);
NMR($CD_3OD$): δ 7.65-7.51, 4.16, 3.93, 3.68, 3.62-3.43, 3.40-3.25, 3.23-3.12, 2.65, 2.58-2.40, 2.17-1.81, 1.81-1.60, 1.53-1.05, 1.05-0.80, 0.97.

Example 4(11)

(3R)-1-butyl-2,5-dioxo-3-((1R)-1-hydroxy-1-cyclohexylmethyl)-9-(4-(N-phenyl-N-methylsulfonylamino)butyl)-1,4,9-triazaspiro[5.5]undecane hydrochloride

TLC: Rf 0.50 (dichloromethane:methanol=10:1);
NMR($CD_3OD$): δ 7.48-7.33, 4.16, 3.93, 3.79, 3.67, 3.60-3.44, 3.30-3.08, 2.93, 2.56-2.25, 2.18-1.81, 1.81-1.60, 1.60-1.05, 1.03-0.81, 0.97.

Example 4(12)

(3R)-1-butyl-2,5-dioxo-3-((1R)-1-hydroxy-1-cyclohexylmethyl)-9-(7-(4-methylaminocarbonylphenoxy)heptyl)-1,4,9-triazaspiro[5.5]undecane hydrochloride

TLC: Rf 0.10 (ethyl acetate);
NMR($CD_3OD$): δ 7.76, 6.81, 4.16, 4.04, 3.93, 3.68, 3.60-3.46, 3.30-3.08, 2.89, 2.54-2.26 , 2.18-1.88, 1.86-1.62, 1.60-1.14, 1.03-0.80, 0.97.

Example 4(13)

(3R)-1-butyl-2,5-dioxo-3-((1R)-1-hydroxy-1-cyclohexylmethyl)-9-(8-(4-methylaminocarbonylphenoxy)octyl)-1,4,9-triazaspiro[5.5]undecane hydrochloride

TLC: Rf 0.11 (ethyl acetate);
NMR($CD_3OD$): δ 7.75, 6.95, 4.16, 4.03, 3.92, 3.68, 3.60-3.46, 3.30-3.08, 2.89, 2.56-2.42 , 2.33, 2.18-1.90, 1.86-1.62, 1.58-1.12, 1.02-0.80, 0.97.

Example 4(14)

(3R)-1-butyl-2,5-dioxo-3-((1R)-1-hydroxy-1-cyclohexylmethyl)-9-(4-(2-methylaminocarbonylphenoxy)butyl)-1,4,9-triazaspiro[5.5]undecane hydrochloride

TLC: Rf 0.47 (dichloromethane:methanol=10:1);
NMR($CD_3OD$): δ 7.67, 7.45, 7.12, 7.03, 4.19, 4.16, 3.96, 3.70, 3.61-3.50, 3.33-3.20, 2.94, 2.56-2.34, 2.16-1.93, 1.80-1.63, 1.45-1.18, 1.00-0.87.

Example 4(15)

(3R)-1-butyl-2,5-dioxo-3-((1R)-1-hydroxy-1-cyclohexylmethyl)-9-(4-(3-methylaminocarbonylphenoxy)butyl)-1,4,9-triazaspiro[5.5]undecane hydrochloride

TLC: Rf 0.36 (dichloromethane:methanol=10:1);
NMR($CD_3OD$): δ 7.39-7.35, 7.09, 4.16, 4.11, 3.96, 3.71, 3.61-3.49, 3.33-3.25, 2.90, 2.52-2.30, 2.17-1.88, 1.80-1.65, 1.45-1.18, 1.00-0.86.

Example 4(16)

(3R)-1-butyl-2,5-dioxo-3-((1R)-1-hydroxy-1-cyclohexylmethyl)-9-(4-(4-methoxyphenylcarbonyl)butyl)-1,4,9-triazaspiro[5.5]undecane hydrochloride

TLC: Rf 0.44 (dichloromethane:methanol=10:I);
NMR($CD_3OD$): δ 7.99, 7.00, 4.16, 3.95, 3.87, 3.70, 3.61-3.48, 3.30-3.15, 3.10, 2.59-2.28, 2.19-1.61, 1.54-1.08, 1.05-0.80, 0.97.

Example 4(17)

(3R)-1-butyl-2,5-dioxo-3-((1R)-1-hydroxy-1-cyclohexylmethyl)-9-(4-(4-dimethylaminocarbonylphenoxy)butyl)-1,4,9-triazaspiro[5.5]undecane hydrochloride

TLC: Rf 0.76 (chloroform:methanol=5:1);
NMR($CD_3OD$): δ 7.40, 7.00, 4.16, 4.10, 3.96, 3.70, 3.63-3.46, 3.34-3.18, 3.07, 2.56, 2.48-2.36, 2.18-1.83, 1.82-1.61, 1.52-1.14, 1.04-0.80, 0.97.

Example 4(18)

(3R)-1-butyl-2,5-dioxo-3-((1R)-1-hydroxy-1-cyclohexylmethyl)-9-(4-(4-cyclopentylaminocarbonylphenoxy)butyl)-1,4,9-triazaspiro[5.5]undecane hydrochloride

TLC: Rf 0.63 (chloroform:methanol=5:1);
NMR($CD_3OD$): δ 7.77, 6.97, 4.29, 4.16, 4.11, 3.97, 3.71, 3.60-3.46, 3.30-3.45, 2.56-2.22, 2.20-1.85, 1.82-1.50, 1.50-1.10, 1.02-0.86, 0.97.

Example 4(19)

(3R)-1-butyl-2,5-dioxo-3-((1R)-1-hydroxy-1-cyclohexylmethyl)-9-(4-(4-(2-methylpropylaminocarbonyl)phenoxy)butyl)-1,4,9-triazaspiro[5.5]undecane hydrochloride

TLC: Rf 0.73 (chloroform:methanol=5:1);
NMR($CD_3OD$): δ 7.78, 6.99, 4.16, 4.11, 3.98, 3.71, 3.62-3.46, 3.32-3.16, 3.17, 2.60-2.24, 2.20-1.83, 1.83-1.60, 1.50-1.10, 1.04-0.86, 0.97 0.95.

Example 4(20)

(3R)-1-butyl-2,5-dioxo-3-((1R)-1-hydroxy-1-cyclohexylmethyl)-9-(4-(4-(2-hydroxyethylaminocarbonyl)phenoxy)butyl)-1,4,9-triazaspiro[5.5]undecane hydrochloride

TLC: Rf 0.56 (chloroform:methanol=5:1);
NMR($CD_3OD$): δ 7.80, 6.98, 4.15, 4.09, 3.69, 3.58-3.42, 3.36-3.16, 2.93, 2.41-2.14, 2.08-1.97, 1.97-1.81, 1.81-1.60, 1.50-1.10, 1.04-0.84, 0.96.

Example 4(21)

(3R)-1-butyl-2,5-dioxo-3-((1R)-1-hydroxy-1-cyclohexylmethyl)-9-(4-(4-(2-(2-hydroxyethoxy)ethylaminocarbonyl) phenoxy)butyl)-1,4,9-triazaspiro[5.5]undecane hydrochloride

TLC: Rf 0.56 (chloroform:methanol=5:1);
NMR($CD_3OD$): δ 7.80, 6.99, 4.16, 4.11, 3.93, 3.73-3.46, 3.38-3.16, 2.64, 2.44, 2.16-1.84, 1.82-1.60, 1.50-1.08, 1.06-0.80, 0.96.

Example 4(22)

(3R)-1-butyl-2,5-dioxo-3-((1R)-1-hydroxy-1-cyclohexylmethyl)-9-(4-(4-(morpholin-4-ylcarbonyl)phenoxy)butyl)-1,4,9-triazaspiro[5.5]undecane hydrochloride

TLC: Rf 0.68 (chloroform:methanol=5:1);
NMR($CD_3OD$): δ 7.39, 7.00, 4.16, 4.10, 3.96, 3.82-3.44, 3.40-3.12, 2.62-2.20, 2.20-1.83, 1.82-1.60, 1.50-1.10, 1.04-0.80, 0.97.

Example 4(23)

(3R)-1-butyl-2,5-dioxo-3-((1R)-1-hydroxy-1-cyclohexylmethyl)-9-(4-(4-(furan-2-ylmethylaminocarbonyl)phenoxy) butyl)-1,4,9-triazaspiro[5.5]undecane hydrochloride

TLC: Rf 0.79 (chloroform:methanol=5:1);
NMR($CD_3OD$): δ 7.80, 7.41, 6.99, 6.34, 6.26, 4.53, 4.16, 4.11, 3.97, 3.71, 3.60-3.48, 3.34-3.16, 2.48-2.28, 2.20-1.86, 1.84-1.60, 1.48-1.12, 1.04-0.80, 0.97.

Example 4(24)

(3R)-1-butyl-2,5-dioxo-3-((1R)-1-hydroxy-1-cyclohexylmethyl)-9-(4-(4-methoxyphenylcarbonyl)phenylmethyl)-1,4,9-triazaspiro[5.5]undecane hydrochloride

TLC: Rf 0.42 (ethyl acetate:methanol=30:1);
NMR($CD_3OD$): δ 7.85-7.77, 7.74, 7.06, 4.47, 4.16, 4.06, 3.90, 3.80, 3.60-3.43 3.30-3.14, 2.59-2.28, 2.19-1.88, 1.84-1.60, 1.55-1.09, 1.06-0.80, 0.95.

Example 4(25)

(3R)-1-butyl-2,5-dioxo-3-((1R)-1-hydroxy-1-cyclohexylmethyl)-9-(4-(4-(2,2,2-trifluoroethylaminocarbonyl)phenoxy)butyl)-1,4,9-triazaspiro[5.5]undecane hydrochloride

TLC: Rf 0.68 (chloroform:methanol=5:1);
NMR($CD_3OD$): δ 7.83, 7.01, 4.16, 4.13, 4.04, 3.97, 3.71, 3.62-3.46, 3.30-3.02, 2.56-2.22, 2.13, 2.04-1.84, 1.81-1.62, 1.45-1.12, 1.02-0.80, 0.97.

Example 4(26)

(3R)-1-butyl-2,5-dioxo-3-((1R)-1-hydroxy-1-cyclohexylmethyl)-9-(4-(2-(4-methylaminocarbonylphenoxy)phenoxy)butyl)-1,4,9-triazaspiro[5.5]undecane hydrochloride

TLC: Rf 0.39 (dichloromethane:methanol=10:1);
NMR($CD_3OD$): δ 7.74, 7.24, 7.15-7.10, 7.02, 6.89, 4.17, 4.06-4.00, 3.79, 3.61-3.48, 3.36-3.16, 3.02-2.97, 2.89, 2.47-0.95.

Example 4(27)

(3R)-1-butyl-2,5-dioxo-3-((1R)-1-hydroxy-1-cyclohexylmethyl)-9-(4-(4-(N-ethyl-N-(2-butynyl)aminocarbonyl)phenoxy)butyl)-1,4,9-triazaspiro[5.5]undecane hydrochloride

TLC: Rf 0.79 (chloroform:methanol=9:1);
NMR($CD_3OD$): δ 7.43, 7.00, 4.16, 4.20-3.90, 4.11, 3.72, 3.62-3.44, 3.34-3.28, 3.26, 2.56-2.20, 2.19-1.82, 1.83, 1.80-1.62, 1.50-1.12, 1.02-0.80, 0.97.

Example 4(28)

(3R)-1-butyl-2,5-dioxo-3-((1R)-1-hydroxy-1-cyclohexylmethyl)-9-(4-(4-methylsulfonylamino-2-methoxyphenoxy)butyl)-1,4,9-triazaspiro[5.5]undecane hydrochloride

TLC: Rf 0.69 (dichloromethane:methanol=5:1);
NMR($CD_3OD$): δ 6.93, 6.91, 6.78, 4.17, 4.05, 3.99, 3.84, 3.73, 3.63-3.50, 3.30-3.12, 2.90, 2.53-1.60, 1.50-1.14, 1.00-0.87, 0.97.

Example 4(29)

(3R)-1-butyl-2,5-dioxo-3-((1R)-1-hydroxy-1-cyclohexylmethyl)-9-(4-(4-methylaminocarbonyl-2-methoxyphenoxy)butyl)-1,4,9-triazaspiro[5.5]undecane hydrochloride

TLC: Rf 0.29 (dichloromethane:methanol=10:1);
NMR($CD_3OD$): δ 7.44, 7.42, 7.00, 4.16, 4.13, 3.97, 3.89, 3.73, 3.63-3.50, 3.30-3.05, 2.90, 2.52-1.18, 1.00-0.87, 0.97.

Example 4(30)

(3R)-1-butyl-2,5-dioxo-3-((1R)-1-hydroxy-1-cyclohexylmethyl)-9-(1-phenyl-1-hydroxyiminomethyl)-1,4,9-triazaspiro [5.5]undecane

TLC: Rf 0.50 (ethyl acetate);
NMR($CDCl_3$): δ 7.48-7.40, 7.20, 6.47, 4.13, 3.98, 3.60-3.31, 2.25-1.90, 1.90-1.20, 0.97.

Example 5

(3R)-1-butyl-2,5-dioxo-3-((1R)-1-hydroxy-1-cyclohexylmethyl)-9-(3-(4-methylaminocarbonylphenoxy)propyl)-1,4,9-triazaspiro[5.5]undecane hydrochloride

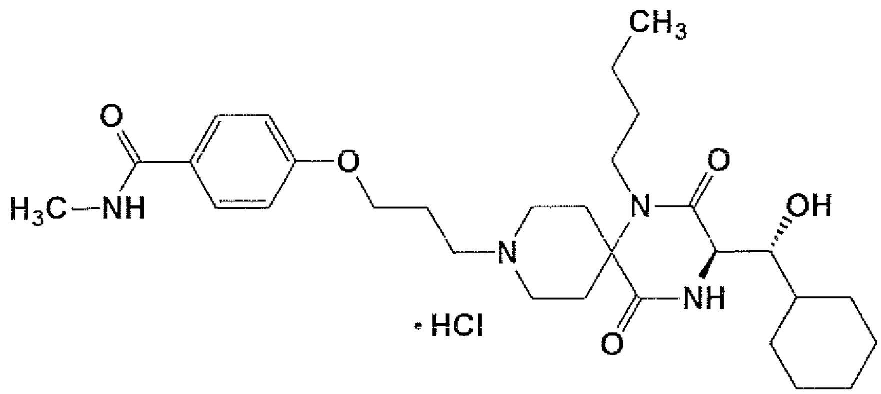

To a solution of the compound prepared in Reference Example 2 (100 mg) and 3-(4-methylaminocarbonylphenoxy)propyl methanesulfonate (89 mg) in dimethylformamide (2 mL) was added triethylamine (0.1 mL) and sodium iodide (77 mg). The mixture was stirred at room temperature overnight. To the reaction mixture was added 1N hydrochloric acid and extracted with ethyl acetate. The organic layer was washed with saturated aqueous solution of sodium sulfite and brine, dried over anhydrous magnesium sulfate and concentrated. The obtained residue was purified by column chromatography on silica gel (ethyl acetate : methanol = 30 : 1-15 : 1) and 4N hydrogen chloride / ethyl acetate solution was added thereto. The reaction mixture was concentrated and washed with t-butylmethyl ether to give the compound of the present invention (59 mg) having the following physical data.
TLC: Rf 0.21 (chloroform:methanol=10:1);
NMR($CD_3OD$): δ 7.78, 7.01, 4.17, 4.24-3.90, 4.17, 3.82-3.02, 2.89, 2.62-1.60, 1.56-0.80, 0.97.

Example 5(1) - Example 5(25)

By the same procedure as described in Example 5 using the corresponding methanesulfonic acid ester respectively instead of 3-(4-methylaminocarbonylphenoxy)propyl methanesulfonate and using the corresponding amine derivatives respectively instead of the compound prepared in Reference Example 2, the following compounds of the present invention were obtained.

Example 5(1)

(3R)-1-butyl-2,5-dioxo-3-((1R)-1-hydroxy-1-cyclohexylmethyl)-9-(2-(4-(4-methylaminocarbonylphenoxy)phenyl) ethyl)-1,4,9-triazaspiro[5.5]undecane hydrochloride

TLC: Rf 0.42 (chloroform:methanol=10:1);
NMR($CD_3OD$): δ 7.80, 7.36, 7.05, 6.99, 4.17, 4.06, 3.90-3.02, 2.90, 2.62-0.82, 0.98.

Example 5(2)

(3R)-1-butyl-2,5-dioxo-3-((1R)-1-hydroxy-1-cyclohexylmethyl)-9-(2-(4-methylaminocarbonylphenoxy)ethyl)-1,4,9-triazaspiro[5.5]undecane hydrochloride

TLC: Rf 0.17 (chloroform:methanol=10:1);
NMR($CD_3OD$): δ 7.82, 7.10, 4.46, 4.17, 4.14, 3.86, 3.76-3.42, 3.66, 3.40-3.00, 2.89, 2.60-1.60, 1.56-0.80, 0.97.

Example 5(3)

(3R)-1-butyl-2,5-dioxo-3-((1R)-1-hydroxy-1-cyclohexylmethyl)-9-(4-(4-methylaminocarbonylphenoxy)-2-butynyl)-1,4,9-triazaspiro[5.5]undecane hydrochloride

TLC: Rf 0.38 (chloroform:methanol=10:1);
NMR($CD_3OD$): δ 7.80, 7.09, 4.96, 4.24-4.02, 4.18, 3.94, 3.64-3.38, 3.36-3.04, 2.89, 2.58-1.86, 1.84-1.56, 1.52-1.10, 1.06-0.80, 0.97.

Example 5(4)

(3R)-1-butyl-2,5-dioxo-3-((1R)-1-hydroxy-1-cyclohexylmethyl)-9-(5-(4-methylaminocarbonylphenoxy)pentyl)-1,4,9-triazaspiro[5.5]undecane hydrochloride

TLC: Rf 0.27 (chloroform:methanol=10:1);
NMR($CD_3OD$): δ 7.76, 6.96, 4.16, 4.07, 3.96, 3.88-3.40, 3.32-3.06, 2.89, 2.58-2.22, 2.20-1.52, 1.50-1.10, 1.08-0.80, 0.97.

Example 5(5)

(3R)-1-butyl-2,5-dioxo-3-((1R)-1-hydroxy-1-cyclohexylmethyl)-9-((2Z)-4-(4-methylaminocarbonylphenoxy)-2-butenyl)-1,4,9-triazaspiro[5.5]undecane hydrochloride

TLC: Rf 0.30 (chloroform:methanol=10:1);
NMR($CD_3OD$): δ 7.79, 7.03, 6.26, 5.86, 4.84-4.74, 4.16, 4.04-3.92, 3.74, 3.62-3.40, 3.38-3.10, 2.89, 2.60-2.24, 2.22-1.84, 1.82-1.58, 1.56-1.08, 1.06-0.80, 0.97.

Example 5(6)

(3R)-1-butyl-2,5-dioxo-3-((1R)-1-hydroxy-1-cyclohexylmethyl)-9-(4-(4-methylaminocarbonylphenoxy)butyl)-1,4,9-triazaspiro[5.5]undecane hydrochloride

TLC: Rf 0.21 (chloroform:methanol=10:1);
NMR($CD_3OD$): δ 7.77, 6.98, 4.16, 4.16-4.06, 3.98, 3.74, 3.64-3.46, 3.40-3.08, 2.89, 2.58-2.24, 2.22-1.60, 1.58-1.10, 1.08-0.80, 0.97.

Example 5(7)

(3R)-1-butyl-2,5-dioxo-3-((1R)-1-hydroxy-1-cyclohexylmethyl)-9-(4-(5-methylaminocarbonylpyridin-2-yloxy)butyl)-1,4,9-triazaspiro[5.5]undecane dihydrochloride

TLC: Rf 0.60 (dichloromethane:methanol=5:1);
NMR($CD_3OD$): δ 8.64, 8.23, 7.02, 4.46, 4.16, 3.95, 3.70, 3.62-3.47, 3.30-3.15, 2.91, 2.61-2.28, 2.18-1.85, 1.84-1.60, 1.52-1.08, 1.05-0.80, 0.97.

Example 5(8)

(3R)-1-butyl-2,5-dioxo-3-((1R)-1-hydroxy-1-cyclohexylmethyl)-9-(4-(4-carboxyphenoxy)butyl)-1,4,9-triazaspiro[5.5] undecane hydrochloride

TLC: Rf 0.41 (dichloromethane:methanol=10:1);
NMR($CD_3OD$): δ 7.96, 6.99, 4.16, 4.12, 3.95, 3.70, 3.60-3.50, 3.35-3.20, 2.65-2.35, 2.20-1.60, 1.55-1.05, 1.00-0.80, 0.96.

Example 5(9)

(3R)-1-butyl-2,5-dioxo-3-((1R)-1-hydroxy-1-cyclohexylmethyl)-9-(2-(2-phenoxyethyloxy)ethyl)-1,4,9-triazaspiro[5.5] undecane hydrochloride

TLC: Rf 0.52 (dichloromethane:methanol=10:1);
NMR($CD_3OD$): δ 7.32-7.21, 6.98-6.88, 4.23-4.12, 4.00, 3.97-3.86, 3.75, 3.66-3.42, 3.42-3.34, 3.30-3.09, 2.53-2.22, 2.15-1.88, 1.83-1.61, 1.51-1.12, 1.04-0.81, 0.96.

Example 5(10)

(3R)-1-butyl-2,5-dioxo-3-((1R)-1-hydroxy-1-cyclohexylmethyl)-9-(2-(4-carboxyphenoxy)ethyl)-1,4,9-triazaspiro[5.5] undecane hydrochloride

TLC: Rf 0.26 (dichloromethane:methanol=10:1);
NMR($CD_3OD$): δ 8.01, 7.10, 4.50, 4.17, 4.12, 3.86, 3.75-3.50, 3.35-3.20, 2.65-2.35, 2.20-1.90, 1.85-1.60, 1.50-1.10, 1.00-0.80, 0.96.

Example 5(11)

(3R)-1-butyl-2,5-dioxo-3-((1R)-1-hydroxy-1-cyclohexylmethyl)-9-(6-(4-carboxyphenoxy)hexyl)-1,4,9-triazaspiro[5.5] undecane hydrochloride

TLC: Rf 0.30 (dichloromethane:methanol=10:1);
NMR($CD_3OD$): δ 7.95, 6.96, 4.16, 4.07, 3.93, 3.68, 3.60-3.50, 3.35-3.10, 2.60-2.30, 2.20-1.10, 1.00-0.80, 0.97.

Example 5(12)

(3R)-1-butyl-2,5-dioxo-3-((1R)-1-hydroxy-1-cyclohexylmethyl)-9-(4-(2-methylaminocarbonylpyridin-5-yloxy)butyl)-1,4,9-triazaspiro[5.5]undecane dihydrochloride

TLC: Rf 0.42 (dichloromethane:methanol=10:1);
NMR($CD_3OD$): δ 8.43, 8.22, 7.86, 4.28, 4.16, 3.96, 3.70, 3.62-3.49, 3.34-3.18, 2.97, 2.59, 2.49-2.40, 2.18-1.89, 1.83-1.61, 1.54-1.10, 1.05-0.81, 0.96.

Example 5(13)

(3R)-1-butyl-2,5-dioxo-3-((1R)-1-hydroxy-1-cyclohexylmethyl)-9-(2-(4-(N,N-bismethylsulfonylamino)phenoxy)ethyl)-1,4,9-triazaspiro[5.5]undecane hydrochloride

TLC: Rf 0.54 (dichloromethane:methanol=10:1);
NMR($CD_3OD$): δ 7.30, 7.02, 4.35, 4.06, 4.01, 3.74, 3.57-3.53, 3.44, 3.30, 3.30-3.06, 2.55-2.20, 2.10-1.80, 1.75-1.50, 1.40-1.00, 1.00-0.70, 0.86.

Example 5(14)

(3R)-1-butyl-2,5-dioxo-3-((1R)-1-hydroxy-1-cyclohexylmethyl)-9-(2-(4-methylsulfonylaminophenoxy)ethyl)-1,4,9-triazaspiro[5.5]undecane hydrochloride

TLC: Rf 0.40 (dichloromethane:methanol=10:1);
NMR($CD_3OD$): δ 7.23, 7.03, 4.39, 4.17, 4.11, 3.85, 3.70-3.50, 3.30-3.10, 2.88, 2.60-2.30, 2.20-1.90, 1.80-1.60, 1.50-1.10, 1.10-0.80, 0.97.

Example 5(15)

(3R)-1-butyl-2,5-dioxo-3-((1R)-1-hydroxy-1-cyclohexylmethyl)-9-(4-diphenylaminobutyl)-1,4,9-triazaspiro[5.5]undecane dihydrochloride

TLC: Rf 0.50 (dichloromethane:methanol=10:1);
NMR($CD_3OD$): δ 7.29-7.20, 7.02-6.96, 6.96-6.88, 4.15, 3.90, 3.80, 3.64, 3.58-3.40, 3.30-3.05, 2.52-2.21, 2.16-1.60, 1.58-1.08, 1.05-0.80, 0.97.

Example 5(16)

(3R)-1-butyl-2,5-dioxo-3-((1R)-1-hydroxy-1-cyclohexylmethyl)-9-(4-acetyl-3,4-dihydro-2H-1,4-benzoxazin-2-ylmethyl)-1,4,9-triazaspiro[5.5]undecane hydrochloride

TLC: Rf 0.53 (dichloromethane:methanol=10:1);
NMR($CD_3OD$): δ 7.40, 7.20-6.95, 4.35, 4.17, 4.12, 3.95-3.40, 3.35-3.20, 2.70-2.30, 2.35, 2.17, 2.05-1.94, 1.85-1.60, 1.60-1.10, 1.00-0.80, 0.98.

Example 5(17)

(3R)-1-butyl-2,5-dioxo-3-((1R)-1-hydroxy-1-cyclohexylmethyl)-9-((2E)-4-phenoxy-2-butenyl)-1,4,9-triazaspiro[5.5]undecane hydrochloride

TLC: Rf 0.53 (ethyl acetate:methanol=9:1);
NMR($CD_3OD$): δ 7.27, 6.95-6.91, 6.29, 6.01, 4.65, 4.16, 3.92, 3.83, 3.67, 3.55-3.40, 3.33-3.13, 2.50-2.25, 2.15-1.92, 1.80-1.60, 1.48-1.12, 0.99-0.83, 0.97.

Example 5(18)

(3R)-1-butyl-2,5-dioxo-3-((1R)-1-hydroxy-1-cyclohexylmethyl)-9-(4-methyl-3,4-dihydro-2H-1,4-benzoxazin-2-ylmethyl)-1,4,9-triazaspiro[5.5]undecane dihydrochloride

TLC: Rf 0.51 (dichloromethane:methanol=10:1);
NMR($CD_3OD$): δ 7.05-6.80, 5.00, 4.17, 4.12, 3.90-3.70, 3.70-3.40, 3.40-3.20, 3.06, 2.80-2.40, 2.20-1.90, 1.85-1.60, 1.60-1.10, 1.10-0.80, 0.98.

Example 5(19)

(3R)-1-butyl-2,5-dioxo-3-((1R)-1-hydroxy-1-cyclohexylmethyl)-9-(4-(1-phenyl-1-ethoxyiminomethyl)phenylmethyl)-1,4,9-triazaspiro[5.5]undecane hydrochloride

TLC: Rf 0.71 (dichloromethane:methanol=10:1);
NMR($CD_3OD$): δ 7.66, 7.60-7.51, 7.49-7.39, 7.39-7.23, 4.42, 4.37, 4.25-4.13, 4.12-3.91, 3.88-3.66, 3.61-3.40, 3.30-3.10, 2.57-2.22, 2.20-1.88, 1.84-1.62, 1.55-1.10, 1.26, 1.25, 1.05-0.80, 0.95, 0.95.

Example 5(20)

(3R)-1-butyl-2,5-dioxo-3-((1R)-1-hydroxy-1-cyclohexylmethyl)-9-(1,2,3,4-tetrahydronaphthalen-2-ylmethyl)-1,4,9-triazaspiro[5.5]undecane hydrochloride

TLC: Rf 0.35 (dichloromethane:methanol=10:1);
NMR($CD_3OD$): δ 7.10-7.05, 4.17, 4.02, 3.76, 3.70-3.50, 3.40-3.20, 3.02, 2.91-2.87, 2.63-2.34, 2.16-1.93, 1,85-1.60, 1.60-1.10, 1.10-0.80, 0.98.

Example 5(21)

(3R)-1-butyl-2,5-dioxo-3-((1R)-1-hydroxy-1-cyclohexylmethyl)-9-((2E)-4-(4-methylaminocarbonylphenoxy)-2-butenyl)-1,4,9-triazaspiro[5.5]undecane hydrochloride

TLC: Rf 0.40 (ethyl acetate:methanol=4:1);
NMR($CD_3OD$): δ 7.78, 7.01, 6.30, 6.01, 4.73, 4.16, 3.95, 3.84, 3.68, 3.56-3.40, 3.30-3.05, 2.89, 2.52-2.35, 2.27, 2.15, 2.07-1.91, 1.81-1.60, 1.48-1.10, 1.00-0.86, 0.97.

Example 5(22)

(3R)-1-butyl-2,5-dioxo-3-((1R)-1-hydroxy-1-cyclohexylmethyl)-9-(3,3-diphenylpropyl)-1,4,9-triazaspiro[5.5]undecane hydrochloride

TLC: Rf 0.62 (ethyl acetate:methanol=9:1);
NMR($CD_3OD$): δ 7.34-7.28, 7.23-7.17, 4.15, 4.04, 3.88, 3.63, 3.57-3.45, 3.30-3.10, 3.09-3.03, 2.60-2.52, 2.50-2.39, 2.29, 2.13-1.91, 1.80-1.60, 1.50-1.15, 1.00-0.86, 0.96.

Example 5(23)

(3R)-1-butyl-2,5-dioxo-3-((1R)-1-hydroxy-1-cyclohexylmethyl)-9-(3-phenylisoxazol-5-ylmethyl)-1,4,9-triazaspiro[5.5]undecane hydrochloride

TLC: Rf 0.93 (chloroform:methanol=5:1);
NMR($CD_3OD$): δ 7.90-7.82, 7.56-7.40, 7.24, 4.70, 4.16, 4.09, 3.84, 3.60-3.44, 3.32-3.10, 3.25, 2.60-2.24, 2.22-2.10, 2.06-1.88, 1.80-1.60, 1.50-1.12, 1.04-0.80, 0.96.

Example 5(24)

(3R)-1-butyl-2,5-dioxo-3-((1R)-1-hydroxy-1-cyclohexylmethyl)-9-(trans-(4-methylaminocarbonylphenoxymethyl)cyclopropylmethyl)-1,4,9-triazaspiro[5.5]undecane hydrochloride

TLC: Rf 0.42 (ethyl acetate:methanol=2:1);
NMR($CD_3OD$): δ 7.76, 6.97, 4.21, 4.17, 3.97, 3.85-3.47, 3.33-3.22, 3.03, 2.89, 2.55-2.32, 2.19-1.93, 1.82-1.62, 1.52-1.12, 1.00-0.79, 0.98.

Example 5(25)

(3R)-1-butyl-2,5-dioxo-3-((1R)-1-hydroxy-1-cyclohexylmethyl)-9-(3-(3-chlorophenyl)-1,2,4-oxathiazol-5-ylmethyl)-1,4,9-triazaspiro[5.5]undecane hydrochloride

TLC: Rf 0.88 (chloroform:methanol=5:1);
NMR($CD_3OD$): δ 8.13, 8.06, 7.62, 7.55, 4.95, 4.27, 4.17, 4.01, 3.84-3.70, 3.55, 3.35-3.14, 3.26, 2.64-2.32, 2.21, 2.08-1.90, 1.82-1.60, 1.58-1.10, 1.02-0.80, 0.98.

Reference Example 3

1-(2-propenyloxycarbonyl)-4-(2-(morpholin-4-yl)ethylaminocarbonyl)-4-(N-(2-butynyl)-N-((2R,3R)-2-amino-3-hydroxy-3-cyclohexylpropanoyl)amino)piperidine

To a solution of 1-(2-propenyloxycarbonyl)-4-oxopiperidine (1.05 g) in methanol (20 mL) was added (2R, 3R)-2-(t-butoxycarbonylamino)-3-cyclohexyl-3-hydroxypropanoic acid (1.5 g), 2-butynylamine hydrochloride (606 mg), diisopropylethylamine (1.0 mL) and 2-(morpholin-4-yl)ethylisocyanide (0.8 mL). The reaction mixture was stirred at 50 °C overnight. At room temperature, the reaction mixture was concentrated and saturated aqueous solution of sodium bicarbonate was added thereto. The water layer was extracted with ethyl acetate, washed with brine, dried over sodium sulfate and concentrated. The residue was dissolved in dichloromethane (10 mL) and trifluoroacetic acid (10 mL) was added thereto on ice bath. The reaction mixture was stirred at room temperature for 2 hours. The reaction mixture was neutralized with saturated aqueous solution of sodium carbonate and extracted with dichloromethane. The extract was washed with water and brine, dried over anhydrous magnesium sulfate and concentrated to give the title compound having the following physical data. The obtained residue was used in the next reaction without further purification.
TLC Rf: 0.16 (chloroform:methanol=9:1).

Reference Example 4

(3R)-1-(2-butynyl)-2,5-dioxo-3-((1R)-1-hydroxy-1-cyclohexylmethyl)-9-(2-propenyloxycarbonyl)-1,4,9-triazaspiro[5.5] undecane

A solution of the compound prepared in Reference Example 3 in 1.25 M acetic acid / ethyl acetate (20 mL) was stirred at 70 °C for 2 hours. The reaction mixture neutralized with saturated aqueous solution of sodium carbonate. The reaction mixture was extracted with ethyl acetate, washed with brine, dried over anhydrous magnesium sulfate and concentrated. The obtained residue was purified by column chromatography on silica gel (ethyl acetate : methanol = 30 : 1) to give the title compound (1.69 g) having the following physical data.
TLC Rf: 0.57 (ethyl acetate:methanol=10:1);
NMR ($CD_3OD$): δ 6.04-5.91, 5.31, 5.21, 4.61-4.58, 4.26, 4.19, 4.05-4.01, 3.90, 3.80-3.52, 3.39, 2.47-2.17, 2.02-1.60, 1.37-1.14, 1.05-0.87.

Reference Example 5

(3R)-1-(2-butynyl)-2,5-dioxo-3-((1R)-1-hydroxy-1-cyclohexylmethyl)-1,4,9-triazaspiro[5.5]undecane hydrochloride

To a solution of the compound prepared in Reference Example 4 (1.69 g) in dichloromethane (40 mL) was added acetic acid (1.4 mL), palladium tetrakistriphenylphosphine (453 mg) and tributyltin hydride (3.2 mL). The reaction mixture was stirred at room temperature for 1 hour and 1N hydrochloric acid was added thereto. The mixture was washed with dichloromethane. The water layer was diluted with 2N sodium hydroxide and extracted with ethyl acetate. The obtained organic layer was washed with brine, dried over anhydrous magnesium sulfate and 4N hydrogen chloride / ethyl acetate solution was added thereto. The reaction mixture was concentrated to give the title compound (834 mg) having the following physical data.
TLC: Rf 0.50 (dichloromethane : methanol=5 : 1);
NMR ($CD_3OD$): δ 4.36, 4.20, 4.00-3.90, 3.72, 3.42-3.30, 2.62-2.40, 2.28-1.92, 1.76-1.65, 1.38-1.15, 1.02-0.84.

Example 6

(3R)-1-(2-butynyl)-2,5-dioxo-3-((1R)-1-hydroxy-1-cyclohexylmethyl)-9-(4-(4-carboxyphenoxy)phenylmethyl)-1,4,9-triazaspiro[5.5]undecane hydrochloride

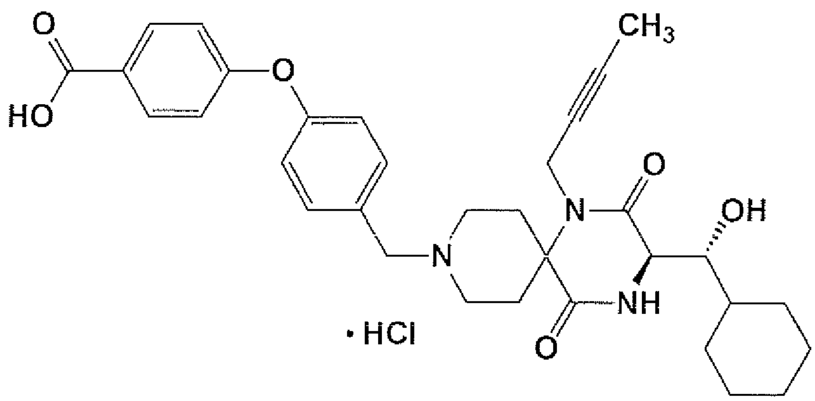

To a solution of the compound prepared in Reference Example 5 (100 mg) and 4-(4-formylphenoxy)benzoic acid (77 mg) in dimethylformamide was added triethylamine (0.04 mL) and sodium triacetoxyborohydride (91 mg) and the mixture was stirred overnight. The reaction mixture was concentrated, purified by column chromatography on silica gel (ethyl acetate : methanol = 9 : 1-5 : 1) and 4N hydrogen chloride / ethyl acetate solution was added thereto. The reaction mixture was concentrated and washed with t-butylmethyl ether to give the compound of the present invention (80 mg) having the following physical data.
TLC: Rf 0.50 (chloroform : methanol=9 : 1);
NMR($CD_3OD$): δ 8.04, 7.61, 7.18, 7.07, 4.40-4.30, 4.38, 4.20, 4.04-3.86, 3.77, 3.56-3.42, 3.30, 2.69, 2.48, 2.36, 2.20, 2.04-1.88, 1.82-1.60, 1.70, 1.40-1.12, 1.03-0.80.

Example 6(1) - 6(31)

By the same procedure as described in Example 6 using the corresponding aldehyde derivatives respectively instead of 4-(4-formylphenoxy)benzoic acid and using the corresponding amine derivatives respectively instead of the compound prepared in Reference Example 5, the following compounds of the present invention were obtained.

Example 6(1)

(3R)-1-butyl-2,5-dioxo-3-((1R)-1-hydroxy-1-(cyclohexen-4-yl)methyl)-9-(4-(4-methylaminocarbonylphenoxy)phenylmethyl)-1,4,9-triazaspiro[5.5]undecane hydrochloride

TLC: Rf 0.17 (dichloromethane:methanol=10:1);
NMR($CD_3OD$): δ 7.84, 7.62, 7.14, 7.07, 5.70-5.60, 4.36, 4.21, 4.11, 4.00, 3.74, 3.60-3.20, 2.91, 2.60-1.60, 1.50-1.25, 1.15, 0.95, 0.94.

Example 6(2)

(3R)-1-butyl-2,5-dioxo-3-((1R)-1-hydroxy-1-(cyclopenten-4-yl)methyl)-9-(4-(4-methylaminocarbonylphenoxy)phenylmethyl)-1,4,9-triazaspiro[5.5]undecane hydrochloride

TLC: Rf 0.76 (dichloromethane:methanol=5:1);
NMR($CD_3OD$): δ 7.84, 7.61, 7.14, 7.07, 5.71-5.67, 4.36, 4.12-3.95, 3.80-3.45, 3.25, 2.91, 2.76-2.02, 1.71, 1.50-1.31, 0.95.

Example 6(3)

(3R)-1-butyl-2,5-dioxo-3-((1R)-1-hydroxy-2-(tetrahydropyran-4-ylidene)ethyl)-9-(4-(4-methylaminocarbonylphenoxy) phenylmethyl)-1,4,9-triazaspiro[5.5]undecane hydrochloride

TLC: Rf 0.66 (dichloromethane:methanol:water=8:2:0.1);
NMR($CD_3OD$): δ 7.84, 7.61, 7.14, 7.07, 5.31, 4.80, 4.36, 4.12-3.10, 2.91, 2.60-2.10, 2.00, 1.70-1.30, 0.95.

Example 6(4)

(3R)-1-butyl-2,5-dioxo-3-((1R)-1-hydroxy-1-(1,3-dithian-2-yl)methyl)-9-(4-(4-methylaminocarbonylphenoxy) phenylmethyl)-1,4,9-triazaspiro[5.5]undecane hydrochloride

TLC: Rf 0.40 (ethyl acetate: methanol=4:1);
NMR($CD_3OD$): δ 7.84, 7.59, 7.15, 7.07, 4.65, 4.36, 4.14, 4.09, 3.99, 3.75, 3.56-3.43, 3.19, 3.03-2.91, 2.91, 2.68-2.11, 2.02-1.95, 1.66, 1.49-1.30, 0.95.

Example 6(5)

(3R)-1-butyl-2,5-dioxo-3-((1R)-1-hydroxy-1-(2,6-dimethylphenyl)methyl)-9-(4-(4-methylaminocarbonylphenoxy) phenylmethyl)-1,4,9-triazaspiro[5.5]undecane hydrochloride

TLC: Rf 0.44 (dichloromethane:methanol=10:1);
NMR($CD_3OD$): δ 7.84, 7.58, 7.15-6.94, 5.47, 4.34, 4.32, 4.29, 4.00, 3.60-3.05, 2.91, 2.41, 2.41, 2.20-2.00, 1.65, 1.45-1.25, 0.93.

Example 6(6)

(3R)-1-butyl-2,5-dioxo-3-((1R)-1-hydroxy-1-(cyclopenten-4-yl)methyl)-9-(4-(4-carboxy-2-methoxyphenylmethyl) phenylmethyl)-1,4,9-triazaspiro[5.5]undecane hydrochloride

TLC: Rf 0.57 (chloroform:methanol:acetic acid=10:1:1);
NMR($CD_3OD$): δ 7.58-7.55, 7.45, 7.34, 7.22, 5.66, 4.30, 4.04, 3.99, 3.97, 3.86, 3.72, 3.55-3.38, 3.19, 2.70, 2.58-2.02, 1.68, 1.50-1.27, 0.94.

Example 6(7)

(3R)-1-butyl-2,5-dioxo-3-((1R)-1-hydroxy-1-(cyclopenten-4-yl)methyl)-9-(4-(4-carboxy-2-methylphenoxy) phenylmethyl)-1,4,9-triazaspiro[5.5]undecane hydrochloride

TLC: Rf 0.60 (chloroform:methanol:acetic acid=10:1:1);
NMR($CD_3OD$): δ 7.98, 7.86, 7.56, 7.07, 6.93, 5.67, 4.35, 4.00, 4.00, 3.76, 3.55-3.42, 3.18, 2.71, 2.59-2.00, 2.28, 1.77-1.29, 0.95.

Example 6(8)

(3R)-1-butyl-2,5-dioxo-3-((1R)-1-hydroxy-1-(cyclopenten-4-yl)methyl)-9-(4-(4-carboxy-3-methylphenoxy) phenylmethyl)-1,4,9-triazaspiro[5.5]undecane hydrochloride

TLC: Rf 0.66 (chloroform:methanol:acetic acid=20:1:1);
NMR($CD_3OD$): δ 7.97, 7.58, 7.16, 6.91, 6.86, 5.67, 4.37, 4.02, 4.00, 3.77, 3.57-3.44, 3.18, 2.71, 2.59-2.00, 2.56, 1.77-1.28, 0.96.

Example 6(9)

(3R)-1-butyl-2,5-dioxo-3-((1R)-1-hydroxy-1-(cyclopenten-4-yl)methyl)-9-(4-(4-carboxyphenoxymethyl)phenylmethyl)-1,4,9-triazaspiro[5.5]undecane hydrochloride

TLC: Rf 0.58 (chloroform:methanol:acetic acid=10:1:1);
NMR($CD_3OD$): δ 7.96, 7.60, 7.56, 7.05, 5.66, 5.23, 4.34, 4.00, 3.98, 3.72, 3.53, 3.53-3.38, 3.17, 2.70, 2.59-2.03, 1.74-1.29, 0.94.

Example 6(10)

(3R)-1-butyl-2,5-dioxo-3-((1R)-1-hydroxy-1-(cyclopenten-4-yl)methyl)-9-(4-(4-carboxyphenoxy)phenylmethyl)-1,4,9-triazaspiro[5.5]undecane hydrochloride

TLC: Rf 0.20 (dichloromethane:methanol=10:1);
NMR($CD_3OD$): δ 8.04, 7.58, 7.18, 7.07, 5.67, 4.37, 4.06, 4.01, 3.76, 3.55-3.46, 3.16, 2.76-1.28, 0.96.

Example 6(11)

(3R)-1-butyl-2,5-dioxo-3-((1R)-1-hydroxy-1-(cyclopenten-4-yl)methyl)-9-(4-(4-aminosulfonylphenoxy)phenylmethyl)-1,4,9-triazaspiro[5.5]undecane hydrochloride

TLC: Rf 0.34 (dichloromethane:methanol=10:1);
NMR($CD_3OD$): δ 7.91, 7.59, 7.19, 7.14, 5.67, 4.38, 4.03, 4.01, 3.78, 3.55-3.47, 3.17, 2.76-1.28, 0.96.

Example 6(12)

(3R)-1-butyl-2,5-dioxo-3-((1R)-1-hydroxy-1-(cyclopenten-4-yl)methyl)-9-(4-(4-fluorophenylmethylcarbonylamino) phenylmethyl)-1,4,9-triazaspiro[5.5]undecane hydrochloride

TLC: Rf 0.32 (dichloromethane:methanol=10:1);
NMR($CD_3OD$): δ 7.71, 7.47, 7.35, 7.05, 5.66, 4.31, 3.99, 3.99, 3.74, 3.68, 3.52, 3.50-3.40, 3.18, 2.75-1.28, 0.94.

Example 6(13)

(3R)-1-butyl-2,5-dioxo-3-((1R)-1-hydroxy-1-(cyclopenten-4-yl)methyl)-9-(4-(4-methylaminocarbonylphenoxymethyl) phenylmethyl)-1,4,9-triazaspiro[5.5]undecane hydrochloride

TLC: Rf 0.46 (dichloromethane:methanol=10:1);
NMR($CD_3OD$): δ 7.77, 7.59, 7.04, 5.70-5.62, 5.21, 4.37, 4.00, 3.99, 3.76, 3.60-3.35, 3.53 3.21, 2.89, 2.72, 2.60-1.95, 1.67, 1.52-1.24, 0.94.

Example 6(14)

(3R)-1-butyl-2,5-dioxo-3-((1R)-1-hydroxy-1-(cyclopenten-4-yl)methyl)-9-(4-(4-methylcarbonylaminophenylmethyl) phenylmethyl)-1,4,9-triazaspiro[5.5]undecane hydrochloride

TLC: Rf 0.43 (dichloromethane:methanol=10:1);
NMR($CD_3OD$): δ 7.46, 7.44, 7.33, 7.25, 5.71-5.61, 4.31, 3.99, 3.99, 3.97, 3.86-3.35, 3.51 3.17, 2.71, 2.60-1.80, 2.09, 1.67, 1.52-1.25, 0.94.

Example 6(15)

(3R)-1-butyl-2,5-dioxo-3-((1R)-1-hydroxy-1-(cyclopenten-4-yl)methyl)-9-(4-(4-carboxy-3-hydroxyphenoxy) phenylmethyl)-1,4,9-triazaspiro[5.5]undecane hydrochloride

TLC: Rf 0.63 (chloroform:methanol=5:1);
NMR($CD_3OD$): δ 7.78, 7.47, 7.02-6.95, 6.44-6.38, 5.66, 4.25, 4.00, 3.91, 3.82-3.39, 3.18, 2.82-2.38, 2.30-2.00, 1.64,

1.50-1.14, 1.08-0.78.

Example 6(16)

(3R)-1-butyl-2,5-dioxo-3-((1R)-1-hydroxy-1-(cyclopenten-4-yl)methyl)-9-(4-methoxyphenylmethyl)-1,4,9-triazaspiro [5.5]undecane hydrochloride

TLC: Rf 0.43 (chloroform:methanol=10:1);
NMR($CD_3OD$): δ 7.45, 7.03, 5.66, 4.29, 3.99, 3.97, 3.82, 3.70, 3.52, 3.60-3.40, 3.15, 2.70, 2.60-2.00, 1.70, 1.50-1.20, 0.95.

Example 6(17)

(3R)-1-butyl-2,5-dioxo-3-((1R)-1-hydroxy-1-(cyclopenten-4-yl)methyl)-9-(4-(4-methylaminocarbonylphenylmethyloxy) phenylmethyl)-1,4,9-triazaspiro[5.5]undecane hydrochloride

TLC: Rf 0.27 (chloroform:methanol=10:1);
NMR($CD_3OD$): δ 7.82, 7.53-7.48, 7.09, 5.65, 5.19, 4.27, 3.99, 3.93, 3.65, 3.53, 3.50-3.40, 3.26, 2.91, 2.70, 2.60-2.30, 2.20-2.00, 1.70, 1.50-1.30, 0.94.

Example 6(18)

(3R)-1-butyl-2,5-dioxo-3-((1R)-1-hydroxy-1-(cyclopenten-4-yl)methyl)-9-(4-(4-carboxy-2-ethoxyphenoxy) phenylmethyl)-1,4,9-triazaspiro[5.5]undecane hydrochloride

TLC: Rf 0.15 (chloroform:methanol=10:1);
NMR($CD_3OD$): δ 7.70, 7.68, 7.48, 7.08, 7.02, 5.66, 4.33, 4.08, 4.00, 3.98, 3.75, 3.55-3.40, 3.20, 2.70, 2.60-2.40, 2.30-2.00, 1.70, 1.60-1.30, 1.24, 0.95.
amorphous
softening point: about 157-161°C

Example 6(19)

(3R)-1-butyl-2,5-dioxo-3-((1R)-1-hydroxy-1-(cyclopenten-4-yl)methyl)-9-(4-(4-methylcarbonylamino-2-methoxyphenoxy)phenylmethyl)-1,4,9-triazaspiro[5.5]undecane hydrochloride

TLC: Rf 0.32 (chloroform:methanol=10:1);
NMR($CD_3OD$): δ 7.51, 7.43, 7.07, 6.98, 6.90, 5.66, 4.29, 4.00 3.99, 3.73, 3.70, 3.53, 3.60-3.40, 3.15, 2.75, 2.60-2.00, 2.13, 1.70, 1.50-1.30, 0.95.

Example 6(20)

(3R)-1-butyl-2,5-dioxo-3-((1R)-1-hydroxy-1-(cyclopenten-4-yl)methyl)-9-(4-(2,4-dimethoxyphenoxy)phenylmethyl)-1,4,9-triazaspiro[5.5]undecane hydrochloride

TLC: Rf 0.56 (ethyl acetate:methanol=10:1);
NMR($CD_3OD$): δ 7.43, 6.96, 6.88, 6.69, 6.53, 5.71-5.61, 4.28, 3.99, 3.98, 3.81, 3.72, 3.72, 3.53, 3.53-3.35, 3.18, 2.70, 2.60-1.98, 1.68, 1.52-1.26, 0.95.

Example 6(21)

(3R)-1-butyl-2,5-dioxo-3-((1R)-1-hydroxy-1-(cyclopenten-4-yl)methyl)-9-(4-(4-hydroxy-2-methoxyphenoxy) phenylmethyl)-1,4,9-triazaspiro[5.5]undecane hydrochloride

TLC: Rf 0.44 (ethyl acetate:methanol=10:1);
NMR($CD_3OD$): δ 7.42, 6.88, 6.85, 6.56, 6.38, 5.71-5.61, 4.28, 3.99, 3.97, 3.72, 3.69, 3.53, 3.53-3.35, 3.18, 2.70, 2.60-1.98, 1.68, 1.52-1.26, 0.95.

Example 6(22)

(3R)-1-butyl-2,5-dioxo-3-((1R)-1-hydroxy-1-(cyclopenten-4-yl)methyl)-9-(4-(4-(2-dimethylaminoethylaminocarbonyl)phenoxy)phenylmethyl)-1,4,9-triazaspiro[5.5]undecane dihydrochloride

TLC: Rf 0.19 (chloroform:methanol:acetic acid=10:1:1);
NMR($CD_3OD$): δ 7.93, 7.63, 7.15, 7.10, 5.67, 4.36, 4.00, 3.99, 3.76, 3.75, 3.57-3.42, 3.38, 3.27, 2.98, 2.72, 2.60-2.00, 1.78-1.30, 0.95.

Example 6(23)

(3R)-1-butyl-2,5-dioxo-3-((1R)-1-hydroxy-1-(cyclopenten-4-yl)methyl)-9-(4-(4-methylcarbonylaminophenoxy)phenylmethyl)-1,4,9-triazaspiro[5.5]undecane hydrochloride

TLC: Rf 0.70 (chloroform:methanol=5:1);
NMR($CD_3OD$): δ 7.60-7.50, 7.06-6.97, 5.66, 4.32, 4.00, 3.98, 3.74, 3.58-3.38, 3.20, 2.70, 2.60-1.92, 2.12, 1.70, 1.52-1.28, 0.95.

Example 6(24)

(3R)-1-butyl-2,5-dioxo-3-((1R)-1-hydroxy-1-(cyclopenten-4-yl)methyl)-9-(4-(4-methylsulfonylaminophenoxy)phenylmethyl)-1,4,9-triazaspiro[5.5]undecane hydrochloride

TLC: Rf 0.74 (ethyl acetate:methanol=5:1);
NMR($CD_3OD$): δ 7.50, 7.29, 7.07, 7.03, 5.67, 4.28, 4.00, 3.96-3.20, 2.96, 2.73-1.28, 0.96.

Example 6(25)

(3R)-1-butyl-2,5-dioxo-3-((1R)-1-hydroxy-1-(cyclopenten-4-yl)methyl)-9-(4-(4-methylaminocarbonyl-2-chlorophenoxy)phenylmethyl)-1,4,9-triazaspiro[5.5]undecane hydrochloride

TLC: Rf 0.50 (dichloromethane:methanol=10:1);
NMR($CD_3OD$): δ 8.00, 7.77, 7.60, 7.13, 7.08, 5.71-5.61, 4.36, 4.00, 4.00, 3.75, 3.60-3.40, 3.53, 3.22, 2.91, 2.71, 2.60-1.98, 1.68, 1.52-1.27, 0.95.

Example 6(26)

(3R)-1-butyl-2,5-dioxo-3-((1R)-1-hydroxy-1-(cyclopenten-4-yl)methyl)-9-(4-(4-methylaminocarbonyl-2-methoxyphenoxy)phenylmethyl)-1,4,9-triazaspiro[5.5]undecane hydrochloride

TLC: Rf 0.42 (dichloromethane:methanol=10:1);
NMR($CD_3OD$): δ 7.59, 7.49, 7.45, 7.08, 6.96, 5.70-5.62, 4.27, 4.00, 3.92, 3.82, 3.68, 3.60-3.32, 3.54, 3.23, 2.93, 2.70, 2.60-1.97, 1.68, 1.52-1.23, 0.95.

Example 6(27)

(3R)-1-butyl-2,5-dioxo-3-((1R)-1-hydroxy-1-(cyclopenten-4-yl)methyl)-9-(4-(4-cyclopropylmethylaminocarbonylphenoxy)phenylmethyl)-1,4,9-triazaspiro[5.5]undecane hydrochloride

TLC: Rf 0.68 (chloroform:methanol=10:1);
NMR($CD_3OD$): δ 8.52, 7.87, 7.57, 7.16, 7.08, 5.67, 4.35, 4.01, 4.00, 3.75, 3.55-3.44, 3.25-3.12, 2.71, 2.59-1.97, 1.76-1.28, 1.10, 0.96, 0.55-0.49, 0.30-0.25.

Example 6(28)

(3R)-1-butyl-2,5-dioxo-3-((1R)-1-hydroxy-1-(cyclopenten-4-yl)methyl)-9-(4-(4-methylaminocarbonylphenylcarbonyl) phenylmethyl)-1,4,9-triazaspiro[5.5]undecane hydrochloride

TLC: Rf 0.36 (chloroform:methanol=10:1);
NMR($CD_3OD$): δ 7.97-7.93, 7.89-7.83, 7.70, 5.66, 4.34, 4.00, 3.90, 3.70-3.45, 3.40-3.10, 2.95, 2.70, 2.60-2.00, 1.70, 1.50-1.25, 0.95.

Example 6(29)

(3R)-1-butyl-2,5-dioxo-3-((1R)-1-hydroxy-1-(cyclopenten-4-yl)methyl)-9-(4-(4-methylaminocarbonylphenylmethyl) phenylmethyl)-1,4,9-triazaspiro[5.5]undecane hydrochloride

TLC: Rf 0.51 (dichloromethane:methanol=10:1);
NMR($CD_3OD$): δ 7.73, 7.46, 7.37, 7.31, 5.72-5.61, 4.32, 4.07, 3.99, 3.99, 3.75, 3.54-3.48, 3.52, 3.12, 2.89, 2.70, 2.60-2.00, 1.68, 1.52-1.24, 0.94.

Example 6(30)

(3R)-1-butyl-2,5-dioxo-3-((1R)-1-hydroxy-1-(cyclopenten-4-yl)methyl)-9-(4-(4-methylsulfonylamino-2-methoxyphenoxy)phenylmethyl)-1,4,9-triazaspiro[5.5]undecane hydrochloride

TLC: Rf 0.53 (dichloromethane:methanol=10:1);
NMR($CD_3OD$): δ 7.46, 7.04, 7.03, 6.92, 6.87, 6.74-6.61, 4.30, 3.99, 3.98, 3.74, 3.73, 3.60-3.38, 3.53, 3.20, 2.99, 2.70, 2.60-2.00, 1.69, 1.52-1.25, 0.95.

Example 6(31)

(3R)-1-butyl-2,5-dioxo-3-((1R)-1-hydroxy-1-(cyclopenten-4-yl)methyl)-9-(4-(4-carboxy-2,6-dimethylphenoxy) phenylmethyl)-1,4,9-triazaspiro[5.5]undecane hydrochloride

TLC: Rf 0.34 (dichloromethane:methanol=10:1);
NMR($CD_3OD$): δ 7.82, 7.52, 6.86, 5.66, 4.30, 4.00, 4.00, 3.70, 3.60-3.40, 3.20, 2.70, 2.60-2.00, 2.14, 1.70, 1.50-1.30, 0.95.

Example 7

(3R)-1-(2-butynyl)-2,5-dioxo-3-((1R)-1-hydroxy-1-cyclohexylmethyl)-9-(4-(4-methylaminocarbonylphenoxy)butyl)-1,4,9-triazaspiro[5,5]undecane hydrochloride

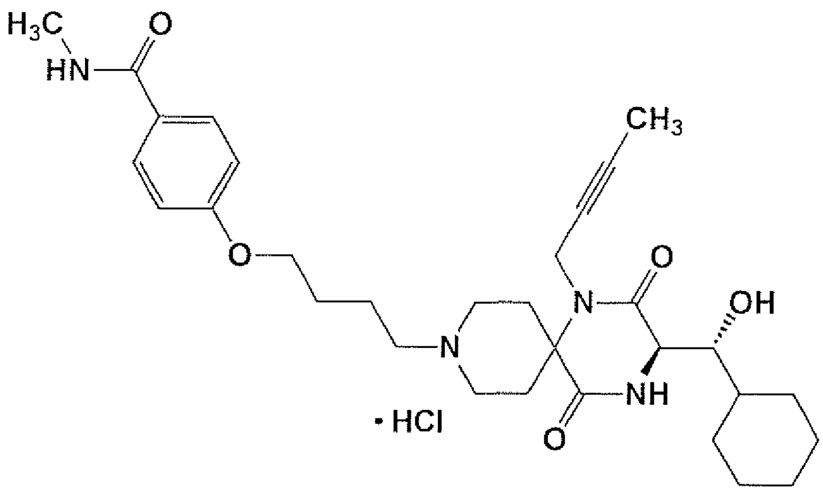

To solution of the compound prepared in Reference Example 5 and 1-bromo-4-(4-methylaminocarbonylphenoxy)butane (99 mg) in dimethylformamide (3 mL) was added triethylamine (0.1 mL) and sodium iodide (86 mg) and the mixture was stirred at room temperature overnight. To the reaction mixture was added 1N hydrochloric acid. The mixture was extracted with ethyl acetate. The organic layer was washed saturated aqueous solution of sodium sulfite and brine, dried over anhydrous sodium sulfate and concentrated. The obtained residue was purified by column chromatography on silica gel (ethyl acetate : methanol = 30 : 1-10 : 1) and 4N hydrogen chloride / ethyl acetate solution was added thereto. The reaction mixture was concentrated and washed with t-butylmethyl ether to give the compound of the present invention (64 mg) having the following physical data.
TLC: Rf 0.44 (chloroform:methanol=9:1);
NMR($CD_3OD$): δ 7.77, 6.98, 4.39, 4.20, 4.11, 4.04-3.88, 3.71, 3.64-3.50, 3.30-3.17, 2.89, 2.73, 2.52-2.32, 2.20, 2.08-1.84, 1.80-1.61, 1.75, 1.36-1.12, 1.04-0.80.

Example 8

(3R)-1-butyl-2,5-dioxo-3-((1R)-1-hydroxy-1-cyclohexylmethyl)-9-(1-(4-(4-methylaminocarbonylphenoxy)phenyl) ethyl)-1,4,9-triazaspiro[5.5]undecane hydrochloride

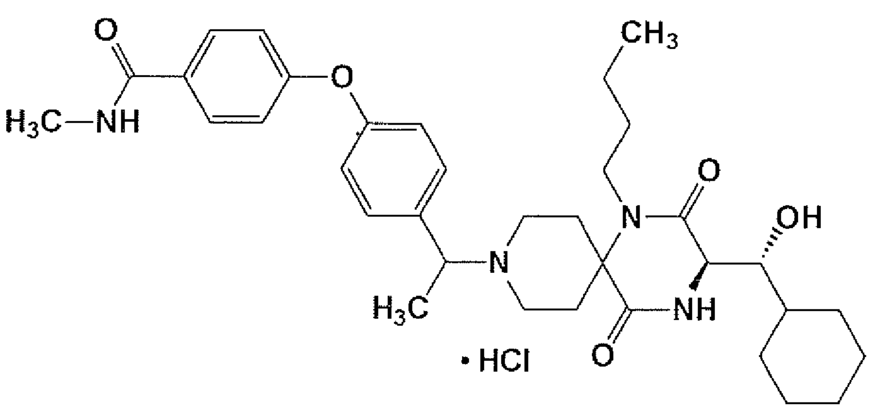

To a solution of N-methyl-4-(4-(1-(4-oxopiperidin-1-yl)ethyl)phenoxy)benzamide (131 mg) in methanol (3 mL) was added (2R,3R)-2-(t-butoxycarbonylamino)-3-cyclohexyl-3-hydroxypropanoic acid (117 mg), butylamine (0.06 mL) and 2-(morpholin-4-yl)ethylisocyanide (0.06 mL). The mixture was stirred at 50°C overnight. At room temperature, to the reaction mixture was added concentrated hydrochloric acid (0.5 mL). The mixture was stirred at 50°C for 2 hours, concentrated and neutralize with saturated sodium bicarbonate. The reaction mixture was extracted with dichloromethane, washed with brine, dried over anhydrous sodium sulfate and concentrated. The obtained residue was dissolved in 1.25 M acetic acid / toluene (3 mL) and heated to 80 °C for 1 hour. After cooling, the reaction mixture was concentrated. The obtained residue was purified by column chromatography on silica gel (ethyl acetate : methanol = 20 : 1-10 : 1) and 4N hydrogen chloride / ethyl acetate solution was added thereto. The reaction mixture was concentrated and washed with t-butylmethyl ether to give the compound of the present invention (118 mg) having the following physical data.
TLC: Rf 0.70 (dichloromethane:methanol=5:1);
NMR($CD_3OD$): δ 7.84, 7.59, 7.15, 7.08, 4.54, 4.13, 3.89, 3.70-3.50, 3.37-3.20, 2.91, 2.60-2.35, 2.16-1.91, 1.81, 1.75-1.72, 1.45-1.14, 1.00-0.94.

Example 8(1) - 8(44)

By the same procedure as described in Example 8 using the corresponding oxopiperidine derivatives respectively instead of N-methyl-4-(4-(1-(4-oxopiperidin-1-yl)ethyl)phenoxy)benzamide, using the corresponding amine derivatives respectively instead of butylamine and using the corresponding amino acid respectively instead of (2R,3R)-2-(t-butoxycarbonylamino)-3-cyclohexyl-3-hydroxypropanoic acid, the following compounds of the present invention were obtained.

Example 8(1)

(3R)-1-(2-propynyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.30 (chloroform:methanol=10:1);
NMR($CD_3OD$): δ 7.84, 7.59, 7.15, 7.08, 4.41, 4.37, 4.21, 4.06-3.95, 3.77, 3.55- 3.46, 3.33, 2.91, 2.72-2.64, 2.51-2.19, 2.04-1.92, 1.80-1.65, 1.34-1.17, 1.00-0.82.

Example 8(2)

(3R)-1-(1-oxidopyridin-3-ylmethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

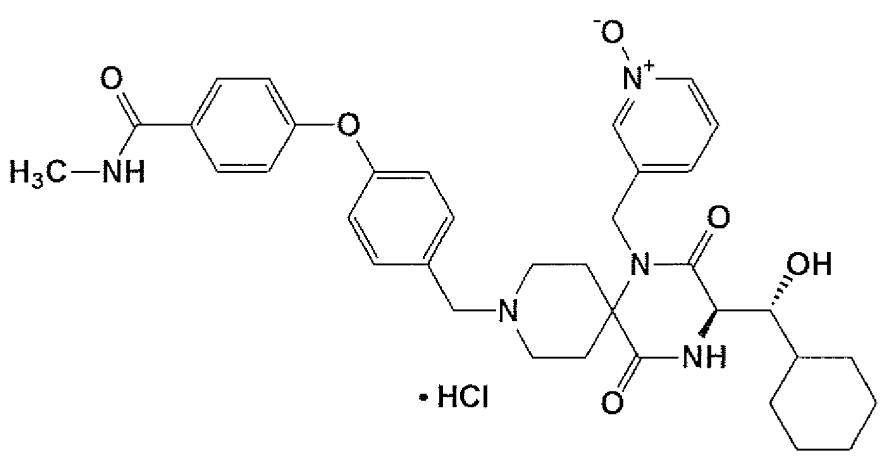

TLC: Rf 0.13 (chloroform:methanol=10:1);
NMR($CD_3OD$): δ 8.94, 8.74, 8.33, 7.92, 7.83, 7.64, 7.10, 7.05, 5.13, 5.05, 4.33, 4.30, 3.89, 3.77, 3.45-3.41, 3.33, 2.91,

2.66, 2.51-2.48, 2.17 -2.07, 1.93, 1.80-1.65, 1.30-1.17, 1.00-0.88.

Example 8(3)

(3R)-1-(1-oxidopyridin-2-ylmethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

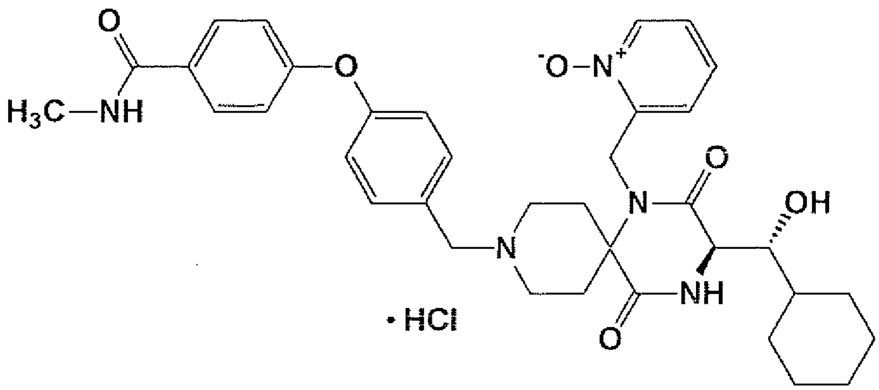

TLC: Rf 0.13 (chloroform:methanol=10:1);
NMR($CD_3OD$): δ 8.59, 7.85, 7.83, 7.63, 7.56, 7.12, 7.05, 5.00, 4.35, 4.30, 3.95, 3.82, 3.50-3.43, 3.33, 2.90, 2.59, 2.50-2.24, 2.09-1.90, 1.80-1.60, 1.28-1.17, 1.00-0.88.

Example 8(4)

(3R)-1-(2-(N,N-dimethylamino)ethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane dihydrochloride

TLC: Rf 0.08 (chloroform:methanol=5:1);
NMR($CD_3OD$): δ 7.84, 7.67, 7.12, 7.07, 4.36, 4.23, 4.01-3.90, 3.72, 3.50-3.44, 3.33-3.24, 3.00, 2.96, 2.91, 2.59-2.43, 2.17-1.94, 1.80-1.65, 1.35-1.15, 1.03-0.82.

Example 8(5)

(3R)-1-ethyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.37 (chloroform:methanol=10:1);
NMR($CD_3OD$): δ 7.84, 7.61, 7.14, 7.07, 4.36, 4.15, 3.99, 3.78-3.59, 3.48-3.44, 3.36, 3.28, 2.91, 2.54-2.36, 2.15-1.93, 1.80-1.65, 1.34-1.15, 1.00-0.86.

Example 8(6)

(3R)-1-cyclopropylmethyl-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.39 (chloroform:methanol=10:1);
NMR($CD_3OD$): δ 7.84, 7.58, 7.15, 7.07, 4.36, 4.20, 4.00, 3.77, 3.56-3.40, 3.34, 3.20, 2.91, 2.53-2.48, 2.29-2.16, 2.03-1.91, 1.76-1.60, 1.39-1.19, 1.05-0.88, 0.53-0.36.

Example 8(7)

(3R)-1-(2-phenylethyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf0.42 (chloroform:methanol=10:1)
NMR($CD_3OD$): δ 7.84, 7.57, 7.33-7.17, 7.15, 7.07, 4.35, 4.23, 4.03, 3.75, 3.55-3.37, 3.23, 3.02, 2.91, 2.74, 2.51-2.46, 2.28, 2.14-1.97, 1.80-1.65, 1.39-1.15, 1.05-0.88.

Example 8(8)

(3R)-1-methyl-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.32 (chloroform:methanol=10:1);
NMR($CD_3OD$): δ 7.84, 7.59, 7.15, 7.07, 4.37, 4.17, 3.98, 3.78, 3.49-3.46, 3.25, 2.95, 2.91, 2.57-2.35, 2.12-1.95, 1.80-1.65, 1.34-1.15, 1.00-0.87.

Example 8(9)

(3R)-1-(3-hydroxybutyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.24 (chloroform:methanol=10:1);
NMR($CD_3OD$): δ 7.85, 7.59, 7.15, 7.08, 4.36, 4.16, 4.02, 3.88-3.74, 3.60, 3.50-3.43, 3.38, 3.27, 2.91, 2.59-2.33, 2.15-1.93, 1.80-1.65, 1.35-1.15, 1.01-0.87.

Example 8(10)

(3R)-1-(2-(pyridin-3-yl)ethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane dihydrochloride

TLC: Rf 0.55 (dichloromethane:methanol=5:1);
NMR($CD_3OD$): δ 8.98, 8.78-8,73, 8.05, 7.84, 7.69, 7.12, 7.07, 4.36, 4.22, 4.09-3.72, 3.63-3.41, 3.33-3.10, 2.91, 2.86, 2.62, 2.44, 2.19-1.97, 1.80-1.65, 1.39-1.15, 1.03-0.87.

Example 8(11)

(3R)-1-(2-(1-oxidopyridin-3-yl)ethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

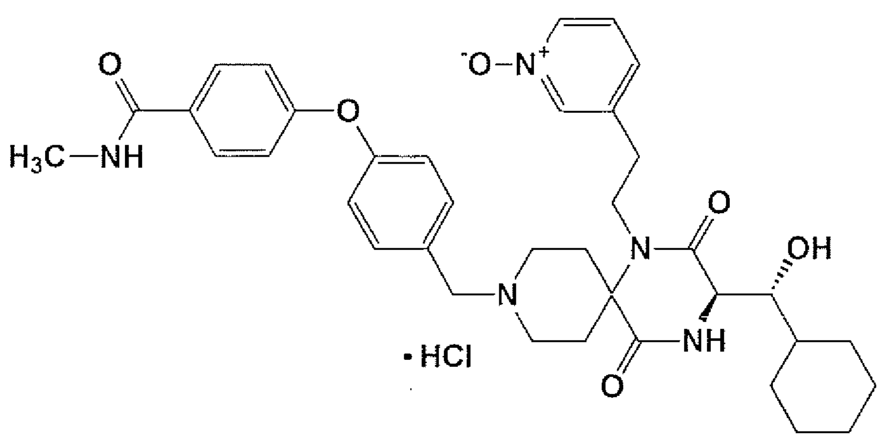

TLC: Rf 0.12 (chloroform:methanol=10:1);
NMR($CD_3OD$): δ 8.89, 8.68, 8.31, 7.88, 7.84, 7.66, 7.13, 7.07, 4.36, 4.21, 4.00, 3.85-3.70, 3.54-3.42, 3.30-3.00, 2.91, 2.75, 2.58-2.43, 2.19-1.96, 1.80-1.70, 1.38-1.15, 1.05-0.87.

Example 8(12)

(3R)-1-(2-(2-propenyloxycarbonylamino)ethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.44 (dichloromethane:methanol=10:1);
NMR($CD_3OD$): δ 7.85, 7.62, 7.14, 7.07, 5.91, 5.28, 5.15, 4.52, 4.36, 4.17, 3.99, 3.82-3.53, 3.52-3.17, 2.91, 2.70-2.25, 2.18-1.87, 1.85-1.60, 1.41-1.10, 1.04-0.80.

Example 8(13)

(3R)-1-ethyl-2,5-dioxo-3-((1R)-1-hydroxy-1-cyclohexylmethyl)-9-phenylmethyl-1,4,9-triazaspiro[5.5]undecane hydrochloride

TLC: Rf 0.54 (ethyl acetate:methanol=4:1);
NMR($CD_3OD$): δ 7.58-7.49, 4.36, 4.15, 4.00, 3.75, 3.61, 3.46-3.42, 3.33-3.25, 2.49-2.23, 2.16-1.92, 1.80-1.63, 1.34-1.15, 1.00-0.86.

Example 8(14)

(3R)-1-methyl-2,5-dioxo-3-((1R)-1-hydroxy-1-cyclohexylmethyl)-9-phenylmethyl-1,4,9-triazaspiro[5.5]undecane hydrochloride

TLC: Rf 0.45 (ethyl acetate:methanol=4:1);
NMR($CD_3OD$): δ 7.58-7.48, 4.36, 4.16, 3.98, 3.78, 3.47-3.43, 3.25, 2.94, 2.59-2.35, 2.10-1.93, 1.80-1.66, 1.33-1.14, 1.00-0.86.

Example 8(15)

(3R)-1-(2-methylaminocarbonylethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.50 (chloroform:methanol:acetic acid=20:4:1);
NMR($CD_3OD$): δ 7.84, 7.58, 7.15, 7.07, 4.37, 4.16, 3.99, 3.85-3.63, 3.58-3.42, 3.26, 2.91, 2.70, 2.62, 2.55-2.20, 2.18-1.90, 1.85-1.60, 1.40-1.05, 1.05-0.80.

Example 8(16)

(3R)-1-(3-methylaminocarbonylpropyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.52 (chloroform:methanol:acetic acid=20:4:1);
NMR($CD_3OD$): δ 7.84, 7.61, 7.15, 7.07, 4.37, 4.16, 4.00, 3.76, 3.58-3.43, 3.30-3.20, 2.91, 2.72, 2.63-2.21, 2.20-1.85, 1.85-1.60, 1.40-1.12, 1.05-0.80.

Example 8(17)

(3R)-1-(2-cyanoethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.37 (dichloromethane:methanol=10:1);
NMR($CD_3OD$): δ 7.84, 7.59, 7.15, 7.07, 4.37, 4.20, 4.01, 3.86-3.70, 3.64-3.44, 3.27, 2.91, 2.88-2.72, 2.58-2.15, 2.08-1.88, 1.84-1.62, 1.41-1.11, 1.05-0.80.

Example 8(18)

(3R)-1-cyclopropyl-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.51 (dichloromethane:methanol=10:1);
NMR($CD_3OD$): δ 7.84, 7.56, 7.14, 7.07, 4.30, 4.18, 3.79, 3.64-3.40, 2.91, 2.80-2.48, 2.35-2.15, 1.98, 1.82-1.60, 1.40-1.09, 1.09-0.83, 0.82-0.66.

Example 8(19)

(3R)-1-hexyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.53 (dichloromethane:methanol=10:1);
NMR($CD_3OD$): δ 7.84, 7.59, 7.14, 7.07, 4.36, 4.15, 3.99, 3.75, 3.59-3.42, 3.33-3.10, 2.91, 2.55-2.24, 2.18-1.87, 1.83-1.62, 1.50-1.10, 1.05-0.80.

Example 8(20)

(3R)-1-heptyl-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.53 (dichloromethane:methanol=10:1);
NMR($CD_3OD$): δ 7.85, 7.61, 7.14, 7.07, 4.36, 4.16, 3.99, 3.75, 3.59-3.42, 3.33-3.05, 2.91, 2.60-2.26, 2.18-1.88, 1.82-1.60, 1.52-1.08, 1.05-0.80.

Example 8(21)

(3R)-1-(2,2,2-trifluoroethyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.51 (dichloromethane:methanol=10:1);
NMR($CD_3OD$): δ 7.84, 7.59, 7.15, 7.07, 4.43, 4.36, 4.30, 4.18-3.92, 3.74, 3.56-3.42, 3.35, 2.91, 2.58, 2.43-2.11, 2.02, 1.89, 1.84-1.60, 1.40-1.06, 1.05-0.80.

Example 8(22)

(3R)-1-butyl-2,5-dioxo-3-((1R)-1-hydroxy-1-cyclohexylmethyl)-9-(4-(4-methylsulfonylaminophenoxy)butyl)-1,4,9-triazaspiro[5.5]undecane hydrochloride

TLC: Rf 0.26 (dichloromethane:methanol=10:1);
NMR : δ 7.18, 6.92, 4.16, 4.04, 3.96, 3.70, 3.62-3.48, 3.30-3.15, 2.87, 2.60-2.30, 2.20-1.60, 1.50-1.10, 1.00-0.80, 0.97.

Example 8(23)

(3R)-1-butyl-2,5-dioxo-3-((1R)-1-hydroxy-1-cyclohexylmethyl)-9-(5-hydroxypyridin-2-ylmethyl)-1,4,9-triazaspiro[5.5] undecane dihydrochloride

TLC: Rf 0.27 (chloroform:methanol=10:1);
NMR($CD_3OD$): δ 8.39, 8.02-7.88, 7.82-7.66, 4.55, 4.15, 4.08, 3.82, 3.64-3.42, 3.38-3.18, 2.74-2.36, 2.20-1.84, 1.82-1.56, 1.54-1.06, 1.04-0.80, 0.95.

Example 8(24)

(3R)-1-butyl-2,5-dioxo-3-((1R)-1-hydroxy-1-cyclohexylmethyl)-9-(5-(5-hydroxypyridin-2-ylmethyloxy)pyridin-2-ylmethyl)-1,4,9-triazaspiro[5.5]undecane trihydrochloride

TLC: Rf 0.23 (chloroform:methanol=10:1);
NMR($CD_3OD$): δ 8.60, 8.37, 8.08-7.98, 7.80-7.64, 5.53, 4.50, 4.16, 4.06, 3.82, 3.64-3.42, 3.40-3.18, 2.72-2.26, 2.20-1.84, 1.82-1.58, 1.56-1.06, 1.04-0.80, 0.96.

Example 8(25)

(3R)-1-butyl-2,5-dioxo-3-((1R)-1-hydroxy-1-cyclohexylmethyl)-9-(4-(N-methyl-N-methylsulfonylamino)phenylmethyl)-1,4,9-triazaspiro[5.5]undecane hydrochloride

TLC: Rf 0.36 (chloroform:methanol=10:1);
NMR($CD_3OD$): δ 7.62, 7.55, 4.37, 4.15, 4.00, 3.76, 3.58-3.40, 3.38-3.14, 3.33, 2.91, 2.58-2.24, 2.18-1.84, 1.82-1.56, 1.50-1.06, 1.04-0.80, 0.95.

Example 8(26)

(3R)-1-pentyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.52 (dichloromethane:methanol=10:1);
NMR($CD_3OD$): δ 7.84, 7.60, 7.15, 7.07, 4.36, 4.15, 4.00, 3.75, 3.59-3.41, 3.30-3.12, 2.91, 2.56-2.24, 2.18-1.88, 1.84-1.60, 1.54-1.06, 1.04-0.80, 0.92.

Example 8(27)

(3R)-1-butyl-2,5-dioxo-3-((1R)-1-hydroxy-1-cyclohexylmethyl)-9-(4-(4-(N-methyl-N-methylsulfonylamino)phenoxy)phenylmethyl)-1,4,9-triazaspiro[5.5]undecane hydrochloride

TLC: Rf 0.51 (chloroform:methanol=10:1);
NMR($CD_3OD$): δ 7.57, 7.45, 7.11, 7.06, 4.34, 4.15, 4.00, 3.72, 3.60-3.38, 3.36-3.12, 3.30, 2.91, 2.58-2.24, 2.20-1.84, 1.82-1.58, 1.54-1.06, 1.04-0.80, 0.95.

Example 8(28)

(3R)-1-butyl-2,5-dioxo-3-((1R)-1-hydroxy-1-cyclohexylmethyl)-9-(5-methylaminocarbonylpyridin-2-ylmethyl)-1,4,9-triazaspiro[5.5]undecane dihydrochloride

TLC: Rf 0.28 (chloroform:methanol=10:1);
NMR($CD_3OD$): δ 9.01, 8.28, 7.65, 4.61, 4.16, 4.04, 3.84, 3.64-3.46, 3.30-3.18, 2.95, 2.64-2.32, 2.20-1.84, 1.82-1.58, 1.56-1.04, 1.02-0.80, 0.98.

Example 8(29)

(3R)-1-butyl-2,5-dioxo-3-((1R)-1-hydroxy-1-cyclohexylmethyl)-9-(1-phenylcarbonylpiperidin-4-ylmethyl)-1,4,9-triazaspiro[5.5]undecane hydrochloride

TLC: Rf 0.34 (chloroform:methanol=10:1);
NMR($CD_3OD$): δ 7.56-7.34, 4.64, 4.16, 3.98, 3.86-3.44, 3.40-2.82, 3.10, 2.64, 2.58-2.38, 2.36-1.58, 1.56-1.06, 1.04-0.80, 0.96.

Example 8(30)

(3R)-1-(2-butynyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.50 (dichloromethane:methanol=10:1);
NMR($CD_3OD$): δ 7.84, 7.59, 7.15, 7.07, 4.39-4.33, 4.37, 4.20, 4.03-3.94, 3.80-3.72, 3.55-3.45, 3.33, 2.91, 2.69, 2.49-2.18, 2.04-1.92, 1.80-1.64, 1.40-1.14, 1.05-0.85.

Example 8(31)

(3R)-1-(3-butynyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.50 (dichloromethane:methanol=10:1);
NMR($CD_3OD$): δ 7.84, 7.59, 7.15, 7.07, 4.37, 4.17, 4.01, 3.79-3.65, 3.50-3.37, 3.27, 2.91, 2.61-1.60, 1.35-1.15, 1.00-0.87.

Example 8(32)

(3R)-1-(3-butenyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.55 (dichloromethane:methanol=10:1);
NMR($CD_3OD$): δ 7.84, 7.59, 7.14, 7.07, 5.83, 5.15-5.03, 4.36, 4.16, 3.99, 3.75, 3.59-3.46, 3.33-3.25, 2.91, 2.51-1.65, 1.34-1.15, 1.00-0.87.

Example 8(33)

(3R)-1-(2-ethylbutyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.50 (dichloromethane:methanol=10:1);
NMR($CD_3OD$): δ 7.84, 7.58, 7.14, 7.07, 4.35, 4.19, 3.91, 3.76, 3.54-3.36, 3.33-3.07, 2.91, 2.56-1.65, 1.44-0.87.

Example 8(34)

(3R)-1-(3-methoxypropyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.40 (dichloromethane:methanol=10:1);
NMR($CD_3OD$): δ 7,84, 7.57, 7.15, 7.07, 4.36, 4,16, 4,01, 3.76, 3.61-3.43, 3.32, 3.30-3.14, 2.91, 2.52-1.92, 1.80-1.65, 1.34-1.15, 1.00-0.87.

Example 8(35)

(3R)-1-(2-ethoxyethyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane hydrochloride

TLC: Rf 0.49 (dichloromethane:methanol=10:1);
NMR($CD_3OD$): δ 7.84, 7.57, 7.15, 7.07, 4.36, 4.17, 3.98, 3.76-3.40, 3.27, 2.91, 2.56-2.25, 2.14-1.91, 1.80-1.65, 1.40-1.15, 1.15, 1.00-0.87.

Example 8(36)

(3R)-1-(2,2,3,3,3-pentafluoropropyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.69 (dichloromethane:methanol=10:1);
NMR($CD_3OD$): δ 7.84, 7.58, 7.15, 7.07, 4.49, 4.36, 4.31, 4.10-4.00, 3.72, 3.52-3.48, 3.35, 2.91, 2.63, 2.44-2.20, 2.03-1.60, 1.34-1.14, 1.01-0.85.

Example 8(37)

(3R)-1-cyclobutylmethyl-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.51(dichloromethane:methanol=10:1);
NMR($CD_3OD$): δ 7.84, 7.58, 7.15, 7.07, 4.35, 4.15, 3.96, 3.76, 3.61-3.45, 3.34- 3.23, 2.91, 2.49-1.72, 1.40-1.15, 0.98-0.90.

Example 8(38)

(3R)-1-((2E)-2-butenyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.43 (chloroform:methanol=9:1);
NMR($CD_3OD$): δ 7.84, 7.60, 7.14, 7.07, 5.72, 5.48, 4.35, 4.20, 4.18, 3.97, 3.87, 3.73, 3.48-3.43, 3.28, 2.91, 2.61-2.30, 2.10-1.90, 1.80-1.60, 1.65, 1.40-1.12, 1.04-0.83.

Example 8(39)

(3R)-1-(2-trans-methylcyclopropylmethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

TLC: Rf 0.46 (chloroform:methanol=9:1);
NMR($CD_3OD$): δ 7.84, 7.59, 7.14, 7.07, 4.35, 4.18, 3.98, 3.77, 3.54-3.44, 3.25-3.11, 2.91, 2.55-2.42, 2.38-2.10, 2.08-1.88, 1.80-1.60, 1.35-1.11, 1.00, 0.96-0.70, 0.54, 0.27.

Example 8(40)

(3R)-1-butyl-2,5-dioxo-3-((1R)-1-hydroxy-1-(cis-4-methylcyclohexyl)methyl)-9-(4-(4-carboxyphenoxy)phenylmethyl)-1,4,9-triazaspiro[5.5]undecane hydrochloride

TLC: Rf 0.66 (chloroform:methanol:acetic acid=10:1:1);
NMR($CD_3OD$): δ 8.04, 7.62, 7.17, 7.07, 4.37, 4.16, 4.00, 3.75, 3.59-3.43, 3.25, 2.57-2.29, 2.12, 1.86, 1.77-1.26, 0.95, 0.94.

Example 8(41)

(3R)-1-butyl-2,5-dioxo-3-((1R)-1-hydroxy-1-(tetrahydrothiopyran-4-yl)methyl)-9-(4-(4-methylaminocarbonylphenoxy)phenylmethyl)-1,4,9-triazaspiro[5.5]undecane hydrochloride

TLC: Rf 0.31 (dichloromethane:methanol=10:1);
NMR($CD_3OD$): δ 7.84, 7.58, 7.15, 7.07, 4.36, 4.17, 4.02, 3.76, 3.60-3.40, 3.16, 2.91, 2.75-2.10, 1.90-1.65, 1.50-1.20, 0.96.

Example 8(42)

(3R)-1-butyl-2,5-dioxo-3-((1R)-1-hydroxy-1-cyclohexylmethyl)-9-(1-methyl-1-phenylethyl)-1,4,9-triazaspiro[5.5] undecane hydrochloride

TLC: Rf 0.35 (ethyl acetate:methanol:water=9:1:0.1);
NMR($CDCl_3$): δ 7.80-7.78, 7.53-7.43, 4.11, 3.98, 3.68, 3.55, 3.40-3.28, 3.25, 2.61, 2.50-2.34, 2.07-1.60, 1.92, 1.45-1.13, 1.00-0.85, 0.95.

Example 8(43)

(3R)-1-butyl-2,5-dioxo-3-((1R)-1-hydroxy-1-cyclohexylmethyl)-9-(1-(4-(4-methylaminocarbonylphenoxy)phenyl) propyl)-1,4,9-triazaspiro[5.5]undecane hydrochloride

TLC: Rf 0.73 (chloroform:methanol=5:1);
NMR($CD_3OD$): δ 7.85, 7.57, 7.16, 7.09, 4.34-4.22, 4.13, 3.93-3.76, 3.68-3.44, 3.42-3.10, 2.99, 2.68-2.16, 2.18-1.84, 1.82-1.60, 1.50-1.08, 1.04-0.76, 0.97, 0.83.

Example 8(44)

(3R)-1-butyl-2,5-dioxo-3-((1R)-1-hydroxy-1-cyclohexylmethyl)-9-(1-(4-(4-carboxyphenoxy)phenyl)ethyl)-1,4,9-triazaspiro[5.5]undecane hydrochloride

TLC: Rf 0.43 (dichloromethane:methanol:water=9:1:0.1);
NMR($CD_3OD$): δ 8.04, 7.60, 7.18, 7.07, 4.57, 4.14, 3.90, 3.80-3.40, 3.40-3.20, 2.60-2.20, 2.20-1.85, 1.85-1.60, 1.81, 1.50-1.10, 1.10-0.80, 0.96.

Example 9

(3R)-1-(2-aminoethyl)-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane dihydrochloride

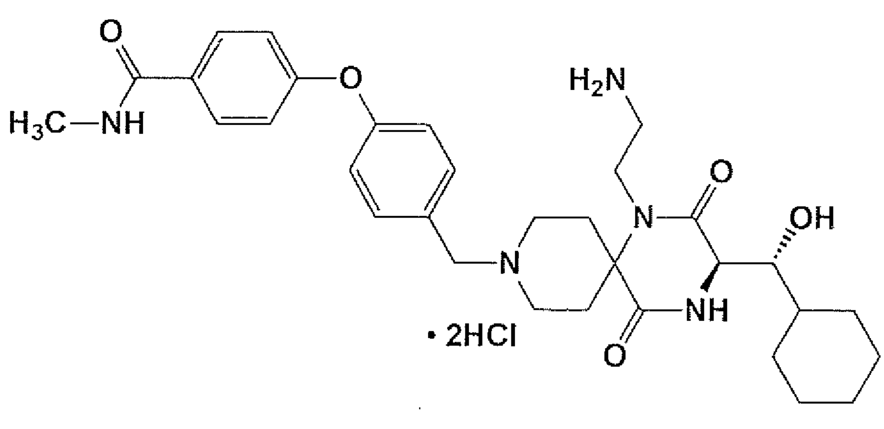

A free form of the compound prepared in Example 8(12) was prepared by using a conventional method. To a suspension of the free form (534 mg) in dichloromethane (8 mL) was added palladium tetrakistriphenylphosphine (47 mg) and tributyltin hydride (0.26 mL) and the mixture was stirred at room temperature for 3 hours. The reaction solvent was removed. To the residue was add aqueous solution of sodium carbonate and the mixture was extracted with chloroform / methanol. The mixture was washed with brine, dried anhydrous sodium sulfate and concentrated. The residue was dissolved in methanol and 4N hydrogen chloride / ethyl acetate solution was added thereto. The reaction mixture was concentrated to give the compound of the present invention (178 mg) having the following physical data.
TLC: Rf 0.33 (n-butanol:acetic acid:water=4:2:1);

NMR($CD_3OD$): δ 7.84, 7.66, 7.12, 7.07, 4.35, 4.23, 4.04-3.65, 3.51-3.40, 3.26, 3.15-3.06, 2.91, 2.65-2.44, 2.16, 2.08-1.91, 1.83-1.62, 1.40-1.12, 1.05-0.80.

Example 10

(3R)-1-(2-methylcarbonylaminoethyl)-2,5-dioxo-3-((1R)-1-hydroxy-1-cyclohexylmethyl)-9-(4-(4-methylaminocarbonylphenoxy)phenylmethyl)-1,4,9-triazaspiro[5.5]undecane hydrochloride

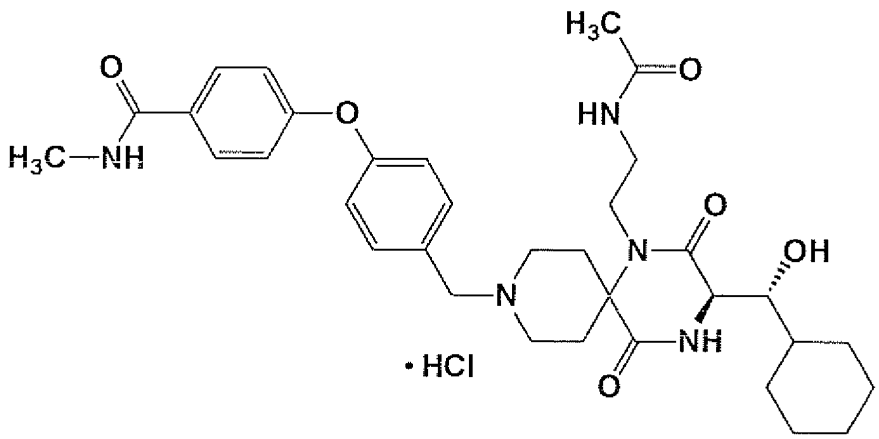

To a suspension of a free form of the compound prepared in Example 9 (44 mg) in chloroform (1 mL) was added triethylamine (0.02 mL). To the mixture was added acetyl chloride (0.01 mL) on ice bath and the mixture was stirred at temperature overnight. To the reaction mixture was added methanol and the mixture was concentrated. The residue was purified by column chromatography on silica gel (ethyl acetate : methanol = 20 : 1-10 : 1) and 4N hydrogen chloride / ethyl acetate solution was added thereto. The reaction mixture was concentrated to give the compound of the present invention (19.3 mg) having the following physical data.
TLC: Rf 0.30 (chloroform:methanol:acetic acid=20:2:1);
NMR($CD_3OD$): δ 7.85, 7.58, 7.15, 7.08, 4.37, 4.17, 3.91, 3.76, 3.60-3.42, 3.40-3.20, 2.91, 2.62-2.41, 2.29, 2.15, 2.08-1.90, 1.95, 1.85-1.62, 1.40-1.08, 1.04-0.80.

Example 11

(3R)-1-butyl-2,5-dioxo-3-((1R)-1-hydroxy-1-cyclohexylmethyl)-9-(4-(4-carboxyphenoxy)phenylmethyl)-9-oxido-1,4,9-triazaspiro[5.5]undecane

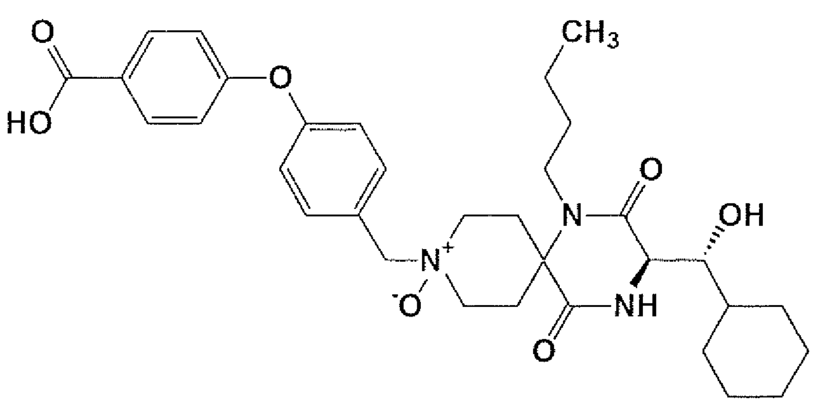

To a solution of the compound prepared in Example 2(1) in methanol was added 1N sodium hydroxide in

equal proportions and the mixture was stirred at room temperature for 30 minutes. The reaction mixture was concentrated and the obtained residue was dissolved in methanol and dichloromethane. To the reaction mixture was added 1.2 N mCPBA and the mixture was stirred for 30 minutes. The reaction mixture was concentrated and the obtained residue was purified by column chromatography on silica gel (ethyl acetate : methanol = 10 : 1) to give the compound of the present invention (55 mg) having the following physical data.
TLC: Rf 0.16 (chloroform:methanol=10:1);
NMR: δ 7.98, 7.84, 7.64, 7.04, 6.94, 5.29, 4.61, 4.20-2.80, 2.80-2.00, 2.00-1.40, 1.40-1.00, 0.90-0.70, 0.80.

Example 12

(3R)-1-butyl-2,5-dioxo-3-((1R)-1-hydroxy-1-cyclohexylmethyl)-9-(1-phenylmethylpiperidin-4-yl)-1,4,9-triazaspiro[5.5] undecane dihydrochloride

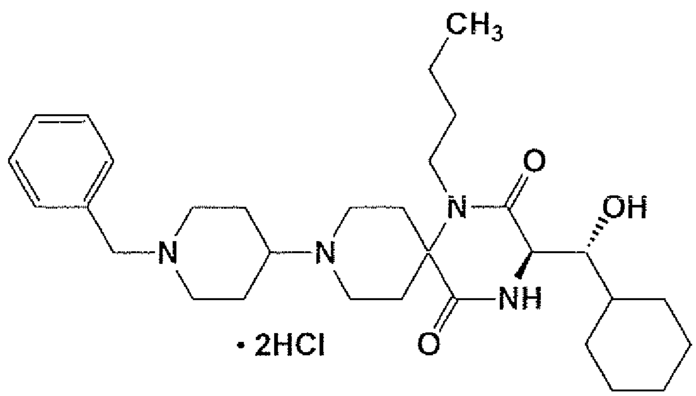

To a solution of the compound prepared in Reference Example 2 (150 mg) in triethylamine (0.07 mL) was added 1-benzyl-4-oxopiperidine (80 mg) and tetraisopropoxytitanium (0.23 mL) and the mixture was stirred at room temperature for 4 hours. To a suspension of the reaction mixture in ethanol (2 mL) was added sodium borohydride (30 mg) and the mixture was stirred overnight. To the reaction mixture was added 2N aqueous ammonia and the precipitated salt was filtrated through CELITE (brand name). The filtrate was extracted with ethyl acetate. The organic layer was washed brine, dried over anhydrous sodium sulfate and concentrated. The obtained residue was purified by column chromatography on silica gel (ethyl acetate : methanol = 40 : 1-8 : 1) and 4N hydrogen chloride / ethyl acetate solution was added thereto. The reaction mixture was concentrated and washed with t-butylmethyl ether to give the compound of the present invention (41 mg) having the following physical data.
TLC: Rf 0.31 (dichloromethane:methanol=10:1);
NMR($CD_3OD$): δ 7.56-7.51, 4.36, 4.15, 4.02, 3.80-3.53, 3.33-3-25, 3.18-3.10, 2.64, 2.55-2.45, 2.20-2.10, 2.05-1.92, 1.80-1.65, 1.41-1.15, 1.00-0.88.

Example 12(1), 12(2)

By the same procedure as described in Example 12 using the corresponding ketone derivatives respectively instead of 1-benzyl-4-oxopiperidine, the following compounds of the present invention were obtained.

Example 12(1)

(3R)-1-butyl-2,5-dioxo-3-((1R)-1-hydroxy-1-cyclohexylmethyl)-9-(1-(4-methylaminocarbonylphenylmethyl)piperidin-4-yl)-1,4,9-triazaspiro[5.5]undecane dihydrochloride

TLC: Rf 0.40 (dichloromethane:methanol=5:1);
NMR($CD_3OD$): δ 7.92, 7.64, 4.40, 4.15, 4.04, 3.75-3.50, 3.33-3.15, 2.93, 2.63-2.49, 2.18-1.90, 1.80-1.65, 1.45-1.19, 1.00-0.90.

Example 12(2)

(3R)-1-butyl-2,5-dioxo-3-((1R)-1-hydroxy-1-cyclohexylmethyl)-9-(1-phenylcarbonylpiperidin-4-yl)-1,4,9-triazaspiro [5.5]undecane hydrochloride

TLC: Rf 0.74 (dichloromethane:methanol=5:1);
NMR($CD_3OD$): δ 7.51-7.42, 4.16, 4.08, 3.81, 3.60-3.50, 3.33-3.16, 2.55-1.70, 1.45-1.20, 1.00-0.94.

Example 13

(3R)-1-butyl-2,5-dioxo-3-((1R)-1-hydroxy-1-cyclohexylmethyl)-9-phenyliminomethyl-1,4,9-triazaspiro[5.5]undecane hydrochloride

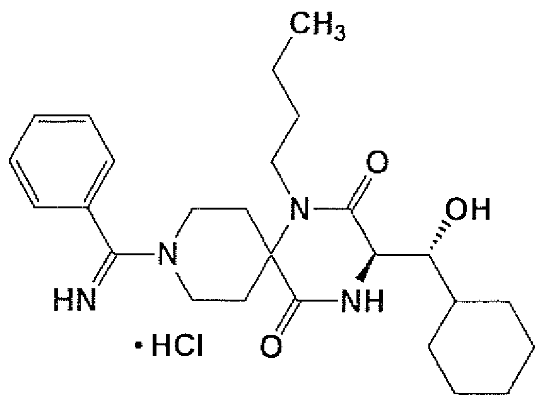

To a solution of the compound prepared in Example 4(30) (37 mg) in methanol (5 mL) was added 20% palladium hydroxide on carbon (3 mg) and the mixture was stirred at room temperature for 12 hours under an atmosphere of hydrogen. After the reaction, the catalyst was filtrated through CELITE (brand name). To the filtrate was added 1N hydrochloric acid and the mixture was concentrated to give the compound of the present invention (35 mg) having the following physical data.
TLC: Rf 0.37 (chloroform:methanol:water=8:2:0.2);
NMR($CDCl_3$): δ 9.40, 9.00, 7.70-7.58, 4.35-3.80, 3.75- 3.15, 2.65-2.30, 2.25-1.86, 1.85-1.60, 1.60-0.65, 0.95.

Example 14(1) - Example 14(9)

By the same procedure as described in Example 2 using the corresponding aldehyde derivatives respectively instead of N-(4-formylphenyl)methanesulfoneamide and using the corresponding amine derivatives respectively instead of the compound prepared in Reference Example 2, the following compounds of the present invention were obtained.

Example 14(1)

(3R)-1-(2-methoxyethyl)-2,5-dioxo-3-((1R)-1-hydroxy-1-cyclohexylmethyl)-9-(1-(4-methylsulfonylaminophenyl)-3,5-dimethylpyrazol-4-ylmethyl)-1,4,9-triazaspiro[5.5]undecane dihydrochloride

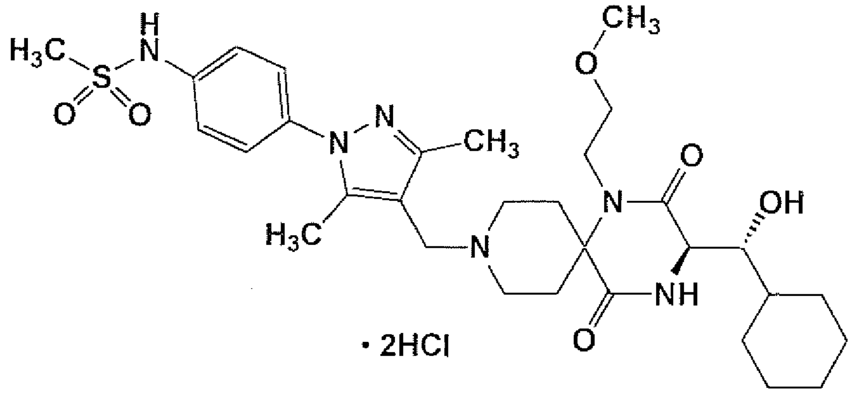

TLC: Rf 0.65 (chloroform:methanol=5:1);
NMR($CD_3OD$): δ 7.44-7.40, 4.30, 4.19, 4.00, 3.84-3.64, 3.62-3.48, 3.46-3.20, 3.03, 2.60-2.44, 2.36, 2.35, 2.30- 1.88, 1.84-1.60, 1.40-1.10, 1.06-0.80.

Example 14(2)

(3R)-1-ethyl-2,5-dioxo-3-((1R)-1-hydroxy-2-methylpropyl)-9-(1-(4-methylsulfonylaminophenyl)-3,5-dimethylpyrazol-4-ylmethyl)-1,4,9-triazaspiro[5.5]undecane dihydrochloride

TLC: Rf 0.56 (chloroform:methanol=5:1);
NMR($CD_3OD$): δ 7.50-7.40, 4.31, 4.15, 4.03, 3.84-3.50, 3.40, 3.20, 3.04, 2.60, 2.42, 2.39, 2.3 9, 2.15, 2.00, 1.20, 1.19, 1.00, 0.98.

Example 14(3)

(3R)-1-ethyl-2,5-dioxo-3-((1R)-1-hydroxy-1-cyclohexylmethyl)-9-(1-(4-methylsulfonylaminophenyl)-3,5-dimethylpyrazol-4-ylmethyl)-1,4,9-triazaspiro[5.5]undecane dihydrochloride

TLC: Rf 0.60 (chloroform:methanol=5:1);
NMR($CD_3OD$): δ 7.50-7.40, 4.30, 4.16, 4.03, 3.84-3.51, 3.50-3.30, 3.03, 2.70-2.28, 2.15, 2.08-1.86, 1.82-1.60, 1.44-1.06, 1.20, 1.04-0.80.

Example 14(4)

(3R)-1-ethyl-2,5-dioxo-3-((1R)-1-hydroxy-1-cyclohexylmethyl)-9-(4-(4-methylsulfonylamino-2-methoxyphenoxy) phenylmethyl)-1,4,9-triazaspiro[5.5]undecane hydrochloride

TLC: Rf 0.40 (chloroform:methanol=10:1);
NMR($CD_3OD$): δ 7.48, 7.05-7.01, 6.89-6.85, 4.29, 4.15, 3.95, 3.74, 3.70, 3.60-3.20, 2.99, 2.60-2.30, 2.20-1.80, 1.80-1.60, 1.40-1.10, 1.17, 1.00-0.80.

Example 14(5)

(3R)-1-ethyl-2,5-dioxo-3-((1R)-1-hydroxy-1-(tetrahydropyran-4-yl)methyl)-9-(4-(4-methylsulfonylamino-2-methoxyphenoxy)phenylmethyl)-1,4,9-triazaspiro[5.5]undecane hydrochloride

TLC: Rf 0.39 (chloroform:methanol=10:1);

NMR($CD_3OD$): δ 7.48, 7.04-7.00, 6.92-6.85, 4.29, 4.11, 4.00-3.80, 3.74, 3.80-3.50, 3.50-3.20, 2.99, 2.60-2.30, 2.20-1.80, 1.40-1.20, 1.18.

Example 14(6)

(3S)-1-butyl-2,5-dioxo-3-cyclohexyl-9-(4-(4-carboxyphenoxy)phenylmethyl)-1,4,9-triazaspiro[5.5]undecane hydrochloride

TLC: Rf 0.46 (dichloromethane:methanol=10:1);
NMR($CD_3OD$): δ 8.04, 7.59, 7.18, 7.07, 4.37, 3.91-3.80, 3.50-3.20, 2.41-1.12, 0.96.

Example 14(7)

(3S)-1-butyl-2,5-dioxo-3-cyclohexyl-9-(4-(4-methylsulfonylaminophenoxy)phenylmethyl)-1,4,9-triazaspiro[5.5] undecane hydrochloride

TLC: Rf 0.57 (dichloromethane:methanol=10:1);
NMR($CD_3OD$): δ 7.52, 7.29, 7.07, 7.03, 4.33, 3.90-3.80, 3.86, 3.48-3.30, 2.96, 2.39-1.12, 0.96.

Example 14(8)

(3R)-1-(2-methoxyethyl)-2,5-dioxo-3-((1R)-1-hydroxy-1-(tetrahydropyran-4-yl)methyl)-9-(4-(4-methylsulfonylamino-2-methoxyphenoxy)phenylmethyl)-1,4,9-triazaspiro[5.5]undecane hydrochloride

TLC: Rf 0.30 (dichloromethane:methanol=10:1);
NMR($CD_3OD$): δ 7.46, 7.04-7.02, 6.91, 6.87, 4.30, 4.14, 4.10, 4.00-3.90, 3.74, 3.70, 3.60-3.30, 3.31, 3.20, 2.99, 2.60-2.20, 2.20-1.80, 1.40-1.20.

Example 14(9)

(3S)-1-butyl-2,5-dioxo-3-cyclohexyl-9-(4-(4-carboxy-2-methoxyphenoxy)phenylmethyl)-1,4,9-triazaspiro[5.5] undecane hydrochloride

TLC: Rf 0.28 (dichloromethane:methanol=10:1);
NMR($CD_3OD$): δ 7.75, 7.67, 7.51, 7.09, 6.99, 4.33, 3.90-3.75, 3.82, 3.60-3.30, 2.50-2.30, 2.20-2.00, 1,90, 1.80-1.10, 0.95.

Example 15(1), 15(2)

By the same procedure as described in Example 6 using the corresponding aldehyde derivatives respectively instead of 4-(4-formylphenoxy)benzoic acid and using the corresponding amine derivatives respectively instead of the compound prepared in Reference Example 5, the following compounds of the present invention were obtained.

Example 15(1)

(3R)-1-(2-butynyl)-2,5-dioxo-3-((1R)-1-hydroxy-1-(tetrahydropyran-4-yl)methyl)-9-(1-(4-methylsulfonylaminophenyl)-3,5-dimethylpyrazol-4-ylmethyl)-1,4,9-triazaspiro[5.5]undecane dihydrochloride

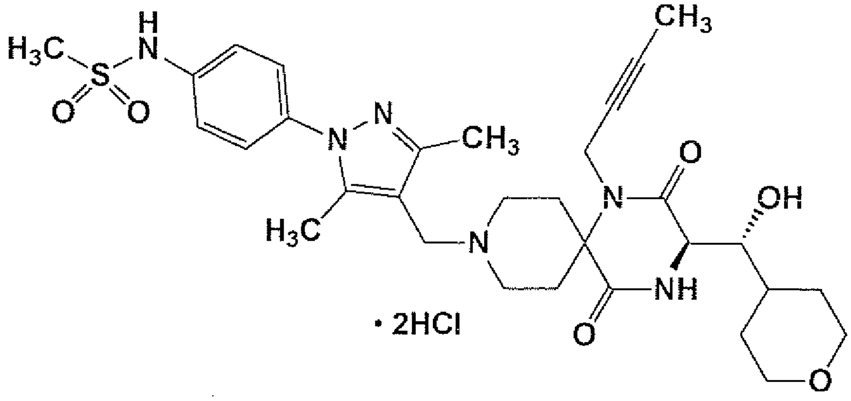

TLC: Rf 0.67 (chloroform:methanol=5:1);
NMR($CD_3OD$): δ 7.50-7.39, 4.46-4.30, 4.31, 4.19, 4.14-3.88, 3.79, 3.68-3.50, 3.39, 3.03, 2.70, 2.51, 2.36, 2.25 , 2.06-1.80, 1.74, 1.40-1.18.

Example 15(2)

(3R)-1-(2-butynyl)-2,5-dioxo-3-((1R)-1-hydroxy-1-(tetrahydropyran-4-yl)methyl)-9-(4-(4-methylsulfonylamino-2-methoxyphenoxy)phenylmethyl)-1,4,9-triazaspiro[5.5]undecane hydrochloride

TLC: Rf 0.42 (dichloromethane:methanol=10:1);
NMR($CD_3OD$): δ 7.44, 7.04-7.00, 6.93, 6.87, 4.31, 4.16, 4.00-3.90, 3.74, 3.70, 3.50-3.20, 2.99, 2.60-2.40, 2.40-2.20, 2.00-1.80, 1.73, 1.30-1.20.

Reference Example 6

1-benzyl-4-(1,4-dioxa-8-azaspiro[4.5]decan-8-yl)piperidin-4-carbonitrile

To a solution of 1,4-dioxa-8-azaspiro[4.5]decane (10 g) in 1,2-dichloroethane (70 mL) was added 1-benzyl-4-oxopiperidine (12.9 mL) and tetraisopropoxytitanium (28.8 mL) and the mixture was stirred at room temperature overnight. The reaction mixture was concentrated and suspended in dichloroethane (100 mL). Trimethylsilylcyanide (11.2 mL) and aluminum chloride (465 mg) was added thereto and the mixture was stirred for 2 hours. The reaction mixture was diluted with ethyl acetate (100 mL) and water (15 mL) was added thereto. The mixture was stirred and the precipitated salt was filtrated through CELITE (brand name). The filtrate was concentrated and to the obtained residue was added t-butylmethyl ether. The mixture was filtrated, washed and dried to give the title compound (13.4 mg) having the following physical data.
TLC: 0.33 (hexane:ethyl acetate=1:1);
NMR: δ 8.20-8.00, 4.67, 4.32-4.26, 3.66-3.54, 3.45-3.34, 2.98-2.94, 2.46-2.42.

Reference Example 7

8-(1-benzyl-4-methylpiperidin-4-yl)-1,4-dioxa-8-azaspiro[4.5]decane

To a solution of the compound prepared in Reference Example 6 (8 g) in tetrahydrofuran (115 mL) was added methyl magnesium bromide (100 mL) at room temperature and the mixture was stirred for 4.5 hours. The reaction mixture was poured in saturated aqueous solution of ammonium chloride to stop the reaction. The mixture was extracted with ethyl acetate, washed with saturated aqueous solution of sodium carbonate and brine, dried over anhydrous sodium sulfate and concentrated to give the title compound (7.69 mg) having the following physical data.

NMR($CD_3OD$): δ 7.35-7.22, 3.91, 3.52, 2.69-2.58, 2.35-2.27, 1.84-1.76, 1.68-1.64, 1.58-1.5 1, 0.94.

Reference Example 8

1'-benzyl-4'-methyl-1,4'-bipiperidin-4-one

To a solution of the compound prepared in Reference Example 7 (7.69 g) in ethyl acetate (100 mL) was added concentrated hydrochloric acid (45 mL) and the mixture was stirred. After the reaction, the reaction mixture was neutralized with aqueous solution of 2N sodium hydroxide and extracted with ethyl acetate. The organic layer was washed with brine, dried over anhydrous sodium sulfate and concentrated to give the title compound (6.16 g) having the following physical data. The compound was used in the next reaction without further purification.
TLC: 0.55 (dichloromethane:methanol=5:1).

Example 16

(3R)-1-butyl-2,5-dioxo-3-((1R)-1-hydroxy-1-cyclohexylmethyl)-9-(1-phenylmethyl-4-methylpiperidin-4-yl)-1,4,9-triazaspiro[5.5]undecane dihydrochloride

By the same procedure as described in Reference Example 1 → Example 1 → Reference Example 2 using the compound prepared in Reference Example 8 instead of benzylpiperidone, the following compounds of the present invention were obtained. The compound was used in the next reaction without further purification.

Example 17

(3R)-1-butyl-2,5-dioxo-3-((1R)-1-hydroxy-1-cyclohexylmethyl)-9-(1-(2,4-dimethyl-1-oxidopyridin-3-ylcarbonyl)-4-methylpiperidin-4-yl-1,4,9-triazaspiro[5.5]undecane hydrochloride

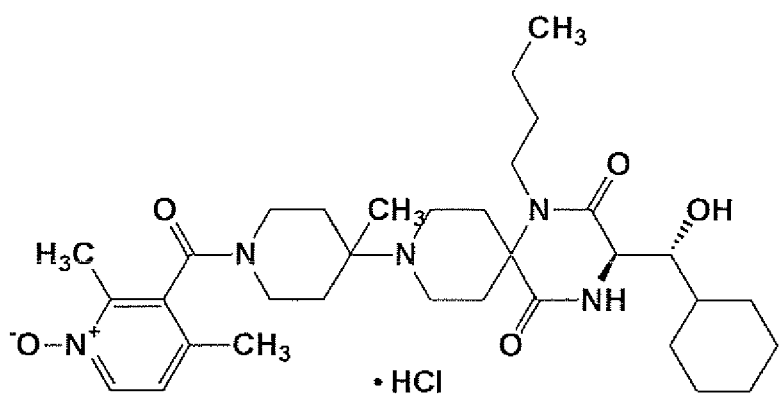

To a solution of the compound prepared in Example 16 (100 mg) in dimethylformamide (2 mL) was added diisopropylethylamine (0.07 mL), 1-ethyl-3-[3-(dimethylamino)propyl]carbodiimide hydrochloride (55 mg), 1-hydroxybenzotriazole (39 mg) and 2,4-dimethylnicotinic acid 1-oxide (39 mg) and the mixture was stirred at room temperature overnight. To the reaction mixture was added water and the mixture was extracted with ethyl acetate. The organic layer was washed with 1N hydrochloric acid and brine, dried over anhydrous sodium sulfate and concentrated. The residue was purified by column chromatography on silica gel (ethyl acetate : methanol = 30 : 1-10 : 1) and preparative thin layer chromatography (dichloromethane methanol = 8 : 1), and converted to hydrochloride salt by using a conventional method to give the compound of the present invention (21 mg) having the following physical data.
TLC: Rf 0.15 (dichloromethane:methanol=10:1);
NMR($CD_3OD$): δ 8.54, 7.58, 4.16, 4.05, 3.79, 3.63-3.06, 2.65-2.38, 2.21-1.92, 1.76-1.65, 1.52, 1.44-1.14, 1.03-0.87.

Formulation example 1

The following components were admixed in a conventional manner, punched out to give 10,000 tablets each containing 50 mg of active ingredient.

| | | |
|---|---|---|
| * | (3R)-1-butyl-2,5-dioxo-3-((1R)-1-hydroxy-2-ethylbutyl)-9-(4-(4-methylaminocarbonyl-2-chlorophenylmethyl)phenylmethyl)-1,4,9-triazaspiro[5.5]undecane hydrochloride | 500 g |
| * | calcium carboxymethyl cellulose (disintegrant) | 20 g |
| * | magnesium stearate(lubricant) | 10 g |
| * | microcrystalline cellulose | 470 g |

Formulation example 2

The following components were admixed in a conventional technique. The solution was sterilized in a conventional technique, filled in ampoules 5 ml each and freeze-dried over in a conventional technique to give 10,000 ampoules each containing 20 mg of active ingredient.

| | | |
|---|---|---|
| * | (3R)-1-butyl-2,5-dioxo-3-((1R)-1-hydroxy-2-ethylbutyl)-9-(4-(4-methylaminocarbonyl-2-chlorophenylmethyl)phenylmethyl)-1,4,9-triazaspiro[5.5]undecane hydrochloride | 200 g |
| * | mannitol | 2000 g |
| * | distilled water | 50 L |

## Claims

**1.** A compound represented by formula (I)

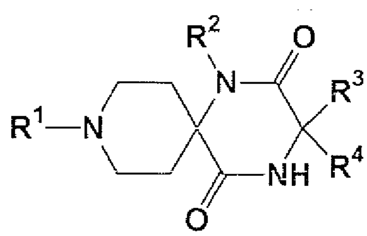

(I)

wherein $R^1$ represents (1) ring 1, or (2) C1-8 alkyl, C2-4 alkenyl or C2-4 alkynyl optionally substituted with 1-3 substituents selected from the following (a)-(i): (a) $-OR^5$, (b)$-COR^6$, (c) $-NR^7R^8$, (d) $-CONR^9R^{10}$, (e) $-NR^{11}COR^{12}$, (f) $-NR^{13}SO_2R^{14}$, (g) ring 1, (h) $=NR^{15}$, (i) $=NOR^{16}$,

$R^5$-$R^{13}$, $R^{15}$ and $R^{16}$ each represents (1) hydrogen, (2) C1-8 alkyl, (3) C2-8 alkenyl, (4) C2-8 alkynyl, (5) ring 1, or (6) C1-8 alkyl, C2-8 alkenyl or C2-8 alkynyl optionally substituted with 1-5 substituents selected from ring 1 and -O-ring 1,

$R^{14}$ represents C1-4 alkyl or ring 1,

ring 1 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 1 may be substituted with 1-5 substituents selected from (1) C1-8 alkyl, (2) C2-8 alkenyl, (3) C2-8 alkynyl, (4) halogen, (5) cyano, (6) ring 2, (7) $-OR^{17}$, (8) $-SR^{18}$, (9) - $NR^{19}R^{20}$, (10) $-COR^{21}$, (11) $-COOR^{22}$, (12) $-CONR^{23}R^{24}$, (13) $-NR^{25}COR^{26}$, (14) - $SO_2NR^{27}R^{28}$, (15) $-NR^{29}SO_2R^{30}$, (16) $-N(SO_2R^{31})_2$, (17) oxo, and (18) C1-8 alkyl, C2-8 alkenyl or C2-8 alkynyl optionally substituted with 1-5 substituents selected from the following (a) -(e); (a) halogen, (b) ring 2, (c) $-OR^{32}$, (d) $-NR^{33}COR^{34}$, (e) $=NOR^{35}$,

$R^{17}$-$R^{29}$ and $R^{32}$-$R^{35}$ each represents (1) hydrogen, (2) C1-8 alkyl, (3) C2-8 alkenyl, (4) C2-8 alkynyl, (5) ring 2, or (6) C1-8 alkyl, C2-8 alkenyl or C2-8 alkynyl optionally substituted with 1-3 substituents selected from the following (a)-(f): (a) ring 2, (b) $-OR^{36}$, (c) - $COOR^{37}$, (d) $-NR^{38}R^{39}$, (e) halogen, (f) $=NR^{40}$,

$R^{30}$ and $R^{31}$ each represents C1-4 alkyl,

$R^{36}$-$R^{40}$ each represents hydrogen or C1-4 alkyl optionally substituted with hydroxyl,

ring 2 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 2 may be substituted with 1-5 substituents selected from (1) C1-8 alkyl, (2) halogen, (3) $-OCF_3$, (4) cyano, (5) ring 3, (6) $-OR^{41}$, (7) $-NR^{42}R^{43}$, (8) $-COR^{44}$, (9) $-COOR^{45}$, (10) $-CONR^{46}R^{47}$, (11) $-NR^{48}COR^{49}$, (12)

$-SO_2NR^{50}R^{51}$, (13) $-NR^{52}SO_2R^{53}$, and (14) - $C(NH_2)=NR^{54}$,

$R^{41}$-$R^{52}$ and $R^{54}$ each represents (1) hydrogen, (2) C1-8 alkyl, (3) C2-8 alkenyl, (4) C2-8 alkynyl, (5) ring 3, (6) $-OR^{55}$, or (7) C1-8 alkyl, C2-8 alkenyl or C2-8 alkynyl optionally substituted with 1-3 substituents selected from the following (a)-(d): (a) ring 3, (b) $-OR^{56}$, (c) $-COOR^{57}$, (d) $-NR^{58}R^{59}$,

$R^{53}$ represents C1-8 alkyl,

$R^{55}$-$R^{59}$ each represents hydrogen or C1-4 alkyl,

ring 3 represents (1) C3-8 mono-carbocyclic aryl which may be partially or fully saturated, or (2) 3-8 membered mono-cyclic hetero aryl containing 1-4 nitrogen atoms, 1-2 oxygen atoms and/or 1-2 sulfur atoms which may be partially or fully saturated,

ring 3 may be substituted with 1-3 of=O or =S,

$R^2$ represents (1) hydrogen, (2) C1-8 alkyl, (3) C2-8 alkenyl, (4) C2-8 alkynyl, (5) ring 4, or (6) C1-8 alkyl, C2-8 alkenyl or C2-8 alkynyl optionally substituted with 1-5 substituents selected from the following (a)-(i): (a) hydrogen, (b) $-OR^{60}$, (c) $-NR^{61}R^{62}$, (d) - $CONR^{63}R^{64}$, (e) $-NR^{65}COR^{66}$, (f) $-NR^{67}SO_2R^{68}$, (g) $NR^{69}COOR^{70}$, (h) ring 4, (i) cyano,

$R^{60}$-$R^{67}$ and $R^{69}$ each represents hydrogen, C1-8 alkyl, C2-8 alkenyl, or C2-8 alkynyl,

$R^{68}$ and $R^{70}$ each represents C1-4 alkyl, C2-4 alkenyl or C2-4 alkynyl,

ring 4 represents phenyl, pyridinyl, or C3-8 cycloalkyl,

ring 4 may be substituted with 1-5 of C1-4 alkyl,

$R^3$ and $R^4$ together with a carbon atom to which they are attached, form C3-8 cycloalkyl, or $R^3$ and $R^4$ each represents (1) hydrogen, (2) C1-8 alkyl, (3) C2-8 alkenyl, (4) C2-8 alkynyl, (5) C1-8 alkyl, C2-8 alkenyl or C2-8 alkynyl optionally substituted with 1-5 substituents selected from the following (a)-(c): (a) ring 5, (b) hydroxyl,

(c) 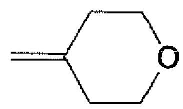 ,

ring 5 represents (1) C3-15 mono-, bi- or tri-carbocyclic aryl which may be partially or fully saturated, or (2) 3- to 15-membered mono-, bi- or tri-cyclic hetero aryl containing 1-4 nitrogen atoms, 1-2 oxygen atoms and/or 1-2 sulfur atoms which may be partially or fully saturated,

ring 5 may be substituted with 1-5 of $-OR^{71}$, C1-4 alkyl or oxo,

$R^{71}$ represents hydrogen or C1-4 alkyl,

a quaternary ammonium salt thereof, an N-oxide thereof, or a salt thereof.

**2.** The compound according to claim 1, which is selected from the group consisting of

(1) (3R)-1-butyl-2,5-dioxo-3-((1R)-1-hydroxy-1-cyclohexylmethyl)-9-(4-(4-methylaminocarbonyl-2-chlorophenoxy)phenylmethyl)-1,4,9-triazaspiro[5.5]undecane,
(2) (3R)-1-butyl-2,5-dioxo-3-((1R)-1-hydroxy-1-cyclohexylmethyl)-9-(4-(4-(5-oxo-4,5-dihydro-1,2,4-thiadiazol-3-yl)phenoxy)phenylmethyl)-1,4,9-triazaspiro[5.5]undecane,
(3) (3R)-1-butyl-2,5-dioxo-3-((1R)-1-hydroxy-2-ethylbutyl)-9-(4-(4-(2-methoxyethylaminocarbonyl)-2-methoxyphenoxy)phenylmethyl)-1,4,9-triazaspiro[5.5]undecane,
(4) (3R)-1-butyl-2,5-dioxo-3-((1R)-1-hydroxy-2-methylpropyl)-9-(4-(4-methylsulfonylamino-2-methoxyphenoxy)phenylmethyl)-1,4,9-triazaspiro[5.5]undecane,
(5) (3R)-1-butyl-2,5-dioxo-3-((1R)-1-hydroxy-1-(tetrahydropyran-4-yl)methyl)-9-(4-(4-(pyrrolidin-1-yl)carbonyl-2-methoxyphenoxy)phenylmethyl)-1,4,9-triazaspiro[5.5]undecane,
(6) (3R)-1-butyl-2,5-dioxo-3-((1R)-1-hydroxy-2-ethylbutyl)-9-(4-(2-methoxy-4-methylsulfonylaminophenoxy)phenylmethyl)-1,4,9-triazaspiro[5.5]undecane,
(7) (3S)-1-butyl-2,5-dioxo-3-(2-methylpropyl)-9-(4-(2-methoxy-4-methylsulfonylaminophenoxy)phenylmethyl)-1,4,9-triazaspiro[5.5]undecane,
(8) (3R)-1-butyl-2,5-dioxo-3-((1R)-1-hydroxy-1-(tetrahydropyran-4-yl)methyl)-9-(4-(4-(2-methylpropyl)carbonylaminophenylmethyl)phenylmethyl)-1,4,9-triazaspiro[5.5]undecane,
(9) (3R)-1-butyl-2,5-dioxo-3((1R)-1-hydroxy-1-(tetrahydropyran-4-yl)methyl)-9-(4-(4-(2-methylpropyl)carbonylamino-2-methoxyphenoxy)phenylmethyl)-1,4,9-triazaspiro[5.5]undecane,
(10) (3R)-1-butyl-2,5-dioxo-3-((1R)-1-hydroxy-1-cyclohexylmethyl)-9-(2-(4-methylaminocarbonylphenoxy)ethyl)-1,4,9-triazaspiro[5.5]undecane,
(11) (3R)-1-butyl-2,5-dioxo-3-((1R)-1-hydroxy-1-cyclohexylmethyl)-9-(5-(4-methylaminocarbonylphenoxy)pentyl)-1,4,9-triazaspiro[5.5]undecane,

(12) (3R)-1-butyl-2,5-dioxo-3-((1R)-1-hydroxy-1-cyclohexylmethyl)-9-(4-(4-methylaminocarbonylphenoxy) butyl)-1,4,9-triazaspiro[5.5]undecane,
(13) (3R)-1-butyl-2,5-dioxo-3-((1R)-1-hydroxy-1-(cyclohexen-4-yl)methyl)-9-(4-(4-methylaminocarbonylphenoxy)phenylmethyl)-1,4,9-triazaspiro[5.5]undecane,
(14) (3R)-1-butyl-2,5-dioxo-3-((1R)-1-hydroxy-1-cyclohexylmethyl)-9-(4-(4-methylsulfonylaminophenoxy) butyl)-1,4,9-triazaspiro[5.5]undecane, and
(15) (3R)-1-pentyl-2,5-dioxo-3-((1R)-1-hydroxy-1-cyclohexylmethyl)-9-(4-(4-methylaminocarbonylphenoxy) phenylmethyl)-1,4,9-triazaspiro[5.5]undecane.

**3.** The compound according to claim 1, which is selected from the group consisting of

(1) (3R)-1-butyl-2,5-dioxo-3-((1R)-1-hydroxy-1-cyclohexylmethyl)-9-(4-(4-cyclopropylmethylaminocarbonylphenoxy)phenylmethyl)-1,4,9-triazaspiro[5.5]undecane,
(2) (3R)-1-butyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(3) (3R)-1-butyl-2,5-dioxo-3-((1R)-1-hydroxy-1-cyclohexylmethyl)-9-(4-(4-methylaminocarbonyl-2-methoxyphenoxy)phenylmethyl)-1,4,9-triazaspiro[5.5]undecane,
(4) (3R)-1-butyl-2,5-dioxo-3-((1R)-1-hydroxy-1-cyclohexylmethyl)-9-(4-(4-carboxy-2-methoxyphenylmethyl) phenylmethyl)-1,4,9-triazaspiro[5.5]undecane,
(5) (3R)-1-butyl-2,5-dioxo-3-((1R)-1-hydroxy-1-cyclohexylmethyl)-9-(4-(4-(N,N,-dimethylaminocarbonyl)-2-methoxyphenoxy)phenylmethyl)-1,4,9-triazaspiro[5.5]undecane,
(6) (3R)-1-butyl-2,5-dioxo-3-((1R)-1-hydroxy-1-cyclohexylmethyl)-9-(4-(4-carboxy-2-ethoxyphenoxy)phenylmethyl)-1,4,9-triazaspiro[5.5]undecane,
(7) (3R)-1-butyl-2,5-dioxo-3-((1R)-1-hydroxy-1-cyclohexylmethyl)-9-(4-(4-carboxy-2,6-dimethylphenoxy)phenylmethyl)-1,4,9-triazaspiro[5.5]undecane,
(8) (3R)-1-pentyl-2,5-dioxo-3-((1R)-1-hydroxy-1-cyclohexylmethyl)-9-(4-(4-carboxy-2-methoxyphenoxy)phenylmethyl)-1,4,9-triazaspiro[5.5]undecane,
(9) (3R)-1-butyl-2,5-dioxo-3-((1R)-1-hydroxy-2-ethylbutyl)-9-(4-(4-carboxy-2-methoxyphenoxy)phenylmethyl)-1,4,9-triazaspiro[5.5]undecane,
(10) (3R)-1-butyl-2,5-dioxo-3-((1R)-1-hydroxy-1-(tetrahydropyran-4-yl)methyl)-9-(4-(4-cyclopropylmethylaminocarbonylphenoxy)phenylmethyl)-1,4,9-triazaspiro[5.5]undecane,
(11) (3R)-1-propyl-2,5-dioxo-3-((1R)-1-hydroxy-1-cyclohexylmethyl)-9-(4-(4-dimethylaminocarbonyl-2-methoxyphenoxy)phenylmethyl)-1,4,9-triazaspiro[5.5]undecane,
(12) (3R)-1-butyl-2,5-dioxo-3-((1R)-1-hydroxy-1-cycloheptylmethyl)-9-(4-(4-carboxy-2-methoxyphenoxy)phenylmethyl)-1,4,9-triazaspiro[5.5]undecane,
(13) (3R)-1-butyl-2,5-dioxo-3-((1R)-1-hydroxy-1-cyclopentylmethyl)-9-(4-(4-carboxy-2-methoxyphenoxy)phenylmethyl)-1,4,9-triazaspiro[5.5]undecane,
(14) (3R)-1-butyl-2,5-dioxo-3-((1R)-1-hydroxy-1-cyclopentylmethyl)-9-(4-(4-carboxy-2-ethoxyphenoxy)phenylmethyl)-1,4,9-triazaspiro[5.5]undecane,
(15) (3R)-1-butyl-2,5-dioxo-3-((1R)-1-hydroxy-1-(tetrahydropyran-4-yl)methyl)-9-(4-(4-cyclopropylmethylaminocarbonyl-2-methoxyphenoxy)phenylmethyl)-1,4,9-triazaspiro[5.5]undecane,
(16) (3R)-1-butyl-2,5-dioxo-3-((1R)-1-hydroxy-1-(tetrahydropyran-4-yl)methyl)-9-(4-(4-methylaminocarbonyl-2-methoxyphenylmethyl)phenylmethyl)-1,4,9-triazaspiro[5.5]undecane,
(17) (3R)-1-butyl-2,5-dioxo-3-((1R)-1-hydroxy-1-(tetrahydropyran-4-yl)methyl)-9-(4-(4-isopropylaminocarbonyl-2-methoxyphenoxy)phenylmethyl)-1,4,9-triazaspiro[5.5]undecane,
(18) (3R)-1-butyl-2,5-dioxo-3-((1R)-1-hydroxy-1-(tetrahydropyran-4-yl)methyl)-9-(4-(4-(2-methylpropyl)aminocarbonyl-2-methoxyphenoxy)phenylmethyl)-1,4,9-triazaspiro[5.5]undecane,
(19) (3R)-1-butyl-2,5-dioxo-3-((1R)-1-hydroxy-1-(tetrahydropyran-4-yl)methyl)-9-(4-(4-(2,2-dimethylpropylaminocarbonyl)-2-methoxyphenoxy)phenylmethyl)-1,4,9-triazaspiro[5.5]undecane,
(20) (3R)-1-butyl-2,5-dioxo-3-((1R)-1-hydroxy-1-(tetrahydropyran-4-yl)methyl)-9-(4-(4-isopropylcarbonylaminophenoxy)phenylmethyl)-1,4,9-triazaspiro[5.5]undecane,
(21) (3R)-1-butyl-2,5-dioxo-3-((1R)-1-hydroxy-1-(tetrahydropyran-4-yl)methyl)-9-(4-(4-(2-methylpropyl)carbonylaminophenoxy)phenylmethyl)-1,4,9-triazaspiro[5.5]undecane,
(22) (3R)-1-butyl-2,5-dioxo-3-((1R)-1-hydroxy-1-(tetrahydropyran-4-yl)methyl)-9-(4-(4-isopropylcarbonylamino-2-methoxyphenoxy)phenylmethyl)-1,4,9-triazaspiro[5.5]undecane,
(23) (3R)-1-butyl-2,5-dioxo-3-((1R)-1-hydroxy-1-(cyclopenten-4-yl)methyl)-9-(4-(4-carboxy-2-methylphenoxy)phenylmethyl)-1,4,9-triazaspiro[5.5]undecane,
(24) (3R)-1-butyl-2,5-dioxo-3-((1R)-1-hydroxy-1-(cyclopenten-4-yl)methyl)-9-(4-(4-carboxy-2-ethoxyphenoxy)phenylmethyl)-1,4,9-triazaspiro[5.5]undecane,
(25) (3R)-1-(2-butynyl)-2,5-dioxo-3-((1R)-1-hydroxy-1-cyclohexylmethyl)-9-(4-(4-methylaminocarbonylphenoxy)butyl)-1,4,9-triazaspiro[5.5]undecane,
(26) (3R)-1-butyl-2,5-dioxo-3-((1R)-1-hydroxy-1-cyclohexylmethyl)-9-(1-(4-(4-methylaminocarbonylphenoxy)phenyl)ethyl)-1,4,9-triazaspiro[5.5]undecane, and
(27) (3R)-1-butyl-2,5-dioxo-3-((1R)-1-hydroxy-1-cyclohexylmethyl)-9-(1-(4-(4-carboxyphenoxy)phenyl)ethyl)-1,4,9-triazaspiro[5.5]undecane.

**4.** The compound according to claim 1, wherein $R^1$ is C1-8 alkyl, C2-4 alkenyl, or C2-4 alkynyl substituted with $-COR^6$, $=NR^{15}$, or $=NOR^{16}$, in which $R^6$, $R^{15}$ and $R^{16}$ have the same meanings as defined in claim 1.

**5.** The compound according to claim 1, wherein at least one of substituents of ring 1 in $R^1$ is $-COR^{12}$, oxo, or $=NOR^{35}$, in which $R^{12}$ and $R^{35}$ have the same meanings as defined in claim 1.

**6.** The compound according to claim 1, wherein at least one of substituents of ring 2 in $R^1$ is $-COR^{44}$ or $-C(NH_2)=NOR^{54}$, in which $R^{44}$ and $R^{54}$ have the same meanings as defined in claim 1.

**7.** The compound according to claim 1, wherein at least one of substituents of ring 3 in $R^1$ is =O or =S.

**8.** A pharmaceutical composition which comprises, as an active ingredient, the compound represented by formula (I) according to claim 1, a quaternary ammonium salt thereof, an N-oxide thereof, or a salt thereof

**9.** A regulator of a chemokine/chemokine receptor, which comprises, as an active ingredient, the compound represented by formula (I) according to claim 1, a quaternary ammonium salt thereof, an N-oxide thereof, or a salt thereof.

**10.** The regulator of a chemokine/chemokine receptor according to claim 9, which is a CCR5 antagonist.

**11.** A pharmaceutical composition for prevention and/or treatment for inflammatory diseases, immunologic diseases, human immunodeficiency virus, allergic diseases, ischemia-reperfusion injury, acute respiratory distress syndrome, shock accompanied by bacterial infection, diabetes mellitus, or metastasis, which comprises, as an active ingredient, the compound represented by formula (I) according to claim 1, a quaternary ammonium salt thereof, an N-oxide thereof, or a salt.

**12.** A method for antagonizing CCR5 in a mammal, which comprises administering to a mammal an effective amount of the compound of formula (I) according to claim 1, a quaternary ammonium salt thereof, an N-oxide thereof, or a salt thereof.

**13.** Use of the compound of formula (I) according to claim 1, a quaternary ammonium salt thereof, an N-oxide thereof, or a salt thereof for the manufacture of a CCR5 antagonist.

## INTERNATIONAL SEARCH REPORT

International application No.
PCT/JP03/11834

**A. CLASSIFICATION OF SUBJECT MATTER**

Int.Cl[7] C07D471/10, A61K31/527, A61P1/04, 1/16, 3/10, 9/00, 11/00, 11/02, 11/06, 13/12, 17/00, 17/06, 19/02, 29/00, 31/04, 31/18, 35/04, 37/02, 37/06, 37/08, 43/00, 25/00,

According to International Patent Classification (IPC) or to both national classification and IPC

**B. FIELDS SEARCHED**

Minimum documentation searched (classification system followed by classification symbols)

Int.Cl[7] C07D471/10, A61K31/527, A61P1/04, 1/16, 3/10, 9/00, 11/00, 11/02, 11/06, 13/12, 17/00, 17/06, 19/02, 29/00, 31/04, 31/18, 35/04, 37/02, 37/06, 37/08, 43/00, 25/00,

Documentation searched other than minimum documentation to the extent that such documents are included in the fields searched

Electronic data base consulted during the international search (name of data base and, where practicable, search terms used)

CA(STN)

**C. DOCUMENTS CONSIDERED TO BE RELEVANT**

| Category* | Citation of document, with indication, where appropriate, of the relevant passages | Relevant to claim No. |
|---|---|---|
| X | WO 01/40227 A1 (Ono Pharmaceutical Co., Ltd.), 07 June, 2001 (07.06.01), Claims & AU 2001016506 A & EP 1236726 A1 & NO 2002002609 A | 1-11,13 |
| P,X | WO 03/35074 A1 (Ono Pharmaceutical Co., Ltd.), 01 May, 2003 (01.05.03), Claims (Family: none) | 1-11,13 |
| P,X | JP 2002-348288 A (Ono Pharmaceutical Co., Ltd.), 04 December, 2002 (04.12.02), Claims (Family: none) | 1-11,13 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 November, 2003 (04.11.03) | 18 November, 2003 (18.11.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/11834

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 02/074770 A1 (Ono Pharmaceutical Co., Ltd.), 26 September, 2002 (26.09.02), Claims (Family: none) | 1-11,13 |
| P,X | WO 02/074769 A1 (Ono Pharmaceutical Co., Ltd.), 26 September, 2002 (26.09.02), Claims (Family: none) | 1-11,13 |
| A | WO 98/31364 A1 (MERCK & CO., INC.), 23 July, 1998 (23.07.98), & JP 2001-508798 A & GB 9707490 A & AU 6133098 A & EP 1003743 A & US 6124319 A | 1-11,13 |
| A | WO 00/14086 A1 (LEUKOSITE, INC. et al.), 16 March, 2000 (16.03.00), & JP 2002-524458 A & CA 2342882 A & AU 2464999 A & EP 1109804 A & US 6288083 B | 1-11,13 |
| X | MAEDA, K. et al., "Novel low molecular weight spirodiketoperazine derivatives potently inhibit R5 HIV-1 infection through their antagonistic effects on CCR5", Journal of Biological Chemistry, 276(37), pages 35194 to 35200, (2001) | 1-11,13 |

Form PCT/ISA/210 (continuation of second sheet) (July 1998)

## INTERNATIONAL SEARCH REPORT

International application No.
PCT/JP03/11834

**Box I Observations where certain claims were found unsearchable (Continuation of item 2 of first sheet)**

This international search report has not been established in respect of certain claims under Article 17(2)(a) for the following reasons:

1. [X] Claims Nos.: 12
   because they relate to subject matter not required to be searched by this Authority, namely:
   The invention as set forth in claim 12 is relevant to method for treatment of the human body by therapy.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/11384

Continuation of A. CLASSIFICATION OF SUBJECT MATTER
(International Patent Classification (IPC))

Int.Cl$^7$ 27/00

(According to International Patent Classification (IPC) or to both national classification and IPC)

Continuation of B. FIELDS SEARCHED
Minimum Documentation Searched (International Patent Classification (IPC))

Int.Cl$^7$ 27/00

Minimum documentation searched (classification system followed by classification symbols)

Form PCT/ISA/210 (extra sheet) (July 1998)